# EUROPEAN PATENT SPECIFICATION

(11) **EP 2 473 630 B1**
(45) Date of publication and mention of the grant of the patent: **08.11.2017**
(21) Application number: 10771551.8
(22) Date of filing: 03.09.2010
(51) Int. Cl.: C12Q 1/68

(54) **OPTIMIZED PROBES AND PRIMERS AND METHODS OF USING SAME FOR THE DETECTION, SCREENING, ISOLATION AND SEQUENCING OF VANCOMYCIN RESISTANCE GENES AND VANCOMYCIN RESISTANT ENTEROCOCCI**
OPTIMIERTE SONDEN UND PRIMER SOWIE VERFAHREN ZU DEREN VERWENDUNG ZUM NACHWEIS, ZUM SCREENING, ZUR ISOLATION UND ZUR SEQUENZIERUNG VON VANCOMYCIN-RESISTENZGENEN UND VANCOMYCINRESISTENTEN ENTEROKOKKEN
SONDES ET AMORCES OPTIMISÉES ET LEURS PROCÉDÉS D'UTILISATION POUR LA DÉTECTION, LE CRIBLAGE, L'ISOLATION ET LE SÉQUENÇAGE DE GÈNES DE RÉSISTANCE À LA VANCOMYCINE ET D'ENTÉROCOQUES RÉSISTANT À LA VANCOMYCINE

(30) Priority: 04.09.2009 US 239940 P
(43) Date of publication of application: 11.07.2012
(73) Proprietor: QIAGEN GmbH, 40724 Hilden (DE)
(72) Inventor: REISKE, Heinz, R., Framingham MA 01701 (US); DOLINGER, David, L., Medway MA 02053 (US); JACOBS, Alice, A., Cambridge MA 02139 (US); ANZOLA, Juan, Manuel, Bogota (CO)
(74) Representative: Uexküll & Stolberg
(86) International application number: PCT/US2010/047867
(87) International publication number: WO 2011/029034

(56) References cited:
- WO-A1-2009/032486
- WO-A1-2009/042851
- WO-A2-98/20157
- WO-A2-99/01571
- WO-A2-2007/023461
- FR-A1- 2 668 489
- FR-A1- 2 699 537
- US-A1- 2003 049 636
- US-A1- 2004 185 478
- US-A1- 2005 058 985
- US-A1- 2005 112 641
- US-A1- 2006 263 810
- US-B1- 6 274 316
- US-B1- 6 569 622
- US-B1- 6 916 906

## Description

### BACKGROUND

*Enterococci* are found within the normal intestinal flora and the female genital tract of humans, other mammals and birds. *Enterococcus* is intrinsically resistant (*i.e.,* resistant to a low level) to β-lactam-based antibiotics (*e.g.,* ampicillin, penicillin) and aminoglycosides (*e.g.,* gentamicin, kanamycin, and neomycin). *Enterococcus* can acquire resistance to glycopeptides, such as vancomycin, and high concentrations of both β-lactam-based antibiotics and aminoglycosides, among others.

The importance of *Enterococcus* in vancomycin-resistant nosocomial infections or hospital acquired infections (HAIs) (vancomycin-resistant *Enterococcus;* VRE), has been the impetus for the development of therapeutic alternatives to vancomycin. In addition to vancomycin resistance in *Enterococci,* there is vancomycin resistance in *Staphylococcus.* Vancomycin resistant *Staphylococcus aureus* (VRSA) are antimicrobial-resistant *Staphylococci.* Patients that develop VRSA infections usually have several underlying health conditions (such as diabetes), previous infections with MRSA, and recent hospitalizations. The spread of VRSA occurs through close physical contact with infected patients or contaminated material. VRE infections can be treated with non-glycopeptide antibiotics such as cephalosporins and aminoglycosides; regardless of the phenotype, susceptibility testing is usually performed on isolates to determine the best course of treatment.

VRE is a threat to immunocompromised individuals, individuals recovering from surgical procedures and those generally in poor health. An individual can be colonized with VRE, which may or may not become a full-blown infection. Although colonized individuals can remain asymptomatic for months, even years, such persons are capable of transmitting VRE to others. VRE is rarely a concern for healthy adults, and is usually cleared from the host without intervention. Infections typically occur at sites such as wounds and urinary tract infections from in dwelling catheters. Infected patients can become septic. VRE is frequently transmitted person-to-person by healthcare workers (HCW) whose hands have become contaminated with VRE that is present in the feces, urine, or blood of an infected or colonized person. VRE can also be spread indirectly via hand contact with open wounds or contaminated environmental surfaces. Colonized individuals could also infect themselves through contact with feces, urine, blood or surfaces contaminated with their own feces, urine or blood. VRE can persist for weeks on environmental surfaces and medical instruments. Consequently, these surfaces are also potential modes of transmission and potential testing areas.

Culture-based diagnostic methods remain the definitive methods of choice for the determination of VRE. Bacterial colonies growing on Bile Esculin Azide plates supplemented with vancomycin (BEAV) could be presumed as VRE based on colonial morphology, but additional culture steps would be required for confirmation. This process can take 48 hours or longer. Culture can also have a high false negative rate. Adoption of nucleic acid-based tests (NAT) (such as polymerase chase reaction (PCR)) has led to diagnostic tests with significantly better turn-around time (2-5 hours), but many of the available tests lack sensitivity and specificity.

Detection of the vancomycin resistance genes from the genus *Enterococcus,* as well as other non-*Enterococcal* genera, would allow for improved treatments of bacterial infections. Furthermore, determination of whether the vancomycin resistance genes are from the *Enterococcal* genera would enable effective treatment decisions. A rapid and accurate diagnostic test panel for the detection of vancomycin-resistance genes and for detection of VRE would provide clinicians with an effective tool for diagnosis and supporting subsequent effective treatment regimens. A rapid screening panel for screening patients at risk for developing vancomycin resistance-associated and VRE-associated diseases would also provide clinicals with an efficient method to screen at-risk patients.

### SUMMARY

Described herein are nucleic acid probes and primers for detecting, isolating and sequencing all known, characterized variants of the *vanA, vanB, vanC1, vanC2*/*3, vanD, vanE,* and *vanG* vancomycin resistance genes (particularly the *vanA* and *vanB* genes) from the genus *Enterococcus,* as well as other non-Enterococcal genera, with a high degree of sensitivity and specificity. Also decribed herein are nucleic acid probes and primers for determining whether the vancomycin resistance genes are from the Enterococcal genera. A diagnostic test that distinguishes multiple drug resistance genes simultaneously and also determines whether the organism is VRE is necessary because such detection is critical in patient and personnel screening and surveillance of inanimate objects to eliminate the transmission of potentially deadly healthcare-associated infections (HAIs).

Patient, personnel and inanimate object screening, combined with barrier isolation and contact precautions of VRE-carriers, has been shown to be effective in controlling VRE infections; in some cases reducing to undetectable levels the VRE in clinical facilities. The assays described herein are critical components of a resistance screening program to screen patients admitted to and personnel working in clinical settings for VRE and VRSA. The assays described herein are also used to screen environmental surfaces for evidence that vancomycin-resistant organisms are or were present in a hospital setting. Additionally, the assays described herein are used to identify or confirm the identification of an isolate as containing vancomycin resistance and whether the organism is from the genera Enterococcus.

Enterococci are common commensal bacteria located in the gut microflora. *Enterococcus faecium* (Efm) *and Enterococcus faecalis* (Efs) are two of the most common Enterococcal species that have been shown to have vancomycin resistance. One marker for Efm and Efs is the *sodA* gene, which encodes the enzyme superoxide dismutase A (Efm *sodA* and Efs *sodA).* The *sodA* gene is frequently used as a bacterial species-specific marker. Other markers, identified through *in silico* analysis, target novel genes from Efm and Efs (Efm novel and Efs novel). An additional marker, a dual marker, identified through *in silico* analysis, binds to novel genes found in both *E. faecium* and *E. faecalis* (Efm/Efs dual).

Many facilities utilize culture-based methods for the determination and detection of antibiotic resistance genes, which requires days to obtain the results. The methods of detection of the resistance markers described herein occurs within a minimal number of hours, allowing clinicians to rapidly determine the appropriate contact precautions or treatment for individuals harboring vancomycin-resistant organisms, avoiding needless precautions for resistance-negative individuals and avoiding the careless use of antibiotics that have no or little treatment efficacy.

One embodiment is directed to an isolated nucleic acid sequence comprising a sequence selected from the group consisting of: SEQ ID NOS: 1-502 and 600-939.

One embodiment is directed to a method of hybridizing one or more isolated nucleic acid sequences comprising a sequence selected from the group consisting of: SEQ ID NOS: 1-502 to a vancomycin-resistance gene sequence, comprising contacting one or more isolated nucleic acid sequences to a sample comprising the vancomycin-resistance gene under conditions suitable for hybridization. In a particular embodiment, the vancomycin-resistance gene sequence is a genomic sequence, a naturally occurring plasmid, a naturally occurring transposable element, a template sequence or a sequence derived from an artificial construct. In a particular embodiment, the method(s) further comprise isolating and/or sequencing the hybridized vancomycin-resistance gene sequence.

One embodiment is directed to a primer set comprising at least one forward primer selected from the group consisting of SEQ ID NOS: 1, 6, 19, 22, 23, 26, 28, 29, 33, 34, 37-42, 45, 48, 53, 59 (*vanA*); 61, 68, 70-72, 75, 81, 83, 89, 93, 94, 103-105, 107 and 111 (*vanB*); 123, 127, 130, 133, 138, 141, 144, 148, 151, 156, 158, 161, 162, 165, 168, 170, 171, 175, 178, 180, 183-185, 188, 191, 193, 194, 196, 198, and 200-204 (*vanC1*); 206, 210, 213, 217, 220, 221, 222, 224-226, 228-238, 240, 242 and 243 (*vanC2*/*3*); 388, 391, 394, 396, 399, 409, 415, 416, 425, 428, 435, 438, 440, 443, 445, 462, 465, 468, 471, 474, 477, 480, 483, 488, 491, 494-498 and 500-502 (*vanD*); 334, 337-380 and 382-387 (*vanE*); 244, 249, 252, 253, 255, 256, 260, 263, 264, 267, 270-272, 275-283, 285, 288, 291, 293, 295, 297-300, 302, 303, 305, 307, 309-311, 313-318, 321, 322, 325-333 *(vanG)*; and at least one reverse primer selected from the group consisting of SEQ ID NOS: SEQ ID NOS: 3, 5, 8, 10, 21, 31, 32, 36, 44, 47, 51, 52, 55 and 60 (*vanA*); 63, 65, 66, 74, 77, 85-88, 90, 91, 95, and 97-102 (*vanB*); 125, 129, 132, 135, 137, 140, 143, 146, 147, 150, 153, 155, 157, 160, 164, 167, 169, 173, 174, 177, 179, 187, 190 and 192 (*vanC1*); 208, 209, 212, 215, 216, 219, 223, 227, 239 and 241 (*vanC2*/*3*); 390, 393, 395, 398, 401, 411, 417, 419, 421, 426, 430, 434, 437, 439, 442, 444, 447-455, 458-461, 464, 467, 470, 473, 476, 479, 482, 486, 490 and 493 *(vanD)*; 336 and 381 *(vanE)*; 246-248, 250, 251, 254, 258, 259, 262, 266, 269, 274, 284, 286, 287, 290, 301, 304, 306, 308, 312, 320 and 324 *(vanG).*

One embodiment is directed to a primer set comprising at least one forward primer selected from the group consisting of SEQ ID NOS: 610 (Efm *sodA*); 670, 679, 683, 691-693 (Efs *sodA*); and at least one reverse primer selected from the group consisting of SEQ ID NOS: SEQ ID NOS: 622 (Efm *sodA*); 710, 716-718, 730, 733 (Efs *sodA).*

One embodiment is directed to a primer set comprising at least one forward primer selected from the group consisting of SEQ ID NOS: 776, 780, 785 (Efm novel); 851, 865, 866, 868 (Efs novel) and at least one reverse primer selected from the group consisting of SEQ ID NOS: 800, 813, 816 (Efm novel); 878, 884, 890, 892, 896 (Efs novel).

One embodiment is directed to a primer set comprising at least one forward primer selected from the group consisting of SEQ ID NO: 936 (Efm/Efs dual); and at least one reverse primer selected from the group consisting of SEQ ID NOS: 938 and 939 (Efm/Efs dual).

One embodiment is directed to a primer set (at least one forward primer and at least one reverse primer) selected from the group consisting of: Groups 1-644 of Tables 5, 6, 8B, 9B, 10B, 11B, and 12.

One embodiment is directed to a method of producing a nucleic acid product, comprising contacting one or more isolated nucleic acid sequences selected from the group consisting of SEQ ID NOS: 1-502 and 600-939 to a sample comprising a vancomycin-resistance gene and/or an Efm and/or Efs *sodA* and/or Efm and/or Efs novel gene or dual marker genes under conditions suitable for nucleic acid polymerization. In a particular embodiment, the nucleic acid product is a *vanA* amplicon produced using at least one forward primer selected from the group consisting of SEQ ID NOS: 1, 6, 19, 22, 23, 26, 28, 29, 33, 34, 37-42, 45, 48, 53 and 59 and at least one reverse primer selected from the group consisting of SEQ ID NOS: 3, 5, 8, 10, 21, 31, 32, 36, 44, 47, 51, 52, 55 and 60. In a particular embodiment, the nucleic acid product is a *vanB* amplicon produced using at least one forward primer selected from the group consisting of SEQ ID NOS: 61, 68, 70-72, 75, 81, 83, 89, 93, 94, 103-105, 107 and 111 and at least one reverse primer selected from the group consisting of SEQ ID NOS: 63, 65, 66, 74, 77, 85-88, 90, 91, 95, and 97-102. In a particular embodiment, the nucleic acid product is a *vanC1* amplicon produced using at least one forward primer selected from the group consisting of SEQ ID NOS: 123, 127, 130, 133, 138, 141, 144, 148, 151, 156, 158, 161, 162, 165, 168, 170, 171, 175, 178, 180, 183-185, 188, 191, 193, 194, 196, 198, and 200-204 and at least one reverse primer selected from the group consisting of SEQ ID NOS: 125, 129, 132, 135, 137, 140, 143, 146, 147, 150, 153, 155, 157, 160, 164, 167, 169, 173, 174, 177, 179, 187, 190 and 192. In a particular embodiment, the nucleic acid product is a *vanC2*/*3* amplicon produced using at least one forward primer selected from the group consisting of SEQ ID NOS: 206, 210, 213, 217, 220, 221, 222, 224-226, 228-238, 240, 242 and 243 and at least one reverse primer selected from the group consisting of SEQ ID NOS: 208, 209, 212, 215, 216, 219, 223, 227, 239 and 241. In a particular embodiment, the nucleic acid product is a *vanD* amplicon produced using at least one forward primer selected from the group consisting of SEQ ID NOS: 388, 391, 394, 396, 399, 409, 415, 416, 425, 428, 435, 438, 440, 443, 445, 462, 465, 468, 471, 474, 477, 480, 483, 488, 491, 494-498 and 500-502 and at least one reverse primer selected from the group consisting of SEQ ID NOS: 390, 393, 395, 398, 401, 411, 417, 419, 421, 426, 430, 434, 437, 439, 442, 444, 447-455, 458-461, 464, 467, 470, 473, 476, 479, 482, 486, 490 and 493. In a particular embodiment, the nucleic acid product is a *vanE* amplicon produced using at least one forward primer selected from the group consisting of SEQ ID NOS: 334, 337-380 and 382-387 and at least one reverse primer selected from the group consisting of SEQ ID NOS: 336 and 381. In a particular embodiment, the nucleic acid product is a *vanG* amplicon produced using at least one forward primer selected from the group consisting of SEQ ID NOS: 244, 249, 252, 253, 255, 256, 260, 263, 264, 267, 270-272, 275-283, 285, 288, 291, 293, 295, 297-300, 302, 303, 305, 307, 309-311, 313-318, 321, 322, 325-333 and at least one reverse primer selected from the group consisting of SEQ ID NOS: 246-248, 250, 251, 254, 258, 259, 262, 266, 269, 274, 284, 286, 287, 290, 301, 304, 306, 308, 312, 320 and 324. In a particular embodiment, the nucleic acid product is a Efm *sodA* amplicon produced using at least one forward primer consisting of SEQ ID NOS: 610 and at least one reverse primer consisting of SEQ ID NOS: 622. In a particular embodiment, the nucleic acid product is a Efs *sodA* amplicon produced using at least one forward primer selected from the group consisting of SEQ ID NOS: 670, 679, 683, 691-693; and at least one reverse primer selected from the group consisting of SEQ ID NOS: 710, 716-718, 730, 733. In a particular embodiment, the nucleic acid product is a Efm novel amplicon produced using at least one forward primer selected from the group consisting of SEQ ID NOS: 776, 780, and 785; and at least one reverse primer selected from the group consisting of SEQ ID NOS: 800, 813, and 816. In a particular embodiment, the nucleic acid product is a Efs novel amplicon produced using at least one forward primer selected from the group consisting of SEQ ID NOS: 851, 865, 866, and 868; and at least one reverse primer selected from the group consisting of SEQ ID NOS: 878, 884, 890, 892, and 896. In a particular embodiment, the nucleic acid product is a Efm dual and Efs dual amplicon produced using at least one forward primer consisting of SEQ ID NO: 936; and at least one reverse primer consisting of SEQ ID NOS: 938 and 939.

One embodiment is directed to a probe that hybridized to an amplicon produced as described herein, *e.g.,* using the primers described herein. In a particular embodiment, the probe comprises a sequence selected from the group consisting of SEQ ID NOS: 2, 4, 7, 9, 11-18, 20, 24, 25, 27, 30, 35, 43, 46, 49, 50, 54, 56-58 (*vanA*); 62, 64, 67, 69, 73, 76, 78-80, 82, 84, 92, 96, 108-110, 112 *(vanB)*; 124, 126, 128, 131, 134, 136, 139, 142, 145, 149, 152, 154, 159, 163, 166, 172, 176, 181, 182, 186, 189, 195, 197, 199, 205 (*vanC1*); 207, 211 (*vanC2*/*3*); 389, 392, 397, 400, 402-408, 410, 412-414, 418, 420, 422-424, 427, 429, 431-433, 436, 441, 446, 457, 463, 466, 469, 472, 475, 478, 481, 484, 485, 487, 489, 492, 499 (*vanD*); 335 (*vanE*); 245, 257, 261, 265, 273, 289, 292, 294, 296, 319, 323 *(vanG).* In a particular embodiment, the probe comprises a sequence selected from the group consisting of SEQ ID NOS: 664, 648, 655 (Efm *sodA*); 737, 743, 747, 752, 754, 755, 756, 757, 758, 760, 766, 768, 769, 770 (Efs *sodA*); 821, 843 (Efm novel); 908, 925 (Efs novel), and 937 (Efm/Efs dual).

In a particular embodiment, the probe(s) is labeled with a detectable label selected from the group consisting of: a fluorescent label, a chemiluminescent label, a quencher, a radioactive label, biotin and gold.

One embodiment is directed to a set of probes that hybridize to an amplicon produced as described herein, *e.g.,* using the primers described herein. In a particular embodiment, a first probe comprises a sequence selected from the group consisting of SEQ ID NOS: 2, 4, 7, 9, 11-18, 20, 24, 25, 27, 30, 35, 43, 46, 49, 50, 54, 56-58 (*vanA*)*,* and a second probe comprises a sequence selected from the group consisting of SEQ ID NOS: 62, 64, 67, 69, 73, 76, 78-80, 82, 84, 92, 96, 108-110, 112 *(vanB).* In a particular embodiment, a first probe comprises a sequence selected from the group consisting of SEQ ID NOS: 2, 4, 7, 9, 11-18, 20, 24, 25, 27, 30, 35, 43, 46, 49, 50, 54, 56-58 *(vanA),* a second probe comprises a sequence selected from the group consisting of SEQ ID NOS: 62, 64, 67, 69, 73, 76, 78-80, 82, 84, 92, 96, 108-110, 112 *(vanB)*, and a third probe comprises SEQ ID NO: 506. In a particular embodiment, a first probe comprises a sequence selected from the group consisting of SEQ ID NOS: 664, 648, 655 (Efm *sodA*); a second probe comprises a sequence selected from the group consisting of SEQ ID NOS: 737, 743, 747, 752, 754, 755, 756, 757, 758, 760, 766, 768, 769, 770 (Efs *sodA*). In a particular embodiment, a first probe comprises a sequence selected from the group consisting of SEQ ID NOS: 664, 648, 655 (Efm *sodA*); a second probe comprises a sequence selected from the group consisting of SEQ ID NOS: 737, 743, 747, 752, 754, 755, 756, 757, 758, 760, 766, 768, 769, 770 (Efs *sodA*); a third probe comprises SEQ ID NO: 506. In a particular embodiment, a first probe comprises a sequence selected from the group consisting of SEQ ID NOS: 821 and 843 (Efm novel); a second probe comprises a sequence selected from the group consisting of SEQ ID NOS: 908 and 925 (Efs novel). In a particular embodiment, a first probe comprises a sequence selected from the group consisting of SEQ ID NOS: 821 and 843 (Efm novel); a second probe comprises a sequence selected from the group consisting of SEQ ID NOS: 908 and 925 (Efs novel); a third probe comprises SEQ ID NO: 506. In a particular embodiment, a probe comprises a sequence consisting of SEQ ID NO: 937 (Efm/Efs dual). In a particular embodiment, a probe comprises a sequence consisting of SEQ ID NO: 937 (Efm/Efs dual) and a second probe comprises SEQ ID NO: 506.

In a particular embodiment, the first probe is labeled with a first detectable label and the second probe is labeled with a second detectable label. In a particular embodiment, the first probe and the second probe are labeled with the same detectable label. In a particular embodiment, the first probe is labeled with a first detectable label, the second probe is labeled with a second detectable label and the third probe is labeled with a third detectable label. One embodiment is directed to a probe that hybridizes directly to the genomic sequences of the target without amplification. In a particular embodiment, the probe comprises a sequence selected from the group consisting of SEQ ID NOS: 2, 4, 7, 9, 11-18, 20, 24, 25, 27, 30, 35, 43, 46, 49, 50, 54, 56-58 (*vanA*); 62, 64, 67, 69, 73, 76, 78-80, 82, 84, 92, 96, 108-110, 112 *(vanB)*; 124, 126, 128, 131, 134, 136, 139, 142, 145, 149, 152, 154, 159, 163, 166, 172, 176, 181, 182, 186, 189, 195, 197, 199, 205 (*vanC1*); 207, 211 (*vanC2*/*3*); 389, 392, 397, 400, 402-408, 410, 412-414, 418, 420, 422-424, 427, 429, 431-433, 436, 441, 446, 457, 463, 466, 469, 472, 475, 478, 481, 484, 485, 487, 489, 492, 499 (*vanD*); 335 (*vanE*); 245, 257, 261, 265, 273, 289, 292, 294, 296, 319, 323 *(vanG).* In a particular embodiment, the probe comprises a sequence selected from the group consisting of SEQ ID NOS: 664, 648, 655 (Efm *sodA*); 737, 743, 747, 752, 754, 755, 756, 757, 758, 760, 766, 768, 769, 770 (Efs *sodA*); 821 and 843 (Efm novel); 908 and 925 (Efs novel); and 937 (Efm/Efs dual).

In a particular embodiment, the probe(s) is labeled with a detectable label selected from the group consisting of: a fluorescent label, a chemiluminescent label, a quencher, a radioactive label, biotin and gold.

One embodiment is directed to a set of probes that hybridize directly to the genomic sequences of the target without amplification. In a particular embodiment, a first probe comprises a sequence selected from the group consisting of SEQ ID NOS: 2, 4, 7, 9, 11-18, 20, 24, 25, 27, 30, 35, 43, 46, 49, 50, 54, 56-58 *(vanA),* and a second probe comprises a sequence selected from the group consisting of SEQ ID NOS: 62, 64, 67, 69, 73, 76, 78-80, 82, 84, 92, 96, 108-110, 112 *(vanB).* In a particular embodiment, a first probe comprises a sequence selected from the group consisting of SEQ ID NOS: 2, 4, 7, 9, 11-18, 20, 24, 25, 27, 30, 35, 43, 46, 49, 50, 54, 56-58 *(vanA)*, a second probe comprises a sequence selected from the group consisting of SEQ ID NOS: 62, 64, 67, 69, 73, 76, 78-80, 82, 84, 92, 96, 108-110, 112 *(vanB)*, a third probe comprises SEQ ID NO: 506. In a particular embodiment, a first probe comprises a sequence selected from the group consisting of SEQ ID NOS: 2, 4, 7, 9, 11-18, 20, 24, 25, 27, 30, 35, 43, 46, 49, 50, 54, 56-58 (*vanA*), a second probe comprises a sequence selected from the group consisting of SEQ ID NOS: 62, 64, 67, 69, 73, 76, 78-80, 82, 84, 92, 96, 108-110, 112 (*vanB*), a third probe comprises SEQ ID NOS: 664, 648, 655 (Efm *sodA*); and a fourth probe comprises a sequence selected from the group consisting of SEQ ID NOS: 737, 743, 747, 752, 754, 755, 756, 757, 758, 760, 766, 768, 769, 770 (Efs *sodA*). In a particular embodiment, a first probe comprises a sequence selected from the group consisting of SEQ ID NOS: 2, 4, 7, 9, 11-18, 20, 24, 25, 27, 30, 35, 43, 46, 49, 50, 54, 56-58 (*vanA*), a second probe comprises a sequence selected from the group consisting of SEQ ID NOS: 62, 64, 67, 69, 73, 76, 78-80, 82, 84, 92, 96, 108-110, 112 (*vanB*), a third probe comprises SEQ ID NOS: 664, 648, 655 (Efm *sodA*); and a fourth probe comprises a sequence selected from the group consisting of SEQ ID NOS: 737, 743, 747, 752, 754, 755, 756, 757, 758, 760, 766, 768, 769, 770 (Efs *sodA*); a fifth probe comprises SEQ ID NO: 506. In a particular embodiment, a first probe comprises a sequence selected from the group consisting of SEQ ID NOS: 2, 4, 7, 9, 11-18, 20, 24, 25, 27, 30, 35, 43, 46, 49, 50, 54, 56-58 (*vanA*), a second probe comprises a sequence selected from the group consisting of SEQ ID NOS: 62, 64, 67, 69, 73, 76, 78-80, 82, 84, 92, 96, 108-110, 112 (*vanB*)*,* a third probe comprises SEQ ID NOS: 821 and 843 (Efm novel); a fourth probe comprises a sequence selected from the group consisting of SEQ ID NOS: 928 and 925 (Efs novel and a fifth probe comprises SEQ ID NO: 506). In a particular embodiment, a first probe comprises a sequence selected from the group consisting of SEQ ID NOS: 2, 4, 7, 9, 11-18, 20, 24, 25, 27, 30, 35, 43, 46, 49, 50, 54, 56-58 (*vanA*), a second probe comprises a sequence selected from the group consisting of SEQ ID NOS: 62, 64, 67, 69, 73, 76, 78-80, 82, 84, 92, 96, 108-110, 112 (*vanB*), a third probe comprises SEQ ID NOS: 937 (Efs/Efm dual) and a fifth probe comprises SEQ ID: 506..

In a particular embodiment, the first probe is labeled with a first detectable label and the second probe is labeled with a second detectable label. In a particular embodiment, the first probe and the second probe are labeled with the same detectable label. In a particular embodiment, the first probe is labeled with a first detectable label, the second probe is labeled with a second detectable label and the third probe is labeled with a third detectable label. In a particular embodiment, the detectable labels are selected from the group consisting of: a fluorescent label, a chemiluminescent label, a quencher, a radioactive label, biotin and gold.

In one embodiment, the probe(s) is fluorescently labeled and the step of detecting the binding of the probe to the amplified product comprises measuring the fluorescence of the sample. In one embodiment, the probe comprises a fluorescent reporter moiety and a quencher of fluorescence-quenching moiety. Upon probe hybridization with the amplified product, the exonuclease activity of a DNA polymerase dissociates the probe's fluorescent reporter and the quencher, resulting in the unquenched emission of fluorescence, which is detected. An increase in the amplified product causes a proportional increase in fluorescence, due to cleavage of the probe and release of the reporter moiety of the probe. The amplified product is quantified in real time as it accumulates. In another embodiment, each probe in the multiplex reaction is labeled with a different distinguishable and detectable label.

In a particular embodiment, the probes are molecular beacons. Molecular beacons are single-stranded probes that form a stem-and-loop structure. A fluorophore is covalently linked to one end of the stem and a quencher is covalently linked to the other end of the stem forming a stem hybrid; fluorescence is quenched when the formation of the stem loop positions the fluorophore proximal to the quencher. When a molecular beacon hybridizes to a target nucleic acid sequence, the probe undergoes a conformational change that results in the dissociation of the stem hybrid and, thus the fluorophore and the quencher move away from each other, enabling the probe to fluoresce brightly. Molecular beacons can be labeled with differently colored fluorophores to detect different target sequences. Any of the probes described herein may be designed and utilized as molecular beacons.

One embodiment is directed a method for detecting a vancomycin-resistance gene(s) in a sample, comprising: (a) contacting the sample with at least one forward primer comprising a sequence selected from the group consisting of: SEQ ID NOS: 1, 6, 19, 22, 23, 26, 28, 29, 33, 34, 37-42, 45, 48, 53 and 59 (*vanA*); 61, 68, 70-72, 75, 81, 83, 89, 93, 94, 103-105, 107 and 111 *(vanB)*; 123, 127, 130, 133, 138, 141, 144, 148, 151, 156, 158, 161, 162, 165, 168, 170, 171, 175, 178, 180, 183-185, 188, 191, 193, 194, 196, 198, and 200-204 (*vanC1*); 206, 210, 213, 217, 220, 221, 222, 224-226, 228-238, 240, 242 and 243 (*vanC2*/*3*); 388, 391, 394, 396, 399, 409, 415, 416, 425, 428, 435, 438, 440, 443, 445, 462, 465, 468, 471, 474, 477, 480, 483, 488, 491, 494-498 and 500-502 (*vanD*); 334, 337-380 and 382-387 (*vanE*); 244, 249, 252, 253, 255, 256, 260, 263, 264, 267, 270-272, 275-283, 285, 288, 291, 293, 295, 297-300, 302, 303, 305, 307, 309-311, 313-318, 321, 322, 325-333 *(vanG)*, and at least one reverse primer comprising a sequence selected from the group consisting of: SEQ ID NOS: 3, 5, 8, 10, 21, 31, 32, 36, 44, 47, 51, 52, 55 and 60 *(vanA)*; 63, 65, 66, 74, 77, 85-88, 90, 91, 95, and 97-102 *(vanB)*; 125, 129, 132, 135, 137, 140, 143, 146, 147, 150, 153, 155, 157, 160, 164, 167, 169, 173, 174, 177, 179, 187, 190 and 192 (*vanC1*); 208, 209, 212, 215, 216, 219, 223, 227, 239 and 241 (*vanC2*/*3*); 390, 393, 395, 398, 401, 411, 417, 419, 421, 426, 430, 434, 437, 439, 442, 444, 447-455, 458-461, 464, 467, 470, 473, 476, 479, 482, 486, 490 and 493 (*vanD*); 336 and 381 (*vanE*); 246-248, 250, 251, 254, 258, 259, 262, 266, 269, 274, 284, 286, 287, 290, 301, 304, 306, 308, 312, 320 and 324 (*vanG*) under conditions such that nucleic acid amplification occurs to yield an amplicon; and (b) contacting the amplicon with one or more probes comprising one or more sequences selected from the group consisting of: SEQ ID NOS: 2, 4, 7, 9, 11-18, 20, 24, 25, 27, 30, 35, 43, 46, 49, 50, 54, 56-58 (*vanA*); 62, 64, 67, 69, 73, 76, 78-80, 82, 84, 92, 96, 108-110, 112 (*vanB*); 124, 126, 128, 131, 134, 136, 139, 142, 145, 149, 152, 154, 159, 163, 166, 172, 176, 181, 182, 186, 189, 195, 197, 199, 205 (*vanC1*); 207, 211 (*vanC2*/*3*); 389, 392, 397, 400, 402-408, 410, 412-414, 418, 420, 422-424, 427, 429, 431-433, 436, 441, 446, 457, 463, 466, 469, 472, 475, 478, 481, 484, 485, 487, 489, 492, 499 *(vanD)*; 335 *(vanE)*; 245, 257, 261, 265, 268, 273, 289, 292, 294, 296, 319, 323 (*vanG*) under conditions such that hybridization of the probe to the amplicon occurs, wherein hybridization of the probe is indicative of a vancomycin-resistance gene(s) in the sample.

One embodiment is directed a method for detecting an Enterococcal Efm *sodA* or Efs *sodA* gene(s) or novel gene or dual marker in a sample, comprising: (a) contacting the sample with at least one forward primer comprising a sequence selected from the group consisting of: SEQ ID NOS: 610 (Efm *sodA*); 670, 679, 683, 691-693 (Efs *sodA)*; 776, 780, 785 (Efm novel); 851, 865, 866, 868 (Efs novel); and 936 (Efm/Efs dual); and at least one reverse primer comprising a sequence selected from the group consisting of SEQ ID NOS: 622 (Efm *sodA*); 710, 716-718, 730, 733 (Efs *sodA*); 800, 813, 816 (Efm novel); 878, 884, 890, 892, 896 (Efs novel); 938 and 939 (Efm/Efs dual) under conditions such that nucleic acid amplification occurs to yield an amplicon; and (b) contacting the amplicon with one or more probes comprising one or more sequences selected from the group consisting of: SEQ ID NOS: 664, 648, 655 (Efm *sodA*); 737, 743, 747, 752, 754, 755, 756, 757, 758, 760, 766, 768, 769, 770 (Efs *sodA*); 821, 843 (Efm novel); 908, 925 (Efs novel); and 937 (Efm/Efs dual).

One embodiment is directed a method for detecting a vancomycin-resistance gene(s) or an *Enterococcal* marker gene in a sample, comprising: (a) contacting the sample with at least one forward primer comprising a sequence selected from the group consisting of: SEQ ID NOS: 1, 6, 19, 22, 23, 26, 28, 29, 33, 34, 37-42, 45, 48, 53 and 59 (*vanA*); 61, 68, 70-72, 75, 81, 83, 89, 93, 94, 103-105, 107 and 111 (*vanB*); 123, 127, 130, 133, 138, 141, 144, 148, 151, 156, 158, 161, 162, 165, 168, 170, 171, 175, 178, 180, 183-185, 188, 191, 193, 194, 196, 198, and 200-204 (*vanC1*); 206, 210, 213, 217, 220, 221, 222, 224-226, 228-238, 240, 242 and 243 (*vanC2*/*3*); 388, 391, 394, 396, 399, 409, 415, 416, 425, 428, 435, 438, 440, 443, 445, 462, 465, 468, 471, 474, 477, 480, 483, 488, 491, 494-498 and 500-502 (*vanD*); 334, 337-380 and 382-387 (*vanE*); 244, 249, 252, 253, 255, 256, 260, 263, 264, 267, 270-272, 275-283, 285, 288, 291, 293, 295, 297-300, 302, 303, 305, 307, 309-311, 313-318, 321, 322, 325-333 (*vanG*)*,* 610 (Efm *sodA*); 670, 679, 683, 691-693 (Efs *sodA*); 776, 780, 785 (Efm novel); 851, 865, 866, 868 (Efs novel); and 936 (Efm/Efs dual), and at least one reverse primer comprising a sequence selected from the group consisting of: SEQ ID NOS: 3, 5, 8, 10, 21, 31, 32, 36, 44, 47, 51, 52, 55 and 60 (*vanA*); 63, 65, 66, 74, 77, 85-88, 90, 91, 95, and 97-102 (*vanB*); 125, 129, 132, 135, 137, 140, 143, 146, 147, 150, 153, 155, 157, 160, 164, 167, 169, 173, 174, 177, 179, 187, 190 and 192 (*vanC1*); 208, 209, 212, 215, 216, 219, 223, 227, 239 and 241 (*vanC2*/*3*); 390, 393, 395, 398, 401, 411, 417, 419, 421, 426, 430, 434, 437, 439, 442, 444, 447-455, 458-461, 464, 467, 470, 473, 476, 479, 482, 486, 490 and 493 (*vanD*); 336 and 381 (*vanE*); 246-248, 250, 251, 254, 258, 259, 262, 266, 269, 274, 284, 286, 287, 290, 301, 304, 306, 308, 312, 320 and 324 *(vanG),* 622 (Efm *sodA*); 710, 716-718, 730, 733 (Efs *sodA*); 800, 813, 816 (Efm novel); 878, 884, 890, 892, 896 (Efs novel); 938 and 939 (Efm/Efs dual) under conditions such that nucleic acid amplification occurs to yield an amplicon; and (b) contacting the amplicon with one or more probes comprising one or more sequences selected from the group consisting of: SEQ ID NOS: 2, 4, 7, 9, 11-18, 20, 24, 25, 27, 30, 35, 43, 46, 49, 50, 54, 56-58 (*vanA*); 62, 64, 67, 69, 73, 76, 78-80, 82, 84, 92, 96, 108-110, 112 (*vanB*); 124, 126, 128, 131, 134, 136, 139, 142, 145, 149, 152, 154, 159, 163, 166, 172, 176, 181, 182, 186, 189, 195, 197, 199, 205 (*vanC1*); 207, 211 (*vanC2*/*3*); 389, 392, 397, 400, 402-408, 410, 412-414, 418, 420, 422-424, 427, 429, 431-433, 436, 441, 446, 457, 463, 466, 469, 472, 475, 478, 481, 484, 485, 487, 489, 492, 499 (*vanD*); 335 (*vanE*); 245, 257, 261, 265, 268, 273, 289, 292, 294, 296, 319, 323 *(vanG),* 664, 648, 655 (Efm *sodA*); 737, 743, 747, 752, 754, 755, 756, 757, 758, 760, 766, 768, 769, 770 (Efs *sodA*); 821, 843 (Efm novel); 908, 925 (Efs novel); and 937 (Efm/Efs dual)under conditions such that hybridization of the probe to the amplicon occurs, wherein hybridization of the probe is indicative of a vancomycin-resistance gene(s) in the sample.

In a particular embodiment, each of the one or more probes is labeled with a different detectable label. In a particular embodiment, the one or more probes are labeled with the same detectable label. In a particular embodiment, the sample is selected from the group consisting of: blood, serum, plasma, enriched peripheral blood mononuclear cells, neoplastic or other tissue obtained from biopsies, cerebrospinal fluid, saliva, fluids collected from the ear, eye, mouth, and respiratory airways, sputum, skin, tears, oropharyngeal swabs, nasopharyngeal swabs, throat swabs, urine, anal-rectal swabs, feces, skin swabs, nasal aspirates, nasal wash, fluids and cells obtained by the perfusion of tissues of both human and animal origin, and fluids and cells derived from the culturing of human cells, including human stem cells and human cartilage or fibroblasts. In one embodiment, the sample is from a human, is non-human in origin, or is derived from an inanimate object or environmental surfaces. In a particular embodiment, the at least one forward primer, the at least one reverse primer and the one or more probes are selected from the group consisting of: Groups 1-212 of Table 5, Groups 213-601 of Table 6, Groups 603-605 of Table 8B, Groups 606-627 of Table 9B, Groups 628-636 of Table 10B, Groups 637-643 of Table 11B, and Group 644 of Table 12. In a particular embodiment, the method(s) further comprise isolating and/or sequencing the vancomycin-resistance gene sequence(s) and/or Enterococcal *sodA or* novel gene or dual marker sequence(s) in a sample.

One embodiment is directed to a primer set or collection of primer sets for amplifying DNA of a vancomycin-resistance gene (*vanA* gene), comprising a nucleotide sequence selected from the group consisting of: (1) SEQ ID NOS: 1 and 3; (2) SEQ ID NOS: 1 and 32; (3) SEQ ID NOS: 1 and 5; (4) SEQ ID NOS: 19 and 21; (5) SEQ ID NOS: 19 and 3; (6) SEQ ID NOS: 19 and 32; (7) SEQ ID NOS: 19 and 47; (8) SEQ ID NOS: 19 and 5; (9) SEQ ID NOS: 19 and 52; (10) SEQ ID NOS: 19 and 55; (11) SEQ ID NOS: 22 and 21; (12) SEQ ID NOS: 22 and 3; (13) SEQ ID NOS: 22 and 32; (14) SEQ ID NOS: 22 and 5; (15) SEQ ID NOS: 23 and 21; (16) SEQ ID NOS: 23 and 3; (17) SEQ ID NOS: 23 and 32; (18) SEQ ID NOS: 23 and 5; (19) SEQ ID NOS: 26 and 3; (20) SEQ ID NOS: 26 and 32; (21) SEQ ID NOS: 26 and 47; (22) SEQ ID NOS: 26 and 5; (23) SEQ ID NOS: 26 and 51; (24) SEQ ID NOS: 28 and 3; (25) SEQ ID NOS: 28 and 32; (26) SEQ ID NOS: 28 and 5; (27) SEQ ID NOS: 29 and 21; (28) SEQ ID NOS: 29 and 3; (29) SEQ ID NOS: 29 and 5; (30) SEQ ID NOS: 29 and 8; (31) SEQ ID NOS: 33 and 21; (32) SEQ ID NOS: 33 and 3; (33) SEQ ID NOS: 33 and 32; (34) SEQ ID NOS: 33 and 5; (35) SEQ ID NOS: 34 and 36; (36) SEQ ID NOS: 34 and 52; (37) SEQ ID NOS: 37 and 3; (38) SEQ ID NOS: 37 and 32; (39) SEQ ID NOS: 37 and 5; (40) SEQ ID NOS: 38 and 3; (41) SEQ ID NOS: 38 and 32; (42) SEQ ID NOS: 38 and 5; (43) SEQ ID NOS: 39 and 3; (44) SEQ ID NOS: 39 and 32; (45) SEQ ID NOS: 39 and 5; (46) SEQ ID NOS: 40 and 3; (47) SEQ ID NOS: 40 and 32; (48) SEQ ID NOS: 40 and 5; (49) SEQ ID NOS: 41 and 21; (50) SEQ ID NOS: 42 and 44; (51) SEQ ID NOS: 45 and 47; (52) SEQ ID NOS: 48 and 47; (53) SEQ ID NOS: 53 and 47; (54) SEQ ID NOS: 59 and 52; (55) SEQ ID NOS: 59 and 60; (56) SEQ ID NOS: 6 and 10; (57) SEQ ID NOS: 6 and 21; (58) SEQ ID NOS: 6 and 3; (59) SEQ ID NOS: 6 and 31; (60) SEQ ID NOS: 6 and 32; (61) SEQ ID NOS: 6 and 5; and (62) SEQ ID NOS: 6 and 8.

One embodiment is directed to a primer set or collection of primer sets for amplifying DNA of a vancomycin-resistance gene (*vanB* gene), comprising a nucleotide sequence selected from the group consisting of: (1) SEQ ID NOS: 103 and 65; (2) SEQ ID NOS: 103 and 66; (3) SEQ ID NOS: 103 and 86; (4) SEQ ID NOS: 103 and 87; (5) SEQ ID NOS: 103 and 88; (6) SEQ ID NOS: 104 and 66; (7) SEQ ID NOS: 105 and 66; (8) SEQ ID NOS: 107 and 66; (9) SEQ ID NOS: 111 and 63; (10) SEQ ID NOS: 111 and 66; (11) SEQ ID NOS: 111 and 88; (12) SEQ ID NOS: 61 and 63; (13) SEQ ID NOS: 61 and 65; (14) SEQ ID NOS: 61 and 66; (15) SEQ ID NOS: 61 and 74; (16) SEQ ID NOS: 61 and 77; (17) SEQ ID NOS: 61 and 97; (18) SEQ ID NOS: 68 and 63; (19) SEQ ID NOS: 68 and 65; (20) SEQ ID NOS: 68 and 66; (21) SEQ ID NOS: 68 and 74; (22) SEQ ID NOS: 68 and 77; (23) SEQ ID NOS: 70 and 63; (24) SEQ ID NOS: 70 and 74; (25) SEQ ID NOS: 70 and 77; (26) SEQ ID NOS: 71 and 63; (27) SEQ ID NOS: 71 and 74; (28) SEQ ID NOS: 71 and 77; (29) SEQ ID NOS: 72 and 74; (30) SEQ ID NOS: 75 and 74; (31) SEQ ID NOS: 81 and 65; (32) SEQ ID NOS: 81 and 66; (33) SEQ ID NOS: 81 and 77; (34) SEQ ID NOS: 81 and 87; (35) SEQ ID NOS: 81 and 88; (36) SEQ ID NOS: 81 and 95; (37) SEQ ID NOS: 83 and 101; (38) SEQ ID NOS: 83 and 65; (39) SEQ ID NOS: 83 and 66; (40) SEQ ID NOS: 83 and 85; (41) SEQ ID NOS: 83 and 86; (42) SEQ ID NOS: 83 and 87; (43) SEQ ID NOS: 83 and 88; (44) SEQ ID NOS: 83 and 95; (45) SEQ ID NOS: 89 and 100; (46) SEQ ID NOS: 89 and 101; (47) SEQ ID NOS: 89 and 102; (48) SEQ ID NOS: 89 and 65; (49) SEQ ID NOS: 89 and 66; (50) SEQ ID NOS: 89 and 85; (51) SEQ ID NOS: 89 and 86; (52) SEQ ID NOS: 89 and 87; (53) SEQ ID NOS: 89 and 88; (54) SEQ ID NOS: 89 and 90; (55) SEQ ID NOS: 89 and 91; (56) SEQ ID NOS: 89 and 95; (57) SEQ ID NOS: 89 and 98; (58) SEQ ID NOS: 89 and 99; (59) SEQ ID NOS: 93 and 101; (60) SEQ ID NOS: 93 and 65; (61) SEQ ID NOS: 93 and 66; (62) SEQ ID NOS: 93 and 85; (63) SEQ ID NOS: 93 and 86; (64) SEQ ID NOS: 93 and 87; (65) SEQ ID NOS: 93 and 88; (66) SEQ ID NOS: 93 and 90; (67) SEQ ID NOS: 93 and 95; (68) SEQ ID NOS: 93 and 98; (69) SEQ ID NOS: 94 and 65; (70) SEQ ID NOS: 94 and 66; (71) SEQ ID NOS: 94 and 87; (72) SEQ ID NOS: 94 and 88; and (73) SEQ ID NOS: 94 and 95.

One embodiment is directed to a primer set or collection of primer sets for amplifying DNA of a vancomycin-resistance gene (*vanC1* gene), comprising a nucleotide sequence selected from the group consisting of: (1) SEQ ID NOS: 123 and 125; (2) SEQ ID NOS: 127 and 129; (3) SEQ ID NOS: 130 and 132; (4) SEQ ID NOS: 133 and 135; (5) SEQ ID NOS: 133 and 137; (6) SEQ ID NOS: 138 and 140; (7) SEQ ID NOS: 141 and 137; (8) SEQ ID NOS: 141 and 143; (9) SEQ ID NOS: 141 and 147; (10) SEQ ID NOS: 141 and 179; (11) SEQ ID NOS: 144 and 137; (12) SEQ ID NOS: 144 and 146; (13) SEQ ID NOS: 144 and 147; (14) SEQ ID NOS: 144 and 157; (15) SEQ ID NOS: 148 and 137; (16) SEQ ID NOS: 148 and 150; (17) SEQ ID NOS: 151 and 153; (18) SEQ ID NOS: 151 and 155; (19) SEQ ID NOS: 156 and 150; (20) SEQ ID NOS: 158 and 160; (21) SEQ ID NOS: 161 and 137; (22) SEQ ID NOS: 161 and 147; (23) SEQ ID NOS: 161 and 150; (24) SEQ ID NOS: 161 and 153; (25) SEQ ID NOS: 161 and 190; (26) SEQ ID NOS: 161 and 192; (27) SEQ ID NOS: 162 and 164; (28) SEQ ID NOS: 165 and 167; (29) SEQ ID NOS: 168 and 169; (30) SEQ ID NOS: 170 and 169; (31) SEQ ID NOS: 171 and 173; (32) SEQ ID NOS: 171 and 174; (33) SEQ ID NOS: 175 and 177; (34) SEQ ID NOS: 178 and 179; (35) SEQ ID NOS: 180 and 146; (36) SEQ ID NOS: 183 and 150; (37) SEQ ID NOS: 184 and 174; (38) SEQ ID NOS: 185 and 187; (39) SEQ ID NOS: 188 and 137; (40) SEQ ID NOS: 188 and 150; (41) SEQ ID NOS: 188 and 190; (42) SEQ ID NOS: 191 and 137; (43) SEQ ID NOS: 191 and 150; (44) SEQ ID NOS: 191 and 153; (45) SEQ ID NOS: 191 and 190; (46) SEQ ID NOS: 191 and 192; (47) SEQ ID NOS: 193 and 137; (48) SEQ ID NOS: 193 and 150; (49) SEQ ID NOS: 193 and 153; (50) SEQ ID NOS: 193 and 190; (51) SEQ ID NOS: 194 and 147; (52) SEQ ID NOS: 194 and 160; (53) SEQ ID NOS: 196 and 147; (54) SEQ ID NOS: 196 and 160; (55) SEQ ID NOS: 198 and 179; (56) SEQ ID NOS: 200 and 179; (57) SEQ ID NOS: 201 and 179; (58) SEQ ID NOS: 202 and 179; (59) SEQ ID NOS: 203 and 147; (60) SEQ ID NOS: 204 and 179; and (61) SEQ ID NOS: 204 and 187.

One embodiment is directed to a primer set or collection of primer sets for amplifying DNA of a vancomycin-resistance gene (*vanC2*/*C3* gene), comprising a nucleotide sequence selected from the group consisting of: (1) SEQ ID NOS: 206 and 208; (2) SEQ ID NOS: 206 and 209; (3) SEQ ID NOS: 206 and 216; (4) SEQ ID NOS: 206 and 219; (5) SEQ ID NOS: 206 and 227; (6) SEQ ID NOS: 210 and 209; (7) SEQ ID NOS: 210 and 212; (8) SEQ ID NOS: 210 and 215; (9) SEQ ID NOS: 210 and 216; (10) SEQ ID NOS: 210 and 219; (11) SEQ ID NOS: 210 and 223; (12) SEQ ID NOS: 210 and 227; (13) SEQ ID NOS: 213 and 215; (14) SEQ ID NOS: 217 and 209; (15) SEQ ID NOS: 217 and 216; (16) SEQ ID NOS: 217 and 219; (17) SEQ ID NOS: 217 and 223; (18) SEQ ID NOS: 217 and 227; (19) SEQ ID NOS: 220 and 209; (20) SEQ ID NOS: 220 and 219; (21) SEQ ID NOS: 220 and 223; (22) SEQ ID NOS: 220 and 227; (23) SEQ ID NOS: 221 and 209; (24) SEQ ID NOS: 221 and 216; (25) SEQ ID NOS: 221 and 219; (26) SEQ ID NOS: 221 and 227; (27) SEQ ID NOS: 222 and 209; (28) SEQ ID NOS: 222 and 216; (29) SEQ ID NOS: 222 and 219; (30) SEQ ID NOS: 222 and 223; (31) SEQ ID NOS: 222 and 227; (32) SEQ ID NOS: 224 and 212; (33) SEQ ID NOS: 224 and 215; (34) SEQ ID NOS: 224 and 216; (35) SEQ ID NOS: 225 and 209; (36) SEQ ID NOS: 225 and 212; (37) SEQ ID NOS: 225 and 216; (38) SEQ ID NOS: 226 and 209; (39) SEQ ID NOS: 226 and 212; (40) SEQ ID NOS: 226 and 216; (41) SEQ ID NOS: 228 and 215; (42) SEQ ID NOS: 229 and 209; (43) SEQ ID NOS: 229 and 215; (44) SEQ ID NOS: 230 and 219; (45) SEQ ID NOS: 231 and 212; (46) SEQ ID NOS: 231 and 215; (47) SEQ ID NOS: 232 and 216; (48) SEQ ID NOS: 233 and 212; (49) SEQ ID NOS: 234 and 215; (50) SEQ ID NOS: 235 and 215; (51) SEQ ID NOS: 235 and 239; (52) SEQ ID NOS: 235 and 241; (53) SEQ ID NOS: 236 and 216; (54) SEQ ID NOS: 237 and 209; (55) SEQ ID NOS: 237 and 215; (56) SEQ ID NOS: 238 and 215; (57) SEQ ID NOS: 240 and 216; (58) SEQ ID NOS: 242 and 216; and (59) SEQ ID NOS: 243 and 215.

One embodiment is directed to a primer set or collection of primer sets for amplifying DNA of a vancomycin-resistance gene (*vanD* gene), comprising a nucleotide sequence selected from the group consisting of: (1) SEQ ID NOS: 388 and 390; (2) SEQ ID NOS: 391 and 393; (3) SEQ ID NOS: 391 and 434; (4) SEQ ID NOS: 394 and 393; (5) SEQ ID NOS: 396 and 398; (6) SEQ ID NOS: 396 and 419; (7) SEQ ID NOS: 396 and 419; (8) SEQ ID NOS: 399 and 401; (9) SEQ ID NOS: 399 and 401; (10) SEQ ID NOS: 399 and 401; (11) SEQ ID NOS: 399 and 401; (12) SEQ ID NOS: 399 and 444; (13) SEQ ID NOS: 399 and 444; (14) SEQ ID NOS: 415 and 401; (15) SEQ ID NOS: 416 and 417; (16) SEQ ID NOS: 435 and 437; (17) SEQ ID NOS: 438 and 439; (18) SEQ ID NOS: 440 and 442; (19) SEQ ID NOS: 443 and 434; (20) SEQ ID NOS: 445 and 447; (21) SEQ ID NOS: 445 and 448; (22) SEQ ID NOS: 445 and 449; (23) SEQ ID NOS: 445 and 450; (24) SEQ ID NOS: 445 and 451; (25) SEQ ID NOS: 445 and 452; (26) SEQ ID NOS: 445 and 453; (27) SEQ ID NOS: 445 and 454; (28) SEQ ID NOS: 445 and 455; (29) SEQ ID NOS: 445 and 459; (30) SEQ ID NOS: 445 and 460; (31) SEQ ID NOS: 445 and 461; (32) SEQ ID NOS: 456 and 458; (33) SEQ ID NOS: 462 and 464; (34) SEQ ID NOS: 465 and 467; (35) SEQ ID NOS: 468 and 470; (36) SEQ ID NOS: 471 and 473; (37) SEQ ID NOS: 474 and 476; (38) SEQ ID NOS: 477 and 479; (39) SEQ ID NOS: 480 and 482; (40) SEQ ID NOS: 483 and 479; (41) SEQ ID NOS: 483 and 486; (42) SEQ ID NOS: 488 and 490; (43) SEQ ID NOS: 491 and 493; (44) SEQ ID NOS: 494 and 486; (45) SEQ ID NOS: 495 and 493; (46) SEQ ID NOS: 496 and 486; (47) SEQ ID NOS: 497 and 486; (48) SEQ ID NOS: 498 and 486; (49) SEQ ID NOS: 500 and 486; (50) SEQ ID NOS: 501 and 486; and (51) SEQ ID NOS: 502 and 493.

One embodiment is directed to a primer set or collection of primer sets for amplifying DNA of a vancomycin-resistance gene (*vanE* gene), comprising a nucleotide sequence selected from the group consisting of: (1) SEQ ID NOS: 334 and 336; (2) SEQ ID NOS: 337 and 336; (3) SEQ ID NOS: 338 and 336; (4) SEQ ID NOS: 338 and 381; (5) SEQ ID NOS: 339 and 336; (6) SEQ ID NOS: 340 and 336; (7) SEQ ID NOS: 341 and 336; (8) SEQ ID NOS: 341 and 381; (9) SEQ ID NOS: 342 and 336; (10) SEQ ID NOS: 342 and 381; (11) SEQ ID NOS: 343 and 336; (12) SEQ ID NOS: 344 and 336; (13) SEQ ID NOS: 344 and 381; (14) SEQ ID NOS: 345 and 336; (15) SEQ ID NOS: 346 and 336; (16) SEQ ID NOS: 347 and 336; (17) SEQ ID NOS: 347 and 381; (18) SEQ ID NOS: 348 and 336; (19) SEQ ID NOS: 348 and 381; (20) SEQ ID NOS: 349 and 336; (21) SEQ ID NOS: 349 and 381; (22) SEQ ID NOS: 350 and 336; (23) SEQ ID NOS: 350 and 381; (24) SEQ ID NOS: 351 and 336; (25) SEQ ID NOS: 352 and 336; (26) SEQ ID NOS: 353 and 336; (27) SEQ ID NOS: 354 and 336; (28) SEQ ID NOS: 355 and 336; (29) SEQ ID NOS: 355 and 381; (30) SEQ ID NOS: 356 and 336; (31) SEQ ID NOS: 357 and 336; (32) SEQ ID NOS: 358 and 336; (33) SEQ ID NOS: 359 and 336; (34) SEQ ID NOS: 359 and 381; (35) SEQ ID NOS: 360 and 336; (36) SEQ ID NOS: 360 and 381; (37) SEQ ID NOS: 361 and 336; (38) SEQ ID NOS: 362 and 336; (39) SEQ ID NOS: 363 and 336; (40) SEQ ID NOS: 364 and 336; (41) SEQ ID NOS: 365 and 336; (42) SEQ ID NOS: 366 and 336; (43) SEQ ID NOS: 367 and 336; (44) SEQ ID NOS: 368 and 336; (45) SEQ ID NOS: 369 and 336; (46) SEQ ID NOS: 370 and 336; (47) SEQ ID NOS: 371 and 336; (48) SEQ ID NOS: 372 and 336; (49) SEQ ID NOS: 373 and 336; (50) SEQ ID NOS: 374 and 336; (51) SEQ ID NOS: 375 and 336; (52) SEQ ID NOS: 376 and 336; (53) SEQ ID NOS: 377 and 336; (54) SEQ ID NOS: 378 and 336; (55) SEQ ID NOS: 379 and 336; (56) SEQ ID NOS: 380 and 336; (57) SEQ ID NOS: 382 and 381; (58) SEQ ID NOS: 383 and 381; (59) SEQ ID NOS: 384 and 381; (60) SEQ ID NOS: 385 and 381; (61) SEQ ID NOS: 386 and 381; and (62) SEQ ID NOS: 387 and 381.

One embodiment is directed to a primer set or collection of primer sets for amplifying DNA of a vancomycin-resistance gene (*vanG* gene), comprising a nucleotide sequence selected from the group consisting of: (1) SEQ ID NOS: 244 and 246; (2) SEQ ID NOS: 244 and 247; (3) SEQ ID NOS: 244 and 248; (4) SEQ ID NOS: 244 and 250; (5) SEQ ID NOS: 244 and 251; (6) SEQ ID NOS: 244 and 254; (7) SEQ ID NOS: 244 and 258; (8) SEQ ID NOS: 244 and 259; (9) SEQ ID NOS: 244 and 284; (10) SEQ ID NOS: 244 and 286; (11) SEQ ID NOS: 244 and 287; (12) SEQ ID NOS: 249 and 246; (13) SEQ ID NOS: 249 and 248; (14) SEQ ID NOS: 249 and 286; (15) SEQ ID NOS: 249 and 301; (16) SEQ ID NOS: 249 and 306; (17) SEQ ID NOS: 249 and 308; (18) SEQ ID NOS: 249 and 312; (19) SEQ ID NOS: 252 and 246; (20) SEQ ID NOS: 252 and 262; (21) SEQ ID NOS: 253 and 246; (22) SEQ ID NOS: 255 and 246; (23) SEQ ID NOS: 256 and 246; (24) SEQ ID NOS: 260 and 258; (25) SEQ ID NOS: 263 and 258; (26) SEQ ID NOS: 264 and 266; (27) SEQ ID NOS: 267 and 269; (28) SEQ ID NOS: 270 and 266; (29) SEQ ID NOS: 270 and 269; (30) SEQ ID NOS: 271 and 266; (31) SEQ ID NOS: 272 and 274; (32) SEQ ID NOS: 275 and 269; (33) SEQ ID NOS: 276 and 269; (34) SEQ ID NOS: 277 and 269; (35) SEQ ID NOS: 278 and 266; (36) SEQ ID NOS: 279 and 269; (37) SEQ ID NOS: 280 and 266; (38) SEQ ID NOS: 280 and 269; (39) SEQ ID NOS: 281 and 269; (40) SEQ ID NOS: 282 and 266; (41) SEQ ID NOS: 282 and 269; (42) SEQ ID NOS: 283 and 269; (43) SEQ ID NOS: 285 and 259; (44) SEQ ID NOS: 288 and 290; (45) SEQ ID NOS: 288 and 304; (46) SEQ ID NOS: 291 and 290; (47) SEQ ID NOS: 293 and 290; (48) SEQ ID NOS: 293 and 304; (49) SEQ ID NOS: 295 and 258; (50) SEQ ID NOS: 297 and 290; (51) SEQ ID NOS: 298 and 290; (52) SEQ ID NOS: 298 and 304; (53) SEQ ID NOS: 299 and 290; (54) SEQ ID NOS: 300 and 290; (55) SEQ ID NOS: 302 and 274; (56) SEQ ID NOS: 303 and 290; (57) SEQ ID NOS: 303 and 304; (58) SEQ ID NOS: 305 and 274; (59) SEQ ID NOS: 305 and 290; (60) SEQ ID NOS: 307 and 274; (61) SEQ ID NOS: 307 and 290; (62) SEQ ID NOS: 309 and 290; (63) SEQ ID NOS: 310 and 290; (64) SEQ ID NOS: 311 and 258; (65) SEQ ID NOS: 313 and 290; (66) SEQ ID NOS: 314 and 290; (67) SEQ ID NOS: 314 and 320; (68) SEQ ID NOS: 315 and 290; (69) SEQ ID NOS: 316 and 290; (70) SEQ ID NOS: 317 and 290; (71) SEQ ID NOS: 318 and 290; (72) SEQ ID NOS: 321 and 258; (73) SEQ ID NOS: 322 and 324; (74) SEQ ID NOS: 325 and 266; (75) SEQ ID NOS: 325 and 274; (76) SEQ ID NOS: 326 and 274; (77) SEQ ID NOS: 327 and 266; (78) SEQ ID NOS: 328 and 274; (79) SEQ ID NOS: 329 and 274; (80) SEQ ID NOS: 330 and 274; (81) SEQ ID NOS: 331 and 274; (82) SEQ ID NOS: 332 and 266; and (83) SEQ ID NOS: 333 and 266.

One embodiment is directed to a primer set or collection of primer sets for amplifying DNA of an Efm *sodA* gene, comprising a nucleotide sequence SEQ ID NOS: 610 and 622.

One embodiment is directed to a primer set or collection of primer sets for amplifying DNA of an Efs *sodA* gene, comprising a nucleotide sequence slected from the from the group consisting of: (1) SEQ ID NOS: 670 and 710; (2) SEQ ID NOS: 670 and 716; (3) SEQ ID NOS: 670 and 717; (4) SEQ ID NOS: 670 and 718; (5) SEQ ID NOS: 670 and 730; (6) SEQ ID NOS: 670 and 733; (7) SEQ ID NOS: 679 and 710; (8) SEQ ID NOS: 679 and 716; (9) SEQ ID NOS: 679 and 717; (10) SEQ ID NOS: 679 and 718; (11) SEQ ID NOS: 679 and 730; (12) SEQ ID NOS: 679 and 733 ; (13) SEQ ID NOS: 683 and 710; (14) SEQ ID NOS: 683 and 716; (15) SEQ ID NOS: 683 and 717; (16) SEQ ID NOS: 683 and 718; (17) SEQ ID NOS: 683 and 730; (18) SEQ ID NOS: 683 and 733; (19) SEQ ID NOS: 691 and 710; (20) SEQ ID NOS: 691 and 716; (21) SEQ ID NOS: 691 and 717; (22) SEQ ID NOS: 691 and 718; (23) SEQ ID NOS: 691 and 730; (24) SEQ ID NOS: 691 and 733; (25) SEQ ID NOS: 692 and 710; (26) SEQ ID NOS: 692 and 716; (27) SEQ ID NOS: 692 and 717; (28) SEQ ID NOS: 692 and 718; (29) SEQ ID NOS: 692 and 730; (30) SEQ ID NOS: 692 and 733; (31) SEQ ID NOS: 693 and 710; (32) SEQ ID NOS: 693 and 716; (33) SEQ ID NOS: 693 and 717; (34) SEQ ID NOS: 693 and 718; (35) SEQ ID NOS: 693 and 730; (36) SEQ ID NOS: 693 and 733.

One embodiment is directed to a primer set or collection of primer sets for amplifying DNA of an Efm novel gene, comprising a nucleotide sequence selected from the from the group consisting of: (1) SEQ ID NOS: 767 and 800; (2) SEQ ID NOS: 776 and 813; (3) SEQ ID NOS: 776 and 816; (4) SEQ ID NOS: 780 and 800; (5) SEQ ID NOS: 780 and 813; (6) SEQ ID NOS: 780 and 816; (7) SEQ ID NOS: 785 and 800; (8) SEQ ID NOS: 785 and 813; and (9) SEQ ID NOS: 785 and 816.

One embodiment is directed to a primer set or collection of primer sets for amplifying DNA of an Efs novel gene, comprising a nucleotide sequence selected from the from the group consisting of: (1) SEQ ID NOS: 851 and 878; (2) SEQ ID NOS: 851 and 884; (3) SEQ ID NOS: 851 and 890; (4) SEQ ID NOS: 851 and 892; (5) SEQ ID NOS: 851 and 896; (6) SEQ ID NOS: 865 and 878; (7) SEQ ID NOS: 865 and 884; (8) SEQ ID NOS: 865 and 890; (9) SEQ ID NOS: 865 and 892; (10) SEQ ID NOS: 865 and 896; (11) SEQ ID NOS: 866 and 878; (12) SEQ ID NOS: 866 and 884 ; (13) SEQ ID NOS: 866 and 890; (14) SEQ ID NOS: 866 and 892; (15) SEQ ID NOS: 866 and 896; (16) SEQ ID NOS: 868 and 878; (17) SEQ ID NOS: 868 and 884; (18) SEQ ID NOS: 868 and 890; (19) SEQ ID NOS: 868 and 892; and (20) SEQ ID NOS: 868 and 896.

One embodiment is directed to a primer set or collection of primer sets for amplifying DNA of Efm/Efs dual genes, comprising a nucleotide sequence consisting of: SEQ ID NOS: 936, 938, and 939.

A particular embodiment is directed to oligonucleotide probes for binding to DNA of a vancomycin-resistance gene(s), comprising a nucleotide sequence selected from the group consisting of: SEQ ID NOS: 2, 4, 7, 9, 11-18, 20, 24, 25, 27, 30, 35, 43, 46, 49, 50, 54, 56-58 (*vanA*); 62, 64, 67, 69, 73, 76, 78-80, 82, 84, 92, 96, 108-110, 112 (*vanB*); 124, 126, 128, 131, 134, 136, 139, 142, 145, 149, 152, 154, 159, 163, 166, 172, 176, 181, 182, 186, 189, 195, 197, 199, 205 (*vanC1*); 207, 211 (*vanC2*/*3*); 389, 392, 397, 400, 402-408, 410, 412-414, 418, 420, 422-424, 427, 429, 431-433, 436, 441, 446, 457, 463, 466, 469, 472, 475, 478, 481, 484, 485, 487, 489, 492, 499 (*vanD*); 335 (*vanE*); and 245, 257, 261, 265, 268, 273, 289, 292, 294, 296, 319, 323 (*vanG*).

A particular embodiment is directed to oligonucleotide probes for binding to DNA of an Efm *sodA* gene or Efs *sodA* gene or Efm novel gene or Efs novel gene or dual genes, comprising a nucleotide sequence selected from the group consisting of: SEQ ID NOS: 664, 648, 655 (Efm *sodA*); 737, 743, 747, 752, 754, 755, 756, 757, 758, 760, 766, 768, 769, 770 (Efs *sodA*); 821 and 843 (Efm novel); 908 and 925 (Efs novel); and 937 (Efm/Efs dual).

One embodiment is directed to the simultaneous detection in a multiplex format of vancomycin resistance, specifically the resistance genes *vanA, vanB, vanC, vanD, vanE* and *vanG.*

One embodiment is directed to the simultaneous detection and differentiation in a multiplex format of the *vanA* and *vanB* resistance genes.

One embodiment is directed to the simultaneous detection in a multiplex format of VRE when an isolate is tested.

One embodiment is directed to primer sets for amplifying DNA of a vancomycin-resistance gene(s) simultaneously, comprising:
(a): (1) SEQ ID NOS: 1 and 3; (2) SEQ ID NOS: 1 and 32; (3) SEQ ID NOS: 1 and 5; (4) SEQ ID NOS: 19 and 21; (5) SEQ ID NOS: 19 and 3; (6) SEQ ID NOS: 19 and 32; (7) SEQ ID NOS: 19 and 47; (8) SEQ ID NOS: 19 and 5; (9) SEQ ID NOS: 19 and 52; (10) SEQ ID NOS: 19 and 55; (11) SEQ ID NOS: 22 and 21; (12) SEQ ID NOS: 22 and 3; (13) SEQ ID NOS: 22 and 32; (14) SEQ ID NOS: 22 and 5; (15) SEQ ID NOS: 23 and 21; (16) SEQ ID NOS: 23 and 3; (17) SEQ ID NOS: 23 and 32; (18) SEQ ID NOS: 23 and 5; (19) SEQ ID NOS: 26 and 3; (20) SEQ ID NOS: 26 and 32; (21) SEQ ID NOS: 26 and 47; (22) SEQ ID NOS: 26 and 5; (23) SEQ ID NOS: 26 and 51; (24) SEQ ID NOS: 28 and 3; (25) SEQ ID NOS: 28 and 32; (26) SEQ ID NOS: 28 and 5; (27) SEQ ID NOS: 29 and 21; (28) SEQ ID NOS: 29 and 3; (29) SEQ ID NOS: 29 and 5; (30) SEQ ID NOS: 29 and 8; (31) SEQ ID NOS: 33 and 21; (32) SEQ ID NOS: 33 and 3; (33) SEQ ID NOS: 33 and 32; (34) SEQ ID NOS: 33 and 5; (35) SEQ ID NOS: 34 and 36; (36) SEQ ID NOS: 34 and 52; (37) SEQ ID NOS: 37 and 3; (38) SEQ ID NOS: 37 and 32; (39) SEQ ID NOS: 37 and 5; (40) SEQ ID NOS: 38 and 3; (41) SEQ ID NOS: 38 and 32; (42) SEQ ID NOS: 38 and 5; (43) SEQ ID NOS: 39 and 3; (44) SEQ ID NOS: 39 and 32; (45) SEQ ID NOS: 39 and 5; (46) SEQ ID NOS: 40 and 3; (47) SEQ ID NOS: 40 and 32; (48) SEQ ID NOS: 40 and 5; (49) SEQ ID NOS: 41 and 21; (50) SEQ ID NOS: 42 and 44; (51) SEQ ID NOS: 45 and 47; (52) SEQ ID NOS: 48 and 47; (53) SEQ ID NOS: 53 and 47; (54) SEQ ID NOS: 59 and 52; (55) SEQ ID NOS: 59 and 60; (56) SEQ ID NOS: 6 and 10; (57) SEQ ID NOS: 6 and 21; (58) SEQ ID NOS: 6 and 3; (59) SEQ ID NOS: 6 and 31; (60) SEQ ID NOS: 6 and 32; (61) SEQ ID NOS: 6 and 5; and (62) SEQ ID NOS: 6 and 8 (forward and reverse primers for amplifying DNA of *vanA,* respectively); and
(b) (1) SEQ ID NOS: 103 and 65; (2) SEQ ID NOS: 103 and 66; (3) SEQ ID NOS: 103 and 86; (4) SEQ ID NOS: 103 and 87; (5) SEQ ID NOS: 103 and 88; (6) SEQ ID NOS: 104 and 66; (7) SEQ ID NOS: 105 and 66; (8) SEQ ID NOS: 107 and 66; (9) SEQ ID NOS: 111 and 63; (10) SEQ ID NOS: 111 and 66; (11) SEQ ID NOS: 111 and 88; (12) SEQ ID NOS: 61 and 63; (13) SEQ ID NOS: 61 and 65; (14) SEQ ID NOS: 61 and 66; (15) SEQ ID NOS: 61 and 74; (16) SEQ ID NOS: 61 and 77; (17) SEQ ID NOS: 61 and 97; (18) SEQ ID NOS: 68 and 63; (19) SEQ ID NOS: 68 and 65; (20) SEQ ID NOS: 68 and 66; (21) SEQ ID NOS: 68 and 74; (22) SEQ ID NOS: 68 and 77; (23) SEQ ID NOS: 70 and 63; (24) SEQ ID NOS: 70 and 74; (25) SEQ ID NOS: 70 and 77; (26) SEQ ID NOS: 71 and 63; (27) SEQ ID NOS: 71 and 74; (28) SEQ ID NOS: 71 and 77; (29) SEQ ID NOS: 72 and 74; (30) SEQ ID NOS: 75 and 74; (31) SEQ ID NOS: 81 and 65; (32) SEQ ID NOS: 81 and 66; (33) SEQ ID NOS: 81 and 77; (34) SEQ ID NOS: 81 and 87; (35) SEQ ID NOS: 81 and 88; (36) SEQ ID NOS: 81 and 95; (37) SEQ ID NOS: 83 and 101; (38) SEQ ID NOS: 83 and 65; (39) SEQ ID NOS: 83 and 66; (40) SEQ ID NOS: 83 and 85; (41) SEQ ID NOS: 83 and 86; (42) SEQ ID NOS: 83 and 87; (43) SEQ ID NOS: 83 and 88; (44) SEQ ID NOS: 83 and 95; (45) SEQ ID NOS: 89 and 100; (46) SEQ ID NOS: 89 and 101; (47) SEQ ID NOS: 89 and 102; (48) SEQ ID NOS: 89 and 65; (49) SEQ ID NOS: 89 and 66; (50) SEQ ID NOS: 89 and 85; (51) SEQ ID NOS: 89 and 86; (52) SEQ ID NOS: 89 and 87; (53) SEQ ID NOS: 89 and 88; (54) SEQ ID NOS: 89 and 90; (55) SEQ ID NOS: 89 and 91; (56) SEQ ID NOS: 89 and 95; (57) SEQ ID NOS: 89 and 98; (58) SEQ ID NOS: 89 and 99; (59) SEQ ID NOS: 93 and 101; (60) SEQ ID NOS: 93 and 65; (61) SEQ ID NOS: 93 and 66; (62) SEQ ID NOS: 93 and 85; (63) SEQ ID NOS: 93 and 86; (64) SEQ ID NOS: 93 and 87; (65) SEQ ID NOS: 93 and 88; (66) SEQ ID NOS: 93 and 90; (67) SEQ ID NOS: 93 and 95; (68) SEQ ID NOS: 93 and 98; (69) SEQ ID NOS: 94 and 65; (70) SEQ ID NOS: 94 and 66; (71) SEQ ID NOS: 94 and 87; (72) SEQ ID NOS: 94 and 88; and (73) SEQ ID NOS: 94 and 95 (forward and reverse primers for amplifying DNA of *vanB,* respectively).

A particular embodiment is directed to oligonucleotide probes for binding to DNA of vancomycin-resistance gene(s), comprising a nucleotide sequence selected from the group consisting of SEQ ID NOS: 2, 4, 7, 9, 11-18, 20, 24, 25, 27, 30, 35, 43, 46, 49, 50, 54, 56-58 (*vanA* probes); and 62, 64, 67, 69, 73, 76, 78, 79, 80, 82, 84, 92, 96, 108-110, 112 (*vanB* probes).

One embodiment is directed to a kit for detecting DNA of a vancomycin-resistance gene(s) in a sample, comprising one or more probes comprising a sequence selected from the group consisting of: SEQ ID NOS: 2, 4, 7, 9, 11-18, 20, 24, 25, 27, 30, 35, 43, 46, 49, 50, 54, 56-58 (*vanA*); 62, 64, 67, 69, 73, 76, 78-80, 82, 84, 92, 96, 108-110, 112 (*vanB*); 124, 126, 128, 131, 134, 136, 139, 142, 145, 149, 152, 154, 159, 163, 166, 172, 176, 181, 182, 186, 189, 195, 197, 199, 205 (*vanC1*); 207, 211 (*vanC2*/*3*); 389, 392, 397, 400, 402-408, 410, 412-414, 418, 420, 422-424, 427, 429, 431-433, 436, 441, 446, 457, 463, 466, 469, 472, 475, 478, 481, 484, 485, 487, 489, 492, 499 (*vanD*); 335 (*vanE*); 245, 257, 261, 265, 268, 273, 289, 292, 294, 296, 319, 323 (*vanG*) In a particular embodiment, the kit further comprises a) at least one forward primer comprising the sequence selected from the group consisting of: SEQ ID NOS: 1, 6, 19, 22, 23, 26, 28, 29, 33, 34, 37-42, 45, 48, 53 and 59 (*vanA*); 61, 68, 70-72, 75, 81, 83, 89, 93, 94, 103-105, 107 and 111 (*vanB*); 123, 127, 130, 133, 138, 141, 144, 148, 151, 156, 158, 161, 162, 165, 168, 170, 171, 175, 178, 180, 183-185, 188, 191, 193, 194, 196, 198, and 200-204 (*vanC1*); 206, 210, 213, 217, 220, 221, 222, 224-226, 228-238, 240, 242 and 243 (*vanC2*/*3*); 388, 391, 394, 396, 399, 409, 415, 416, 425, 428, 435, 438, 440, 443, 445, 462, 465, 468, 471, 474, 477, 480, 483, 488, 491, 494-498 and 500-502 (*vanD*); 334, 337-380 and 382-387 (*vanE*); 244, 249, 252, 253, 255, 256, 260, 263, 264, 267, 270-272, 275-283, 285, 288, 291, 293, 295, 297-300, 302, 303, 305, 307, 309-311, 313-318, 321, 322, 325-333 *(vanG);* and b) at least one reverse primer comprising the sequence selected from the group consisting of: SEQ ID NOS: SEQ ID NOS: SEQ ID NOS: 3, 5, 8, 10, 21, 31, 32, 36, 44, 47, 51, 52, 55 and 60 (*vanA*); 63, 65, 66, 74, 77, 85-88, 90, 91, 95, and 97-102 *(vanB);* 125, 129, 132, 135, 137, 140, 143, 146, 147, 150, 153, 155, 157, 160, 164, 167, 169, 173, 174, 177, 179, 187, 190 and 192 (*vanC1*); 208, 209, 212, 215, 216, 219, 223, 227, 239 and 241 (*vanC2*/*3*); 390, 393, 395, 398, 401, 411, 417, 419, 421, 426, 430, 434, 437, 439, 442, 444, 447-455, 458-461, 464, 467, 470, 473, 476, 479, 482, 486, 490 and 493 *(vanD);* 336 and 381 *(vanE);* 246-248, 250, 251, 254, 258, 259, 262, 266, 269, 274, 284, 286, 287, 290, 301, 304, 306, 308, 312, 320 and 324 (*vanG*)*.*

One embodiment is directed to a kit for detecting DNA of a Efm *sodA* or Efs *sodA* gene or Efm novel gene or Efs novel gene or dual genes, in a sample, comprising one or more probes comprising a sequence selected from the group consisting of: SEQ ID NOS: 664, 648, 655 (Efm *sodA*); 737, 743, 747, 752, 754, 755, 756, 757, 758, 760, 766, 768, 769, 770 (Efs *sodA*); 821 and 843 (Efm novel); 908 and 925 (Efs novel); and 937 (Efm/Efs dual). In a particular embodiment, the kit further comprises a) at least one forward primer comprising the sequence selected from the group consisting of: SEQ ID NOS: 610 (Efm *sodA*); 670, 679, 683, 691-693 (Efs *sodA*); 776, 780, 785 (Efm novel); 851, 865, 866, 868 (Efs novel); and 936 (Efm/Efs dual); and b) at least one reverse primer comprising a sequence selected from the group consisting of SEQ ID NOS: 622 (Efm *sodA*); 710, 716-718, 730, 733 (Efs *sodA*); 800, 813, 816 (Efm novel); 878, 884, 890, 892, 896 (Efs novel); 938 and 939 (Efm/Efs dual).

One embodiment is directed to a kit for detecting DNA of a vancomycin-resistance gene(s) or *Enterococcal* marker gene in a sample, comprising one or more probes comprising a sequence selected from the group consisting of: SEQ ID NOS: 2, 4, 7, 9, 11-18, 20, 24, 25, 27, 30, 35, 43, 46, 49, 50, 54, 56-58 (*vanA*); 62, 64, 67, 69, 73, 76, 78-80, 82, 84, 92, 96, 108-110, 112 (*vanB*); 124, 126, 128, 131, 134, 136, 139, 142, 145, 149, 152, 154, 159, 163, 166, 172, 176, 181, 182, 186, 189, 195, 197, 199, 205 (*vanC1*); 207, 211 (*vanC2*/*3*); 389, 392, 397, 400, 402-408, 410, 412-414, 418, 420, 422-424, 427, 429, 431-433, 436, 441, 446, 457, 463, 466, 469, 472, 475, 478, 481, 484, 485, 487, 489, 492, 499 (*vanD*); 335 (*vanE*); 245, 257, 261, 265, 268, 273, 289, 292, 294, 296, 319, 323 (*vanG*)*,* 664, 648, 655 (Efm *sodA*); 737, 743, 747, 752, 754, 755, 756, 757, 758, 760, 766, 768, 769, 770 (Efs *sodA*); 821 and 843 (Efm novel); 908 and 925 (Efs novel); and 937 (Efm/Efs dual). In a particular embodiment, the kit further comprises a) at least one forward primer comprising the sequence selected from the group consisting of: SEQ ID NOS: 1, 6, 19, 22, 23, 26, 28, 29, 33, 34, 37-42, 45, 48, 53 and 59 (*vanA*); 61, 68, 70-72, 75, 81, 83, 89, 93, 94, 103-105, 107 and 111 (*vanB*); 123, 127, 130, 133, 138, 141, 144, 148, 151, 156, 158, 161, 162, 165, 168, 170, 171, 175, 178, 180, 183-185, 188, 191, 193, 194, 196, 198, and 200-204 (*vanC1*); 206, 210, 213, 217, 220, 221, 222, 224-226, 228-238, 240, 242 and 243 (*vanC2*/*3*); 388, 391, 394, 396, 399, 409, 415, 416, 425, 428, 435, 438, 440, 443, 445, 462, 465, 468, 471, 474, 477, 480, 483, 488, 491, 494-498 and 500-502 (*vanD*); 334, 337-380 and 382-387 (*vanE*); 244, 249, 252, 253, 255, 256, 260, 263, 264, 267, 270-272, 275-283, 285, 288, 291, 293, 295, 297-300, 302, 303, 305, 307, 309-311, 313-318, 321, 322, 325-333 (*vanG*)*,* 610 (Efm *sodA*); 670, 679, 683, 691-693 (Efs *sodA*); 776, 780, 785 (Efm novel); 851, 865, 866, 868 (Efs novel); and 936 (Efm/Efs dual); and b) at least one reverse primer comprising the sequence selected from the group consisting of: SEQ ID NOS: SEQ ID NOS: SEQ ID NOS: 3, 5, 8, 10, 21, 31, 32, 36, 44, 47, 51, 52, 55 and 60 (*vanA*); 63, 65, 66, 74, 77, 85-88, 90, 91, 95, and 97-102 (*vanB*); 125, 129, 132, 135, 137, 140, 143, 146, 147, 150, 153, 155, 157, 160, 164, 167, 169, 173, 174, 177, 179, 187, 190 and 192 (*vanC1*); 208, 209, 212, 215, 216, 219, 223, 227, 239 and 241 (*vanC2*/*3*); 390, 393, 395, 398, 401, 411, 417, 419, 421, 426, 430, 434, 437, 439, 442, 444, 447-455, 458-461, 464, 467, 470, 473, 476, 479, 482, 486, 490 and 493 (*vanD*); 336 and 381 (*vanE*); 246-248, 250, 251, 254, 258, 259, 262, 266, 269, 274, 284, 286, 287, 290, 301, 304, 306, 308, 312, 320 and 324 (*vanG*), 622 (Efm *sodA*); 710, 716-718, 730, 733 (Efs *sodA*); 800, 813, 816 (Efm novel); 878, 884, 890, 892, 896 (Efs novel); 938 and 939 (Efm/Efs dual)..

In a particular embodiment, the kit further comprises reagents for isolating and/or sequencing the vancomycin-resistance gene(s) in the sample. In a particular embodiment, the one or more probes are labeled with different detectable labels. In a particular embodiment, the one or more probes are labeled with the same detectable labels. In a particular embodiment, the at least one forward primer, the at least one reverse primer and the one or more probes are selected from the group consisting of: Groups 1-212 of Table 5, Groups 213-601 of Table 6, Groups 603-605 of Table 8B, Groups 606-627 of Table 9B, Groups 628-636 of Table 10B, Groups 637-643 of Table 11B, and Group 644 of Table 12.

One embodiment is directed to a method for diagnosing a condition, syndrome or disease in a human associated with a vancomycin-resistant organism, comprising: a) contacting a sample with at least one forward and reverse primer set selected from the group consisting of: Groups 1-502 of Tables 5 and 6; b) conducting an amplification reaction, thereby producing an amplicon; and c) detecting the amplicon using one or more probes selected from the group consisting of: SEQ ID NOS: 2, 4, 7, 9, 11-18, 20, 24, 25, 27, 30, 35, 43, 46, 49, 50, 54, 56-58 (*vanA*); 62, 64, 67, 69, 73, 76, 78-80, 82, 84, 92, 96, 108-110, 112 (*vanB*); 124, 126, 128, 131, 134, 136, 139, 142, 145, 149, 152, 154, 159, 163, 166, 172, 176, 181, 182, 186, 189, 195, 197, 199, 205 (*vanC1*); 207, 211 (*vanC2*/*3*); 389, 392, 397, 400, 402-408, 410, 412-414, 418, 420, 422-424, 427, 429, 431-433, 436, 441, 446, 457, 463, 466, 469, 472, 475, 478, 481, 484, 485, 487, 489, 492, 499 (*vanD*); 335 (*vanE*); 245, 257, 261, 265, 268, 273, 289, 292, 294, 296, 319, 323 (*vanG*); wherein the generation of an amplicon is indicative of the presence of an organism resistant to vancomycin in the sample. In a particular embodiment, the sample is blood, serum, plasma, enriched peripheral blood mononuclear cells, neoplastic or other tissue obtained from biopsies, cerebrospinal fluid, saliva, fluids collected from the ear, eye, mouth, and respiratory airways, sputum, skin, tears, oropharyngeal swabs, nasopharyngeal swabs, throat swabs, urine, anal-rectal swabs, feces, skin swabs, nasal aspirates, nasal wash, fluids and cells obtained by the perfusion of tissues of both human and animal origin, and fluids and cells derived from the culturing of human cells, including human stem cells and human cartilage, fibroblasts or samples derived from inanimate objects. A sample may be collected from more than one collection site, *e.g.,* blood and an anal-rectal swab. In a particular embodiment, the complications, conditions, syndromes or diseases in humans associated with a vancomycin-resistant organism are selected from the group consisting of: infections at indwelling sites and wounds, urinary tract infections, sepsis, infections from indwelling urinary or central venous catheters, and infections from abdominal or cardiothoracic surgery.

One embodiment is directed to a method for diagnosing a condition, syndrome or disease in a human associated with an *Enterococcal* organism, comprising: a) contacting a sample with at least one forward and reverse primer set selected from the group consisting of: Groups 603-644 of Tables 8B, 9B, 10B, 11B, and 12; b) conducting an amplification reaction, thereby producing an amplicon; and c) detecting the amplicon using one or more probes selected from the group consisting of: SEQ ID NOS: 664, 648, 655 (Efm *sodA*); 737, 743, 747, 752, 754, 755, 756, 757, 758, 760, 766, 768, 769, 770 (Efs *sodA*); 821 and 843 (Efm novel); 908 and 925 (Efs novel); and 937 (Efm/Efs dual); wherein the generation of an amplicon is indicative of the presence of an *Enterococcal* organism in the sample. In a particular embodiment, the sample is blood, serum, plasma, enriched peripheral blood mononuclear cells, neoplastic or other tissue obtained from biopsies, cerebrospinal fluid, saliva, fluids collected from the ear, eye, mouth, and respiratory airways, sputum, skin, tears, oropharyngeal swabs, nasopharyngeal swabs, throat swabs, urine, anal-rectal swabs, feces, skin swabs, nasal aspirates, nasal wash, fluids and cells obtained by the perfusion of tissues of both human and animal origin, and fluids and cells derived from the culturing of human cells, including human stem cells and human cartilage, fibroblasts or samples derived from inanimate objects. A sample may be collected from more than one collection site, *e.g.,* blood and an anal-rectal swab. In a particular embodiment, the complications, conditions, syndromes or diseases in humans associated with a vancomycin-resistant organism are selected from the group consisting of: infections at indwelling sites and wounds, urinary tract infections, sepsis, infections from indwelling urinary or central venous catheters, and infections from abdominal or cardiothoracic surgery.

One embodiment is directed to a kit for amplifying and sequencing DNA of a vancomycin-resistance gene(s) in a sample, comprising: a) at least one forward primer comprising the sequence selected from the group consisting of: SEQ ID NOS: 1, 6, 19, 22, 23, 26, 28, 29, 33, 34, 37-42, 45, 48, 53 and 59 (*vanA*); 61, 68, 70-72, 75, 81, 83, 89, 93, 94, 103-105, 107 and 111 (*vanB*); 123, 127, 130, 133, 138, 141, 144, 148, 151, 156, 158, 161, 162, 165, 168, 170, 171, 175, 178, 180, 183-185, 188, 191, 193, 194, 196, 198, and 200-204 (*vanC1*); 206, 210, 213, 217, 220, 221, 222, 224-226, 228-238, 240, 242 and 243 (*vanC2*/*3*); 388, 391, 394, 396, 399, 409, 415, 416, 425, 428, 435, 438, 440, 443, 445, 462, 465, 468, 471, 474, 477, 480, 483, 488, 491, 494-498 and 500-502 (*vanD*); 334, 337-380 and 382-387 (*vanE*); 244, 249, 252, 253, 255, 256, 260, 263, 264, 267, 270-272, 275-283, 285, 288, 291, 293, 295, 297-300, 302, 303, 305, 307, 309-311, 313-318, 321, 322, 325-333 (*vanG*); and b) at least one reverse primer comprising the sequence selected from the group consisting of: SEQ ID NOS: 3, 5, 8, 10, 21, 31, 32, 36, 44, 47, 51, 52, 55 and 60 (*vanA*); 63, 65, 66, 74, 77, 85-88, 90, 91, 95, and 97-102 (*vanB*); 125, 129, 132, 135, 137, 140, 143, 146, 147, 150, 153, 155, 157, 160, 164, 167, 169, 173, 174, 177, 179, 187, 190 and 192 (*vanC1*); 208, 209, 212, 215, 216, 219, 223, 227, 239 and 241 (*vanC2*/*3*); 390, 393, 395, 398, 401, 411, 417, 419, 421, 426, 430, 434, 437, 439, 442, 444, 447-455, 458-461, 464, 467, 470, 473, 476, 479, 482, 486, 490 and 493 (*vanD*); 336 and 381 (*vanE*); 246-248, 250, 251, 254, 258, 259, 262, 266, 269, 274, 284, 286, 287, 290, 301, 304, 306, 308, 312, 320 and 324 (*vanG*); and c) reagents for the sequencing of amplified DNA fragments.

One embodiment is directed to a kit for amplifying and sequencing DNA of an Enterococci specific gene in a sample, comprising: a) at least one forward primer comprising the sequence selected from the group consisting of: SEQ ID NOS: 610 (Efm *sodA*); 670, 679, 683, 691-693 (Efs *sodA*); 776, 780, 785 (Efm novel); 851, 865, 866, 868 (Efs novel); and 936 (Efm/Efs dual); and b) at least one reverse primer comprising a sequence selected from the group consisting of SEQ ID NOS: 622 (Efm *sodA*); 710, 716-718, 730, 733 (Efs *sodA*); 800, 813, 816 (Efm novel); 878, 884, 890, 892, 896 (Efs novel); 938 and 939 (Efm/Efs dual); and c) reagents for the sequencing of amplified DNA fragments.

In a particular embodiment, the sample is blood, serum, plasma, enriched peripheral blood mononuclear cells, neoplastic or other tissue obtained from biopsies, cerebrospinal fluid, saliva, fluids collected from the ear, eye, mouth, and respiratory airways, sputum, skin, tears, oropharyngeal swabs, nasopharyngeal swabs, throat swabs, urine, anal-rectal swabs, feces, skin swabs, nasal aspirates, nasal wash, fluids and cells obtained by the perfusion of tissues of both human and animal origin, and fluids and cells derived from the culturing of human cells, including human stem cells and human cartilage, fibroblasts or samples derived from inanimate objects. In a particular embodiment, the complications, conditions, syndromes or diseases in humans associated with a vancomycin-resistant organism are selected from the group consisting of: skin infections, such as boils, impetigo, cellulitis, and scalded skin syndrome; food poisoning, leading to abdominal cramps, nausea, vomiting, and diarrhea; bacteremia, resulting in a persistent fever and other signs of blood poisoning; toxic shock syndrome, resulting in high fever, nausea, vomiting, rash on palms and soles, confusion, muscle aches, seizures, headache; and septic arthritis, resulting in joint swelling, severe pain in the affected joint, fever, and shaking chills.

One embodiment is directed to an internal control plasmid and vancomycin-resistance positive control plasmids. The non-competitive internal control plasmid is a synthetic target that does not occur naturally in clinical sample types for which this assay is intended. The synthetic target sequence incorporates an artificial, random polynucleotide sequence with a known GC content. The synthetic target sequence is:
5'GCGAAGTGAGAATACGCCGTGTCGCAGTTTCCTTGAGCAGTGTCTCTAAATGCC TCAAACCGTCGCATTTTTGGTTATAGCAGTAACTATATGGAGGTCCGTAGGCGGC GTGCGTGGGGGCACCAAACTCATCCAACGGTCGACTGCGCCTGTAGGGTCTTAA GAAGCGGCACCTCAGACCGATAGCATAGCACTTAAAGAGGAATTGAATAATCAA GATGGGTATCCGACCGACGCGGAGTGACCGAGGAAGAGGACCCTGCATGTATCC TGAGAGTATAGTTGTCAGAGCAGCAATTGATTCACCACCAAGGGACTTAGTCT 3' (SEQ ID NO: 503). This internal control is detected by a forward primer (SEQ ID NO: 504), a reverse primer (SEQ ID NO: 505) and a probe (SEQ ID NO: 506). A plasmid vector containing the internal control target sequence (SEQ ID NO: 503) is included in the assay. The internal control plasmid is added directly to the reaction mix to monitor the integrity of the PCR reagents and the presence of PCR inhibitors.

The vancomycin-resistance positive control plasmid contain partial sequences for one or more of the vancomycin resistance targets (*i. e. vanA, vanB, vanC, etc.*), respectively. The positive control plasmids comprise forward primer, probe and reverse primer sequences for the given vancomycin resistance. An artificial polynucleotide sequence is inserted within the positive control sequence corresponding to the given target to allow the amplicon generated by the target primer pairs to be differentiated from the amplicon derived by the same primer pairs from a natural target by size, by a unique restriction digest profile, and by a probe directed against the artificial sequence. The positive control plasmids are intended to be used as a control to confirm that the assay is performing within specifications.

The oligonucleotides of the present invention and their resulting amplicons do not cross react and, thus, will work together without negatively impacting each other. The primers and probes of the present invention do not cross react with other potentially contaminating species that would be present in a sample matrix.

One embodiment is directed to a method of hybridizing one or more isolated nucleic acid sequences comprising a sequence selected from the group consisting of: SEQ ID NOS: 606-939 to a *Enterococcus faecium* specific gene and/or *Enterococcus faecalis* specific gene, comprising contacting one or more isolated nucleic acid sequences to a sample comprising the *Enterococcus faecium* specific gene and/or *Enterococcus faecalis* specific gene under conditions suitable for hybridization.

One embodiment is directed to a method of hybridizing one or more isolated nucleic acid sequences comprising a sequence selected from the group consisting of: SEQ ID NOS: 1-502 and 600-939 to a vancomycin-resistance gene and/or an *Enterococcus faecium* specific gene and/or an *Enterococcus faecalis* specific gene, comprising contacting one or more isolated nucleic acid sequences to a sample comprising the vancomycin-resistance gene and/or the *Enterococcus faecium* specific gene and/or *Enterococcus faecalis* specific gene under conditions suitable for hybridization.

One embodiment is directed to a kit for detecting an *Enterococcus faecium* specific gene and/or an *Enterococcus faecalis* specific gene in a sample, comprising one or more probes comprising a sequence selected from the group consisting of: SEQ ID NOS: 606-939.

One embodiment is directed to a kit for detecting a vancomycin-resistance gene and/or an *Enterococcus faecium* specific gene and/or *Enterococcus faecalis* specific gene in a sample, comprising one or more probes comprising a sequence selected from the group consisting of: SEQ ID NOS: 1-502 and 600-939.

### BRIEF DESCRIPTION OF THE DRAWINGS

FIG. 1 is a plot diagram of the amplification of the *vanA* synthetic construct.
FIG. 2 is a plot diagram of the amplification of the *vanB* synthetic construct.
FIG. 3 is an electropherogram and legend showing the migration of *vanA* and *vanB* PCR products during gel electrophoresis.
FIG. 4 is a plot diagram of the amplification of the *E. faecium sodA* oligonucleotide solution, *E. faecalis sodA* oligonucleotide solution, and both *E. faecium* and *E. faecalis* dual oligonucleotide solution.
FIG. 5 is an electropherogram and legend showing the migration of the *E. faecium sodA, E. faecalis sodA,* and both *E. faecium* and *E. faecalis* dual PCR products during gel electrophoresis.

### DETAILED DESCRIPTION

A diagnostic or screening test that can detect multiple resistance genes simultaneously (the *van* genes), as well as determine whether a sample contains VRE, is necessary, as vancomycin resistant organisms are the major causative agents, for example, of HAIs.

Described herein are optimized probes and primers that, alone or in various combinations, allow for the amplification, detection, isolation, and sequencing of vancomycin genes that can be found in clinical isolates, including *Enterococcal* and *Staphylococcal* pathogens. Specific probes and primers, *i.e*., probes and primers that detect all known and characterized vancomycin have been discovered and are described herein. Nucleic acid primers and probes for detecting bacterial genetic material, especially the resistance genes *vanA* and *vanB,* and methods for designing and optimizing the respective primer and probe sequences, are described. Also provided are nucleic acid primers and probes for detecting the resistance genes *vanC, vanD, vanE* and *vanG.* Also provided are nucleic acid primers and probes for detecting the genus *Enterococci.*

The primers and probes described herein can be used, for example, to screen patients for the presence of the *vanA, vanB, vanC, vanD, vanE* and *vanG* resistance genes, Efs *sodA,* Efm *sodA,* Efs novel, Efm novel, Efm/Efs dual, *e.g.,* in clinical isolates, including *Enterococcal* and *Staphylococcal* pathogens, in a multiplex format.

The primers and probes disclosed herein can be used for the detection of the vancomycin-resistance genes in a multiplex format to allow detection of vancomycin resistant organisms (including VRE and vancomycin resistant *Staphylococcus aureus* (VRSA). Currently, the vancomycin-resistance genes are tested separately; however, the multiplex format option of the present description allows relative comparisons to be made between these prevalent resistance genes.

### Vancomycin Resistance

The importance of *Enterococcus* in vancomycin-resistant nosocomial infections or hospital acquired infections (HAIs) (vancomycin-resistant *Enterococcus*; VRE), has been the impetus for the development of therapeutic alternatives to vancomycin. One such alternative that is currently in use in the United States and European Union is linezolid. Linezolid is of the oxazolidinone class and is frequently used on multi-drug resistant bacteria, such as methicillin-resistant *Staphylococcus aureus* (MRSA) and VRE. Vancomycin resistance is classified according to six phenotypes: VanA, VanB, VanC (C1, C2, C3), VanD, VanE and VanG. VanA and VanB are inducible and transferable, while VanC, VanD, VanE and VanG are constitutive and non-transferable. In general, the VanA and VanB phenotypes are the most clinically important and found most often in *E. faecium* and *E. faecalis.* The VanC phenotype may also be clinically important as it is frequently associated with resistance infections caused by other *Enterococcus* species (such as *E. gallinarum, E. casseliflavus,* and *E. flavescens*)*.* It is less clear if the VanD, VanE and VanG phenotypes are clinically important. Vancomycin-resistant *E. faecium* and *E. faecalis* corresponding to the VanD, VanE, or VanG phenotypes have been isolated, but the prevalence of these relative to the VanA and VanB phenotypes in these species is not known and may be much lower in clinical settings. (Cetinkaya et al., Clin. Microbiol. Rev. 13:686-707 (2000); McKessar et al., Antimicrob. Agents Chemother. 44:3224-3228 (2000); Perichon et al., Antimicrob. Agents Chemother. 41:2016-2018 (1997); Boyd et al., Antimicrob. Agents Chemother. 46:1977-1979 (2002); Domingo et al., Antimicrob. Agents Chemother. 49:4784-4786 (2005)).

The presence of non-enterococcal vancomycin resistance may not alter present treatment or control measures. However, since *vanB* genes may be transferred from intestinal flora to enterococcal species, knowledge of potential reservoirs of glycopeptide resistance genes is critical for maintaining VRE infection control over VRE and other vancomycin-resistant species. (Ballard et al., Antimicrob. Agents Chemother. 49:77-81 (2005); Ballard et al., Antimicrob. Agents Chemother. 49:1688-1694 (2005); Domingo et al., J. Antimicrob. Chemother. 55:466-474 (2005)).

The mechanism of vancomycin resistance involves substituting the D-alanine terminating residue of cell wall precursors to which vancomycin binds, with a D-lactate residue- VanA, VanB, or VanD phenotypes, or D-serine residue - VanC and VanE phenotypes, and presumably the VanG phenotype. The modified cell wall precursors have a lower affinity for vancomycin binding, neutralizing its effect. The VanA phenotype is highly resistant to vancomycin and another glycopeptide-class antibiotic, teicoplanin. The VanB phenotype is associated with moderate to high levels of vancomycin resistance, but sensitivity to teicoplanin. The VanC, VanE and VanG phenotypes are associated with lower levels of resistance to both vancomycin and teicoplanin, while the VanD phenotype is associated with moderate levels of resistance to both vancomycin and teicoplanin (de Lalla et al., Antimicrob Agents Chemother. 36:2192-2196 (1992); McKessar et al., Antimicrob. Agents Chemother. 44: 3224-3228 (2000); Perichon et al., Antimicrob. Agents Chemother. 41:2016-2018 (1997); Leclercq et al., Clin. Infect. Dis. 24:545-556 (1997); Yean et al., BMC Microbiology 7:112 (2007); Arthur et al., J. Bacteriol. 175:117-127 (1993)). Table 1 lists the minimal inhibitory concentration (MIC) for vancomycin and teicoplanin for each phenotype. (Cetinkaya et al., Clin. Microbiol. Rev. 13:686-707 (2000); McKessar et al., Antimicrob. Agents Chemother. 44: 3224-3228 (2000)).

**Table 1. Minimal inhibitory concentration (MIC) for vancomycin and teicoplanin for the resistance genes vanA, vanB, vanC, vanD, vanE and vanG**

| **Phenotype** | **Allele** | **MIC (µg/mL) vancomycin** | **MIC (µg/mL) teicoplanin** |
|---|---|---|---|
| VanA | *vanA* | 64 - >1000 (high) | 16 - 512 (high) |
| VanB | *vanB* | 4 - 1024 (can be high) | ≤0.5 (sensitive) |
| VanC | *vanC* | 2 - 32 (low) | ≤0.5 (sensitive) |
| VanD | *vanD* | 128 (moderate) | 4 (moderate) |
| VanE | *vanE* | 16 (low) | 0.5 (sensitive) |
| VanG | *vanG* | 12- 16 (low) | 0.5 (sensitive) |

VRE infections can be treated with non-glycopeptide antibiotics such as cephalosporins and aminoglycosides; regardless of the phenotype, susceptibility testing is performed on isolates to determine the best course of treatment.

VRE is a threat to immunocompromised individuals, individuals recovering from surgical procedures and those generally in poor health. An individual can be colonized with VRE, which may or may not become a full-blown infection. Although colonized individuals can remain asymptomatic for months, such persons are capable of transmitting VRE to others. VRE is rarely a concern for healthy adults, and is usually cleared from the host without intervention.

Diagnosis of VRE can be determined using bacteriological and molecular-based diagnostic tests to identify the type of vancomycin resistance (*i.e.* VanA, VanB, VanC phenotypes, etc.) and the infecting/colonizing *Enterococcus* species. Once VRE is identified, the isolate is subjected to further tests to predict its susceptibility to antibiotics (for treatment of infections) and, in some cases, is speciated to enable the infection to be tracked (for infection control).

Risk factors for VRE infection or colonization include indwelling urinary or central venous catheters; recent abdominal or cardiothoracic surgery; prolonged and/or frequent hospital stays; hospital stay on an ICU, oncology, or transplant ward; stay in a long-term care facility (LTCF); and prior treatment with vancomycin, cephalosporins, metronidazole or clindamycin, or multiple antibiotics.

A reduction or eradication of VRE can occur upon implementation of control measures. VRE incidence can be decreased in hospitals in which patient surveillance cultures are used in concert with barrier isolation of colonized patients. Active infection-control intervention, relying heavily on surveillance cultures to guide the isolation of colonized patients, is important to reducing and even eradicating VRE (Ostrowsky et al., N Engl J Med. 344:1427-1433 (2001)).

Active Surveillance Cultures (ACS), combined with contact precautions, has also been described as effective in VRE reduction and sustaining long-term control. Conversely, long-term VRE increases are observed in institutions not utilizing this approach. (Management of Multidrug-Resistant Organisms in Healthcare Settings, HIPAC/CDC (2006)).

Additional control methods include administrative controls, such as tracking and trending VRE infections/colonizations and establishing a system whereby VRE positive results trigger specific responses (*i.e.* administrative controls). Control methods will require screening. Testing has been shown to correlate with reduction in VRE occurrence or re-occurrence.

Culture-based methods are widely used to screen patients for the presence of VRE. Bacterial colonies growing on Bile Esculin Azide plates supplemented with vancomycin (BEAV) are preliminarily identified as VRE based on colonial morphology, but additional culture steps would be required for definitive confirmation. The Bile Esculin test differentiates enterococci and group D streptococci from non-group D viridans group streptococci. Bile Esculin positive colonies appear black and are preliminarily identified as enterococci before Gram-staining. Gram-positive cocci (*Enterococcus* is Gram-positive) are then plated on a blood agar plate for isolation. The Gram status of the isolates is confirmed and they are subsequently checked for catalase and pyrrolinodyl peptidase activity. Catalase-negative and pyrrolinodyl peptidase positive isolates can be reported as *Enterococcus* spp. Positive isolates subjected to susceptibility testing can be classified as VRE if the minimal inhibitory concentration (MIC) of vancomycin is 32 µg/mL (Moellering, R., Clin Infect Dis. 14:1173-6 (1992)).

The use of automated systems allows one to speciate the *Enterococcus* isolate. Microscopic observation of motility can also be used to speciate *Enterococcus.* This entire process can take several days, with the first result suggesting *Enterococcus* obtained in 24-48 hours. These putative vancomycin-resistant isolates can be confirmed more quickly, perhaps within hours, by polymerase chain reaction (PCR) detection of any of the vancomycin-resistance markers. In addition, patient's clinical specimens can be screened rapidly using a PCR test designed to detect vancomycin-resistance genes. A positive result would suggest the need for barrier isolation, while a negative result may establish that barrier precautions are unnecessary.

In addition to VRE infections/colonizations, another form of vancomycin resistant bacteria has been observed. Vancomycin resistant *Staphylococcus aureus* (VRSA) are antimicrobial-resistant *Staphylococci.* Patients that develop VRSA infections usually have several underlying health conditions (such as diabetes), previous infections with MRSA, and recent hospitalizations. The spread of VRSA occurs through close physical contact with infected patients or contaminated material.

### Assays

Tables 2 and 3 demonstrate possible diagnostic outcome scenarios using the probes and primers described herein in diagnostic methods.

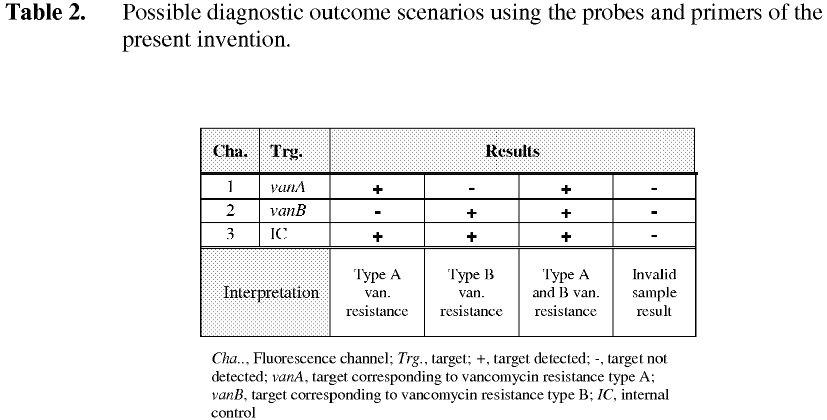

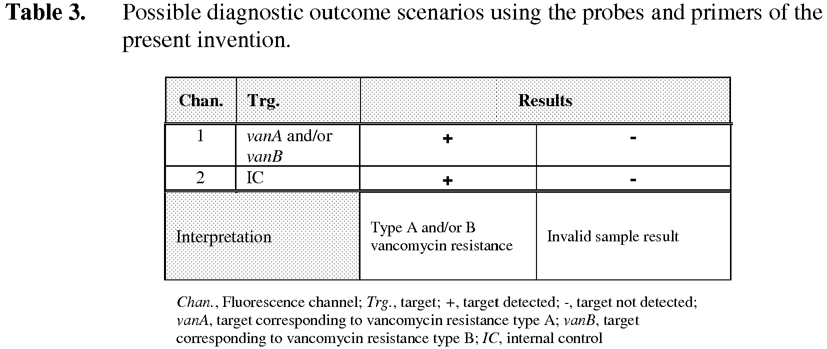

Detection of the internal control (IC) indicates that the sample result is valid, where an absence of a signal corresponding to the IC indicates either an invalid result or that one or more of the specific targets is at a high starting concentration. A signal indicating a high starting concentration of specific target in the absence of an internal control signal is considered to be a valid sample result.

The advantages of a multiplex format are: (1) simplified and improved testing and analysis; (2) increased efficiency and cost-effectiveness; (3) decreased turnaround time (increased speed of reporting results); (4) increased productivity (less equipment time needed); and (5) coordination/standardization of results for patients for multiple organisms (reduces error from inter-assay variation).

Screening and diagnosis of the vancomycin resistance genes and VRE can lead to earlier and more effective treatment of a subject. The methods for diagnosing and detecting vancomycin resistance and VRE described herein can be coupled with effective treatment therapies (*e.g.*, antibiotics). The antibiotic classes comprising non-glycopeptides such as cephalosporins and aminoglycosides are often prescribed for treatment of a vancomycin resistant infection. The treatments for such infections will depend upon the clinical disease state of the patient, as determinable by one of skill in the art.

The present description therefore provides a method for specifically detecting the presence of antibiotic resistance genes in a given sample using the primers and probes provided herein. Of particular interest in this regard is the ability of the disclosed primers and probes, as well as those that can be designed according to the disclosed methods, to specifically detect all or a majority of presently characterized strains of known, characterized vancomycin-resistance genes. The optimized primers and probes are useful, therefore, for identifying and diagnosing the causative or contributing agents of disease caused by VRE, whereupon an appropriate treatment can then be administered to the individual to eradicate the bacteria.

The present description provides one or more sets of primers that can anneal to all currently identified vancomycin-resistance genes and the genus *Enterococci* and thereby amplify a target from a biological sample. The present description provides, for example, at least a first primer and at least a second primer for the vancomycin resistance genes *vanA, vanB, vanC, vanD, van E* and *vanG,* and the genus *Enterococci,* each of which comprises a nucleotide sequence designed according to the inventive principles disclosed herein, which are used together to amplify DNA from vancomycin-resistance genes and *Enterococci* in a mixed-flora sample in a multiplex assay.

Also provided herein are probes that hybridize to the vancomycin-resistance gene sequences and *Enterococci* sequences and/or amplified products derived from the vancomycin-resistance gene sequences and *Enterococci* sequences. A probe can be labeled, for example, such that when it binds to an amplified or unamplified target sequence, or after it has been cleaved after binding, a fluorescent signal is emitted that is detectable under various spectroscopy and light measuring apparatuses. The use of a labeled probe, therefore, can enhance the sensitivity of detection of a target in an amplification reaction of DNA of vancomycin-resistance genes because it permits the detection of bacterial-derived DNA at low template concentrations that might not be conducive to visual detection as a gel-stained amplification product.

Primers and probes are sequences that anneal to a bacterial genomic or bacterial genomic derived sequence, *e.g.,* the antibiotic resistance genes of *Enterococcus* and/or *Staphylococcus* sequences, *e.g.,* VRE and/or VRSA sequences (the "target" sequences). The target sequence can be, for example, an antibiotic resistance gene or a bacterial genome. In one embodiment, the entire gene sequence can be "scanned" for optimized primers and probes useful for detecting the antibiotic resistance genes. In other embodiments, particular regions of the gene can be scanned, *e.g*., regions that are documented in the literature as being useful for detecting multiple genes, regions that are conserved, or regions where sufficient information is available in, for example, a public database, with respect to the antibiotic resistance genes.

Sets or groups of primers and probes are generated based on the target to be detected. The set of all possible primers and probes can include, for example, sequences that include the variability at every site based on the known antibiotic resistance gene, or the primers and probes can be generated based on a consensus sequence of the target. The primers and probes are generated such that the primers and probes are able to anneal to a particular sequence under high stringency conditions. For example, one of skill in the art recognizes that for any particular sequence, it is possible to provide more than one oligonucleotide sequence that will anneal to the particular target sequence, even under high stringency conditions. The set of primers and probes to be sampled includes, for example, all such oligonucleotides for all known and characterized vancomycin resistance genes and for the genus *Enterococci.* Alternatively, the primers and probes include all such oligonucleotides for a given consensus sequence for a target.

Typically, stringent hybridization and washing conditions are used for nucleic acid molecules over about 500 bp. Stringent hybridization conditions include a solution comprising about 1 M Na⁺ at 25°C to 30°C below the Tm; *e.g.,* 5 × SSPE, 0.5% SDS, at 65°C; *see,* Ausubel, et al., Current Protocols in Molecular Biology, Greene Publishing, 1995; Sambrook et al., Molecular Cloning: A Laboratory Manual, Cold Spring Harbor Press, 1989). Tm is dependent on both the G+C content and the concentration of salt ions, *e.g.,* Na⁺ and K^{+.} A formula to calculate the Tm of nucleic acid molecules greater than about 500 bp is Tm = 81.5 + 0.41(%(G+C)) - log₁₀[Na⁺]. Washing conditions are generally performed at least at equivalent stringency conditions as the hybridization. If the background levels are high, washing can be performed at higher stringency, such as around 15°C below the Tm.

The set of primers and probes, once determined as described above, are optimized for hybridizing to a plurality of antibiotic resistance genes by employing scoring and/or ranking steps that provide a positive or negative preference or "weight" to certain nucleotides in a target nucleic acid strain sequence. If a consensus sequence is used to generate the full set of primers and probes, for example, then a particular primer sequence is scored for its ability to anneal to the corresponding sequence of every known native target sequence. Even if a probe were originally generated based on a consensus, the validation of the probe is in its ability to specifically anneal and detect every, or a large majority of, target sequences. The particular scoring or ranking steps performed depend upon the intended use for the primer and/or probe, the particular target nucleic acid sequence, and the number of resistance genes of that target nucleic acid sequence. The methods of the description provide optimal primer and probe sequences because they hybridize to all or a subset of vancomycin resistance genes and the genus *Enterococci.* Once optimized oligonucleotides are identified that can anneal to such genes, the sequences can then further be optimized for use, for example, in conjunction with another optimized sequence as a "primer set" or for use as a probe. A "primer set" is defined as at least one forward primer and one reverse primer.

Described herein are methods for using the primers and probes for producing a nucleic acid product, for example, comprising contacting one or more nucleic acid sequences of SEQ ID NOS: 1-502 and 600-939 to a sample comprising the vancomycin-resistance genes and the *Enterococci* marker sequences under conditions suitable for nucleic acid polymerization. The primers and probes can additionally be used to sequence the DNA of the vancomycin-resistance genes and the *Enterococci* marker sequences, or used as diagnostics to, for example, detect vancomycin resistance genes in a clinical isolate sample, *e.g*., obtained from a subject, *e.g.,* a mammalian subject. Particular combinations for amplifying DNA of vancomycin-resistance genes include, for example, using at least one forward primer selected from the group consisting of: SEQ ID NOS: 1, 6, 19, 22, 23, 26, 28, 29, 33, 34, 37-42, 45, 48, 53 and 59 (*vanA*); 61, 68, 70-72, 75, 81, 83, 89, 93, 94, 103-105, 107 and 111 (*vanB*); 123, 127, 130, 133, 138, 141, 144, 148, 151, 156, 158, 161, 162, 165, 168, 170, 171, 175, 178, 180, 183-185, 188, 191, 193, 194, 196, 198, and 200-204 (*vanC1*); 206, 210, 213, 217, 220, 221, 222, 224-226, 228-238, 240, 242 and 243 (*vanC2*/*3*); 388, 391, 394, 396, 399, 409, 415, 416, 425, 428, 435, 438, 440, 443, 445, 462, 465, 468, 471, 474, 477, 480, 483, 488, 491, 494-498 and 500-502 (*vanD*); 334, 337-380 and 382-387 (*vanE*); 244, 249, 252, 253, 255, 256, 260, 263, 264, 267, 270-272, 275-283, 285, 288, 291, 293, 295, 297-300, 302, 303, 305, 307, 309-311, 313-318, 321, 322, 325-333 (*vanG*), and using at least one reverse primer selected from the group consisting of: SEQ ID NOS: 3, 5, 8, 10, 21, 31, 32, 36, 44, 47, 51, 52, 55 and 60 (*vanA*); 63, 65, 66, 74, 77, 85-88, 90, 91, 95, and 97-102 (*vanB*); 125, 129, 132, 135, 137, 140, 143, 146, 147, 150, 153, 155, 157, 160, 164, 167, 169, 173, 174, 177, 179, 187, 190 and 192 (*vanC1*); 208, 209, 212, 215, 216, 219, 223, 227, 239 and 241 (*vanC2*/*3*); 390, 393, 395, 398, 401, 411, 417, 419, 421, 426, 430, 434, 437, 439, 442, 444, 447-455, 458-461, 464, 467, 470, 473, 476, 479, 482, 486, 490 and 493 (*vanD*); 336 and 381 (*vanE*); and 246-248, 250, 251, 254, 258, 259, 262, 266, 269, 274, 284, 286, 287, 290, 301, 304, 306, 308, 312, 320 and 324 *(vanG).* Particular combinations for amplifying DNA of *Enterococci* marker sequences include, for example, using at least one forward primer selected from the group consisting of: SEQ ID NOS: 610 (Efm *sodA*); 670, 679, 683, 691-693 (Efs *sodA*); 776, 780, 785 (Efm novel); 851, 865, 866, 868 (Efs novel); and 936 (Efm/Efs dual); and using at least one reverse primer selected from the group consisting of: SEQ ID NOS: 622 (Efm *sodA*); 710, 716-718, 730, 733 (Efs *sodA*); 800, 813, 816 (Efm novel); 878, 884, 890, 892, 896 (Efs novel); 938 and 939 (Efm/Efs dual).

Methods are described for detecting vancomycin resistance genes in a sample, for example, comprising (1) contacting at least one forward and reverse primer set, *e.g.,* SEQ ID NOS: SEQ ID NOS: SEQ ID NOS: 1, 6, 19, 22, 23, 26, 28, 29, 33, 34, 37-42, 45, 48, 53 and 59 (*vanA*); 61, 68, 70-72, 75, 81, 83, 89, 93, 94, 103-105, 107 and 111 (*vanB*); 123, 127, 130, 133, 138, 141, 144, 148, 151, 156, 158, 161, 162, 165, 168, 170, 171, 175, 178, 180, 183-185, 188, 191, 193, 194, 196, 198, and 200-204 (*vanC1*); 206, 210, 213, 217, 220, 221, 222, 224-226, 228-238, 240, 242 and 243 (*vanC2*/*3*); 388, 391, 394, 396, 399, 409, 415, 416, 425, 428, 435, 438, 440, 443, 445, 462, 465, 468, 471, 474, 477, 480, 483, 488, 491, 494-498 and 500-502 (*vanD*); 334, 337-380 and 382-387 (*vanE*); 244, 249, 252, 253, 255, 256, 260, 263, 264, 267, 270-272, 275-283, 285, 288, 291, 293, 295, 297-300, 302, 303, 305, 307, 309-311, 313-318, 321, 322, 325-333 (*vanG*) (forward primers) and SEQ ID NOS: 3, 5, 8, 10, 21, 31, 32, 36, 44, 47, 51, 52, 55 and 60 (*vanA*); 63, 65, 66, 74, 77, 85-88, 90, 91, 95, and 97-102 (*vanB*); 125, 129, 132, 135, 137, 140, 143, 146, 147, 150, 153, 155, 157, 160, 164, 167, 169, 173, 174, 177, 179, 187, 190 and 192 (*vanC1*); 208, 209, 212, 215, 216, 219, 223, 227, 239 and 241 (*vanC2*/*3*); 390, 393, 395, 398, 401, 411, 417, 419, 421, 426, 430, 434, 437, 439, 442, 444, 447-455, 458-461, 464, 467, 470, 473, 476, 479, 482, 486, 490 and 493 (*vanD*); 336 and 381 (*vanE*); 246-248, 250, 251, 254, 258, 259, 262, 266, 269, 274, 284, 286, 287, 290, 301, 304, 306, 308, 312, 320 and 324 (*vanG*) (reverse primers) to a sample; (2) conducting an amplification; and (3) detecting the generation of an amplified product, wherein the generation of an amplified product indicates the presence of vancomycin genes from *Enterococcus* and/or *Staphylococcus* pathogens in a clinical isolate sample.

Methods are described for detecting the *Enterococci* marker sequences in a sample, for example, comprising (1) contacting at least one forward and reverse primer set, *e.g.,* SEQ ID NOS: 610 (Efm *sodA*); 670, 679, 683, 691-693 (Efs *sodA*); 776, 780, 785 (Efm novel); 851, 865, 866, 868 (Efs novel); and 936 (Efm/Efs dual) (forward primers); and SEQ ID NOS: 622 (Efm *sodA*); 710, 716-718, 730, 733 (Efs *sodA*); 800, 813, 816 (Efm novel); 878, 884, 890, 892, 896 (Efs novel); 938 and 939 (Efm/Efs dual) (reverse primers) to a sample; (2) conducting an amplification; and (3) detecting the generation of an amplified product, wherein the generation of an amplified product indicates the presence of *Enterococcus* in a clinical isolate sample.

The detection of amplicons using probes described herein can be performed, for example, using a labeled probe, *e.g.,* the probe comprising a nucleotide sequence selected from the group consisting of: SEQ ID NOS: 2, 4, 7, 9, 11-18, 20, 24, 25, 27, 30, 35, 43, 46, 49, 50, 54, 56-58 (*vanA*); 62, 64, 67, 69, 73, 76, 78-80, 82, 84, 92, 96, 108-110, 112 (*vanB*); 124, 126, 128, 131, 134, 136, 139, 142, 145, 149, 152, 154, 159, 163, 166, 172, 176, 181, 182, 186, 189, 195, 197, 199, 205 (*vanC1*); 207, 211 (*vanC2*/*3*); 389, 392, 397, 400, 402-408, 410, 412-414, 418, 420, 422-424, 427, 429, 431-433, 436, 441, 446, 457, 463, 466, 469, 472, 475, 478, 481, 484, 485, 487, 489, 492, 499 (*vanD*); 335 (*vanE*); 245, 257, 261, 265, 268, 273, 289, 292, 294, 296, 319, 323 *(vanG)*; 664, 648, 655 (Efm *sodA*); 737, 743, 747, 752, 754, 755, 756, 757, 758, 760, 766, 768, 769, 770 (Efs *sodA*); 821, 843 (Efm novel); 908, 925 (Efs novel); and 937 (Efm/Efs dual) that hybridizes to one of the strands of the amplicon generated by at least one forward and reverse primer set. The probe(s) can be, for example, fluorescently labeled, thereby indicating that the detection of the probe involves measuring the fluorescence of the sample of the bound probe, *e.g.,* after bound probes have been isolated. Probes can also be fluorescently labeled in such a way, for example, such that they only fluoresce upon hybridizing to their target, thereby eliminating the need to isolate hybridized probes. The probe can also comprise a fluorescent reporter moiety and a quencher of fluorescence moiety. Upon probe hybridization with the amplified product, the exonuclease activity of a DNA polymerase can be used to dissociate the probe's reporter and quencher, resulting in the unquenched emission of fluorescence, which is detected. An increase in the amplified product causes a proportional increase in fluorescence, due to cleavage of the probe and release of the reporter moiety of the probe. The amplified product is quantified in real time as it accumulates. For multiplex reactions involving more than one distinct probe, each of the probes can be labeled with a different distinguishable and detectable label.

The probes can be molecular beacons. Molecular beacons are single-stranded probes that form a stem-loop structure. A fluorophore can be, for example, covalently linked to one end of the stem and a quencher can be covalently linked to the other end of the stem forming a stem hybrid. When a molecular beacon hybridizes to a target nucleic acid sequence, the probe undergoes a conformational change that results in the dissociation of the stem hybrid and, thus the fluorophore and the quencher move away from each other, enabling the probe to fluoresce brightly. Molecular beacons can be labeled with differently colored fluorophores to detect different target sequences. Any of the probes described herein can be modified and utilized as molecular beacons.

Primer or probe sequences can be ranked according to specific hybridization parameters or metrics that assign a score value indicating their ability to anneal to bacterial strains under highly stringent conditions. Where a primer set is being scored, a "first" or "forward" primer is scored and the "second" or "reverse"-oriented primer sequences can be optimized similarly but with potentially additional parameters, followed by an optional evaluation for primer dimmers, for example, between the forward and reverse primers.

The scoring or ranking steps that are used in the methods of determining the primers and probes include, for example, the following parameters: a target sequence score for the target nucleic acid sequence(s), *e.g*., the PriMD® score; a mean conservation score for the target nucleic acid sequence(s); a mean coverage score for the target nucleic acid sequence(s); 100% conservation score of a portion (*e.g.,* 5' end, center, 3' end) of the target nucleic acid sequence(s); a species score; a strain score; a subtype score; a serotype score; an associated disease score; a year score; a country of origin score; a duplicate score; a patent score; and a minimum qualifying score. Other parameters that are used include, for example, the number of mismatches, the number of critical mismatches (*e.g*., mismatches that result in the predicted failure of the sequence to anneal to a target sequence), the number of native strain sequences that contain critical mismatches, and predicted Tm values. The term "Tm" refers to the temperature at which a population of double-stranded nucleic acid molecules becomes half-dissociated into single strands. Methods for calculating the Tm of nucleic acids are known in the art (Berger and Kimmel (1987) Meth. Enzymol., Vol. 152: Guide To Molecular Cloning Techniques, San Diego: Academic Press, Inc. and Sambrook et al. (1989) Molecular Cloning: A Laboratory Manual, (2nd ed.) Vols. 1-3, Cold Spring Harbor Laboratory).

The resultant scores represent steps in determining nucleotide or whole target nucleic acid sequence preference, while tailoring the primer and/or probe sequences so that they hybridize to a plurality of target nucleic acid sequences. The methods of determining the primers and probes also can comprise the step of allowing for one or more nucleotide changes when determining identity between the candidate primer and probe sequences and the target nucleic acid sequences, or their complements.

In another embodiment, the methods of determining the primers and probes comprise the steps of comparing the candidate primer and probe nucleic acid sequences to "exclusion nucleic acid sequences" and then rejecting those candidate nucleic acid sequences that share identity with the exclusion nucleic acid sequences. In another embodiment, the methods comprise the steps of comparing the candidate primer and probe nucleic acid sequences to "inclusion nucleic acid sequences" and then rejecting those candidate nucleic acid sequences that do not share identity with the inclusion nucleic acid sequences.

In other embodiments of the methods of determining the primers and probes, optimizing primers and probes comprises using a polymerase chain reaction (PCR) penalty score formula comprising at least one of a weighted sum of: primer Tm - optimal Tm; difference between primer Tms; amplicon length - minimum amplicon length; and distance between the primer and a TaqMan® probe. The optimizing step also can comprise determining the ability of the candidate sequence to hybridize with the most target nucleic acid strain sequences (*e.g*., the most target organisms or genes). In another embodiment, the selecting or optimizing step comprises determining which sequences have mean conservation scores closest to 1, wherein a standard of deviation on the mean conservation scores is also compared.

In other embodiments, the methods further comprise the step of evaluating which target nucleic acid sequences are hybridized by an optimal forward primer and an optimal reverse primer, for example, by determining the number of base pair differences between target nucleic acid sequences in a database. For example, the evaluating step can comprise performing an *in silico* polymerase chain reaction, involving (1) rejecting the forward primer and/or reverse primer if it does not meet inclusion or exclusion criteria; (2) rejecting the forward primer and/or reverse primer if it does not amplify a medically valuable nucleic acid; (3) conducting a BLAST analysis to identify forward primer sequences and/or reverse primer sequences that overlap with a published and/or patented sequence; (4) and/or determining the secondary structure of the forward primer, reverse primer, and/or target. In an embodiment, the evaluating step includes evaluating whether the forward primer sequence, reverse primer sequence, and/or probe sequence hybridizes to sequences in the database other than the nucleic acid sequences that are representative of the target strains.

The present description provides oligonucleotides that have preferred primer and probe qualities. These qualities are specific to the sequences of the optimized probes, however, one of skill in the art would recognize that other molecules with similar sequences could also be used. The oligonucleotides provided herein comprise a sequence that shares at least about 60-70% identity with a sequence described in Tables 5, 6, 8A, 8B, 9A, 9B, 10A, 10B, 11A, 11B, and 12. In addition, the sequences can be incorporated into longer sequences, provided they function to specifically anneal to and identify bacterial strains. In another embodiment, the description provides a nucleic acid comprising a sequence that shares at least about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100% identity with the sequences of Tables 5, 6, 8A, 8B, 9A, 9B, 10A, 10B, 11A, 11B, and 12 or complement thereof. The terms "homology" or "identity" or "similarity" refer to sequence relationships between two nucleic acid molecules and can be determined by comparing a nucleotide position in each sequence when aligned for purposes of comparison. The term "homology" refers to the relatedness of two nucleic acid or protein sequences. The term "identity" refers to the degree to which nucleic acids are the same between two sequences. The term "similarity" refers to the degree to which nucleic acids are the same, but includes neutral degenerate nucleotides that can be substituted within a codon without changing the amino acid identity of the codon, as is well known in the art. The primer and/or probe nucleic acid sequences of the description are complementary to the target nucleic acid sequence. The probe and/or primer nucleic acid sequences of the description are optimal for identifying numerous strains of a target nucleic acid, *e.g.,* vancomycin-resistance genes and the *Enterococci* marker sequences. In an embodiment, the nucleic acids of the description are primers for the synthesis (*e.g*., amplification) of target nucleic acid sequences and/or probes for identification, isolation, detection, or analysis of target nucleic acid sequences, *e.g*., an amplified target nucleic acid that is amplified using the primers of the description. The present oligonucleotides hybridize with more than one antibiotic resistance gene (gene as determined by differences in its sequence). The probes and primers provided herein can, for example, allow for the detection of currently identified vancomycin resistance genes or a subset thereof. In addition, the primers and probes of the present invention, depending on the vancomycin resistance gene sequence(s), can allow for the detection of previously unidentified antibiotic resistance genes and VRE. The methods of the description provide for optimal primers and probes, and sets thereof, and combinations of sets thereof, which can hybridize with a larger number of targets than available primers and probes.

In other aspects, the description also provides vectors (*e.g*., plasmid, phage, expression), cell lines (*e.g*., mammalian, insect, yeast, bacterial), and kits comprising any of the sequences of the description described herein. The description further provides known or previously unknown target nucleic acid strain sequences that are identified, for example, using the methods of the description. In an embodiment, the target nucleic acid sequence is an amplification product. In another embodiment, the target nucleic acid sequence is a native or synthetic nucleic acid. The primers, probes, and target nucleic acid sequences, vectors, cell lines, and kits can have any number of uses, such as diagnostic, investigative, confirmatory, monitoring, predictive or prognostic.

Diagnostic kits that comprise one or more of the oligonucleotides described herein, which are useful for screening for and/or detecting the presence of vancomycin resistance and VRE in an individual and/or from a sample, are provided herein. An individual can be a human male, human female, human adult, human child, or human fetus. An individual can also be any mammal, reptile, avian, fish, or amphibian. Hence, an individual can be a primate, pig, horse, cattle, sheep, dog, rabbit, guinea pig, rodent, bird or fish. A sample includes any item, surface, material, clothing, or environment, for example, sewage or water treatment plants, in which it may be desirable to test for the presence of vancomycin resistance genes and VRE. Thus, for instance, the present description includes testing door handles, faucets, table surfaces, elevator buttons, chairs, toilet seats, sinks, kitchen surfaces, children's cribs, bed linen, pillows, keyboards, and so on, for the presence of vancomycin resistance genes and VRE.

A probe of the present description can comprise a label such as, for example, a fluorescent label, a chemiluminescent label, a radioactive label, biotin, gold, dendrimers, aptamer, enzymes, proteins, quenchers and molecular motors. In an embodiment, the probe is a hydrolysis probe, such as, for example, a TaqMan® probe. In other embodiments, the probes of the description are molecular beacons, any fluorescent probes, and probes that are replaced by any double stranded DNA binding dyes (*e. g., SYBR Green*® *1*)*.*

Oligonucleotides of the present description do not only include primers that are useful for conducting the aforementioned amplification reactions, but also include oligonucleotides that are attached to a solid support, such as, for example, a microarray, multiwell plate, column, bead, glass slide, polymeric membrane, glass microfiber, plastic tubes, cellulose, and carbon nanostructures. Hence, detection of vancomycin resistance genes and VRE can be performed by exposing such an oligonucleotide-covered surface to a sample such that the binding of a complementary strain DNA sequence to a surface-attached oligonucleotide elicits a detectable signal or reaction.

Oligonucleotides of the present description also include primers for isolating and sequencing nucleic acid sequences derived from any identified or yet to be isolated and identified vancomycin-resistance gene and VRE.

One embodiment of the description uses solid support-based oligonucleotide hybridization methods to detect gene expression. Solid support-based methods suitable for practicing the present description are widely known and are described (PCT application WO 95/11755; Huber et al., Anal. Biochem., 299:24, 2001; Meiyanto et al., Biotechniques, 31:406, 2001; Relogio et al., Nucleic Acids Res., 30:e51, 2002). Any solid surface to which oligonucleotides can be bound, covalently or non-covalently, can be used. Such solid supports include, but are not limited to, filters, polyvinyl chloride dishes, silicon or glass based chips.

In certain embodiments, the nucleic acid molecule can be directly bound to the solid support or bound through a linker arm, which is typically positioned between the nucleic acid sequence and the solid support. A linker arm that increases the distance between the nucleic acid molecule and the substrate can increase hybridization efficiency. There are a number of ways to position a linker arm. In one common approach, the solid support is coated with a polymeric layer that provides linker arms with a plurality of reactive ends/sites. A common example of this type is glass slides coated with polylysine (U.S. Patent No. 5,667, 976), which are commercially available. Alternatively, the linker arm can be synthesized as part of or conjugated to the nucleic acid molecule, and then this complex is bonded to the solid support. One approach, for example, takes advantage of the extremely high affinity biotin-streptavidin interaction. The streptavidin-biotinylated reaction is stable enough to withstand stringent washing conditions and is sufficiently stable that it is not cleaved by laser pulses used in some detection systems, such as matrix-assisted laser desorption/ionization time of flight (MALDI-TOF) mass spectrometry. Therefore, streptavidin can be covalently attached to a solid support, and a biotinylated nucleic acid molecule will bind to the streptavidin-coated surface. In one version of this method, an amino-coated silicon wafer is reacted with the n-hydroxysuccinimido-ester of biotin and complexed with streptavidin. Biotinylated oligonucleotides are bound to the surface at a concentration of about 20 fmol DNA per mm².

One can alternatively directly bind DNA to the support using carbodiimides, for example. In one such method, the support is coated with hydrazide groups, and then treated with carbodiimide. Carboxy-modified nucleic acid molecules are then coupled to the treated support. Epoxide-based chemistries are also being employed with amine modified oligonucleotides. Other chemistries for coupling nucleic acid molecules to solid substrates are known to those of skill in the art.

The nucleic acid molecules, *e.g.,* the primers and probes of the present description, must be delivered to the substrate material, which is suspected of containing or is being tested for the presence of vancomycin resistance genes and VRE. Because of the miniaturization of the arrays, delivery techniques must be capable of positioning very small amounts of liquids in very small regions, very close to one another and amenable to automation. Several techniques and devices are available to achieve such delivery. Among these are mechanical mechanisms (*e.g*., arrayers from GeneticMicroSystems, MA, USA) and ink-jet technology. Very fine pipets can also be used.

Other formats are also suitable within the context of this description. For example, a 96-well format with fixation of the nucleic acids to a nitrocellulose or nylon membrane can also be employed.

After the nucleic acid molecules have been bound to the solid support, it is often useful to block reactive sites on the solid support that are not consumed in binding to the nucleic acid molecule. In the absence of the blocking step, excess primers and/or probes can, to some extent, bind directly to the solid support itself, giving rise to non-specific binding. Non-specific binding can sometimes hinder the ability to detect low levels of specific binding. A variety of effective blocking agents (*e.g*., milk powder, serum albumin or other proteins with free amine groups, polyvinylpyrrolidine) can be used and others are known to those skilled in the art (U.S. Patent No. 5,994,065). The choice depends at least in part upon the binding chemistry.

One embodiment uses oligonucleotide arrays, *e.g*., microarrays, that can be used to simultaneously observe the expression of a number of vancomycin resistance genes and VRE. Oligonucleotide arrays comprise two or more oligonucleotide probes provided on a solid support, wherein each probe occupies a unique location on the support. The location of each probe can be predetermined, such that detection of a detectable signal at a given location is indicative of hybridization to an oligonucleotide probe of a known identity. Each predetermined location can contain more than one molecule of a probe, but each molecule within the predetermined location has an identical sequence. Such predetermined locations are termed features. There can be, for example, from 2, 10, 100, 1,000, 2,000 or 5,000 or more of such features on a single solid support. In one embodiment, each oligonucleotide is located at a unique position on an array at least 2, at least 3, at least 4, at least 5, at least 6, or at least 10 times.

Oligonucleotide probe arrays for detecting gene expression can be made and used according to conventional techniques described (Lockhart et al., Nat. Biotech., 14:1675-1680, 1996; McGall et al., Proc. Natl. Acad. Sci. USA, 93:13555, 1996; Hughes et al., Nat. Biotechnol., 19:342, 2001). A variety of oligonucleotide array designs are suitable for the practice of this description. Generally, a detectable molecule, also referred to herein as a label, can be incorporated or added to an array's probe nucleic acid sequences. Many types of molecules can be used within the context of this description. Such molecules include, but are not limited to, fluorochromes, chemiluminescent molecules, chromogenic molecules, radioactive molecules, mass spectrometry tags, proteins, and the like. Other labels will be readily apparent to one skilled in the art.

Oligonucleotide probes used in the methods of the present description, including microarray techniques, can be generated using PCR. PCR primers used in generating the probes are chosen, for example, based on the sequences of Tables 6-8. In one embodiment, oligonucleotide control probes also are used. Exemplary control probes can fall into at least one of three categories referred to herein as (1) normalization controls, (2) expression level controls and (3) negative controls. In microarray methods, one or more of these control probes can be provided on the array with the inventive cell cycle gene-related oligonucleotides.

Normalization controls correct for dye biases, tissue biases, dust, slide irregularities, malformed slide spots, *etc.* Normalization controls are oligonucleotide or other nucleic acid probes that are complementary to labeled reference oligonucleotides or other nucleic acid sequences that are added to the nucleic acid sample to be screened. The signals obtained from the normalization controls, after hybridization, provide a control for variations in hybridization conditions, label intensity, reading efficiency and other factors that can cause the signal of a perfect hybridization to vary between arrays. The normalization controls also allow for the semi-quantification of the signals from other features on the microarray. In one embodiment, signals (*e.g*., fluorescence intensity or radioactivity) read from all other probes used in the method are divided by the signal from the control probes, thereby normalizing the measurements.

Virtually any probe can serve as a normalization control. Hybridization efficiency varies, however, with base composition and probe length. Preferred normalization probes are selected to reflect the average length of the other probes being used, but they also can be selected to cover a range of lengths. Further, the normalization control(s) can be selected to reflect the average base composition of the other probe(s) being used. In one embodiment, only one or a few normalization probes are used, and they are selected such that they hybridize well (*i.e.,* without forming secondary structures) and do not match any test probes. In one embodiment, the normalization controls are mammalian genes.

"Negative control" probes are not complementary to any of the test oligonucleotides (*i.e.,* the inventive cell cycle gene-related oligonucleotides), normalization controls, or expression controls. In one embodiment, the negative control is a mammalian gene that is not complementary to any other sequence in the sample.

The terms "background" and "background signal intensity" refer to hybridization signals resulting from non-specific binding or other interactions between the labeled target nucleic acids (e.g., mRNA present in the biological sample) and components of the oligonucleotide array. Background signals also can be produced by intrinsic fluorescence of the array components themselves. A single background signal can be calculated for the entire array, or a different background signal can be calculated for each target nucleic acid. In one embodiment, background is calculated as the average hybridization signal intensity for the lowest 5 to 10 percent of the oligonucleotide probes being used, or, where a different background signal is calculated for each target gene, for the lowest 5 to 10 percent of the probes for each gene. Where the oligonucleotide probes corresponding to a particular target hybridize well and, hence, appear to bind specifically to a target sequence, they should not be used in a background signal calculation. Alternatively, background can be calculated as the average hybridization signal intensity produced by hybridization to probes that are not complementary to any sequence found in the sample (*e.g*., probes directed to nucleic acids of the opposite sense or to genes not found in the sample). In microarray methods, background can be calculated as the average signal intensity produced by regions of the array that lack any oligonucleotides probes at all.

In an alternative embodiment, the nucleic acid molecules are directly or indirectly coupled to an enzyme. Following hybridization, a chromogenic substrate is applied and the colored product is detected by a camera, such as a charge-coupled camera. Examples of such enzymes include alkaline phosphatase, horseradish peroxidase and the like. A probe can be labeled with an enzyme or, alternatively, the probe is labeled with a moiety that is capable of binding to another moiety that is linked to the enzyme. For example, in the biotin-streptavidin interaction, the streptavidin is conjugated to an enzyme such as horseradish peroxidase (HRP). A chromogenic substrate is added to the reaction and is processed/cleaved by the enzyme. The product of the cleavage forms a color, either in the UV or visible spectrum. In another embodiment, streptavidin alkaline phosphatase can be used in a labeled streptavidin-biotin immunoenzymatic antigen detection system.

The description also provides methods of labeling nucleic acid molecules with cleavable mass spectrometry tags (CMST; U.S. Patent Application No: 60/279,890). After an assay is complete, and the uniquely CMST-labeled probes are distributed across the array, a laser beam is sequentially directed to each member of the array. The light from the laser beam both cleaves the unique tag from the tag-nucleic acid molecule conjugate and volatilizes it. The volatilized tag is directed into a mass spectrometer. Based on the mass spectrum of the tag and knowledge of how the tagged nucleotides were prepared, one can unambiguously identify the nucleic acid molecules to which the tag was attached (WO 9905319).

The nucleic acids, primers and probes of the present description can be labeled readily by any of a variety of techniques. When the diversity panel is generated by amplification, the nucleic acids can be labeled during the reaction by incorporation of a labeled dNTP or use of labeled amplification primer. If the amplification primers include a promoter for an RNA polymerase, a post-reaction labeling can be achieved by synthesizing RNA in the presence of labeled NTPs. Amplified fragments that were unlabeled during amplification or unamplified nucleic acid molecules can be labeled by one of a number of end labeling techniques or by a transcription method, such as nick-translation, random-primed DNA synthesis. Details of these methods are known to one of skill in the art and are set out in methodology books. Other types of labeling reactions are performed by denaturation of the nucleic acid molecules in the presence of a DNA-binding molecule, such as RecA, and subsequent hybridization under conditions that favor the formation of a stable RecA-incorporated DNA complex.

In another embodiment, PCR-based methods are used to detect gene expression. These methods include reverse-transcriptase-mediated polymerase chain reaction (RT-PCR) including real-time and endpoint quantitative reverse-transcriptase-mediated polymerase chain reaction (Q-RTPCR). These methods are well known in the art. For example, methods of quantitative PCR can be carried out using kits and methods that are commercially available from, for example, Applied BioSystems and Stratagene®. See also Kochanowski, Quantitative PCR Protocols (Humana Press, 1999); Innis *et al., supra*.; Vandesompele et al., Genome Biol., 3: RESEARCH0034, 2002; Stein, Cell Mol. Life Sci. 59:1235, 2002.

The forward and reverse amplification primers and internal hybridization probe is designed to hybridize specifically and uniquely with one nucleotide sequence derived from the transcript of a target gene. In one embodiment, the selection criteria for primer and probe sequences incorporates constraints regarding nucleotide content and size to accommodate TaqMan® requirements. SYBR Green® can be used as a probe-less Q-RTPCR alternative to the TaqMan®-type assay, discussed above (ABI Prism® 7900 Sequence Detection System User Guide Applied Biosystems, chap. 1-8, App. A-F. (2002)). A device measures changes in fluorescence emission intensity during PCR amplification. The measurement is done in "real time," that is, as the amplification product accumulates in the reaction. Other methods can be used to measure changes in fluorescence resulting from probe digestion. For example, fluorescence polarization can distinguish between large and small molecules based on molecular tumbling (U.S. Patent No. 5,593,867).

The primers and probes of the present description may anneal to or hybridize to various *Enterococcus* and/or *Staphylococcus* genetic material or genetic material derived therefrom, or other genetic material derived therefrom, such as RNA, DNA, cDNA, or a PCR product.

A "sample" that is tested for the presence of vancomycin resistance genes and VRE includes, but is not limited to a tissue sample, such as, for example, blood, serum, plasma, enriched peripheral blood mononuclear cells, neoplastic or other tissue obtained from biopsies, cerebrospinal fluid, saliva, fluids collected from the ear, eye, mouth, and respiratory airways, sputum, skin, tears, oropharyngeal swabs, nasopharyngeal swabs, throat swabs, urine, anal-rectal swabs, feces, skin swabs, nasal aspirates, nasal wash, fluids and cells obtained by the perfusion of tissues of both human and animal origin, and fluids and cells derived from the culturing of human cells, including human stem cells and human cartilage or fibroblasts. The tissue sample may be fresh, fixed, preserved, or frozen. A sample also includes any item, surface, material, or clothing, or environment, for example, sewage or water treatment plants, in which it may be desirable to test for the presence of vancomycin resistance genes and VRE. Thus, for instance, the present description includes testing door handles, faucets, table surfaces, elevator buttons, chairs, toilet seats, sinks, kitchen surfaces, children's cribs, bed linen, pillows, keyboards, and so on, for the presence of vancomycin resistance genes and VRE.

The target nucleic acid strain that is amplified may be RNA or DNA or a modification thereof. Thus, the amplifying step can comprise isothermal or non-isothermal reactions, such as polymerase chain reaction, Scorpion® primers, molecular beacons, SimpleProbes®, HyBeacons®, cycling probe technology, Invader Assay, self-sustained sequence replication, nucleic acid sequence-based amplification, ramification amplifying method, hybridization signal amplification method, rolling circle amplification, multiple displacement amplification, thermophilic strand displacement amplification, transcription-mediated amplification, ligase chain reaction, signal mediated amplification of RNA, split promoter amplification, Q-Beta replicase, isothermal chain reaction, one cut event amplification, loop-mediated isothermal amplification, molecular inversion probes, ampliprobe, headloop DNA amplification, and ligation activated transcription. The amplifying step can be conducted on a solid support, such as a multiwell plate, array, column, bead, glass slide, polymeric membrane, glass microfiber, plastic tubes, cellulose, and carbon nanostructures. The amplifying step also comprises *in situ* hybridization. The detecting step can comprise gel electrophoresis, fluorescence resonant energy transfer, or hybridization to a labeled probe, such as a probe labeled with biotin, at least one fluorescent moiety, an antigen, a molecular weight tag, and a modifier of probe Tm. The detection step can also comprise the incorporation of a label (e.g., fluorescent or radioactive) during an extension reaction. The detecting step comprises measuring fluorescence, mass, charge, and/or chemiluminescence.

The target nucleic acid strain may not need amplification and may be RNA or DNA or a modification thereof. If amplification is not necessary, the target nucleic acid strain can be denatured to enable hybridization of a probe to the target nucleic acid sequence.

Hybridization may be detected in a variety of ways and with a variety of equipment. In general, the methods can be categorized as those that rely upon detectable molecules incorporated into the diversity panels and those that rely upon measurable properties of double-stranded nucleic acids (e.g., hybridized nucleic acids) that distinguish them from single-stranded nucleic acids (e.g., unhybridized nucleic acids). The latter category of methods includes intercalation of dyes, such as, for example, ethidium bromide, into double-stranded nucleic acids, differential absorbance properties of double and single stranded nucleic acids, binding of proteins that preferentially bind double-stranded nucleic acids, and the like.

### EXEMPLIFICATION

### Example 1. Scoring a Set of Predicted Annealing Oligonucleotides

Each of the sets of primers and probes selected is ranked by a combination of methods as individual primers and probes and as a primer/probe set. This involves one or more methods of ranking (e.g., joint ranking, hierarchical ranking , and serial ranking) where sets of primers and probes are eliminated or included based on any combination of the following criteria, and a weighted ranking again based on any combination of the following criteria, for example: (A) Percentage Identity to Target Strains; (B) Conservation Score; (C) Coverage Score; (D) Strain/Subtype/Serotype Score; (E) Associated Disease Score; (F) Duplicates Sequences Score; (G) Year and Country of Origin Score; (H) Patent Score, and (I) Epidemiology Score.

### (A) Percentage Identity

A percentage identity score is based upon the number of target nucleic acid strain (e.g., native) sequences that can hybridize with perfect conservation (the sequences are perfectly complimentary) to each primer or probe of a primer set and probe set. If the score is less than 100%, the program ranks additional primer set and probe sets that are not perfectly conserved. This is a hierarchical scale for percent identity starting with perfect complimentarity, then one base degeneracy through to the number of degenerate bases that would provide the score closest to 100%. The position of these degenerate bases would then be ranked. The methods for calculating the conservation is described under section B.

### (i) Individual Base Conservation Score

A set of conservation scores is generated for each nucleotide base in the consensus sequence and these scores represent how many of the target nucleic acid strains sequences have a particular base at this position. For example, a score of 0.95 for a nucleotide with an adenosine, and 0.05 for a nucleotide with a cytidine means that 95% of the native sequences have an A at that position and 5% have a C at that position. A perfectly conserved base position is one where all the target nucleic acid strain sequences have the same base (either an A, C, G, or T/U) at that position. If there is an equal number of bases *(e.g.,* 50% A & 50% T) at a position, it is identified with an N.

### (ii) Candidate Primer/Probe Sequence Conservation

An overall conservation score is generated for each candidate primer or probe sequence that represents how many of the target nucleic acid strain sequences will hybridize to the primers or probes. A candidate sequence that is perfectly complimentary to all the target nucleic acid strain sequences will have a score of 1.0 and rank the highest. For example, illustrated below in Table 4 are three different 10-base candidate probe sequences that are targeted to different regions of a consensus target nucleic acid strain sequence. Each candidate probe sequence is compared to a total of 10 native sequences.

**Table 4.**

| | | | | | | | | | | |
|---|---|---|---|---|---|---|---|---|---|---|
| #1. | A | A | A | C | A | C | G | T | G | C |
| | 0.7 | 1.0 | 1.0 | 1.0 | 1.0 | 1.0 | 1.0 | 1.0 | 1.0 | 1.0 |
| (SEQ ID NO:507) | | | | | | | | | | |
| →Number of target nucleic acid strain sequences that are perfectly complimentary - 7. Three out of the ten sequences do not have an A at position 1. | | | | | | | | | | |
| #2. | C | C | T | T | G | T | T | C | C | A |
| | 1.0 | 0.9 | 1.0 | 0.9 | 0.9 | 1.0 | 1.0 | 1.0 | 1.0 | 1.0 |
| (SEQ ID NO:508) | | | | | | | | | | |
| →Number of target nucleic acid strain sequences that are perfectly complimentary - 7, 8, or 9. At least one target nucleic acid strain does not have a C at position 2, T at position 4, or G at position 5. These differences may all be on one target nucleic acid strain molecule or may be on two or three separate molecules. | | | | | | | | | | |
| #3. | C | A | G | G | G | A | C | G | A | T |
| | 1.0 | 1.0 | 1.0 | 1.0 | 1.0 | 0.9 | 0.8 | 1.0 | 1.0 | 1.0 |
| (SEQ ID NO:509) | | | | | | | | | | |
| →Number of target nucleic acid strain sequences that are perfectly complimentary - 7 or 8. At least one target nucleic acid strain does not have an A at position 6 and at least two target nucleic acid strain do not have a C at position 7. These differences may all be on one target nucleic acid strain molecule or may be on two separate molecules. | | | | | | | | | | |

A simple arithmetic mean for each candidate sequence would generate the same value of 0.97. The number of target nucleic acid strain sequences identified by each candidate probe sequence, however, can be very different. Sequence #1 can only identify 7 native sequences because of the 0.7 (out of 1.0) score by the first base - A. Sequence #2 has three bases each with a score of 0.9; each of these could represent a different or shared target nucleic acid strain sequence. Consequently, Sequence #2 can identify 7, 8 or 9 target nucleic acid strain sequences. Similarly, Sequence #3 can identify 7 or 8 of the target nucleic acid strain sequences. Sequence #2 would, therefore, be the best choice if all the three bases with a score of 0.9 represented the same 9 target nucleic acid strain sequences.

### (iii) Overall Conservation Score of the Primer and Probe Set - Percent Identity

The same method described in (ii) when applied to the complete primer set and probe set will generate the percent identity for the set (see A above). For example, using the same sequences illustrated above, if Sequences #1 and #2 are primers and Sequence #3 is a probe, then the percent identity for the target can be calculated from how many of the target nucleic acid sequences are identified with perfect complementarity to all three primer/probe sequences. The percent identity could be no better than 0.7 (7 out of 10 target nucleic acid strain sequences) but as little as 0.1 if each of the degenerate bases reflects a different target nucleic acid strain sequence. Again, an arithmetic mean of these three sequences would be 0.97. As none of the above examples were able to capture all the target nucleic acid strain sequences because of the degeneracy (scores of less than 1.0), the ranking system takes into account that a certain amount of degeneracy can be tolerated under normal hybridization conditions, for example, during a polymerase chain reaction. The ranking of these degeneracies is described in (iv) below.

An *in silico* evaluation determines how many native sequences *(e.g.,* original sequences submitted to public databases) are identified by a given candidate primer/probe set. The ideal candidate primer/probe set is one that can perform PCR and the sequences are perfectly complementary to all the known native sequences that were used to generate the consensus sequence. If there is no such candidate, then the sets are ranked according to how many degenerate bases can be accepted and still hybridize to just the target sequence during the PCR and yet identify all the native sequences.

The hybridization conditions, for TaqMan® as an example, are: 10-50 mM Tris-HCl pH 8.3, 50 mM KCl, 0.1-0.2% Triton® X-100 or 0.1 % Tween®, 1-5 mM MgCl₂. The hybridization is performed at 58-60°C for the primers and 68-70°C for the probe. The *in silico* PCR identifies native sequences that are not amplifiable using the candidate primers and probe set. The rules can be as simple as counting the number of degenerate bases to more sophisticated approaches based on exploiting the PCR criteria used by the PriMD® software. Each target nucleic acid strain sequence has a value or weight (see Score assignment above). If the failed target nucleic acid strain sequence is medically valuable, the primer/probe set is rejected. This *in silico* analysis provides a degree of confidence for a given genotype and is important when new sequences are added to the databases. New target nucleic acid strain sequences are automatically entered into both the "include" and "exclude" categories. Published primer and probes will also be ranked by the PriMD software.

### (iv) Position (5' to 3') Of The Base Conservation Score

In an embodiment, primers do not have bases in the terminal five positions at the 3' end with a score less than 1. This is one of the last parameters to be relaxed if the method fails to select any candidate sequences. The next best candidate having a perfectly conserved primer would be one where the poorer conserved positions are limited to the terminal bases at the 5' end. The closer the poorer conserved position is to the 5' end, the better the score. For probes, the position criteria are different. For example, with a TaqMan® probe, the most destabilizing effect occurs in the center of the probe. The 5' end of the probe is also important as this contains the reporter molecule that must be cleaved, following hybridization to the target, by the polymerase to generate a sequence-specific signal. The 3' end is less critical. Therefore, a sequence with a perfectly conserved middle region will have the higher score. The remaining ends of the probe are ranked in a similar fashion to the 5' end of the primer. Thus, the next best candidate to a perfectly conserved TaqMan® probe would be one where the poorer conserved positions are limited to the terminal bases at either the 5' or 3' ends. The hierarchical scoring will select primers with only one degeneracy first, then primers with two degeneracies next and so on. The relative position of each degeneracy will then be ranked favoring those that are closest to the 5' end of the primers and those closest to the 3' end of the TaqMan® probe. If there are two or more degenerate bases in a primer and probe set the ranking will initially select the sets where the degeneracies occur on different sequences.

### B. Coverage Score

The total number of aligned sequences is considered under a coverage score. A value is assigned to each position based on how many times that position has been reported or sequenced. Alternatively, coverage can be defined as how representative the sequences are of the known strains, subtypes etc., or their relevance to a certain diseases. For example, the target nucleic acid strain sequences for a particular gene may be very well conserved and show complete coverage but certain strains are not represented in those sequences.

A sequence is included if it aligns with any part of the consensus sequence, which is usually a whole gene or a functional unit, or has been described as being a representative of this gene. Even though a base position is perfectly conserved it may only represent a fraction of the total number of sequences (for example, if there are very few sequences). For example, region A of a gene shows a 100% conservation from 20 sequence entries while region B in the same gene shows a 98% conservation but from 200 sequence entries. There is a relationship between conservation and coverage if the sequence shows some persistent variability. As more sequences are aligned, the conservation score falls, but this effect is lessened as the number of sequences gets larger. Unless the number of sequences is very small (e.g., under 10) the value of the coverage score is small compared to that of the conservation score. To obtain the best consensus sequence, artificial spaces are allowed to be introduced. Such spaces are not considered in the coverage score.

### C. Strain/Subtype/Serotype Score

A value is assigned to each strain or subtype or serotype based upon its relevance to a disease. For example, bacterial strains and/or species that are linked to high frequencies of infection will have a higher score than strains that are generally regarded as benign. The score is based upon sufficient evidence to automatically associate a particular strain with a disease. For example, certain strains of adenovirus are not associated with diseases of the upper respiratory system. Accordingly, there will be sequences included in the consensus sequence that are not associated with diseases of the upper respiratory system.

### D. Associated Disease Score

The associated disease score pertains to strains that are not known to be associated with a particular disease (to differentiate from D above). Here, a value is assigned only if the submitted sequence is directly linked to the disease and that disease is pertinent to the assay.

### E. Duplicate Sequences Score

If a particular sequence has been sequenced more than once it will have an effect on representation, for example, a strain that is represented by 12 entries in GenBank of which six are identical and the other six are unique. Unless the identical sequences can be assigned to different strains/subtypes (usually by sequencing other gene or by immunology methods) they will be excluded from the scoring.

### F. Year and Country of Origin Score

The year and country of origin scores are important in terms of the age of the human population and the need to provide a product for a global market. For example, strains identified or collected many years ago may not be relevant today. Furthermore, it is probably difficult to obtain samples that contain these older strains. Certain divergent strains from more obscure countries or sources may also be less relevant to the locations that will likely perform clinical tests, or may be more important for certain countries (e.g., North America, Europe, or Asia).

### G. Patent Score

Candidate target strain sequences published in patents are searched electronically and annotated such that patented regions are excluded. Alternatively, candidate sequences are checked against a patented sequence database.

### H. Minimum Qualifying Score

The minimum qualifying score is determined by expanding the number of allowed mismatches in each set of candidate primers and probes until all possible native sequences are represented *(e.g.,* has a qualifying hit).

### I. Other

A score is given to based on other parameters, such as relevance to certain patients *(e.g.,* pediatrics, immunocompromised) or certain therapies *(e.g.,* target those strains that respond to treatment) or epidemiology. The prevalence of an organism/strain and the number of times it has been tested for in the community can add value to the selection of the candidate sequences. If a particular strain is more commonly tested then selection of it would be more likely. Strain identification can be used to select better vaccines.

### Example 2. Primer/Probe Evaluation

Once the candidate primers and probes have received their scores and have been ranked, they are evaluated using any of a number of methods of the description, such as BLAST analysis and secondary structure analysis.

### A. BLAST Analysis

The candidate primer/probe sets are submitted to BLAST analysis to check for possible overlap with any published sequences that might be missed by the Include/Exclude function. It also provides a useful summary.

### B. Secondary Structure

The methods of the present description include analysis of nucleic acid secondary structure. This includes the structures of the primers and/or probes, as well as their intended target strain sequences. The methods and software of the description predict the optimal temperatures for annealing, but assumes that the target *(e.g.,* RNA or DNA) does not have any significant secondary structure. For example, if the starting material is RNA, the first stage is the creation of a complimentary strand of DNA (cDNA) using a specific primer. This is usually performed at temperatures where the RNA template can have significant secondary structure thereby preventing the annealing of the primer. Similarly, after denaturation of a double stranded DNA target (for example, an amplicon after PCR), the binding of the probe is dependent on there being no major secondary structure in the amplicon.

The methods of the description can either use this information as a criteria for selecting primers and probes or evaluate any secondary structure of a selected sequence, for example, by cutting and pasting candidate primer or probe sequences into a commercial internet link that uses software dedicated to analyzing secondary structure, such as, for example, MFOLD (Zuker et al. (1999) Algorithms and Thermodynamics for RNA Secondary Structure Prediction: A Practical Guide in RNA Biochemistry and Biotechnology, J. Barciszewski and B.F.C. Clark, eds., NATO ASI Series, Kluwer Academic Publishers).

### C. Evaluating the Primer and Probe Sequences

The methods and software of the description may also analyze any nucleic acid sequence to determine its suitability in a nucleic acid amplification-based assay. For example, it can accept a competitor's primer set and determine the following information: (1) How it compares to the primers of the description (*e.g.,* overall rank, PCR and conservation ranking, etc.); (2) How it aligns to the exclude libraries (e.g., assessing cross-hybridization) - also used to compare primer and probe sets to newly published sequences; and (3) If the sequence has been previously published. This step requires keeping a database of sequences published in scientific journals, posters, and other presentations.

### Example 3. Multiplexing

The Exclude/Include capability is ideally suited for designing multiplex reactions. The parameters for designing multiple primer and probe sets adhere to a more stringent set of parameters than those used for the initial Exclude/Include function. Each set of primers and probe, together with the resulting amplicon, is screened against the other sets that constitute the multiplex reaction. As new targets are accepted, their sequences are automatically added to the Exclude category.

The database is designed to interrogate the online databases to determine and acquire, if necessary, any new sequences relevant to the targets. These sequences are evaluated against the optimal primer/probe set. If they represent a new genotype or strain, then a multiple sequence alignment may be required.

### Example 4. Sequences Identified for Detecting the Antibiotic Resistance Genes vanA, vanB, vanC, vanD, vanE and vanG Gene Variants

The set of primers and probes were then scored according to the methods described herein to identify the optimized primers and probes of Table 5 (*vanA* and *vanB*)*,* and Table 6 (*vanC, vanD, vanE* and *vanG*)*.* It should be noted that the primers, as they are sequences that anneal to a plurality of all identified or unidentified vancomycin-resistance genes, can also be used as probes either in the presence or absence of amplification of a sample.

**Table 5. Optimized Primers and Probes for the Detection of vanA and vanB Resistance Genes.**

| **Group No.** | **Forward Primer** | **Probe** | **Reverse Primer** |
|---|---|---|---|
| ***vanA Sets*** | | | |
| 1 | SEQ ID NO: 1 | SEQ ID NO: 2 | SEQ ID NO: 3 |
| | TTGTGCGGTATTGGGAAACAGT | TGATTTGGTCCACCTCGCCAACAACTAACGC | CGACTTCCTGATGAATACGAAAGATTCC |
| 2 | SEQ ID NO: 1 | SEQ ID NO: 4 | SEQ ID NO: 3 |
| | TTGTGCGGTATTGGGAAACAGT | CCTGATTTGGTCCACCTCGCCAACAACTAACG | CGACTTCCTGATGAATACGAAAGATTCC |
| 3 | SEQ ID NO: 1 | SEQ ID NO: 2 | SEQ ID NO: 5 |
| | TTGTGCGGTATTGGGAAACAGT | TGATTTGGTCCACCTCGCCAACAACTAACGC | CTCGACTTCCTGATGAATACGAAAGATTC |
| 4 | SEQ ID NO: 1 | SEQ ID NO: 4 | SEQ ID NO: 5 |
| | TTGTGCGGTATTGGGAAACAGT | CCTGATTTGGTCCACCTCGCCAACAACTAACG | CTCGACTTCCTGATGAATACGAAAGATTC |
| 5 | SEQ ID NO: 6 | SEQ ID NO: 7 | SEQ ID NO: 8 |
| | CGCGTTCAGGCTCATCCTT | TCACAGCCCGAAACAGCCTGCTCAATTAAGAT TTTGC | ACTGTTTCCCAATACCGCACAAC |
| 6 | SEQ ID NO: 6 | SEQ ID NO: 9 | SEQ ID NO:10 |
| | CGCGTTCAGGCTCATCCTT | CTCACAGCCCGAAACAGCCTGCTCAATTAAGA TTT | ACTGTTTCCCAATACCGCACAA |
| 7 | SEQ ID NO: 6 | SEQ ID NO: 11 | SEQ ID NO:10 |
| | CGCGTTCAGGCTCATCCTT | CTCACAGCCCGAAACAGCCTGCTCAATTAAGA TT | ACTGTTTCCCAATACCGCACAA |
| 8 | SEQ ID NO: 6 | SEQ ID NO: 12 | SEQ ID NO:10 |
| | CGCGTTCAGGCTCATCCTT | CTCACAGCCCGAAACAGCCTGCTCAATTAAGA TTTTG | ACTGTTTCCCAATACCGCACAA |
| 9 | SEQ ID NO: 6 | SEQ ID NO: 13 | SEQ ID NO:10 |
| | CGCGTTCAGGCTCATCCTT | CTCACAGCCCGAAACAGCCTGCTCAATTAAGA T | ACTGTTTCCCAATACCGCACAA |
| 10 | SEQ ID NO: 6 | SEQ ID NO: 14 | SEQ ID NO: 8 |
| | CGCGTTCAGGCTCATCCTT | ACAGCCCGAAACAGCCTGCTCAATTAAGATTTT GCT | ACTGTTTCCCAATACCGCACAAC |
| 11 | SEQ ID NO: 6 | SEQ ID NO: 15 | SEQ ID NO:10 |
| | CGCGTTCAGGCTCATCCTT | TCACAGCCCGAAACAGCCTGCTCAATTAAGAT | ACTGTTTCCCAATACCGCACAA |
| 12 | SEQ ID NO: 6 | SEQ ID NO: 16 | SEQ ID NO:10 |
| | CGCGTTCAGGCTCATCCTT | TCACAGCCCGAAACAGCCTGCTCAATTAAGAT T | ACTGTTTCCCAATACCGCACAA |
| 13 | SEQ ID NO: 6 | SEQ ID NO: 17 | SEQ ID NO:10 |
| | CGCGTTCAGGCTCATCCTT | TCACAGCCCGAAACAGCCTGCTCAATTAAGAT TTTG | ACTGTTTCCCAATACCGCACAA |
| 14 | SEQ ID NO: 6 | SEQ ID NO: 14 | SEQ ID NO:10 |
| | CGCGTTCAGGCTCATCCTT | ACAGCCCGAAACAGCCTGCTCAATTAAGATTTT GCT | ACTGTTTCCCAATACCGCACAA |
| 15 | SEQ ID NO: 6 | SEQ ID NO: 18 | SEQ ID NO:10 |
| | CGCGTTCAGGCTCATCCTT | CAGCCCGAAACAGCCTGCTCAATTAAGATTTTG CT | ACTGTTTCCCAATACCGCACAA |
| 16 | SEQ ID NO: 19 | SEQ ID NO: 20 | SEQ ID NO: 21 |
| | AGCAAAATCTTAATTGAGCAGGC TGTTT | CCCAATACCGCACAACCGACCTCACAG | GGTCCACCTCGCCAACA |
| 17 | SEQ ID NO: 22 | SEQ ID NO: 20 | SEQ ID NO: 21 |
| | CAGCAAAATCTTAATTGAGCAGG CTGTTT | CCCAATACCGCACAACCGACCTCACAG | GGTCCACCTCGCCAACA |
| 18 | SEQ ID NO: 6 | SEQ ID NO: 20 | SEQ ID NO: 21 |
| | CGCGTTCAGGCTCATCCTT | CCCAATACCGCACAACCGACCTCACAG | GGTCCACCTCGCCAACA |
| 19 | SEQ ID NO: 23 | SEQ ID NO: 20 | SEQ ID NO: 21 |
| | GCAAAATCTTAATTGAGCAGGCT GTTT | CCCAATACCGCACAACCGACCTCACAG | GGTCCACCTCGCCAACA |
| 20 | SEQ ID NO: 6 | SEQ ID NO: 24 | SEQ ID NO: 21 |
| | CGCGTTCAGGCTCATCCTT | CAATACCGCACAACCGACCTCACAGCC | GGTCCACCTCGCCAACA |
| 21 | SEQ ID NO: 6 | SEQ ID NO: 25 | SEQ ID NO: 3 |
| | CGCGTTCAGGCTCATCCTT | CTGCAGCCTGATTTGGTCCACCTCGC | CGACTTCCTGATGAATACGAAAGATTCC |
| 22 | SEQ ID NO: 22 | SEQ ID NO: 25 | SEQ ID NO: 3 |
| | CAGCAAAATCTTAATTGAGCAGG CTGTTT | CTGCAGCCTGATTTGGTCCACCTCGC | CGACTTCCTGATGAATACGAAAGATTCC |
| 23 | SEQ ID NO: 19 | SEQ ID NO: 25 | SEQ ID NO: 3 |
| | AGCAAAATCTTAATTGAGCAGGC TGTTT | CTGCAGCCTGATTTGGTCCACCTCGC | CGACTTCCTGATGAATACGAAAGATTCC |
| 24 | SEQ ID NO: 26 | SEQ ID NO: 27 | SEQ ID NO: 3 |
| | GGCTGTGAGGTCGGTTGTG | TGCAGCCTGATTTGGTCCACCTCGC | CGACTTCCTGATGAATACGAAAGATTCC |
| 25 | SEQ ID NO: 22 | SEQ ID NO: 27 | SEQ ID NO: 3 |
| | CAGCAAAATCTTAATTGAGCAGG | TGCAGCCTGATTTGGTCCACCTCGC | CGACTTCCTGATGAATACGAAAGATTCC |
| | CTGTTT | | |
| 26 | SEQ ID NO: 19 | SEQ ID NO: 27 | SEQ ID NO: 3 |
| | AGCAAAATCTTAATTGAGCAGGC TGTTT | TGCAGCCTGATTTGGTCCACCTCGC | CGACTTCCTGATGAATACGAAAGATTCC |
| 27 | SEQ ID NO: 23 | SEQ ID NO: 25 | SEQ ID NO: 3 |
| | GCAAAATCTTAATTGAGCAGGCT GTTT | CTGCAGCCTGATTTGGTCCACCTCGC | CGACTTCCTGATGAATACGAAAGATTCC |
| 28 | SEQ ID NO: 22 | SEQ ID NO: 25 | SEQ ID NO: 5 |
| | CAGCAAAATCTTAATTGAGCAGG CTGTTT | CTGCAGCCTGATTTGGTCCACCTCGC | CTCGACTTCCTGATGAATACGAAAGATTC |
| 29 | SEQ ID NO: 19 | SEQ ID NO: 25 | SEQ ID NO: 5 |
| | AGCAAAATCTTAATTGAGCAGGC TGTTT | CTGCAGCCTGATTTGGTCCACCTCGC | CTCGACTTCCTGATGAATACGAAAGATTC |
| 30 | SEQ ID NO: 6 | SEQ ID NO: 25 | SEQ ID NO: 5 |
| | CGCGTTCAGGCTCATCCTT | CTGCAGCCTGATTTGGTCCACCTCGC | CTCGACTTCCTGATGAATACGAAAGATTC |
| 31 | SEQ ID NO: 28 | SEQ ID NO: 27 | SEQ ID NO: 3 |
| | GGCTGTGAGGTCGGTTGT | TGCAGCCTGATTTGGTCCACCTCGC | CGACTTCCTGATGAATACGAAAGATTCC |
| 32 | SEQ ID NO: 29 | SEQ ID NO: 7 | SEQ ID NO: 8 |
| | GGCGCGTTCAGGCTCATC | TCACAGCCCGAAACAGCCTGCTCAATTAAGAT TTTGC | ACTGTTTCCCAATACCGCACAAC |
| 33 | SEQ ID NO: 23 | SEQ ID NO: 25 | SEQ ID NO: 5 |
| | GCAAAATCTTAATTGAGCAGGCT GTTT | CTGCAGCCTGATTTGGTCCACCTCGC | CTCGACTTCCTGATGAATACGAAAGATTC |
| 34 | SEQ ID NO: 23 | SEQ ID NO: 27 | SEQ ID NO: 3 |
| | GCAAAATCTTAATTGAGCAGGCT GTTT | TGCAGCCTGATTTGGTCCACCTCGC | CGACTTCCTGATGAATACGAAAGATTCC |
| 35 | SEQ ID NO: 19 | SEQ ID NO: 27 | SEQ ID NO: 5 |
| | AGCAAAATCTTAATTGAGCAGGC TGTTT | TGCAGCCTGATTTGGTCCACCTCGC | CTCGACTTCCTGATGAATACGAAAGATTC |
| 36 | SEQ ID NO: 26 | SEQ ID NO: 27 | SEQ ID NO: 5 |
| | GGCTGTGAGGTCGGTTGTG | TGCAGCCTGATTTGGTCCACCTCGC | CTCGACTTCCTGATGAATACGAAAGATTC |
| 37 | SEQ ID NO: 22 | SEQ ID NO: 27 | SEQ ID NO: 5 |
| | CAGCAAAATCTTAATTGAGCAGG CTGTTT | TGCAGCCTGATTTGGTCCACCTCGC | CTCGACTTCCTGATGAATACGAAAGATTC |
| 38 | SEQ ID NO: 28 | SEQ ID NO: 27 | SEQ ID NO: 5 |
| | GGCTGTGAGGTCGGTTGT | TGCAGCCTGATTTGGTCCACCTCGC | CTCGACTTCCTGATGAATACGAAAGATTC |
| 39 | SEQ ID NO: 19 | SEQ ID NO: 30 | SEQ ID NO: 3 |
| | AGCAAAATCTTAATTGAGCAGGC TGTTT | CAGCCTGATTTGGTCCACCTCGCCA | CGACTTCCTGATGAATACGAAAGATTCC |
| 40 | SEQ ID NO: 23 | SEQ ID NO: 27 | SEQ ID NO: 5 |
| | GCAAAATCTTAATTGAGCAGGCT GTTT | TGCAGCCTGATTTGGTCCACCTCGC | CTCGACTTCCTGATGAATACGAAAGATTC |
| 41 | SEQ ID NO: 6 | SEQ ID NO: 14 | SEQ ID NO: 31 |
| | CGCGTTCAGGCTCATCCTT | ACAGCCCGAAACAGCCTGCTCAATTAAGATTTT GCT | CACTGTTTCCCAATACCGCACAA |
| 42 | SEQ ID NO: 6 | SEQ ID NO: 25 | SEQ ID NO: 32 |
| | CGCGTTCAGGCTCATCCTT | CTGCAGCCTGATTTGGTCCACCTCGC | GCTCGACTTCCTGATGAATACGAAA |
| 43 | SEQ ID NO: 19 | SEQ ID NO: 25 | SEQ ID NO: 32 |
| | AGCAAAATCTTAATTGAGCAGGC TGTTT | CTGCAGCCTGATTTGGTCCACCTCGC | GCTCGACTTCCTGATGAATACGAAA |
| 44 | SEQ ID NO: 22 | SEQ ID NO: 25 | SEQ ID NO: 32 |
| | CAGCAAAATCTTAATTGAGCAGG CTGTTT | CTGCAGCCTGATTTGGTCCACCTCGC | GCTCGACTTCCTGATGAATACGAAA |
| 45 | SEQ ID NO: 23 | SEQ ID NO: 25 | SEQ ID NO: 32 |
| | GCAAAATCTTAATTGAGCAGGCT GTTT | CTGCAGCCTGATTTGGTCCACCTCGC | GCTCGACTTCCTGATGAATACGAAA |
| 46 | SEQ ID NO: 22 | SEQ ID NO: 27 | SEQ ID NO: 32 |
| | CAGCAAAATCTTAATTGAGCAGG CTGTTT | TGCAGCCTGATTTGGTCCACCTCGC | GCTCGACTTCCTGATGAATACGAAA |
| 47 | SEQ ID NO: 26 | SEQ ID NO: 27 | SEQ ID NO: 32 |
| | GGCTGTGAGGTCGGTTGTG | TGCAGCCTGATTTGGTCCACCTCGC | GCTCGACTTCCTGATGAATACGAAA |
| 48 | SEQ ID NO: 28 | SEQ ID NO: 27 | SEQ ID NO: 32 |
| | GGCTGTGAGGTCGGTTGT | TGCAGCCTGATTTGGTCCACCTCGC | GCTCGACTTCCTGATGAATACGAAA |
| 49 | SEQ ID NO: 19 | SEQ ID NO: 27 | SEQ ID NO: 32 |
| | AGCAAAATCTTAATTGAGCAGGC TGTTT | TGCAGCCTGATTTGGTCCACCTCGC | GCTCGACTTCCTGATGAATACGAAA |
| 50 | SEQ ID NO: 23 | SEQ ID NO: 27 | SEQ ID NO: 32 |
| | GCAAAATCTTAATTGAGCAGGCT GTTT | TGCAGCCTGATTTGGTCCACCTCGC | GCTCGACTTCCTGATGAATACGAAA |
| 51 | SEQ ID NO: 33 | SEQ ID NO: 20 | SEQ ID NO: 21 |
| | GGCGCGTTCAGGCTCAT | CCCAATACCGCACAACCGACCTCACAG | GGTCCACCTCGCCAACA |
| 52 | SEQ ID NO: 29 | SEQ ID NO: 20 | SEQ ID NO: 21 |
| | GGCGCGTTCAGGCTCATC | CCCAATACCGCACAACCGACCTCACAG | GGTCCACCTCGCCAACA |
| 53 | SEQ ID NO: 33 | SEQ ID NO: 24 | SEQ ID NO: 21 |
| | GGCGCGTTCAGGCTCAT | CAATACCGCACAACCGACCTCACAGCC | GGTCCACCTCGCCAACA |
| 54 | SEQ ID NO: 34 | SEQ ID NO: 35 | SEQ ID NO: 36 |
| | GGGCTGTGAGGTCGGTTGT | CGTACTGCAGCCTGATTTGGTCCACCTCG | GCTCGACTTCCTGATGAATACGAAAGAT |
| 55 | SEQ ID NO: 37 | SEQ ID NO: 25 | SEQ ID NO: 3 |
| | GAGGTCGGTTGTGCGGTATTG | CTGCAGCCTGATTTGGTCCACCTCGC | CGACTTCCTGATGAATACGAAAGATTCC |
| 56 | SEQ ID NO: 33 | SEQ ID NO: 25 | SEQ ID NO: 3 |
| | GGCGCGTTCAGGCTCAT | CTGCAGCCTGATTTGGTCCACCTCGC | CGACTTCCTGATGAATACGAAAGATTCC |
| 57 | SEQ ID NO: 38 | SEQ ID NO: 25 | SEQ ID NO: 3 |
| | AGGTCGGTTGTGCGGTATTG | CTGCAGCCTGATTTGGTCCACCTCGC | CGACTTCCTGATGAATACGAAAGATTCC |
| 58 | SEQ ID NO: 39 | SEQ ID NO: 25 | SEQ ID NO: 3 |
| | TGAGGTCGGTTGTGCGGTATT | CTGCAGCCTGATTTGGTCCACCTCGC | CGACTTCCTGATGAATACGAAAGATTCC |
| 59 | SEQ ID NO: 40 | SEQ ID NO: 25 | SEQ ID NO: 3 |
| | GAGGTCGGTTGTGCGGTATT | CTGCAGCCTGATTTGGTCCACCTCGC | CGACTTCCTGATGAATACGAAAGATTCC |
| 60 | SEQ ID NO: 1 | SEQ ID NO: 25 | SEQ ID NO: 3 |
| | TTGTGCGGTATTGGGAAACAGT | CTGCAGCCTGATTTGGTCCACCTCGC | CGACTTCCTGATGAATACGAAAGATTCC |
| 61 | SEQ ID NO: 29 | SEQ ID NO: 25 | SEQ ID NO: 3 |
| | GGCGCGTTCAGGCTCATC | CTGCAGCCTGATTTGGTCCACCTCGC | CGACTTCCTGATGAATACGAAAGATTCC |
| 62 | SEQ ID NO: 37 | SEQ ID NO: 27 | SEQ ID NO: 3 |
| | GAGGTCGGTTGTGCGGTATTG | TGCAGCCTGATTTGGTCCACCTCGC | CGACTTCCTGATGAATACGAAAGATTCC |
| 63 | SEQ ID NO: 38 | SEQ ID NO: 27 | SEQ ID NO: 3 |
| | AGGTCGGTTGTGCGGTATTG | TGCAGCCTGATTTGGTCCACCTCGC | CGACTTCCTGATGAATACGAAAGATTCC |
| 64 | SEQ ID NO: 39 | SEQ ID NO: 27 | SEQ ID NO: 3 |
| | TGAGGTCGGTTGTGCGGTATT | TGCAGCCTGATTTGGTCCACCTCGC | CGACTTCCTGATGAATACGAAAGATTCC |
| 65 | SEQ ID NO: 40 | SEQ ID NO: 27 | SEQ ID NO: 3 |
| | GAGGTCGGTTGTGCGGTATT | TGCAGCCTGATTTGGTCCACCTCGC | CGACTTCCTGATGAATACGAAAGATTCC |
| 66 | SEQ ID NO: 33 | SEQ ID NO: 25 | SEQ ID NO: 5 |
| | GGCGCGTTCAGGCTCAT | CTGCAGCCTGATTTGGTCCACCTCGC | CTCGACTTCCTGATGAATACGAAAGATTC |
| 67 | SEQ ID NO: 40 | SEQ ID NO: 25 | SEQ ID NO: 5 |
| | GAGGTCGGTTGTGCGGTATT | CTGCAGCCTGATTTGGTCCACCTCGC | CTCGACTTCCTGATGAATACGAAAGATTC |
| 68 | SEQ ID NO: 37 | SEQ ID NO: 25 | SEQ ID NO: 5 |
| | GAGGTCGGTTGTGCGGTATTG | CTGCAGCCTGATTTGGTCCACCTCGC | CTCGACTTCCTGATGAATACGAAAGATTC |
| 69 | SEQ ID NO: 38 | SEQ ID NO: 25 | SEQ ID NO: 5 |
| | AGGTCGGTTGTGCGGTATTG | CTGCAGCCTGATTTGGTCCACCTCGC | CTCGACTTCCTGATGAATACGAAAGATTC |
| 70 | SEQ ID NO: 39 | SEQ ID NO: 25 | SEQ ID NO: 5 |
| | TGAGGTCGGTTGTGCGGTATT | CTGCAGCCTGATTTGGTCCACCTCGC | CTCGACTTCCTGATGAATACGAAAGATTC |
| 71 | SEQ ID NO: 1 | SEQ ID NO: 25 | SEQ ID NO: 5 |
| | TTGTGCGGTATTGGGAAACAGT | CTGCAGCCTGATTTGGTCCACCTCGC | CTCGACTTCCTGATGAATACGAAAGATTC |
| 72 | SEQ ID NO: 1 | SEQ ID NO: 27 | SEQ ID NO: 3 |
| | TTGTGCGGTATTGGGAAACAGT | TGCAGCCTGATTTGGTCCACCTCGC | CGACTTCCTGATGAATACGAAAGATTCC |
| 73 | SEQ ID NO: 29 | SEQ ID NO: 25 | SEQ ID NO: 5 |
| | GGCGCGTTCAGGCTCATC | CTGCAGCCTGATTTGGTCCACCTCGC | CTCGACTTCCTGATGAATACGAAAGATTC |
| 74 | SEQ ID NO: 38 | SEQ ID NO: 27 | SEQ ID NO: 5 |
| | AGGTCGGTTGTGCGGTATTG | TGCAGCCTGATTTGGTCCACCTCGC | CTCGACTTCCTGATGAATACGAAAGATTC |
| 75 | SEQ ID NO: 40 | SEQ ID NO: 27 | SEQ ID NO: 5 |
| | GAGGTCGGTTGTGCGGTATT | TGCAGCCTGATTTGGTCCACCTCGC | CTCGACTTCCTGATGAATACGAAAGATTC |
| 76 | SEQ ID NO: 37 | SEQ ID NO: 27 | SEQ ID NO: 5 |
| | GAGGTCGGTTGTGCGGTATTG | TGCAGCCTGATTTGGTCCACCTCGC | CTCGACTTCCTGATGAATACGAAAGATTC |
| 77 | SEQ ID NO: 39 | SEQ ID NO: 27 | SEQ ID NO: 5 |
| | TGAGGTCGGTTGTGCGGTATT | TGCAGCCTGATTTGGTCCACCTCGC | CTCGACTTCCTGATGAATACGAAAGATTC |
| 78 | SEQ ID NO: 1 | SEQ ID NO: 27 | SEQ ID NO: 5 |
| | TTGTGCGGTATTGGGAAACAGT | TGCAGCCTGATTTGGTCCACCTCGC | CTCGACTTCCTGATGAATACGAAAGATTC |
| 79 | SEQ ID NO: 1 | SEQ ID NO: 25 | SEQ ID NO: 32 |
| | TTGTGCGGTATTGGGAAACAGT | CTGCAGCCTGATTTGGTCCACCTCGC | GCTCGACTTCCTGATGAATACGAAA |
| 80 | SEQ ID NO: 38 | SEQ ID NO: 25 | SEQ ID NO: 32 |
| | AGGTCGGTTGTGCGGTATTG | CTGCAGCCTGATTTGGTCCACCTCGC | GCTCGACTTCCTGATGAATACGAAA |
| 81 | SEQ ID NO: 40 | SEQ ID NO: 25 | SEQ ID NO: 32 |
| | GAGGTCGGTTGTGCGGTATT | CTGCAGCCTGATTTGGTCCACCTCGC | GCTCGACTTCCTGATGAATACGAAA |
| 82 | SEQ ID NO: 33 | SEQ ID NO: 25 | SEQ ID NO: 32 |
| | GGCGCGTTCAGGCTCAT | CTGCAGCCTGATTTGGTCCACCTCGC | GCTCGACTTCCTGATGAATACGAAA |
| 83 | SEQ ID NO: 37 | SEQ ID NO: 25 | SEQ ID NO: 32 |
| | GAGGTCGGTTGTGCGGTATTG | CTGCAGCCTGATTTGGTCCACCTCGC | GCTCGACTTCCTGATGAATACGAAA |
| 84 | SEQ ID NO: 39 | SEQ ID NO: 25 | SEQ ID NO: 32 |
| | TGAGGTCGGTTGTGCGGTATT | CTGCAGCCTGATTTGGTCCACCTCGC | GCTCGACTTCCTGATGAATACGAAA |
| 85 | SEQ ID NO: 40 | SEQ ID NO: 27 | SEQ ID NO: 32 |
| | GAGGTCGGTTGTGCGGTATT | TGCAGCCTGATTTGGTCCACCTCGC | GCTCGACTTCCTGATGAATACGAAA |
| 86 | SEQ ID NO: 37 | SEQ ID NO: 27 | SEQ ID NO: 32 |
| | GAGGTCGGTTGTGCGGTATTG | TGCAGCCTGATTTGGTCCACCTCGC | GCTCGACTTCCTGATGAATACGAAA |
| 87 | SEQ ID NO: 1 | SEQ ID NO: 27 | SEQ ID NO: 32 |
| | TTGTGCGGTATTGGGAAACAGT | TGCAGCCTGATTTGGTCCACCTCGC | GCTCGACTTCCTGATGAATACGAAA |
| 88 | SEQ ID NO: 38 | SEQ ID NO: 27 | SEQ ID NO: 32 |
| | AGGTCGGTTGTGCGGTATTG | TGCAGCCTGATTTGGTCCACCTCGC | GCTCGACTTCCTGATGAATACGAAA |
| 89 | SEQ ID NO: 39 | SEQ ID NO: 27 | SEQ ID NO: 32 |
| | TGAGGTCGGTTGTGCGGTATT | TGCAGCCTGATTTGGTCCACCTCGC | GCTCGACTTCCTGATGAATACGAAA |
| 90 | SEO ID NO: 41 | SEQ ID NO: 20 | SEQ ID NO: 21 |
| | GGCAAGACAATATGACAGCAAA ATCTTAATTG | CCCAATACCGCACAACCGACCTCACAG | GGTCCACCTCGCCAACA |
| 91 | SEQ ID NO: 42 | SEQ ID NO: 43 | SEQ ID NO: 44 |
| | CAGCTACGTTTACCTATCCTGTTT TTGTTAAG | CCGGCGCGTTCAGGCTCATCCTT | GCCTGCTCAATTAAGATTTTGCTGTCAT |
| 92 | SEQ ID NO: 45 | SEQ ID NO: 46 | SEQ ID NO: 47 |
| | TGGCGAGGTGGACCAAATCA | ACTGCGTTTTCAGAGCCTTTTTCCGGCTC | GGTCTGCGGGAACGGTTAT |
| 93 | SEQ ID NO: 48 | SEQ ID NO: 49 | SEQ ID NO: 47 |
| | GTTGGCGAGGTGGACCAAAT | AGAGCCTTTTTCCGGCTCGACTTCCTGAT | GGTCTGCGGGAACGGTTAT |
| 94 | SEQ ID NO: 26 | SEQ ID NO: 50 | SEQ ID NO: 47 |
| | GGCTGTGAGGTCGGTTGTG | ACTGCGTTTTCAGAGCCTTTTTCCGGCTCG | GGTCTGCGGGAACGGTTAT |
| 95 | SEQ ID NO: 26 | SEQ ID NO: 27 | SEQ ID NO: 51 |
| | GGCTGTGAGGTCGGTTGTG | TGCAGCCTGATTTGGTCCACCTCGC | CTGCGTTTTCAGAGCCTTTTTCC |
| 96 | SEQ ID NO: 34 | SEQ ID NO: 50 | SEQ ID NO: 52 |
| | GGGCTGTGAGGTCGGTTGT | ACTGCGTTTTCAGAGCCTTTTTCCGGCTCG | AGGTCTGCGGGAACGGTTAT |
| 97 | SEQ ID NO: 53 | SEQ ID NO: 54 | SEQ ID NO: 47 |
| | AATCAGGCTGCAGTACGGAATCT TT | TGCGTTTTCAGAGCCTTTTTCCGGCTCG | GGTCTGCGGGAACGGTTAT |
| 98 | SEQ ID NO: 19 | SEQ ID NO: 2 | SEQ ID NO: 51 |
| | AGCAAAATCTTAATTGAGCAGGC TGTTT | TGATTTGGTCCACCTCGCCAACAACTAACGC | CTGCGTTTTCAGAGCCTTTTTCC |
| 99 | SEQ ID NO: 19 | SEQ ID NO: 56 | SEQ ID NO: 52 |
| | AGCAAAATCTTAATTGAGCAGGC TGTTT | TGCGTTTTCAGAGCCTTTTTCCGGCTCGAC | AGGTCTGCGGGAACGGTTAT |
| 100 | SEQ ID NO: 19 | SEQ ID NO: 50 | SEQ ID NO: 52 |
| | AGCAAAATCTTAATTGAGCAGGC TGTTT | ACTGCGTTTTCAGAGCCTTTTTCCGGCTCG | AGGTCTGCGGGAACGGTTAT |
| 101 | SEQ ID NO: 19 | SEQ ID NO: 57 | SEQ ID NO: 52 |
| | AGCAAAATCTTAATTGAGCAGGC TGTTT | AACTGCGTTTTCAGAGCCTTTTTCCGGCTCG | AGGTCTGCGGGAACGGTTAT |
| 102 | SEQ ID NO: 19 | SEQ ID NO: 54 | SEQ ID NO: 47 |
| | AGCAAAATCTTAATTGAGCAGGC TGTTT | TGCGTTTTCAGAGCCTTTTTCCGGCTCG | GGTCTGCGGGAACGGTTAT |
| 103 | SEQ ID NO: 19 | SEQ ID NO: 46 | SEQ ID NO: 47 |
| | AGCAAAATCTTAATTGAGCAGGC TGTTT | ACTGCGTTTTCAGAGCCTTTTTCCGGCTC | GGTCTGCGGGAACGGTTAT |
| 104 | SEQ ID NO: 19 | SEQ ID NO: 58 | SEQ ID NO: 3 |
| | AGCAAAATCTTAATTGAGCAGGC TGTTT | TGTTTCCCAATACCGCACAACCGACCTCAC | CGACTTCCTGATGAATACGAAAGATTCC |
| 105 | SEQ ID NO: 59 | SEQ ID NO: 56 | SEQ ID NO: 52 |
| | GCAGTACGGAATCTTTCGTATTC ATCAG | TGCGTTTTCAGAGCCTTTTTCCGGCTCGAC | AGGTCTGCGGGAACGGTTAT |
| 106 | SEQ ID NO: 59 | SEQ ID NO: 54 | SEQ ID NO: 60 |
| | GCAGTACGGAATCTTTCGTATTC ATCAG | TGCGTTTTCAGAGCCTTTTTCCGGCTCG | GGTCTGCGGGAACGGTTA |

| ***vanB* Sets** | | | |
|---|---|---|---|
| 107 | SEQ ID NO: 61 | SEQ ID NO: 62 | SEQ ID NO: 63 |
| | AAATCACTGGCCTACATTCTTAC A | TTCTATCGCAGCGTTAAGTTCTTCCGTACC | CGAAATCGCTTGCTCAATTAAGAT |
| 108 | SEQ ID NO: 61 | SEQ ID NO: 64 | SEQ ID NO: 65 |
| | AAATCACTGGCCTACATTCTTAC A | TATCGCAGCGTTAAGTTCTTCCGTACCGTTTA | GCTTGCTCAATTAAGATTTTTCCATCA |
| 109 | SEQ ID NO: 61 | SEQ ID NO: 64 | SEQ ID NO: 66 |
| | AAATCACTGGCCTACATTCTTAC A | TATCGCAGCGTTAAGTTCTTCCGTACCGTTTA | GCTCAATTAAGATTTTTCCATCATATTGTC |
| 110 | SEQ ID NO: 61 | SEQ ID NO: 62 | SEQ ID NO: 66 |
| | AAATCACTGGCCTACATTCTTAC A | TTCTATCGCAGCGTTAAGTTCTTCCGTACC | GCTCAATTAAGATTTTTCCATCATATTGTC |
| 111 | SEQ ID NO: 61 | SEQ ID NO: 67 | SEQ ID NO: 66 |
| | AAATCACTGGCCTACATTCTTAC A | CTTCTATCGCAGCGTTAAGTTCTTCCGTACC | GCTCAATTAAGATTTTTCCATCATATTGTC |
| 112 | SEQ ID NO: 68 | SEQ ID NO: 64 | SEQ ID NO: 65 |
| | AAATCACTGGCCTACATTCTTAC AAAA | TATCGCAGCGTTAAGTTCTTCCGTACCGTTTA | GCTTGCTCAATTAAGATTTTTCCATCA |

| **Group No.** | **Forward Primer** | **Probe** | **Reverse Primer** |
|---|---|---|---|
| 113 | SEQ ID NO: 68 | SEQ ID NO: 64 | SEQ ID NO: 66 |
| | AAATCACTGGCCTACATTCTTAC AAAA | TATCGCAGCGTTAAGTTCTTCCGTACCGTTTA | GCTCAATTAAGATTTTTCCATCATATTGTC |
| 114 | SEQ ID NO: 68 | SEQ ID NO: 62 | SEQ ID NO: 66 |
| | AAATCACTGGCCTACATTCTTAC AAAA | TTCTATCGCAGCGTTAAGTTCTTCCGTACC | GCTCAATTAAGATTTTTCCATCATATTGTC |
| 115 | SEQ ID NO: 68 | SEQ ID NO: 67 | SEQ ID NO: 66 |
| | AAATCACTGGCCTACATTCTTAC AAAA | CTTCTATCGCAGCGTTAAGTTCTTCCGTACC | GCTCAATTAAGATTTTTCCATCATATTGTC |
| 116 | SEQ ID NO: 61 | SEQ ID NO: 69 | SEQ ID NO: 63 |
| | AAATCACTGGCCTACATTCTTAC A | TTTTCCATCATATTGTCCTGCCGCTTCTATCG | CGAAATCGCTTGCTCAATTAAGAT |
| 117 | SEQ ID NO: 70 | SEQ ID NO: 69 | SEQ ID NO: 63 |
| | AAATCACTGGCCTACATTCTTAC AA | TTTTCCATCATATTGTCCTGCCGCTTCTATCG | CGAAATCGCTTGCTCAATTAAGAT |
| 118 | SEQ ID NO: 71 | SEQ ID NO: 69 | SEQ ID NO: 63 |
| | AAATCACTGGCCTACATTCTTAC AAA | TTTTCCATCATATTGTCCTGCCGCTTCTATCG | CGAAATCGCTTGCTCAATTAAGAT |
| 119 | SEQ ID NO: 68 | SEQ ID NO: 69 | SEQ ID NO: 63 |
| | AAATCACTGGCCTACATTCTTAC AAAA | TTTTCCATCATATTGTCCTGCCGCTTCTATCG | CGAAATCGCTTGCTCAATTAAGAT |
| 120 | SEQ ID NO: 72 | SEQ ID NO: 73 | SEQ ID NO: 74 |
| | AATCACTGGCCTACATTCTTACA A | AGATTTTTCCATCATATTGTCCTGCCGCTTCTAT | CCGAAATCGCTTGCTCAATTA |
| 121 | SEQ ID NO: 75 | SEQ ID NO: 73 | SEQ ID NO: 74 |
| | AATCACTGGCCTACATTCTTACA AA | AGATTTTTCCATCATATTGTCCTGCCGCTTCTAT | CCGAAATCGCTTGCTCAATTA |
| 122 | SEQ ID NO: 70 | SEQ ID NO: 76 | SEQ ID NO: 77 |
| | AAATCACTGGCCTACATTCTTAC AA | TAAGATTTTTCCATCATATTGTCCTGCCGCTTCT | CCGAAATCGCTTGCTCAAT |
| 123 | SEQ ID NO: 71 | SEQ ID NO: 76 | SEQ ID NO: 77 |
| | AAATCACTGGCCTACATTCTTAC AAA | TAAGATTTTTCCATCATATTGTCCTGCCGCTTCT | CCGAAATCGCTTGCTCAAT |
| 124 | SEQ ID NO: 61 | SEQ ID NO: 76 | SEQ ID NO: 77 |
| | AAATCACTGGCCTACATTCTTAC A | TAAGATTTTTCCATCATATTGTCCTGCCGCTTCT | CCGAAATCGCTTGCTCAAT |
| 125 | SEQ ID NO: 68 | SEQ ID NO: 76 | SEQ ID NO: 77 |
| | AAATCACTGGCCTACATTCTTAC AAAA | TAAGATTTTTCCATCATATTGTCCTGCCGCTTCT | CCGAAATCGCTTGCTCAAT |
| 126 | SEQ ID NO: 71 | SEQ ID NO: 78 | SEQ ID NO: 77 |
| | AAATCACTGGCCTACATTCTTAC AAA | AAGATTTTTCCATCATATTGTCCTGCCGCTTCT A | CCGAAATCGCTTGCTCAAT |
| 127 | SEQ ID NO: 61 | SEQ ID NO: 79 | SEQ ID NO: 77 |
| | AAATCACTGGCCTACATTCTTAC A | AAGATTTTTCCATCATATTGTCCTGCCGCTTCT | CCGAAATCGCTTGCTCAAT |
| 128 | SEQ ID NO: 70 | SEQ ID NO: 80 | SEQ ID NO: 77 |
| | AAATCACTGGCCTACATTCTTAC AA | AAGATTTTTCCATCATATTGTCCTGCCGCTTCT AT | CCGAAATCGCTTGCTCAAT |
| 129 | SEQ ID NO: 70 | SEQ ID NO: 79 | SEQ ID NO: 77 |
| | AAATCACTGGCCTACATTCTTAC AA | AAGATTTTTCCATCATATTGTCCTGCCGCTTCT | CCGAAATCGCTTGCTCAAT |
| 130 | SEQ ID NO: 71 | SEQ ID NO: 80 | SEQ ID NO: 77 |
| | AAATCACTGGCCTACATTCTTAC AAA | AAGATTTTTCCATCATATTGTCCTGCCGCTTCT AT | CCGAAATCGCTTGCTCAAT |
| 131 | SEQ ID NO: 61 | SEQ ID NO: 78 | SEQ ID NO: 77 |
| | AAATCACTGGCCTACATTCTTAC A | AAGATTTTTCCATCATATTGTCCTGCCGCTTCT A | CCGAAATCGCTTGCTCAAT |
| 132 | SEQ ID NO: 71 | SEQ ID NO: 79 | SEQ ID NO: 77 |
| | AAATCACTGGCCTACATTCTTAC AAA | AAGATTTTTCCATCATATTGTCCTGCCGCTTCT | CCGAAATCGCTTGCTCAAT |
| 133 | SEQ ID NO: 70 | SEQ ID NO: 78 | SEQ ID NO: 77 |
| | AAATCACTGGCCTACATTCTTAC AA | AAGATTTTTCCATCATATTGTCCTGCCGCTTCT A | CCGAAATCGCTTGCTCAAT |
| 134 | SEQ ID NO: 61 | SEQ ID NO: 80 | SEQ ID NO: 77 |
| | AAATCACTGGCCTACATTCTTAC A | AAGATTTTTCCATCATATTGTCCTGCCGCTTCT AT | CCGAAATCGCTTGCTCAAT |
| 135 | SEQ ID NO: 68 | SEQ ID NO: 80 | SEQ ID NO: 77 |
| | AAATCACTGGCCTACATTCTTAC AAAA | AAGATTTTTCCATCATATTGTCCTGCCGCTTCT AT | CCGAAATCGCTTGCTCAAT |
| 136 | SEQ ID NO: 68 | SEQ ID NO: 79 | SEQ ID NO: 77 |
| | AAATCACTGGCCTACATTCTTAC AAAA | AAGATTTTTCCATCATATTGTCCTGCCGCTTCT | CCGAAATCGCTTGCTCAAT |
| 137 | SEQ ID NO: 68 | SEQ ID NO: 78 | SEQ ID NO: 77 |
| | AAATCACTGGCCTACATTCTTAC | AAGATTTTTCCATCATATTGTCCTGCCGCTTCT | CCGAAATCGCTTGCTCAAT |
| | AAAA | A | |
| 138 | SEQ ID NO: 81 | SEQ ID NO: 82 | SEQ ID NO: 77 |
| | CTTACCTACCCTGTCTTTGTGA | AGATTTTTCCATCATATTGTCCTGCCGCTTCT | CCGAAATCGCTTGCTCAAT |
| 139 | SEQ ID NO: 83 | SEQ ID NO: 84 | SEQ ID NO: 85 |
| | TTACCTACCCTGTCTTTGTGAAG | CACGGTCAGGTTCGTCCTTTGGC | CGCTTGCTCAATTAAGATTTTTCCA |
| 140 | SEQ ID NO: 83 | SEQ ID NO: 84 | SEQ ID NO: 86 |
| | TTACCTACCCTGTCTTTGTGAAG | CACGGTCAGGTTCGTCCTTTGGC | CGCTTGCTCAATTAAGATTTTTCCAT |
| 141 | SEQ ID NO: 83 | SEQ ID NO: 84 | SEQ ID NO: 65 |
| | TTACCTACCCTGTCTTTGTGAAG | CACGGTCAGGTTCGTCCTTTGGC | GCTTGCTCAATTAAGATTTTTCCATCA |
| 142 | SEQ ID NO: 83 | SEQ ID NO: 84 | SEQ ID NO: 87 |
| | TTACCTACCCTGTCTTTGTGAAG | CACGGTCAGGTTCGTCCTTTGGC | GCTTGCTCAATTAAGATTTTTCCATCAT |
| 143 | SEQ ID NO: 83 | SEQ ID NO: 84 | SEQ ID NO: 88 |
| | TTACCTACCCTGTCTTTGTGAAG | CACGGTCAGGTTCGTCCTTTGGC | GCTTGCTCAATTAAGATTTTTCCATCATA |
| 144 | SEQ ID NO: 89 | SEQ ID NO: 84 | SEQ ID NO: 90 |
| | ACCTACCCTGTCTTTGTGAAG | CACGGTCAGGTTCGTCCTTTGGC | TCGCTTGCTCAATTAAGATTTTTCC |
| 145 | SEQ ID NO: 89 | SEQ ID NO: 84 | SEQ ID NO: 85 |
| | ACCTACCCTGTCTTTGTGAAG | CACGGTCAGGTTCGTCCTTTGGC | CGCTTGCTCAATTAAGATTTTTCCA |
| 146 | SEQ ID NO: 89 | SEQ ID NO: 84 | SEQ ID NO: 86 |
| | ACCTACCCTGTCTTTGTGAAG | CACGGTCAGGTTCGTCCTTTGGC | CGCTTGCTCAATTAAGATTTTTCCAT |
| 147 | SEQ ID NO: 89 | SEQ ID NO: 84 | SEQ ID NO: 65 |
| | ACCTACCCTGTCTTTGTGAAG | CACGGTCAGGTTCGTCCTTTGGC | GCTTGCTCAATTAAGATTTTTCCATCA |
| 148 | SEQ ID NO: 89 | SEQ ID NO: 84 | SEQ ID NO: 87 |
| | ACCTACCCTGTCTTTGTGAAG | CACGGTCAGGTTCGTCCTTTGGC | GCTTGCTCAATTAAGATTTTTCCATCAT |
| 149 | SEQ ID NO: 89 | SEQ ID NO: 84 | SEQ ID NO: 91 |
| | ACCTACCCTGTCTTTGTGAAG | CACGGTCAGGTTCGTCCTTTGGC | ATCGCTTGCTCAATTAAGATTTTTCC |
| 150 | SEQ ID NO: 89 | SEQ ID NO: 84 | SEQ ID NO: 88 |
| | ACCTACCCTGTCTTTGTGAAG | CACGGTCAGGTTCGTCCTTTGGC | GCTTGCTCAATTAAGATTTTTCCATCATA |
| 151 | SEQ ID NO: 61 | SEQ ID NO: 92 | SEQ ID NO: 74 |
| | AAATCACTGGCCTACATTCTTAC A | TTTTTCCATCATATTGTCCTGCCGCTTCTATCG | CCGAAATCGCTTGCTCAATTA |
| 152 | SEQ ID NO: 70 | SEQ ID NO: 92 | SEQ ID NO: 74 |
| | AAATCACTGGCCTACATTCTTAC AA | TTTTTCCATCATATTGTCCTGCCGCTTCTATCG | CCGAAATCGCTTGCTCAATTA |
| 153 | SEQ ID NO: 71 | SEQ ID NO: 92 | SEQ ID NO: 74 |
| | AAATCACTGGCCTACATTCTTAC AAA | TTTTTCCATCATATTGTCCTGCCGCTTCTATCG | CCGAAATCGCTTGCTCAATTA |
| 154 | SEQ ID NO: 68 | SEQ ID NO: 92 | SEQ ID NO: 74 |
| | AAATCACTGGCCTACATTCTTAC AAAA | TTTTTCCATCATATTGTCCTGCCGCTTCTATCG | CCGAAATCGCTTGCTCAATTA |
| 155 | SEQ ID NO: 93 | SEQ ID NO: 84 | SEQ ID NO: 65 |
| | TACCTACCCTGTCTTTGTGAAG | CACGGTCAGGTTCGTCCTTTGGC | GCTTGCTCAATTAAGATTTTTCCATCA |
| 156 | SEQ ID NO: 93 | SEQ ID NO: 84 | SEQ ID NO: 87 |
| | TACCTACCCTGTCTTTGTGAAG | CACGGTCAGGTTCGTCCTTTGGC | GCTTGCTCAATTAAGATTTTTCCATCAT |
| 157 | SEQ ID NO: 93 | SEQ ID NO: 84 | SEQ ID NO: 88 |
| | TACCTACCCTGTCTTTGTGAAG | CACGGTCAGGTTCGTCCTTTGGC | GCTTGCTCAATTAAGATTTTTCCATCATA |
| 158 | SEQ ID NO: 93 | SEQ ID NO: 84 | SEQ ID NO: 90 |
| | TACCTACCCTGTCTTTGTGAAG | CACGGTCAGGTTCGTCCTTTGGC | TCGCTTGCTCAATTAAGATTTTTCC |
| 159 | SEQ ID NO: 93 | SEQ ID NO: 84 | SEQ ID NO: 85 |
| | TACCTACCCTGTCTTTGTGAAG | CACGGTCAGGTTCGTCCTTTGGC | CGCTTGCTCAATTAAGATTTTTCCA |
| 160 | SEQ ID NO: 93 | SEQ ID NO: 84 | SEQ ID NO: 86 |
| | TACCTACCCTGTCTTTGTGAAG | CACGGTCAGGTTCGTCCTTTGGC | CGCTTGCTCAATTAAGATTTTTCCAT |
| 161 | SEQ ID NO: 94 | SEQ ID NO: 84 | SEQ ID NO: 65 |
| | CTTACCTACCCTGTCTTTGTGAA | CACGGTCAGGTTCGTCCTTTGGC | GCTTGCTCAATTAAGATTTTTCCATCA |
| 162 | SEQ ID NO: 94 | SEQ ID NO: 84 | SEQ ID NO: 87 |
| | CTTACCTACCCTGTCTTTGTGAA | CACGGTCAGGTTCGTCCTTTGGC | GCTTGCTCAATTAAGATTTTTCCATCAT |
| 163 | SEQ ID NO: 94 | SEQ ID NO: 84 | SEQ ID NO: 88 |
| | CTTACCTACCCTGTCTTTGTGAA | CACGGTCAGGTTCGTCCTTTGGC | GCTTGCTCAATTAAGATTTTTCCATCATA |
| 164 | SEQ ID NO: 83 | SEQ ID NO: 84 | SEQ ID NO: 95 |
| | TTACCTACCCTGTCTTTGTGAAG | CACGGTCAGGTTCGTCCTTTGGC | CTTGCTCAATTAAGATTTTTCCATCATATTGTC |
| 165 | SEQ ID NO: 61 | SEQ ID NO: 96 | SEQ ID NO: 97 |
| | AAATCACTGGCCTACATTCTTAC A | CGCCTCCGGCTTGTCACCTTT | ACAAAGACAGGGTAGGTAAGC |
| 166 | SEQ ID NO: 89 | SEQ ID NO: 84 | SEQ ID NO: 95 |
| | ACCTACCCTGTCTTTGTGAAG | CACGGTCAGGTTCGTCCTTTGGC | CTTGCTCAATTAAGATTTTTCCATCATATTGTC |
| 167 | SEQ ID NO: 89 | SEQ ID NO: 84 | SEQ ID NO: 66 |
| | ACCTACCCTGTCTTTGTGAAG | CACGGTCAGGTTCGTCCTTTGGC | GCTCAATTAAGATTTTTCCATCATATTGTC |
| 168 | SEQ ID NO: 83 | SEQ ID NO: 84 | SEQ ID NO: 66 |
| | TTACCTACCCTGTCTTTGTGAAG | CACGGTCAGGTTCGTCCTTTGGC | GCTCAATTAAGATTTTTCCATCATATTGTC |
| 169 | SEQ ID NO: 93 | SEQ ID NO: 84 | SEQ ID NO: 66 |
| | TACCTACCCTGTCTTTGTGAAG | CACGGTCAGGTTCGTCCTTTGGC | GCTCAATTAAGATTTTTCCATCATATTGTC |
| 170 | SEQ ID NO: 89 | SEQ ID NO: 84 | SEQ ID NO: 98 |
| | ACCTACCCTGTCTTTGTGAAG | CACGGTCAGGTTCGTCCTTTGGC | TCGCTTGCTCAATTAAGATTTTTCCA |
| 171 | SEQ ID NO: 61 | SEQ ID NO: 69 | SEQ ID NO: 74 |
| | AAATCACTGGCCTACATTCTTAC A | TTTTCCATCATATTGTCCTGCCGCTTCTATCG | CCGAAATCGCTTGCTCAATTA |
| 172 | SEQ ID NO: 70 | SEQ ID NO: 69 | SEQ ID NO: 74 |
| | AAATCACTGGCCTACATTCTTAC AA | TTTTCCATCATATTGTCCTGCCGCTTCTATCG | CCGAAATCGCTTGCTCAATTA |
| 173 | SEQ ID NO: 71 | SEQ ID NO: 69 | SEQ ID NO: 74 |
| | AAATCACTGGCCTACATTCTTAC AAA | TTTTCCATCATATTGTCCTGCCGCTTCTATCG | CCGAAATCGCTTGCTCAATTA |
| 174 | SEQ ID NO: 89 | SEQ ID NO: 84 | SEQ ID NO: 99 |
| | ACCTACCCTGTCTTTGTGAAG | CACGGTCAGGTTCGTCCTTTGGC | ATCGCTTGCTCAATTAAGATTTTTCCA |
| 175 | SEQ ID NO: 68 | SEQ ID NO: 69 | SEQ ID NO: 74 |
| | AAATCACTGGCCTACATTCTTAC AAAA | TTTTCCATCATATTGTCCTGCCGCTTCTATCG | CCGAAATCGCTTGCTCAATTA |
| 176 | SEQ ID NO: 89 | SEQ ID NO: 84 | SEQ ID NO: 100 |
| | ACCTACCCTGTCTTTGTGAAG | CACGGTCAGGTTCGTCCTTTGGC | TCGCTTGCTCAATTAAGATTTTTCCAT |
| 177 | SEQ ID NO: 93 | SEQ ID NO: 84 | SEQ ID NO: 95 |
| | TACCTACCCTGTCTTTGTGAAG | CACGGTCAGGTTCGTCCTTTGGC | CTTGCTCAATTAAGATTTTTCCATCATATTGTC |
| 178 | SEQ ID NO: 89 | SEQ ID NO: 84 | SEQ ID NO: 101 |
| | ACCTACCCTGTCTTTGTGAAG | CACGGTCAGGTTCGTCCTTTGGC | CGCTTGCTCAATTAAGATTTTTCC |
| 179 | SEQ ID NO: 83 | SEQ ID NO: 84 | SEQ ID NO: 101 |
| | TTACCTACCCTGTCTTTGTGAAG | CACGGTCAGGTTCGTCCTTTGGC | CGCTTGCTCAATTAAGATTTTTCC |
| 180 | SEQ ID NO: 93 | SEQ ID NO: 84 | SEQ ID NO: 101 |
| | TACCTACCCTGTCTTTGTGAAG | CACGGTCAGGTTCGTCCTTTGGC | CGCTTGCTCAATTAAGATTTTTCC |
| 181 | SEQ ID NO: 94 | SEQ ID NO: 84 | SEQ ID NO: 95 |
| | CTTACCTACCCTGTCTTTGTGAA | CACGGTCAGGTTCGTCCTTTGGC | CTTGCTCAATTAAGATTTTTCCATCATATTGTC |
| 182 | SEQ ID NO: 89 | SEQ ID NO: 84 | SEQ ID NO: 102 |
| | ACCTACCCTGTCTTTGTGAAG | CACGGTCAGGTTCGTCCTTTGGC | ATCGCTTGCTCAATTAAGATTTTTCCAT |
| 183 | SEQ ID NO: 93 | SEQ ID NO: 84 | SEQ ID NO: 98 |
| | TACCTACCCTGTCTTTGTGAAG | CACGGTCAGGTTCGTCCTTTGGC | TCGCTTGCTCAATTAAGATTTTTCCA |
| 184 | SEQ ID NO: 94 | SEQ ID NO: 84 | SEQ ID NO: 66 |
| | CTTACCTACCCTGTCTTTGTGAA | CACGGTCAGGTTCGTCCTTTGGC | GCTCAATTAAGATTTTTCCATCATATTGTC |
| 185 | SEQ ID NO: 61 | SEQ ID NO: 92 | SEQ ID NO: 77 |
| | AAATCACTGGCCTACATTCTTAC A | TTTTTCCATCATATTGTCCTGCCGCTTCTATCG | CCGAAATCGCTTGCTCAAT |
| 186 | SEQ ID NO: 70 | SEQ ID NO: 92 | SEQ ID NO: 77 |
| | AAATCACTGGCCTACATTCTTAC AA | TTTTTCCATCATATTGTCCTGCCGCTTCTATCG | CCGAAATCGCTTGCTCAAT |
| 187 | SEQ ID NO: 71 | SEQ ID NO: 92 | SEQ ID NO: 77 |
| | AAATCACTGGCCTACATTCTTAC AAA | TTTTTCCATCATATTGTCCTGCCGCTTCTATCG | CCGAAATCGCTTGCTCAAT |
| 188 | SEQ ID NO: 81 | SEQ ID NO: 84 | SEQ ID NO: 88 |
| | CTTACCTACCCTGTCTTTGTGA | CACGGTCAGGTTCGTCCTTTGGC | GCTTGCTCAATTAAGATTTTTCCATCATA |
| 189 | SEQ ID NO: 81 | SEQ ID NO: 84 | SEQ ID NO: 65 |
| | CTTACCTACCCTGTCTTTGTGA | CACGGTCAGGTTCGTCCTTTGGC | GCTTGCTCAATTAAGATTTTTCCATCA |
| 190 | SEQ ID NO: 81 | SEQ ID NO: 84 | SEQ ID NO: 87 |
| | CTTACCTACCCTGTCTTTGTGA | CACGGTCAGGTTCGTCCTTTGGC | GCTTGCTCAATTAAGATTTTTCCATCAT |
| 191 | SEQ ID NO: 68 | SEQ ID NO: 92 | SEQ ID NO: 77 |
| | AAATCACTGGCCTACATTCTTAC AAAA | TTTTTCCATCATATTGTCCTGCCGCTTCTATCG | CCGAAATCGCTTGCTCAAT |
| 192 | SEQ ID NO: 103 | SEQ ID NO: 84 | SEQ ID NO: 86 |
| | TTACCTACCCTGTCTTTGTGAA | CACGGTCAGGTTCGTCCTTTGGC | CGCTTGCTCAATTAAGATTTTTCCAT |
| 193 | SEQ ID NO: 103 | SEQ ID NO: 84 | SEQ ID NO: 66 |
| | TTACCTACCCTGTCTTTGTGAA | CACGGTCAGGTTCGTCCTTTGGC | GCTCAATTAAGATTTTTCCATCATATTGTC |
| 194 | SEQ ID NO: 103 | SEQ ID NO: 84 | SEQ ID NO: 65 |
| | TTACCTACCCTGTCTTTGTGAA | CACGGTCAGGTTCGTCCTTTGGC | GCTTGCTCAATTAAGATTTTTCCATCA |
| 195 | SEQ ID NO: 103 | SEQ ID NO: 84 | SEQ ID NO: 87 |
| | TTACCTACCCTGTCTTTGTGAA | CACGGTCAGGTTCGTCCTTTGGC | GCTTGCTCAATTAAGATTTTTCCATCAT |
| 196 | SEQ ID NO: 103 | SEQ ID NO: 84 | SEQ ID NO: 88 |
| | TTACCTACCCTGTCTTTGTGAA | CACGGTCAGGTTCGTCCTTTGGC | GCTTGCTCAATTAAGATTTTTCCATCATA |
| 197 | SEQ ID NO: 81 | SEQ ID NO: 84 | SEQ ID NO: 66 |
| | CTTACCTACCCTGTCTTTGTGA | CACGGTCAGGTTCGTCCTTTGGC | GCTCAATTAAGATTTTTCCATCATATTGTC |
| 198 | SEQ ID NO: 71 | SEQ ID NO: 69 | SEQ ID NO: 77 |
| | AAATCACTGGCCTACATTCTTAC AAA | TTTTCCATCATATTGTCCTGCCGCTTCTATCG | CCGAAATCGCTTGCTCAAT |
| 199 | SEQ ID NO: 61 | SEQ ID NO: 69 | SEQ ID NO: 77 |
| | AAATCACTGGCCTACATTCTTAC | TTTTCCATCATATTGTCCTGCCGCTTCTATCG | CCGAAATCGCTTGCTCAAT |
| | A | | |
| 200 | SEQ ID NO: 70 | SEQ ID NO: 69 | SEQ ID NO: 77 |
| | AAATCACTGGCCTACATTCTTAC AA | TTTTCCATCATATTGTCCTGCCGCTTCTATCG | CCGAAATCGCTTGCTCAAT |
| 201 | SEQ ID NO: 81 | SEQ ID NO: 84 | SEQ ID NO: 95 |
| | CTTACCTACCCTGTCTTTGTGA | CACGGTCAGGTTCGTCCTTTGGC | CTTGCTCAATTAAGATTTTTCCATCATATTGTC |
| 202 | SEQ ID NO: 68 | SEQ ID NO: 69 | SEQ ID NO: 77 |
| | AAATCACTGGCCTACATTCTTAC AAAA | TTTTCCATCATATTGTCCTGCCGCTTCTATCG | CCGAAATCGCTTGCTCAAT |
| 203 | SEQ ID NO: 104 | SEQ ID NO: 84 | SEQ ID NO: 66 |
| | GCTTACCTACCCTGTCTTTGT | CACGGTCAGGTTCGTCCTTTGGC | GCTCAATTAAGATTTTTCCATCATATTGTC |
| 204 | SEQ ID NO: 105 | SEQ ID NO: 84 | SEQ ID NO: 66 |
| | CGCTTACCTACCCTGTCTTT | CACGGTCAGGTTCGTCCTTTGGC | GCTCAATTAAGATTTTTCCATCATATTGTC |
| 205 | SEQ ID NO: 89 | SEQ ID NO: 106 | SEQ ID NO: 66 |
| | ACCTACCCTGTCTTTGTGAAG | AGGTTCGTCCTTTGGCGTAACCAAAGTAAAC | GCTCAATTAAGATTTTTCCATCATATTGTC |
| 206 | SEQ ID NO: 107 | SEQ ID NO: 84 | SEQ ID NO: 66 |
| | CGCTTACCTACCCTGTCTT | CACGGTCAGGTTCGTCCTTTGGC | GCTCAATTAAGATTTTTCCATCATATTGTC |
| 207 | SEQ ID NO: 103 | SEQ ID NO: 108 | SEQ ID NO: 66 |
| | TTACCTACCCTGTCTTTGTGAA | CAGGTTCGTCCTTTGGCGTAACCA | GCTCAATTAAGATTTTTCCATCATATTGTC |
| 208 | SEQ ID NO: 93 | SEQ ID NO: 109 | SEQ ID NO: 66 |
| | TACCTACCCTGTCTTTGTGAAG | CCAAAGGACGAACCTGACCGTGC | GCTCAATTAAGATTTTTCCATCATATTGTC |
| 209 | SEQ ID NO: 89 | SEQ ID NO: 110 | SEQ ID NO: 66 |
| | ACCTACCCTGTCTTTGTGAAG | AGGACGAACCTGACCGTGCC | GCTCAATTAAGATTTTTCCATCATATTGTC |
| 210 | SEQ ID NO: 111 | SEQ ID NO: 112 | SEQ ID NO: 63 |
| | ATCACTGGCCTACATTCTTACA | CGCTTACCTACCCTGTCTTTGTGAAGC | CGAAATCGCTTGCTCAATTAAGAT |
| 211 | SEQ ID NO: 111 | SEQ ID NO: 112 | SEQ ID NO: 66 |
| | ATCACTGGCCTACATTCTTACA | CGCTTACCTACCCTGTCTTTGTGAAGC | GCTCAATTAAGATTTTTCCATCATATTGTC |
| 212 | SEQ ID NO: 111 | SEQ ID NO: 112 | SEQ ID NO: 88 |
| | ATCACTGGCCTACATTCTTACA | CGCTTACCTACCCTGTCTTTGTGAAGC | GCTTGCTCAATTAAGATTTTTCCATCATA |

A PCR primer set for amplifying a *vanA* gene comprises at least one of the following sets of primer sequences: (1) SEQ ID NOS: 1 and 3; (2) SEQ ID NOS: 1 and 32; (3) SEQ ID NOS: 1 and 5; (4) SEQ ID NOS: 19 and 21; (5) SEQ ID NOS: 19 and 3; (6) SEQ ID NOS: 19 and 32; (7) SEQ ID NOS: 19 and 47; (8) SEQ ID NOS: 19 and 5; (9) SEQ ID NOS: 19 and 52; (10) SEQ ID NOS: 19 and 55; (11) SEQ ID NOS: 22 and 21; (12) SEQ ID NOS: 22 and 3; (13) SEQ ID NOS: 22 and 32; (14) SEQ ID NOS: 22 and 5; (15) SEQ ID NOS: 23 and 21; (16) SEQ ID NOS: 23 and 3; (17) SEQ ID NOS: 23 and 32; (18) SEQ ID NOS: 23 and 5; (19) SEQ ID NOS: 26 and 3; (20) SEQ ID NOS: 26 and 32; (21) SEQ ID NOS: 26 and 47; (22) SEQ ID NOS: 26 and 5; (23) SEQ ID NOS: 26 and 51; (24) SEQ ID NOS: 28 and 3; (25) SEQ ID NOS: 28 and 32; (26) SEQ ID NOS: 28 and 5; (27) SEQ ID NOS: 29 and 21; (28) SEQ ID NOS: 29 and 3; (29) SEQ ID NOS: 29 and 5; (30) SEQ ID NOS: 29 and 8; (31) SEQ ID NOS: 33 and 21; (32) SEQ ID NOS: 33 and 3; (33) SEQ ID NOS: 33 and 32; (34) SEQ ID NOS: 33 and 5; (35) SEQ ID NOS: 34 and 36; (36) SEQ ID NOS: 34 and 52; (37) SEQ ID NOS: 37 and 3; (38) SEQ ID NOS: 37 and 32; (39) SEQ ID NOS: 37 and 5; (40) SEQ ID NOS: 38 and 3; (41) SEQ ID NOS: 38 and 32; (42) SEQ ID NOS: 38 and 5; (43) SEQ ID NOS: 39 and 3; (44) SEQ ID NOS: 39 and 32; (45) SEQ ID NOS: 39 and 5; (46) SEQ ID NOS: 40 and 3; (47) SEQ ID NOS: 40 and 32; (48) SEQ ID NOS: 40 and 5; (49) SEQ ID NOS: 41 and 21; (50) SEQ ID NOS: 42 and 44; (51) SEQ ID NOS: 45 and 47; (52) SEQ ID NOS: 48 and 47; (53) SEQ ID NOS: 53 and 47; (54) SEQ ID NOS: 59 and 52; (55) SEQ ID NOS: 59 and 60; (56) SEQ ID NOS: 6 and 10; (57) SEQ ID NOS: 6 and 21; (58) SEQ ID NOS: 6 and 3; (59) SEQ ID NOS: 6 and 31; (60) SEQ ID NOS: 6 and 32; (61) SEQ ID NOS: 6 and 5; and (62) SEQ ID NOS: 6 and 8.

The preceding numbering of the 62 sets of primers does not correspond exactly to the "Group" numbering scheme in Table 5 because certain groups use the same primer set, but different internal probes. For example, Groups 1 and 2 of Table 5 each employ the forward primer of SEQ ID NO: 1 and the reverse primer of SEQ ID NO: 3, but different internal probes in each instance, e.g., SEQ ID NOS: 2 and 4. Accordingly, primer set "(1)" of the preceding passage implies any one of Groups 1 or 2 of Table 5.

A probe for binding to an amplicon(s) of a *vanA* gene, or to a *vanA* gene target, comprises at least one of the following probe sequences: SEQ ID NOS: 2, 4, 7, 9, 11-18, 20, 24, 25, 27, 30, 35, 43, 46, 49, 50, 54, 56, 57, 58 (*vanA* probes).

A PCR primer set for amplifying a *vanB* gene comprises at least one of the following sets of primer sequences: (1) SEQ ID NOS: 103 and 65; (2) SEQ ID NOS: 103 and 66; (3) SEQ ID NOS: 103 and 86; (4) SEQ ID NOS: 103 and 87; (5) SEQ ID NOS: 103 and 88; (6) SEQ ID NOS: 104 and 66; (7) SEQ ID NOS: 105 and 66; (8) SEQ ID NOS: 107 and 66; (9) SEQ ID NOS: 111 and 63; (10) SEQ ID NOS: 111 and 66; (11) SEQ ID NOS: 111 and 88; (12) SEQ ID NOS: 61 and 63; (13) SEQ ID NOS: 61 and 65; (14) SEQ ID NOS: 61 and 66; (15) SEQ ID NOS: 61 and 74; (16) SEQ ID NOS: 61 and 77; (17) SEQ ID NOS: 61 and 97; (18) SEQ ID NOS: 68 and 63; (19) SEQ ID NOS: 68 and 65; (20) SEQ ID NOS: 68 and 66; (21) SEQ ID NOS: 68 and 74; (22) SEQ ID NOS: 68 and 77; (23) SEQ ID NOS: 70 and 63; (24) SEQ ID NOS: 70 and 74; (25) SEQ ID NOS: 70 and 77; (26) SEQ ID NOS: 71 and 63; (27) SEQ ID NOS: 71 and 74; (28) SEQ ID NOS: 71 and 77; (29) SEQ ID NOS: 72 and 74; (30) SEQ ID NOS: 75 and 74; (31) SEQ ID NOS: 81 and 65; (32) SEQ ID NOS: 81 and 66; (33) SEQ ID NOS: 81 and 77; (34) SEQ ID NOS: 81 and 87; (35) SEQ ID NOS: 81 and 88; (36) SEQ ID NOS: 81 and 95; (37) SEQ ID NOS: 83 and 101; (38) SEQ ID NOS: 83 and 65; (39) SEQ ID NOS: 83 and 66; (40) SEQ ID NOS: 83 and 85; (41) SEQ ID NOS: 83 and 86; (42) SEQ ID NOS: 83 and 87; (43) SEQ ID NOS: 83 and 88; (44) SEQ ID NOS: 83 and 95; (45) SEQ ID NOS: 89 and 100; (46) SEQ ID NOS: 89 and 101; (47) SEQ ID NOS: 89 and 102; (48) SEQ ID NOS: 89 and 65; (49) SEQ ID NOS: 89 and 66; (50) SEQ ID NOS: 89 and 85; (51) SEQ ID NOS: 89 and 86; (52) SEQ ID NOS: 89 and 87; (53) SEQ ID NOS: 89 and 88; (54) SEQ ID NOS: 89 and 90; (55) SEQ ID NOS: 89 and 91; (56) SEQ ID NOS: 89 and 95; (57) SEQ ID NOS: 89 and 98; (58) SEQ ID NOS: 89 and 99; (59) SEQ ID NOS: 93 and 101; (60) SEQ ID NOS: 93 and 65; (61) SEQ ID NOS: 93 and 66; (62) SEQ ID NOS: 93 and 85; (63) SEQ ID NOS: 93 and 86; (64) SEQ ID NOS: 93 and 87; (65) SEQ ID NOS: 93 and 88; (66) SEQ ID NOS: 93 and 90; (67) SEQ ID NOS: 93 and 95; (68) SEQ ID NOS: 93 and 98; (69) SEQ ID NOS: 94 and 65; (70) SEQ ID NOS: 94 and 66; (71) SEQ ID NOS: 94 and 87; (72) SEQ ID NOS: 94 and 88; and (73) SEQ ID NOS: 94 and 95.

The preceding numbering of the 73 sets of primers does not correspond exactly to the "Group" numbering scheme in Table 5 because certain groups use the same primer set, but different internal probes. For example, Groups 109-111 of Table 5 each employ the forward primer of SEQ ID NO: 61 and the reverse primer of SEQ ID NO: 66, but different internal probes in each instance, e.g., SEQ ID NOS: 64, 62, and 67, respectively. Accordingly, primer set "(14)" of the preceding passage implies any one of Groups 109-111 of Table 5.

A probe for binding to an amplicon(s) of a *vanB* gene, or to a *vanB* gene target, comprises at least one of the following probe sequences: SEQ ID NOS: 62, 64, 67, 69, 73, 76, 78, 79, 80, 82, 84, 92, 96, 108, 109, 110 and 112 (*vanB* probes).

Any set of primers can be used simultaneously in a multiplex reaction with one or more other primer sets, so that multiple amplicons are amplified simultaneously.

**Table 6. Optimized Primers and Probes for the Detection of vanC, vanD, vanE and vanG Resistance Genes.**

| **Group No.** | **Forward Primer** | **Probe** | **Reverse Primer** |
|---|---|---|---|
| ***vanC1* Sets** | | | |
| 213 | SEQ ID NO: 123 | SEQ ID NO: 124 | SEQ ID NO: 125 |
| | ATGTATGAACAAATGGCTCTTGCATCA AC | CGCTAGTGCTCCCACTTTGCTTTTATCCCGCT A | GATCAACACAGTAGAACCGTAAGCAAAAG |
| 214 | SEQ ID NO: 123 | SEQ ID NO: 126 | SEQ ID NO: 125 |
| | ATGTATGAACAAATGGCTCTTGCATCA AC | CGCTAGTGCTCCCACTTTGCTTTTATCCCGC | GATCAACACAGTAGAACCGTAAGCAAAAG |
| 215 | SEQ ID NO: 127 | SEQ ID NO: 128 | SEQ ID NO: 129 |
| | AAAAGGGATCACAAAAGTAACTGACA AAACAG | CGCAGCCAATTTCAATACCCGCTATCGCC | CCAATCGTCAATTGCTCATTTCCTAAGAT |
| 216 | SEQ ID NO: 130 | SEQ ID NO: 131 | SEQ ID NO: 132 |
| | TCGATCGTTTTATTCAAGACCATGGAT TC | CCCTTTTGAAGAACCGGCTTCATTCGGCT | GATCAACACAGTAGAACCGTAAGCA |
| 217 | SEQ ID NO: 133 | SEQ ID NO: 134 | SEQ ID NO: 135 |
| | AGGGATCACAAAAGTAACTGACAAAA CAG | TGCCGCAGCCAATTTCAATACCCGCTA | ACCAATCGTCAATTGCTCATTTCCTA |
| 218 | SEQ ID NO: 133 | SEQ ID NO: 136 | SEQ ID NO: 137 |
| | AGGGATCACAAAAGTAACTGACAAAA CAG | AAGATGCCGCAGCCAATTTCAATACCCGC | ACCAATCGTCAATTGCTCATTTCCT |
| 219 | SEQ ID NO: 138 | SEQ ID NO: 139 | SEQ ID NO: 140 |
| | TCGATCGTTTTATTCAAGACCATGGAT TC | CCCTTTTGAAGAACCGGCTTCATTCGGCT | ACCGTAAGCAAAAGCAGTCGTTA |
| 220 | SEQ ID NO: 141 | SEQ ID NO: 142 | SEQ ID NO: 143 |
| | TTAACGACTGCTTTTGCTTACGGTTCT | ACCCGCTATCGCCTTTTGGATCAACACAGT | GTCGACAAGAGAAATCGCATCACA |
| 221 | SEQ ID NO: 144 | SEQ ID NO: 145 | SEQ ID NO: 146 |
| | CTTTTGCTTACGGTTCTACTGTGTTGA | CGCAGCCAATTTCAATACCCGCTATCGCC | ACAAGAGAAATCGCATCACAAGCA |
| 222 | SEQ ID NO: 141 | SEQ ID NO: 142 | SEQ ID NO: 147 |
| | TTAACGACTGCTTTTGCTTACGGTTCT | ACCCGCTATCGCCTTTTGGATCAACACAGT | CGCATCACAAGCACCAATCG |
| 223 | SEQ ID NO: 148 | SEQ ID NO: 136 | SEQ ID NO: 137 |
| | TCTGCATTAACGACTGCTTTTGCT | AAGATGCCGCAGCCAATTTCAATACCCGC | ACCAATCGTCAATTGCTCATTTCCT |
| 224 | SEQ ID NO: 148 | SEQ ID NO: 149 | SEQ ID NO: 150 |
| | TCTGCATTAACGACTGCTTTTGCT | TGCCGCAGCCAATTTCAATACCCGCTA | ACCAATCGTCAATTGCTCATTTCCTA |
| 225 | SEQ ID NO: 144 | SEQ ID NO: 145 | SEQ ID NO: 147 |
| | CTTTTGCTTACGGTTCTACTGTGTTGA | CGCAGCCAATTTCAATACCCGCTATCGCC | CGCATCACAAGCACCAATCG |
| 226 | SEQ ID NO: 141 | SEQ ID NO: 136 | SEQ ID NO: 137 |
| | TTAACGACTGCTTTTGCTTACGGTTCT | AAGATGCCGCAGCCAATTTCAATACCCGC | ACCAATCGTCAATTGCTCATTTCCT |
| 227 | SEQ ID NO: 151 | SEQ ID NO: 152 | SEQ ID NO: 153 |
| | GCTTTTGCTTACGGTTCTACTGTGTT | AGATGCCGCAGCCAATTTCAATACCCGC | CACCAATCGTCAATTGCTCATTTCCTA |
| 228 | SEQ ID NO: 151 | SEQ ID NO: 154 | SEQ ID NO: 155 |
| | GCTTTTGCTTACGGTTCTACTGTGTT | CGCAGCCAATTTCAATACCCGCTATCGCCTTT | CACCAATCGTCAATTGCTCATTTCCTAA |
| 229 | SEQ ID NO: 156 | SEQ ID NO: 149 | SEQ ID NO: 150 |
| | GCTTTTGCTTACGGTTCTACTGTGTTG | TGCCGCAGCCAATTTCAATACCCGCTA | ACCAATCGTCAATTGCTCATTTCCTA |
| 230 | SEQ ID NO: 144 | SEQ ID NO: 136 | SEQ ID NO: 137 |
| | CTTTTGCTTACGGTTCTACTGTGTTGA | AAGATGCCGCAGCCAATTTCAATACCCGC | ACCAATCGTCAATTGCTCATTTCCT |
| 231 | SEQ ID NO: 144 | SEQ ID NO: 145 | SEQ ID NO: 157 |
| | CTTTTGCTTACGGTTCTACTGTGTTGA | CGCAGCCAATTTCAATACCCGCTATCGCC | ACCAATCGTCAATTGCTCATTTCCTAAG |
| 232 | SEQ ID NO: 158 | SEQ ID NO: 159 | SEQ ID NO: 160 |
| | ACTGTGTTGATCCAAAAGGCGATAG | [?] TTCCTAAGATGCCGCAGCCAATTTCAATACC CG | GAAATCGCATCACAAGCACCAATC |
| 233 | SEQ ID NO: 161 | SEQ ID NO: 159 | SEQ ID NO: 147 |
| | CTACTGTGTTGATCCAAAAGGCGATA | TTCCTAAGATGCCGCAGCCAATTTCAATACC CG | CGCATCACAAGCACCAATCG |
| 234 | SEQ ID NO: 162 | SEQ ID NO: 163 | SEQ ID NO: 164 |
| | TTTATTCAAGACCATGGATTCCCGATC TTTATC | TCCCTTTTGAAGAACCGGCTTCATTCGGCT | GCGCTGTTTTGTCAGTTACTTTTGTG |
| 235 | SEQ ID NO: 165 | SEQ ID NO: 166 | SEQ ID NO: 167 |
| | TTCAAGACCATGGATTCCCGATCTTTA TCA | TGATCCCTTTTGAAGAACCGGCTTCATTCGGC | TGGAGCGCTGTTTTGTCAGTTAC |
| 236 | SEQ ID NO: 168 | SEQ ID NO: 166 | SEQ ID NO: 169 |
| | ATTCAAGACCATGGATTCCCGATCTTT ATCA | TGATCCCTTTTGAAGAACCGGCTTCATTCGGC | GGAGCGCTGTTTTGTCAGTTACTT |
| 237 | SEQ ID NO: 170 | SEQ ID NO: 166 | SEQ ID NO: 169 |
| | CAAGACCATGGATTCCCGATCTTTAT | TGATCCCTTTTGAAGAACCGGCTTCATTCGGC | GGAGCGCTGTTTTGTCAGTTACTT |
| 238 | SEQ ID NO: 171 | SEQ ID NO: 172 | SEQ ID NO: 173 |
| | AAGACCATGGATTCCCGATCTTTATCA | TCCCTTTTGAAGAACCGGCTTCATTCGGC | TGGAGCGCTGTTTTGTCAGTTA |
| 239 | SEQ ID NO: 171 | SEQ ID NO: 166 | SEQ ID NO: 174 |
| | AAGACCATGGATTCCCGATCTTTATCA | TGATCCCTTTTGAAGAACCGGCTTCATTCGGC | TGGAGCGCTGTTTTGTCAGTT |
| 240 | SEQ ID NO: 175 | SEQ ID NO: 176 | SEQ ID NO: 177 |
| | ACGGTTCTACTGTGTTGATCCAAAAGG | AGATGCCGCAGCCAATTTCAATACCCGCT | CACCAATCGTCAATTGCTCATTTCCT |
| 241 | SEQ ID NO: 178 | SEQ ID NO: 142 | SEQ ID NO: 179 |
| | TCTGCATTAACGACTGCTTTTGCTTA | ACCCGCTATCGCCTTTTGGATCAACACAGT | TAAGATGCCGCAGCCAATTTCA |
| 242 | SEQ ID NO: 180 | SEQ ID NO: 181 | SEQ ID NO: 146 |
| | AAAGGCGATAGCGGGTATTGAAATTG | CCAATCGTCAATTGCTCATTTCCTAAGATGCC GCAG | ACAAGAGAAATCGCATCACAAGCA |
| 243 | SEQ ID NO: 175 | SEQ ID NO: 182 | SEQ ID NO: 177 |
| | ACGGTTCTACTGTGTTGATCCAAAAGG | AAGATGCCGCAGCCAATTTCAATACCCGCT | CACCAATCGTCAATTGCTCATTTCCT |
| 244 | SEQ ID NO: 183 | SEQ ID NO: 149 | SEQ ID NO: 150 |
| | TACGGTTCTACTGTGTTGATCCAAAAG G | TGCCGCAGCCAATTTCAATACCCGCTA | ACCAATCGTCAATTGCTCATTTCCTA |
| 245 | SEQ ID NO: 184 | SEQ ID NO: 166 | SEQ ID NO: 174 |
| | AGACCATGGATTCCCGATCTTTATCA | TGATCCCTTTTGAAGAACCGGCTTCATTCGGC | TGGAGCGCTGTTTTGTCAGTT |
| 246 | SEQ ID NO: 185 | SEQ ID NO: 186 | SEQ ID NO: 187 |
| | CTGCATTAACGACTGCTTTTGCTTAC | ACCCGCTATCGCCTTTTGGATCAACACAGTA G | CTAAGATGCCGCAGCCAATTTC |
| 247 | SEQ ID NO: 188 | SEQ ID NO: 189 | SEQ ID NO: 190 |
| | CGGTTCTACTGTGTTGATCCAAAAGG | TGCCGCAGCCAATTTCAATACCCGCT | CACCAATCGTCAATTGCTCATTTCC |
| 248 | SEQ ID NO: 188 | SEQ ID NO: 152 | SEQ ID NO: 137 |
| | CGGTTCTACTGTGTTGATCCAAAAGG | AGATGCCGCAGCCAATTTCAATACCCGC | ACCAATCGTCAATTGCTCATTTCCT |
| 249 | SEQ ID NO: 188 | SEQ ID NO: 149 | SEQ ID NO: 150 |
| | CGGTTCTACTGTGTTGATCCAAAAGG | TGCCGCAGCCAATTTCAATACCCGCTA | ACCAATCGTCAATTGCTCATTTCCTA |
| 250 | SEQ ID NO: 191 | SEQ ID NO: 136 | SEQ ID NO: 192 |
| | TCTACTGTGTTGATCCAAAAGGCGATA | AAGATGCCGCAGCCAATTTCAATACCCGC | GCACCAATCGTCAATTGCTCATTTC |
| 251 | SEQ ID NO: 193 | SEQ ID NO: 152 | SEQ ID NO: 153 |
| | TTCTACTGTGTTGATCCAAAAGGCGAT A | AGATGCCGCAGCCAATTTCAATACCCGC | CACCAATCGTCAATTGCTCATTTCCTA |
| 252 | SEQ ID NO: 193 | SEQ ID NO: 136 | SEQ ID NO: 190 |
| | TTCTACTGTGTTGATCCAAAAGGCGAT A | AAGATGCCGCAGCCAATTTCAATACCCGC | CACCAATCGTCAATTGCTCATTTCC |
| 253 | SEQ ID NO: 191 | SEQ ID NO: 152 | SEQ ID NO: 192 |
| | TCTACTGTGTTGATCCAAAAGGCGATA | AGATGCCGCAGCCAATTTCAATACCCGC | GCACCAATCGTCAATTGCTCATTTC |
| 254 | SEQ ID NO: 194 | SEQ ID NO: 195 | SEQ ID NO: 160 |
| | CAAAAGGCGATAGCGGGTATTGAAA | TCAATTGCTCATTTCCTAAGATGCCGCAGCCA | GAAATCGCATCACAAGCACCAATC |
| 255 | SEQ ID NO: 193 | SEQ ID NO: 152 | SEQ ID NO: 190 |
| | TTCTACTGTGTTGATCCAAAAGGCGAT A | AGATGCCGCAGCCAATTTCAATACCCGC | CACCAATCGTCAATTGCTCATTTCC |
| 256 | SEQ ID NO: 191 | SEQ ID NO: 152 | SEQ ID NO: 190 |
| | TCTACTGTGTTGATCCAAAAGGCGATA | AGATGCCGCAGCCAATTTCAATACCCGC | CACCAATCGTCAATTGCTCATTTCC |
| 257 | SEQ ID NO: 193 | SEQ ID NO: 136 | SEQ ID NO: 137 |
| | TTCTACTGTGTTGATCCAAAAGGCGAT A | AAGATGCCGCAGCCAATTTCAATACCCGC | ACCAATCGTCAATTGCTCATTTCCT |
| 258 | SEQ ID NO: 196 | SEQ ID NO: 197 | SEQ ID NO: 160 |
| | AAAAGGCGATAGCGGGTATTGAAA | TCAATTGCTCATTTCCTAAGATGCCGCAGCCA A | GAAATCGCATCACAAGCACCAATC |
| 259 | SEQ ID NO: 198 | SEQ ID NO: 142 | SEQ ID NO: 179 |
| | ATTAACGACTGCTTTTGCTTACGGTTC T | ACCCGCTATCGCCTTTTGGATCAACACAGT | TAAGATGCCGCAGCCAATTTCA |
| 260 | SEQ ID NO: 193 | SEQ ID NO: 152 | SEQ ID NO: 137 |
| | TTCTACTGTGTTGATCCAAAAGGCGAT A | AGATGCCGCAGCCAATTTCAATACCCGC | ACCAATCGTCAATTGCTCATTTCCT |
| 261 | SEQ ID NO: 191 | SEQ ID NO: 152 | SEQ ID NO: 153 |
| | TCTACTGTGTTGATCCAAAAGGCGATA | AGATGCCGCAGCCAATTTCAATACCCGC | CACCAATCGTCAATTGCTCATTTCCTA |
| 262 | SEQ ID NO: 193 | SEQ ID NO: 152 | SEQ ID NO: 150 |
| | TTCTACTGTGTTGATCCAAAAGGCGAT A | AGATGCCGCAGCCAATTTCAATACCCGC | ACCAATCGTCAATTGCTCATTTCCTA |
| 263 | SEQ ID NO: 191 | SEQ ID NO: 136 | SEQ ID NO: 190 |
| | TCTACTGTGTTGATCCAAAAGGCGATA | AAGATGCCGCAGCCAATTTCAATACCCGC | CACCAATCGTCAATTGCTCATTTCC |
| 264 | SEQ ID NO: 196 | SEQ ID NO: 195 | SEQ ID NO: 160 |
| | AAAAGGCGATAGCGGGTATTGAAA | TCAATTGCTCATTTCCTAAGATGCCGCAGCCA | GAAATCGCATCACAAGCACCAATC |
| 265 | SEQ ID NO: 161 | SEQ ID NO: 136 | SEQ ID NO: 192 |
| | CTACTGTGTTGATCCAAAAGGCGATA | AAGATGCCGCAGCCAATTTCAATACCCGC | GCACCAATCGTCAATTGCTCATTTC |
| 266 | SEQ ID NO: 193 | SEQ ID NO: 199 | SEQ ID NO: 150 |
| | TTCTACTGTGTTGATCCAAAAGGCGAT A | TGCCGCAGCCAATTTCAATACCCGC | ACCAATCGTCAATTGCTCATTTCCTA |
| 267 | SEQ ID NO: 141 | SEQ ID NO: 142 | SEQ ID NO: 179 |
| | TTAACGACTGCTTTTGCTTACGGTTCT | ACCCGCTATCGCCTTTTGGATCAACACAGT | TAAGATGCCGCAGCCAATTTCA |
| 268 | SEQ ID NO: 161 | SEQ ID NO: 152 | SEQ ID NO: 153 |
| | CTACTGTGTTGATCCAAAAGGCGATA | AGATGCCGCAGCCAATTTCAATACCCGC | CACCAATCGTCAATTGCTCATTTCCTA |
| 269 | SEQ ID NO: 191 | SEQ ID NO: 199 | SEQ ID NO: 150 |
| | TCTACTGTGTTGATCCAAAAGGCGATA | TGCCGCAGCCAATTTCAATACCCGC | ACCAATCGTCAATTGCTCATTTCCTA |
| 270 | SEQ ID NO: 161 | SEQ ID NO: 136 | SEQ ID NO: 190 |
| | CTACTGTGTTGATCCAAAAGGCGATA | AAGATGCCGCAGCCAATTTCAATACCCGC | CACCAATCGTCAATTGCTCATTTCC |
| 271 | SEQ ID NO: 191 | SEQ ID NO: 136 | SEQ ID NO: 137 |
| | TCTACTGTGTTGATCCAAAAGGCGATA | AAGATGCCGCAGCCAATTTCAATACCCGC | ACCAATCGTCAATTGCTCATTTCCT |
| 272 | SEQ ID NO: 161 | SEQ ID NO: 152 | SEQ ID NO: 190 |
| | CTACTGTGTTGATCCAAAAGGCGATA | AGATGCCGCAGCCAATTTCAATACCCGC | CACCAATCGTCAATTGCTCATTTCC |
| 273 | SEQ ID NO: 191 | SEQ ID NO: 152 | SEQ ID NO: 137 |
| | TCTACTGTGTTGATCCAAAAGGCGATA | AGATGCCGCAGCCAATTTCAATACCCGC | ACCAATCGTCAATTGCTCATTTCCT |
| 274 | SEQ ID NO: 191 | SEQ ID NO: 152 | SEQ ID NO: 150 |
| | TCTACTGTGTTGATCCAAAAGGCGATA | AGATGCCGCAGCCAATTTCAATACCCGC | ACCAATCGTCAATTGCTCATTTCCTA |
| 275 | SEQ ID NO: 161 | SEQ ID NO: 136 | SEQ ID NO: 137 |
| | CTACTGTGTTGATCCAAAAGGCGATA | AAGATGCCGCAGCCAATTTCAATACCCGC | ACCAATCGTCAATTGCTCATTTCCT |
| 276 | SEQ ID NO: 200 | SEQ ID NO: 142 | SEQ ID NO: 179 |
| | TAACGACTGCTTTTGCTTACGGTTCT | ACCCGCTATCGCCTTTTGGATCAACACAGT | TAAGATGCCGCAGCCAATTTCA |
| 277 | SEQ ID NO: 161 | SEQ ID NO: 152 | SEQ ID NO: 137 |
| | CTACTGTGTTGATCCAAAAGGCGATA | AGATGCCGCAGCCAATTTCAATACCCGC | ACCAATCGTCAATTGCTCATTTCCT |
| 278 | SEQ ID NO: 161 | SEQ ID NO: 152 | SEQ ID NO: 150 |
| | CTACTGTGTTGATCCAAAAGGCGATA | AGATGCCGCAGCCAATTTCAATACCCGC | ACCAATCGTCAATTGCTCATTTCCTA |
| 279 | SEQ ID NO: 161 | SEQ ID NO: 199 | SEQ ID NO: 150 |
| | CTACTGTGTTGATCCAAAAGGCGATA | TGCCGCAGCCAATTTCAATACCCGC | ACCAATCGTCAATTGCTCATTTCCTA |
| 280 | SEQ ID NO: 201 | SEQ ID NO: 142 | SEQ ID NO: 179 |
| | AACGACTGCTTTTGCTTACGGTTCT | ACCCGCTATCGCCTTTTGGATCAACACAGT | TAAGATGCCGCAGCCAATTTCA |
| 281 | SEQ ID NO: 194 | SEQ ID NO: 195 | SEQ ID NO: 147 |
| | CAAAAGGCGATAGCGGGTATTGAAA | TCAATTGCTCATTTCCTAAGATGCCGCAGCCA | CGCATCACAAGCACCAATCG |
| 282 | SEQ ID NO: 202 | SEQ ID NO: 142 | SEQ ID NO: 179 |
| | ACGACTGCTTTTGCTTACGGTTCT | ACCCGCTATCGCCTTTTGGATCAACACAGT | TAAGATGCCGCAGCCAATTTCA |
| 283 | SEQ ID NO: 203 | SEQ ID NO: 195 | SEQ ID NO: 147 |
| | CAAAAGGCGATAGCGGGTATTGAA | TCAATTGCTCATTTCCTAAGATGCCGCAGCCA | CGCATCACAAGCACCAATCG |
| 284 | SEQ ID NO: 194 | SEQ ID NO: 197 | SEQ ID NO: 147 |
| | CAAAAGGCGATAGCGGGTATTGAAA | TCAATTGCTCATTTCCTAAGATGCCGCAGCCA A | CGCATCACAAGCACCAATCG |
| 285 | SEQ ID NO: 204 | SEQ ID NO: 205 | SEQ ID NO: 187 |
| | CGACTGCTTTTGCTTACGGTTCT | TACCCGCTATCGCCTTTTGGATCAACACAGT | CTAAGATGCCGCAGCCAATTTC |
| 286 | SEQ ID NO: 203 | SEQ ID NO: 197 | SEQ ID NO: 147 |
| | CAAAAGGCGATAGCGGGTATTGAA | TCAATTGCTCATTTCCTAAGATGCCGCAGCCA A | CGCATCACAAGCACCAATCG |
| 287 | SEQ ID NO: 204 | SEQ ID NO: 142 | SEQ ID NO: 187 |
| | CGACTGCTTTTGCTTACGGTTCT | ACCCGCTATCGCCTTTTGGATCAACACAGT | CTAAGATGCCGCAGCCAATTTC |
| 288 | SEQ ID NO: 204 | SEQ ID NO: 142 | SEQ ID NO: 179 |
| | CGACTGCTTTTGCTTACGGTTCT | ACCCGCTATCGCCTTTTGGATCAACACAGT | TAAGATGCCGCAGCCAATTTCA |
| 289 | SEQ ID NO: 196 | SEQ ID NO: 195 | SEQ ID NO: 147 |
| | AAAAGGCGATAGCGGGTATTGAAA | TCAATTGCTCATTTCCTAAGATGCCGCAGCCA | CGCATCACAAGCACCAATCG |
| 290 | SEQ ID NO: 196 | SEQ ID NO: 197 | SEQ ID NO: 147 |
| | AAAAGGCGATAGCGGGTATTGAAA | TCAATTGCTCATTTCCTAAGATGCCGCAGCCA A | CGCATCACAAGCACCAATCG |
| ***vanC2*/*3* Sets** | | | |
| 291 | SEQ ID NO: 206 | SEQ ID NO: 207 | SEQ ID NO: 208 |
| | CGCCATTGCCTGAAACGATTG | AGTCTTGGTCTTAAAGGTCTTGCTCGCATCGA CT | TCAAGTATAGTTCTCCTTGATCCGTGACA |
| 292 | SEQ ID NO: 206 | SEQ ID NO: 207 | SEQ ID NO: 209 |
| | CGCCATTGCCTGAAACGATTG | AGTCTTGGTCTTAAAGGTCTTGCTCGCATCGA CT | AGTATAGTTCTCCTTGATCCGTGACA |
| 293 | SEQ ID NO: 210 | SEQ ID NO: 211 | SEQ ID NO: 212 |
| | CCTGAAACGATTGAAACCAAGGTCAA | AGTCGATGCGAGCAAGACCTTTAAGACCAAG ACT | AAGTATAGTTCTCCTTGATCCGTGACA |
| 294 | SEQ ID NO: 210 | SEQ ID NO: 211 | SEQ ID NO: 209 |
| | CCTGAAACGATTGAAACCAAGGTCAA | AGTCGATGCGAGCAAGACCTTTAAGACCAAG ACT | AGTATAGTTCTCCTTGATCCGTGACA |
| 295 | SEQ ID NO: 213 | SEQ ID NO: 214 | SEQ ID NO: 215 |
| | GCGCCATTGCCTGAAACGAT | AAGTCGATGCGAGCAAGACCTTTAAGACCAA GACT | AGTATAGTTCTCCTTGATCCGTGACAAA |
| 296 | SEQ ID NO: 210 | SEQ ID NO: 211 | SEQ ID NO: 215 |
| | CCTGAAACGATTGAAACCAAGGTCAA | AGTCGATGCGAGCAAGACCTTTAAGACCAAG ACT | AGTATAGTTCTCCTTGATCCGTGACAAA |
| 297 | SEQ ID NO: 210 | SEO ID NO: 211 | SEQ ID NO: 216 |
| | CCTGAAACGATTGAAACCAAGGTCAA | AGTCGATGCGAGCAAGACCTTTAAGACCAAG ACT | GTATAGTTCTCCTTGATCCGTGACAAAAA |
| 298 | SEQ ID NO: 217 | SEQ ID NO: 218 | SEQ ID NO: 219 |
| | CGCCATTGCCTGAAACGATTGAA | CGATGCGAGCAAGACCTTTAAGACCAAGACT ACG | TTCTCCTTGATCCGTGACAAAAAAGT |
| 299 | SEQ ID NO: 220 | SEQ ID NO: 218 | SEQ ID NO: 219 |
| | GCGCCATTGCCTGAAACG | CGATGCGAGCAAGACCTTTAAGACCAAGACT ACG | TTCTCCTTGATCCGTGACAAAAAAGT |
| 300 | SEQ ID NO: 221 | SEQ ID NO: 218 | SEQ ID NO: 219 |
| | GCCATTGCCTGAAACGATTGAAAC | CGATGCGAGCAAGACCTTTAAGACCAAGACT ACG | TTCTCCTTGATCCGTGACAAAAAAGT |
| 301 | SEQ ID NO: 222 | SEQ ID NO: 218 | SEQ ID NO: 219 |
| | CTGCGCCATTGCCTGAAAC | CGATGCGAGCAAGACCTTTAAGACCAAGACT ACG | TTCTCCTTGATCCGTGACAAAAAAGT |
| 302 | SEQ ID NO: 210 | SEQ ID NO: 218 | SEQ ID NO: 219 |
| | CCTGAAACGATTGAAACCAAGGTCAA | CGATGCGAGCAAGACCTTTAAGACCAAGACT ACG | TTCTCCTTGATCCGTGACAAAAAAGT |
| 303 | SEQ ID NO: 206 | SEQ ID NO: 218 | SEQ ID NO: 219 |
| | CGCCATTGCCTGAAACGATTG | CGATGCGAGCAAGACCTTTAAGACCAAGACT ACG | TTCTCCTTGATCCGTGACAAAAAAGT |
| 304 | SEQ ID NO: 222 | SEQ ID NO: 218 | SEQ ID NO: 216 |
| | CTGCGCCATTGCCTGAAAC | CGATGCGAGCAAGACCTTTAAGACCAAGACT ACG | GTATAGTTCTCCTTGATCCGTGACAAAAA |
| 305 | SEQ ID NO: 217 | SEQ ID NO: 218 | SEQ ID NO: 223 |
| | CGCCATTGCCTGAAACGATTGAA | CGATGCGAGCAAGACCTTTAAGACCAAGACT ACG | AGTTCTCCTTGATCCGTGACAAAAAA |
| 306 | SEQ ID NO: 221 | SEQ ID NO: 218 | SEQ ID NO: 209 |
| | GCCATTGCCTGAAACGATTGAAAC | CGATGCGAGCAAGACCTTTAAGACCAAGACT ACG | AGTATAGTTCTCCTTGATCCGTGACA |
| 307 | SEQ ID NO: 224 | SEQ ID NO: 211 | SEQ ID NO: 216 |
| | CCTGAAACGATTGAAACCAAGGTCAA AG | AGTCGATGCGAGCAAGACCTTTAAGACCAAG ACT | GTATAGTTCTCCTTGATCCGTGACAAAAA |
| 308 | SEQ ID NO: 225 | SEQ ID NO: 211 | SEQ ID NO: 216 |
| | CTGAAACGATTGAAACCAAGGTCAAA G | AGTCGATGCGAGCAAGACCTTTAAGACCAAG ACT | GTATAGTTCTCCTTGATCCGTGACAAAAA |
| 309 | SEQ ID NO: 226 | SEQ ID NO: 211 | SEQ ID NO: 209 |
| | CTGAAACGATTGAAACCAAGGTCAAA GAA | AGTCGATGCGAGCAAGACCTTTAAGACCAAG ACT | AGTATAGTTCTCCTTGATCCGTGACA |
| 310 | SEQ ID NO: 222 | SEQ ID NO: 218 | SEQ ID NO: 209 |
| | CTGCGCCATTGCCTGAAAC | CGATGCGAGCAAGACCTTTAAGACCAAGACT ACG | AGTATAGTTCTCCTTGATCCGTGACA |
| 311 | SEQ ID NO: 206 | SEQ ID NO: 218 | SEQ ID NO: 227 |
| | CGCCATTGCCTGAAACGATTG | CGATGCGAGCAAGACCTTTAAGACCAAGACT ACG | TATAGTTCTCCTTGATCCGTGACAAAAAAG |
| 312 | SEQ ID NO: 220 | SEQ ID NO: 218 | SEQ ID NO: 223 |
| | GCGCCATTGCCTGAAACG | CGATGCGAGCAAGACCTTTAAGACCAAGACT ACG | AGTTCTCCTTGATCCGTGACAAAAAA |
| 313 | SEQ ID NO: 228 | SEQ ID NO: 211 | SEQ ID NO: 215 |
| | CCTGAAACGATTGAAACCAAGGTCAA A | AGTCGATGCGAGCAAGACCTTTAAGACCAAG ACT | AGTATAGTTCTCCTTGATCCGTGACAAA |
| 314 | SEQ ID NO: 206 | SEQ ID NO: 218 | SEQ ID NO: 209 |
| | CGCCATTGCCTGAAACGATTG | CGATGCGAGCAAGACCTTTAAGACCAAGACT ACG | AGTATAGTTCTCCTTGATCCGTGACA |
| 315 | SEQ ID NO: 217 | SEQ ID NO: 218 | SEQ ID NO: 227 |
| | CGCCATTGCCTGAAACGATTGAA | CGATGCGAGCAAGACCTTTAAGACCAAGACT ACG | TATAGTTCTCCTTGATCCGTGACAAAAAAG |
| 316 | SEQ ID NO: 221 | SEQ ID NO: 218 | SEQ ID NO: 216 |
| | GCCATTGCCTGAAACGATTGAAAC | CGATGCGAGCAAGACCTTTAAGACCAAGACT ACG | GTATAGTTCTCCTTGATCCGTGACAAAAA |
| 317 | SEQ ID NO: 210 | SEQ ID NO: 218 | SEQ ID NO: 216 |
| | CCTGAAACGATTGAAACCAAGGTCAA | CGATGCGAGCAAGACCTTTAAGACCAAGACT ACG | GTATAGTTCTCCTTGATCCGTGACAAAAA |
| 318 | SEQ ID NO: 229 | SEQ ID NO: 211 | SEQ ID NO: 215 |
| | CTGAAACGATTGAAACCAAGGTCAAA GA | AGTCGATGCGAGCAAGACCTTTAAGACCAAG ACT | AGTATAGTTCTCCTTGATCCGTGACAAA |
| 319 | SEQ ID NO: 226 | SEQ ID NO: 211 | SEQ ID NO: 216 |
| | CTGAAACGATTGAAACCAAGGTCAAA GAA | AGTCGATGCGAGCAAGACCTTTAAGACCAAG ACT | GTATAGTTCTCCTTGATCCGTGACAAAAA |
| 320 | SEQ ID NO: 222 | SEQ ID NO: 218 | SEQ ID NO: 223 |
| | CTGCGCCATTGCCTGAAAC | CGATGCGAGCAAGACCTTTAAGACCAAGACT ACG | AGTTCTCCTTGATCCGTGACAAAAAA |
| 321 | SEQ ID NO: 230 | SEQ ID NO: 218 | SEQ ID NO: 219 |
| | CCGTCCCTGCGCCATT | CGATGCGAGCAAGACCTTTAAGACCAAGACT ACG | TTCTCCTTGATCCGTGACAAAAAAGT |
| 322 | SEQ ID NO: 210 | SEQ ID NO: 218 | SEQ ID NO: 223 |
| | CCTGAAACGATTGAAACCAAGGTCAA | CGATGCGAGCAAGACCTTTAAGACCAAGACT ACG | AGTTCTCCTTGATCCGTGACAAAAAA |
| 323 | SEQ ID NO: 224 | SEQ ID NO: 211 | SEQ ID NO: 215 |
| | CCTGAAACGATTGAAACCAAGGTCAA AG | AGTCGATGCGAGCAAGACCTTTAAGACCAAG ACT | AGTATAGTTCTCCTTGATCCGTGACAAA |
| 324 | SEQ ID NO: 225 | SEQ ID NO: 211 | SEQ ID NO: 212 |
| | CTGAAACGATTGAAACCAAGGTCAAA G | AGTCGATGCGAGCAAGACCTTTAAGACCAAG ACT | AAGTATAGTTCTCCTTGATCCGTGACA |
| 325 | SEQ ID NO: 206 | SEQ ID NO: 218 | SEQ ID NO: 216 |
| | CGCCATTGCCTGAAACGATTG | CGATGCGAGCAAGACCTTTAAGACCAAGACT ACG | GTATAGTTCTCCTTGATCCGTGACAAAAA |
| 326 | SEQ ID NO: 210 | SEQ ID NO: 218 | SEQ ID NO: 209 |
| | CCTGAAACGATTGAAACCAAGGTCAA | CGATGCGAGCAAGACCTTTAAGACCAAGACT ACG | AGTATAGTTCTCCTTGATCCGTGACA |
| 327 | SEQ ID NO: 210 | SEQ ID NO: 218 | SEQ ID NO: 227 |
| | CCTGAAACGATTGAAACCAAGGTCAA | CGATGCGAGCAAGACCTTTAAGACCAAGACT ACG | TATAGTTCTCCTTGATCCGTGACAAAAAAG |
| 328 | SEQ ID NO: 231 | SEQ ID NO: 211 | SEQ ID NO: 215 |
| | TGAAACGATTGAAACCAAGGTCAAAG | AGTCGATGCGAGCAAGACCTTTAAGACCAAG | AGTATAGTTCTCCTTGATCCGTGACAAA |
| | A | ACT | |
| 329 | SEQ ID NO: 222 | SEQ ID NO: 218 | SEQ ID NO: 227 |
| | CTGCGCCATTGCCTGAAAC | CGATGCGAGCAAGACCTTTAAGACCAAGACT ACG | TATAGTTCTCCTTGATCCGTGACAAAAAAG |
| 330 | SEQ ID NO: 217 | SEQ ID NO: 218 | SEQ ID NO: 216 |
| | CGCCATTGCCTGAAACGATTGAA | CGATGCGAGCAAGACCTTTAAGACCAAGACT ACG | GTATAGTTCTCCTTGATCCGTGACAAAAA |
| 331 | SEQ ID NO: 229 | SEQ ID NO: 211 | SEQ ID NO: 209 |
| | CTGAAACGATTGAAACCAAGGTCAAA GA | AGTCGATGCGAGCAAGACCTTTAAGACCAAG ACT | AGTATAGTTCTCCTTGATCCGTGACA |
| 332 | SEQ ID NO: 226 | SEQ ID NO: 211 | SEQ ID NO: 212 |
| | CTGAAACGATTGAAACCAAGGTCAAA GAA | AGTCGATGCGAGCAAGACCTTTAAGACCAAG ACT | AAGTATAGTTCTCCTTGATCCGTGACA |
| 333 | SEQ ID NO: 217 | SEQ ID NO: 218 | SEQ ID NO: 209 |
| | CGCCATTGCCTGAAACGATTGAA | CGATGCGAGCAAGACCTTTAAGACCAAGACT ACG | AGTATAGTTCTCCTTGATCCGTGACA |
| 334 | SEQ ID NO: 220 | SEQ ID NO: 218 | SEQ ID NO: 227 |
| | GCGCCATTGCCTGAAACG | CGATGCGAGCAAGACCTTTAAGACCAAGACT ACG | TATAGTTCTCCTTGATCCGTGACAAAAAAG |
| 335 | SEQ ID NO: 224 | SEQ ID NO: 211 | SEQ ID NO: 212 |
| | CCTGAAACGATTGAAACCAAGGTCAA AG | AGTCGATGCGAGCAAGACCTTTAAGACCAAG ACT | AAGTATAGTTCTCCTTGATCCGTGACA |
| 336 | SEQ ID NO: 225 | SEQ ID NO: 211 | SEQ ID NO: 209 |
| | CTGAAACGATTGAAACCAAGGTCAAA G | AGTCGATGCGAGCAAGACCTTTAAGACCAAG ACT | AGTATAGTTCTCCTTGATCCGTGACA |
| 337 | SEQ ID NO: 220 | SEQ ID NO: 218 | SEQ ID NO: 209 |
| | GCGCCATTGCCTGAAACG | CGATGCGAGCAAGACCTTTAAGACCAAGACT ACG | AGTATAGTTCTCCTTGATCCGTGACA |
| 338 | SEQ ID NO: 221 | SEQ ID NO: 218 | SEQ ID NO: 227 |
| | GCCATTGCCTGAAACGATTGAAAC | CGATGCGAGCAAGACCTTTAAGACCAAGACT ACG | TATAGTTCTCCTTGATCCGTGACAAAAAAG |
| 339 | SEQ ID NO: 231 | SEQ ID NO: 211 | SEQ ID NO: 212 |
| | TGAAACGATTGAAACCAAGGTCAAAG A | AGTCGATGCGAGCAAGACCTTTAAGACCAAG ACT | AAGTATAGTTCTCCTTGATCCGTGACA |
| 340 | SEQ ID NO: 232 | SEQ ID NO: 211 | SEQ ID NO: 216 |
| | ACGATTGAAACCAAGGTCAAAGAACA AG | AGTCGATGCGAGCAAGACCTTTAAGACCAAG ACT | GTATAGTTCTCCTTGATCCGTGACAAAAA |
| 341 | SEQ ID NO: 233 | SEQ ID NO: 211 | SEQ ID NO: 212 |
| | TGAAACGATTGAAACCAAGGTCAAAG AAC | AGTCGATGCGAGCAAGACCTTTAAGACCAAG ACT | AAGTATAGTTCTCCTTGATCCGTGACA |
| 342 | SEQ ID NO: 234 | SEQ ID NO: 211 | SEQ ID NO: 215 |
| | TGAAACGATTGAAACCAAGGTCAAAG AACA | AGTCGATGCGAGCAAGACCTTTAAGACCAAG ACT | AGTATAGTTCTCCTTGATCCGTGACAAA |
| 343 | SEQ ID NO: 235 | SEQ ID NO: 207 | SEQ ID NO: 239 |
| | AACGATTGAAACCAAGGTCAAAGAAC A | AGTCTTGGTCTTAAAGGTCTTGCTCGCATCGA CT | AAGTATAGTTCTCCTTGATCCGTGACAAA |
| 344 | SEQ ID NO: 236 | SEQ ID NO: 211 | SEQ ID NO: 216 |
| | ACGATTGAAACCAAGGTCAAAGAACA A | AGTCGATGCGAGCAAGACCTTTAAGACCAAG ACT | GTATAGTTCTCCTTGATCCGTGACAAAAA |
| 345 | SEQ ID NO: 237 | SEQ ID NO: 211 | SEQ ID NO: 215 |
| | GAAACGATTGAAACCAAGGTCAAAGA AC | AGTCGATGCGAGCAAGACCTTTAAGACCAAG ACT | AGTATAGTTCTCCTTGATCCGTGACAAA |
| 346 | SEQ ID NO: 238 | SEQ ID NO: 211 | SEQ ID NO: 215 |
| | GAAACGATTGAAACCAAGGTCAAAGA ACA | AGTCGATGCGAGCAAGACCTTTAAGACCAAG ACT | AGTATAGTTCTCCTTGATCCGTGACAAA |
| 347 | SEQ ID NO: 240 | SEQ ID NO: 211 | SEQ ID NO: 216 |
| | AAACGATTGAAACCAAGGTCAAAGAA CAAG | AGTCGATGCGAGCAAGACCTTTAAGACCAAG ACT | GTATAGTTCTCCTTGATCCGTGACAAAAA |
| 348 | SEQ ID NO: 235 | SEQ ID NO: 211 | SEQ ID NO: 215 |
| | AACGATTGAAACCAAGGTCAAAGAAC A | AGTCGATGCGAGCAAGACCTTTAAGACCAAG ACT | AGTATAGTTCTCCTTGATCCGTGACAAA |
| 349 | SEQ ID NO: 237 | SEQ ID NO: 211 | SEQ ID NO: 209 |
| | GAAACGATTGAAACCAAGGTCAAAGA AC | AGTCGATGCGAGCAAGACCTTTAAGACCAAG ACT | AGTATAGTTCTCCTTGATCCGTGACA |
| 350 | SEQ ID NO: 235 | SEQ ID NO: 207 | SEQ ID NO: 241 |
| | AACGATTGAAACCAAGGTCAAAGAAC A | AGTCTTGGTCTTAAAGGTCTTGCTCGCATCGA CT | AGTATAGTTCTCCTTGATCCGTGACAAAAA |
| 351 | SEQ ID NO: 242 | SEQ ID NO: 211 | SEQ ID NO: 216 |
| | AAACGATTGAAACCAAGGTCAAAGAA CA | AGTCGATGCGAGCAAGACCTTTAAGACCAAG ACT | GTATAGTTCTCCTTGATCCGTGACAAAAA |
| 352 | SEQ ID NO: 243 | SEQ ID NO: 211 | SEQ ID NO: 215 |
| | AAACGATTGAAACCAAGGTCAAAGAA CAA | AGTCGATGCGAGCAAGACCTTTAAGACCAAG ACT | AGTATAGTTCTCCTTGATCCGTGACAAA |

| ***vanG* Sets** | | | |
|---|---|---|---|
| 353 | SEQ ID NO: 244 | SEQ ID NO: 245 | SEQ ID NO: 246 |
| | TCTGGCGAAATTGTATTTAATGAGGTA AACA | CAATACCAGGCTTTACCTCGCACAGTCGC | AGACCAATGCCTTTCATCATATTTGGA |
| 354 | SEQ ID NO: 244 | SEQ ID NO: 245 | SEQ ID NO: 247 |
| | TCTGGCGAAATTGTATTTAATGAGGTA AACA | CAATACCAGGCTTTACCTCGCACAGTCGC | GATAGACCAATGCCTTTCATCATATTTGGATA |
| 355 | SEQ ID NO: 244 | SEQ ID NO: 245 | SEQ ID NO: 248 |
| | TCTGGCGAAATTGTATTTAATGAGGTA AACA | CAATACCAGGCTTTACCTCGCACAGTCGC | GATAGACCAATGCCTTTCATCATATTTGGA |
| 356 | SEQ ID NO: 249 | SEQ ID NO: 245 | SEQ ID NO: 246 |
| | ACCGTCTGGCGAAATTGTATTTAATGA | CAATACCAGGCTTTACCTCGCACAGTCGC | AGACCAATGCCTTTCATCATATTTGGA |
| 357 | SEQ ID NO: 244 | SEQ ID NO: 245 | SEQ ID NO: 250 |
| | TCTGGCGAAATTGTATTTAATGAGGTA AACA | CAATACCAGGCTTTACCTCGCACAGTCGC | CGATAGACCAATGCCTTTCATCATATTTGG |
| 358 | SEQ ID NO: 244 | SEQ ID NO: 245 | SEQ ID NO: 251 |
| | TCTGGCGAAATTGTATTTAATGAGGTA AACA | CAATACCAGGCTTTACCTCGCACAGTCGC | CGATAGACCAATGCCTTTCATCATATTTGGATA |
| 359 | SEQ ID NO: 252 | SEQ ID NO: 245 | SEQ ID NO: 246 |
| | CACCGTCTGGCGAAATTGTATTTAATG | CAATACCAGGCTTTACCTCGCACAGTCGC | AGACCAATGCCTTTCATCATATTTGGA |
| 360 | SEQ ID NO: 253 | SEQ ID NO: 245 | SEQ ID NO: 246 |
| | ACACCGTCTGGCGAAATTGTAT | CAATACCAGGCTTTACCTCGCACAGTCGC | AGACCAATGCCTTTCATCATATTTGGA |
| 361 | SEQ ID NO: 244 | SEQ ID NO: 245 | SEQ ID NO: 254 |
| | TCTGGCGAAATTGTATTTAATGAGGTA AACA | CAATACCAGGCTTTACCTCGCACAGTCGC | AACGATAGACCAATGCCTTTCATCATATTT |
| 362 | SEQ ID NO: 255 | SEQ ID NO: 245 | SEQ ID NO: 246 |
| | ACACCGTCTGGCGAAATTGTATTTA | CAATACCAGGCTTTACCTCGCACAGTCGC | AGACCAATGCCTTTCATCATATTTGGA |
| 363 | SEQ ID NO: 256 | SEQ ID NO: 245 | SEQ ID NO: 246 |
| | ACACCGTCTGGCGAAATTGTATT | CAATACCAGGCTTTACCTCGCACAGTCGC | AGACCAATGCCTTTCATCATATTTGGA |
| 364 | SEQ ID NO: 249 | SEQ ID NO: 245 | SEQ ID NO: 248 |
| | ACCGTCTGGCGAAATTGTATTTAATGA | CAATACCAGGCTTTACCTCGCACAGTCGC | GATAGACCAATGCCTTTCATCATATTTGGA |
| 365 | SEQ ID NO: 244 | SEQ ID NO: 257 | SEQ ID NO: 258 |
| | TCTGGCGAAATTGTATTTAATGAGGTA AACA | AGGCTTTACCTCGCACAGTCGCTATCCAA | GAACGATAGACCAATGCCTTTCATCA |
| 366 | SEQ ID NO: 244 | SEQ ID NO: 257 | SEQ ID NO: 259 |
| | TCTGGCGAAATTGTATTTAATGAGGTA AACA | AGGCTTTACCTCGCACAGTCGCTATCCAA | GAACGATAGACCAATGCCTTTCATCATA |
| 367 | SEQ ID NO: 260 | SEQ ID NO: 261 | SEQ ID NO: 258 |
| | ACCGTCTGGCGAAATTGTATTTAATGA G | CCAGGCTTTACCTCGCACAGTCGCTATCC | GAACGATAGACCAATGCCTTTCATCA |
| 368 | SEQ ID NO: 252 | SEQ ID NO: 257 | SEQ ID NO: 262 |
| | CACCGTCTGGCGAAATTGTATTTAATG | AGGCTTTACCTCGCACAGTCGCTATCCAA | GAACGATAGACCAATGCCTTTCATCAT |
| 369 | SEQ ID NO: 263 | SEQ ID NO: 257 | SEQ ID NO: 258 |
| | TTTATACACCGTCTGGCGAAATTGT | AGGCTTTACCTCGCACAGTCGCTATCCAA | GAACGATAGACCAATGCCTTTCATCA |
| 370 | SEQ ID NO: 264 | SEQ ID NO: 265 | SEQ ID NO: 266 |
| | GCCGAAGCAGAAAAACGGATACA | CCACTCTGGAAAAACCCGAACAGCCCAGA | CAATTTCGCCAGACGGTGTATAAAACATA |
| 371 | SEQ ID NO: 267 | SEQ ID NO: 268 | SEQ ID NO: 269 |
| | GCCGAAGCAGAAAAACGGATACAA | TATCCACTCTGGAAAAACCCGAACAGCCCA | ACAATTTCGCCAGACGGTGTATAAAA |
| 372 | SEQ ID NO: 270 | SEQ ID NO: 265 | SEQ ID NO: 266 |
| | TGCCGAAGCAGAAAAACGGATAC | CCACTCTGGAAAAACCCGAACAGCCCAGA | CAATTTCGCCAGACGGTGTATAAAACATA |
| 373 | SEQ ID NO: 270 | SEQ ID NO: 268 | SEQ ID NO: 269 |
| | TGCCGAAGCAGAAAAACGGATAC | TATCCACTCTGGAAAAACCCGAACAGCCCA | ACAATTTCGCCAGACGGTGTATAAAA |
| 374 | SEQ ID NO: 271 | SEQ ID NO: 265 | SEQ ID NO: 266 |
| | ATGCCGAAGCAGAAAAACGGATAC | CCACTCTGGAAAAACCCGAACAGCCCAGA | CAATTTCGCCAGACGGTGTATAAAACATA |
| 375 | SEQ ID NO: 272 | SEQ ID NO: 273 | SEQ ID NO: 274 |
| | AAGGATTGATGCCGAAGCAGAA | CTGGAAAAACCCGAACAGCCCAGAGCTT | GCCAGACGGTGTATAAAACATATCCA |
| 376 | SEQ ID NO: 275 | SEQ ID NO: 268 | SEQ ID NO: 269 |
| | ATGCCGAAGCAGAAAAACGGATA | TATCCACTCTGGAAAAACCCGAACAGCCCA | ACAATTTCGCCAGACGGTGTATAAAA |
| 377 | SEQ ID NO: 276 | SEQ ID NO: 268 | SEQ ID NO: 269 |
| | GATGCCGAAGCAGAAAAACGGATA | TATCCACTCTGGAAAAACCCGAACAGCCCA | ACAATTTCGCCAGACGGTGTATAAAA |
| 378 | SEQ ID NO: 277 | SEQ ID NO: 268 | SEQ ID NO: 269 |
| | AGGATTGATGCCGAAGCAGAAAA | TATCCACTCTGGAAAAACCCGAACAGCCCA | ACAATTTCGCCAGACGGTGTATAAAA |
| 379 | SEQ ID NO: 278 | SEQ ID NO: 265 | SEQ ID NO: 266 |
| | AAGGATTGATGCCGAAGCAGAAAAAC | CCACTCTGGAAAAACCCGAACAGCCCAGA | CAATTTCGCCAGACGGTGTATAAAACATA |
| 380 | SEQ ID NO: 279 | SEQ ID NO: 268 | SEQ ID NO: 269 |
| | AAGGATTGATGCCGAAGCAGAAA | TATCCACTCTGGAAAAACCCGAACAGCCCA | ACAATTTCGCCAGACGGTGTATAAAA |
| 381 | SEQ ID NO: 280 | SEQ ID NO: 265 | SEQ ID NO: 266 |
| | CAAGGATTGATGCCGAAGCAGAA | CCACTCTGGAAAAACCCGAACAGCCCAGA | CAATTTCGCCAGACGGTGTATAAAACATA |
| 382 | SEQ ID NO: 281 | SEQ ID NO: 268 | SEQ ID NO: 269 |
| | AAGGATTGATGCCGAAGCAGAAAA | TATCCACTCTGGAAAAACCCGAACAGCCCA | ACAATTTCGCCAGACGGTGTATAAAA |
| 383 | SEQ ID NO: 280 | SEQ ID NO: 268 | SEQ ID NO: 269 |
| | CAAGGATTGATGCCGAAGCAGAA | TATCCACTCTGGAAAAACCCGAACAGCCCA | ACAATTTCGCCAGACGGTGTATAAAA |
| 384 | SEQ ID NO: 282 | SEQ ID NO: 265 | SEQ ID NO: 266 |
| | GCAAGGATTGATGCCGAAGCA | CCACTCTGGAAAAACCCGAACAGCCCAGA | CAATTTCGCCAGACGGTGTATAAAACATA |
| 385 | SEQ ID NO: 283 | SEQ ID NO: 268 | SEQ ID NO: 269 |
| | CAAGGATTGATGCCGAAGCAGAAA | TATCCACTCTGGAAAAACCCGAACAGCCCA | ACAATTTCGCCAGACGGTGTATAAAA |
| 386 | SEQ ID NO: 282 | SEQ ID NO: 268 | SEQ ID NO: 269 |
| | GCAAGGATTGATGCCGAAGCA | TATCCACTCTGGAAAAACCCGAACAGCCCA | ACAATTTCGCCAGACGGTGTATAAAA |
| 387 | SEQ ID NO: 244 | SEQ ID NO: 257 | SEQ ID NO: 284 |
| | TCTGGCGAAATTGTATTTAATGAGGTA AACA | AGGCTTTACCTCGCACAGTCGCTATCCAA | ACATACAGACCTATCAGCTTATCCAACA |
| 388 | SEQ ID NO: 285 | SEQ ID NO: 257 | SEQ ID NO: 259 |
| | CGGGTTTTTCCAGAGTGGATATGT | AGGCTTTACCTCGCACAGTCGCTATCCAA | GAACGATAGACCAATGCCTTTCATCATA |
| 389 | SEQ ID NO: 244 | SEQ ID NO: 257 | SEQ ID NO: 286 |
| | TCTGGCGAAATTGTATTTAATGAGGTA AACA | AGGCTTTACCTCGCACAGTCGCTATCCAA | ACATACAGACCTATCAGCTTATCCAACATT |
| 390 | SEQ ID NO: 244 | SEQ ID NO: 257 | SEQ ID NO: 287 |
| | TCTGGCGAAATTGTATTTAATGAGGTA AACA | AGGCTTTACCTCGCACAGTCGCTATCCAA | ACATACAGACCTATCAGCTTATCCAACAT |
| 391 | SEQ ID NO: 288 | SEQ ID NO: 289 | SEQ ID NO: 290 |
| | TCAAGCGGCTTTTTGATTATACAGAG AAAT | TTTCTGCTTCGGCATCAATCCTTGCAGGC | CAGCCCAGAGCTTTATATATGGTTACAG |
| 392 | SEQ ID NO: 291 | SEQ ID NO: 292 | SEQ ID NO: 290 |
| | TCAAGCGGCTTTTTTGATTATACAGAG AAATA | TTCTGCTTCGGCATCAATCCTTGCAGGC | CAGCCCAGAGCTTTATATATGGTTACAG |
| 393 | SEQ ID NO: 293 | SEQ ID NO: 294 | SEQ ID NO: 290 |
| | TCAAGCGGCTTTTTGATTATACAGAG A | TTTTCTGCTTCGGCATCAATCCTTGCAGGC | CAGCCCAGAGCTTTATATATGGTTACAG |
| 394 | SEQ ID NO: 295 | SEQ ID NO: 296 | SEQ ID NO: 258 |
| | GTTCGGGTTTTTCCAGAGTGGAT | ACCAGGCTTTACCTCGCACAGTCGC | GAACGATAGACCAATGCCTTTCATCA |
| 395 | SEQ ID NO: 293 | SEQ ID NO: 289 | SEQ ID NO: 290 |
| | TCAAGCGGCTTTTTTGATTATACAGAG A | TTTCTGCTTCGGCATCAATCCTTGCAGGC | CAGCCCAGAGCTTTATATATGGTTACAG |
| 396 | SEQ ID NO: 288 | SEQ ID NO: 294 | SEQ ID NO: 290 |
| | TCAAGCGGCTTTTTTGATTATACAGAG AAAT | TTTTCTGCTTCGGCATCAATCCTTGCAGGC | CAGCCCAGAGCTTTATATATGGTTACAG |
| 397 | SEQ ID NO: 297 | SEQ ID NO: 294 | SEQ ID NO: 290 |
| | TCAAGCGGCTTTTTTGATTATACAGAG AAATATAC | TTTTCTGCTTCGGCATCAATCCTTGCAGGC | CAGCCCAGAGCTTTATATATGGTTACAG |
| 398 | SEQ ID NO: 291 | SEQ ID NO: 289 | SEQ ID NO: 290 |
| | TCAAGCGGCTTTTTTGATTATACAGAG AAATA | TTTCTGCTTCGGCATCAATCCTTGCAGGC | CAGCCCAGAGCTTTATATATGGTTACAG |
| 399 | SEQ ID NO: 249 | SEQ ID NO: 257 | SEQ ID NO: 286 |
| | ACCGTCTGGCGAAATTGTATTTAATGA | AGGCTTTACCTCGCACAGTCGCTATCCAA | ACATACAGACCTATCAGCTTATCCAACATT |
| 400 | SEQ ID NO: 298 | SEQ ID NO: 292 | SEQ ID NO: 290 |
| | GTCAAGCGGCTTTTTGATTATACAGA GA | TTCTGCTTCGGCATCAATCCTTGCAGGC | CAGCCCAGAGCTTTATATATGGTTACAG |
| 401 | SEQ ID NO: 298 | SEQ ID NO: 294 | SEQ ID NO: 290 |
| | GTCAAGCGGCTTTTTGATTATACAGA GA | TTTTCTGCTTCGGCATCAATCCTTGCAGGC | CAGCCCAGAGCTTTATATATGGTTACAG |
| 402 | SEQ ID NO: 298 | SEQ ID NO: 289 | SEQ ID NO: 290 |
| | GTCAAGCGGCTTTTTGATTATACAGA GA | TTTCTGCTTCGGCATCAATCCTTGCAGGC | CAGCCCAGAGCTTTATATATGGTTACAG |
| 403 | SEQ ID NO: 299 | SEQ ID NO: 294 | SEQ ID NO: 290 |
| | TGTCAAGCGGCTTTTTGATTATACAG A | TTTTCTGCTTCGGCATCAATCCTTGCAGGC | CAGCCCAGAGCTTTATATATGGTTACAG |
| 404 | SEQ ID NO: 300 | SEQ ID NO: 289 | SEQ ID NO: 290 |
| | TGTCAAGCGGCTTTTTGATTATACAG AGA | TTTCTGCTTCGGCATCAATCCTTGCAGGC | CAGCCCAGAGCTTTATATATGGTTACAG |
| 405 | SEQ ID NO: 249 | SEQ ID NO: 257 | SEQ ID NO: 301 |
| | ACCGTCTGGCGAAATTGTATTTAATGA | AGGCTTTACCTCGCACAGTCGCTATCCAA | CACATACAGACCTATCAGCTTATCCAACATT |
| 406 | SEQ ID NO: 299 | SEQ ID NO: 289 | SEQ ID NO: 290 |
| | TGTCAAGCGGCTTTTTGATTATACAG A | TTTCTGCTTCGGCATCAATCCTTGCAGGC | CAGCCCAGAGCTTTATATATGGTTACAG |
| 407 | SEQ ID NO: 300 | SEQ ID NO: 292 | SEQ ID NO: 290 |
| | TGTCAAGCGGCTTTTTGATTATACAG AGA | TTCTGCTTCGGCATCAATCCTTGCAGGC | CAGCCCAGAGCTTTATATATGGTTACAG |
| 408 | SEQ ID NO: 302 | SEQ ID NO: 273 | SEQ ID NO: 274 |
| | ATCTTCAAAGATATATATGCCTGCAAG GATTG | CTGGAAAAACCCGAACAGCCCAGAGCTT | GCCAGACGGTGTATAAAACATATCCA |
| 409 | SEQ ID NO: 299 | SEQ ID NO: 292 | SEQ ID NO: 290 |
| | TGTCAAGCGGCTTTTTTGATTATACAG A | TTCTGCTTCGGCATCAATCCTTGCAGGC | CAGCCCAGAGCTTTATATATGGTTACAG |
| 410 | SEQ ID NO: 300 | SEQ ID NO: 294 | SEQ ID NO: 290 |
| | TGTCAAGCGGCTTTTTGATTATACAG AGA | TTTTCTGCTTCGGCATCAATCCTTGCAGGC | CAGCCCAGAGCTTTATATATGGTTACAG |
| 411 | SEQ ID NO: 303 | SEQ ID NO: 292 | SEQ ID NO: 290 |
| | CTGTCAAGCGGCTTTTTTGATTATACA G | TTCTGCTTCGGCATCAATCCTTGCAGGC | CAGCCCAGAGCTTTATATATGGTTACAG |
| 412 | SEQ ID NO: 293 | SEQ ID NO: 294 | SEQ ID NO: 304 |
| | TCAAGCGGCTTTTTTGATTATACAGAG A | TTTTCTGCTTCGGCATCAATCCTTGCAGGC | GAACAGCCCAGAGCTTTATATATGGTTAC |
| 413 | SEQ ID NO: 305 | SEQ ID NO: 294 | SEQ ID NO: 290 |
| | CTGTCAAGCGGCTTTTTGATTATACA GA | TTTTCTGCTTCGGCATCAATCCTTGCAGGC | CAGCCCAGAGCTTTATATATGGTTACAG |
| 414 | SEQ ID NO: 288 | SEQ ID NO: 289 | SEQ ID NO: 304 |
| | TCAAGCGGCTTTTTTGATTATACAGAG AAAT | TTTCTGCTTCGGCATCAATCCTTGCAGGC | GAACAGCCCAGAGCTTTATATATGGTTAC |
| 415 | SEQ ID NO: 305 | SEQ ID NO: 289 | SEQ ID NO: 290 |
| | CTGTCAAGCGGCTTTTTTGATTATACA GA | TTTCTGCTTCGGCATCAATCCTTGCAGGC | CAGCCCAGAGCTTTATATATGGTTACAG |
| 416 | SEQ ID NO: 303 | SEQ ID NO: 294 | SEQ ID NO: 290 |
| | CTGTCAAGCGGCTTTTTTGATTATACA G | TTTTCTGCTTCGGCATCAATCCTTGCAGGC | CAGCCCAGAGCTTTATATATGGTTACAG |
| 417 | SEQ ID NO: 249 | SEQ ID NO: 257 | SEQ ID NO: 306 |
| | ACCGTCTGGCGAAATTGTATTTAATGA | AGGCTTTACCTCGCACAGTCGCTATCCAA | CCACATACAGACCTATCAGCTTATCCA |
| 418 | SEQ ID NO: 298 | SEQ ID NO: 289 | SEQ ID NO: 304 |
| | GTCAAGCGGCTTTTTTGATTATACAGA GA | TTTCTGCTTCGGCATCAATCCTTGCAGGC | GAACAGCCCAGAGCTTTATATATGGTTAC |
| 419 | SEQ ID NO: 307 | SEQ ID NO: 292 | SEQ ID NO: 290 |
| | ACTGTCAAGCGGCTTTTTTGATTATAC A | TTCTGCTTCGGCATCAATCCTTGCAGGC | CAGCCCAGAGCTTTATATATGGTTACAG |
| 420 | SEQ ID NO: 249 | SEQ ID NO: 257 | SEQ ID NO: 308 |
| | ACCGTCTGGCGAAATTGTATTTAATGA | AGGCTTTACCTCGCACAGTCGCTATCCAA | TCCACATACAGACCTATCAGCTTATCC |
| 421 | SEQ ID NO: 309 | SEQ ID NO: 294 | SEQ ID NO: 290 |
| | ACTGTCAAGCGGCTTTTTTGATTATAC AG | TTTTCTGCTTCGGCATCAATCCTTGCAGGC | CAGCCCAGAGCTTTATATATGGTTACAG |
| 422 | SEQ ID NO: 310 | SEQ ID NO: 289 | SEQ ID NO: 290 |
| | ACTGTCAAGCGGCTTTTTTGATTATAC AGA | TTTCTGCTTCGGCATCAATCCTTGCAGGC | CAGCCCAGAGCTTTATATATGGTTACAG |
| 423 | SEQ ID NO: 309 | SEQ ID NO: 292 | SEQ ID NO: 290 |
| | ACTGTCAAGCGGCTTTTTTGATTATAC AG | TTCTGCTTCGGCATCAATCCTTGCAGGC | CAGCCCAGAGCTTTATATATGGTTACAG |
| 424 | SEQ ID NO: 311 | SEQ ID NO: 261 | SEQ ID NO: 258 |
| | GGGCTGTTCGGGTTTTTCCA | CCAGGCTTTACCTCGCACAGTCGCTATCC | GAACGATAGACCAATGCCTTTCATCA |
| 425 | SEQ ID NO: 249 | SEQ ID NO: 257 | SEQ ID NO: 312 |
| | ACCGTCTGGCGAAATTGTATTTAATGA | AGGCTTTACCTCGCACAGTCGCTATCCAA | TTCCACATACAGACCTATCAGCTTATCC |
| 426 | SEQ ID NO: 313 | SEQ ID NO: 292 | SEQ ID NO: 290 |
| | AACTGTCAAGCGGCTTTTTTGATTATA CA | TTCTGCTTCGGCATCAATCCTTGCAGGC | CAGCCCAGAGCTTTATATATGGTTACAG |
| 427 | SEQ ID NO: 314 | SEQ ID NO: 292 | SEQ ID NO: 290 |
| | AACTGTCAAGCGGCTTTTTTGATTATA CAG | TTCTGCTTCGGCATCAATCCTTGCAGGC | CAGCCCAGAGCTTTATATATGGTTACAG |
| 428 | SEQ ID NO: 303 | SEQ ID NO: 292 | SEQ ID NO: 304 |
| | CTGTCAAGCGGCTTTTTGATTATACA G | TTCTGCTTCGGCATCAATCCTTGCAGGC | GAACAGCCCAGAGCTTTATATATGGTTAC |
| 429 | SEQ ID NO: 315 | SEQ ID NO: 292 | SEQ ID NO: 290 |
| | GAACTGTCAAGCGGCTTTTTTGATTAT AC | TTCTGCTTCGGCATCAATCCTTGCAGGC | CAGCCCAGAGCTTTATATATGGTTACAG |
| 430 | SEQ ID NO: 316 | SEQ ID NO: 292 | SEQ ID NO: 290 |
| | CGAACTGTCAAGCGGCTTTTTTGAT | TTCTGCTTCGGCATCAATCCTTGCAGGC | CAGCCCAGAGCTTTATATATGGTTACAG |
| 431 | SEQ ID NO: 317 | SEQ ID NO: 292 | SEQ ID NO: 290 |
| | TCGAACTGTCAAGCGGCTTTTTT | TTCTGCTTCGGCATCAATCCTTGCAGGC | CAGCCCAGAGCTTTATATATGGTTACAG |
| 432 | SEQ ID NO: 318 | SEQ ID NO: 292 | SEQ ID NO: 290 |
| | ATCGAACTGTCAAGCGGCTTTTT | TTCTGCTTCGGCATCAATCCTTGCAGGC | CAGCCCAGAGCTTTATATATGGTTACAG |
| 433 | SEQ ID NO: 314 | SEQ ID NO: 319 | SEQ ID NO: 320 |
| | AACTGTCAAGCGGCTTTTTGATTATA CAG | TCTGCTTCGGCATCAATCCTTGCAGGC | CCGAACAGCCCAGAGCTTTA |
| 434 | SEQ ID NO: 321 | SEQ ID NO: 261 | SEQ ID NO: 258 |
| | AACCATATATAAAGCTCTGGGCTGTTC | CCAGGCTTTACCTCGCACAGTCGCTATCC | GAACGATAGACCAATGCCTTTCATCA |
| 435 | SEQ ID NO: 322 | SEQ ID NO: 323 | SEQ ID NO: 324 |
| | CTGCAAGGATTGATGCCGAAGCA | ATCCACTCTGGAAAAACCCGAACAGCCCAGA | GGATAGCGACTGTGCGAGGTA |
| 436 | SEQ ID NO: 325 | SEQ ID NO: 273 | SEQ ID NO: 274 |
| | GTCAAGCGGCTTTTTTGATTATACAGA GAAATA | CTGGAAAAACCCGAACAGCCCAGAGCTT | GCCAGACGGTGTATAAAACATATCCA |
| 437 | SEQ ID NO: 305 | SEQ ID NO: 273 | SEQ ID NO: 274 |
| | CTGTCAAGCGGCTTTTTTGATTATACA GA | CTGGAAAAACCCGAACAGCCCAGAGCTT | GCCAGACGGTGTATAAAACATATCCA |
| 438 | SEQ ID NO: 307 | SEQ ID NO: 273 | SEQ ID NO: 274 |
| | ACTGTCAAGCGGCTTTTTTGATTATAC A | CTGGAAAAACCCGAACAGCCCAGAGCTT | GCCAGACGGTGTATAAAACATATCCA |
| 439 | SEQ ID NO: 326 | SEQ ID NO: 273 | SEQ ID NO: 274 |
| | CGAACTGTCAAGCGGCTTTTTTGATTA | CTGGAAAAACCCGAACAGCCCAGAGCTT | GCCAGACGGTGTATAAAACATATCCA |
| 440 | SEQ ID NO: 327 | SEQ ID NO: 265 | SEQ ID NO: 266 |
| | GTCAAGCGGCTTTTTTGATTATACAGA GAAAT | CCACTCTGGAAAAACCCGAACAGCCCAGA | CAATTTCGCCAGACGGTGTATAAAACATA |
| 441 | SEQ ID NO: 325 | SEQ ID NO: 265 | SEQ ID NO: 266 |
| | GTCAAGCGGCTTTTTTGATTATACAGA GAAATA | CCACTCTGGAAAAACCCGAACAGCCCAGA | CAATTTCGCCAGACGGTGTATAAAACATA |
| 442 | SEQ ID NO: 328 | SEQ ID NO: 273 | SEQ ID NO: 274 |
| | AATCGAACTGTCAAGCGGCTTTTTT | CTGGAAAAACCCGAACAGCCCAGAGCTT | GCCAGACGGTGTATAAAACATATCCA |
| 443 | SEQ ID NO: 329 | SEQ ID NO: 273 | SEQ ID NO: 274 |
| | AAATCGAACTGTCAAGCGGCTTTTT | CTGGAAAAACCCGAACAGCCCAGAGCTT | GCCAGACGGTGTATAAAACATATCCA |
| 444 | SEQ ID NO: 330 | SEQ ID NO: 273 | SEQ ID NO: 274 |
| | GAAATCGAACTGTCAAGCGGCTTTT | CTGGAAAAACCCGAACAGCCCAGAGCTT | GCCAGACGGTGTATAAAACATATCCA |
| 445 | SEQ ID NO: 331 | SEQ ID NO: 273 | SEQ ID NO: 274 |
| | GAAATCGAACTGTCAAGCGGCTTTTT | CTGGAAAAACCCGAACAGCCCAGAGCTT | GCCAGACGGTGTATAAAACATATCCA |
| 446 | SEQ ID NO: 332 | SEQ ID NO: 265 | SEQ ID NO: 266 |
| | ATCGAACTGTCAAGCGGCTTTTTT | CCACTCTGGAAAAACCCGAACAGCCCAGA | CAATTTCGCCAGACGGTGTATAAAACATA |
| 447 | SEQ ID NO: 333 | SEQ ID NO: 265 | SEQ ID NO: 266 |
| | AATCGAACTGTCAAGCGGCTTTT | CCACTCTGGAAAAACCCGAACAGCCCAGA | CAATTTCGCCAGACGGTGTATAAAACATA |

| ***VanE* Sets** | | | |
|---|---|---|---|
| 448 | SEQ ID NO: 334 | SEQ ID NO: 335 | SEQ ID NO: 336 |
| | GGTATCGGAGCTGCAGCAAT | TCCCACAGCCAATTTCTACCCCTTTCACTTCC T | CTGATTTGGTCACATTCTCCAACGA |
| 449 | SEQ ID NO: 337 | SEQ ID NO: 335 | SEQ ID NO: 336 |
| | TGGTGTAAAAAGCACCCCTAGTATGA | TCCCACAGCCAATTTCTACCCCTTTCACTTCC T | CTGATTTGGTCACATTCTCCAACGA |
| 450 | SEQ ID NO: 338 | SEQ ID NO: 335 | SEQ ID NO: 336 |
| | AGACGAAGCTTCAAAATATGATAGCC GTAT | TCCCACAGCCAATTTCTACCCCTTTCACTTCC T | CTGATTTGGTCACATTCTCCAACGA |
| 451 | SEQ ID NO: 339 | SEQ ID NO: 335 | SEQ ID NO: 336 |
| | CAGCAATCTCCATGAATAAAATAATG CTCCAT | TCCCACAGCCAATTTCTACCCCTTTCACTTCC T | CTGATTTGGTCACATTCTCCAACGA |
| 452 | SEQ ID NO: 340 | SEQ ID NO: 335 | SEQ ID NO: 336 |
| | TGGTGTAAAAAGCACCCCTAGTATGAT | TCCCACAGCCAATTTCTACCCCTTTCACTTCC T | CTGATTTGGTCACATTCTCCAACGA |
| 453 | SEQ ID NO: 341 | SEQ ID NO: 335 | SEQ ID NO: 336 |
| | TTGGTGTAAAAAGCACCCCTAGTATGA | TCCCACAGCCAATTTCTACCCCTTTCACTTCC T | CTGATTTGGTCACATTCTCCAACGA |
| 454 | SEQ ID NO: 342 | SEQ ID NO: 335 | SEQ ID NO: 336 |
| | TTGGTGTAAAAGCACCCCTAGTATGA T | TCCCACAGCCAATTTCTACCCCTTTCACTTCC T | CTGATTTGGTCACATTCTCCAACGA |
| 455 | SEQ ID NO: 343 | SEQ ID NO: 335 | SEQ ID NO: 336 |
| | TGGTGTAAAAAGCACCCCTAGTATGAT TA | TCCCACAGCCAATTTCTACCCCTTTCACTTCC T | CTGATTTGGTCACATTCTCCAACGA |
| 456 | SEQ ID NO: 344 | SEQ ID NO: 335 | SEQ ID NO: 336 |
| | TTGGTGTAAAAGCACCCCTAGTATGA TTA | TCCCACAGCCAATTTCTACCCCTTTCACTTCC T | CTGATTTGGTCACATTCTCCAACGA |
| 457 | SEQ ID NO: 345 | SEQ ID NO: 335 | SEQ ID NO: 336 |
| | ACCCCTAGTATGATTATAGAAAAGGG ACAAGA | TCCCACAGCCAATTTCTACCCCTTTCACTTCC T | CTGATTTGGTCACATTCTCCAACGA |
| 458 | SEQ ID NO: 346 | SEQ ID NO: 335 | SEQ ID NO: 336 |
| | AGGGACAAGACCTACAAAAAGTCGAT | TCCCACAGCCAATTTCTACCCCTTTCACTTCC T | CTGATTTGGTCACATTCTCCAACGA |
| 459 | SEQ ID NO: 347 | SEQ ID NO: 335 | SEQ ID NO: 336 |
| | AAAGGGACAAGACCTACAAAAAGTCG AT | TCCCACAGCCAATTTCTACCCCTTTCACTTCC T | CTGATTTGGTCACATTCTCCAACGA |
| 460 | SEQ ID NO: 348 | SEQ ID NO: 335 | SEQ ID NO: 336 |
| | TTGGTGTAAAAAGCACCCCTAGTATGA TT | TCCCACAGCCAATTTCTACCCCTTTCACTTCC T | CTGATTTGGTCACATTCTCCAACGA |
| 461 | SEQ ID NO: 349 | SEQ ID NO: 335 | SEQ ID NO: 336 |

| **Group No.** | **Forward Primer** | **Probe** | **Reverse Primer** |
|---|---|---|---|
| | TGCAGCAATCTCCATGAATAAAATAAT GCT | TCCCACAGCCAATTTCTACCCCTTTCACTTCC T | CTGATTTGGTCACATTCTCCAACGA |
| 462 | SEQ ID NO: 350 | SEQ ID NO: 335 | SEQ ID NO: 336 |
| | TAGACGAAGCTTCAAAATATGATAGC CGTAT | TCCCACAGCCAATTTCTACCCCTTTCACTTCC T | CTGATTTGGTCACATTCTCCAACGA |
| 463 | SEQ ID NO: 351 | SEQ ID NO: 335 | SEQ ID NO: 336 |
| | GAGCTGCAGCAATCTCCATGAATAAA AT | TCCCACAGCCAATTTCTACCCCTTTCACTTCC T | CTGATTTGGTCACATTCTCCAACGA |
| 464 | SEQ ID NO: 352 | SEQ ID NO: 335 | SEQ ID NO: 336 |
| | GAGCTGCAGCAATCTCCATGAATAAA ATAA | TCCCACAGCCAATTTCTACCCCTTTCACTTCC T | CTGATTTGGTCACATTCTCCAACGA |
| 465 | SEQ ID NO: 353 | SEQ ID NO: 335 | SEQ ID NO: 336 |
| | TGAGGCAGGCTCATCAAAAGG | TCCCACAGCCAATTTCTACCCCTTTCACTTCC T | CTGATTTGGTCACATTCTCCAACGA |
| 466 | SEQ ID NO: 354 | SEQ ID NO: 335 | SEQ ID NO: 336 |
| | TGGTGTAAAAAGCACCCCTAGTATGAT T | TCCCACAGCCAATTTCTACCCCTTTCACTTCC T | CTGATTTGGTCACATTCTCCAACGA |
| 467 | SEQ ID NO: 355 | SEQ ID NO: 335 | SEQ ID NO: 336 |
| | GCAATCTCCATGAATAAAATAATGCTC CATCA | TCCCACAGCCAATTTCTACCCCTTTCACTTCC T | CTGATTTGGTCACATTCTCCAACGA |
| 468 | SEQ ID NO: 356 | SEQ ID NO: 335 | SEQ ID NO: 336 |
| | AGCTGCAGCAATCTCCATGAATAAAA T | TCCCACAGCCAATTTCTACCCCTTTCACTTCC T | CTGATTTGGTCACATTCTCCAACGA |
| 469 | SEQ ID NO: 357 | SEQ ID NO: 335 | SEQ ID NO: 336 |
| | AAAAGGGACAAGACCTACAAAAAGTC GAT | TCCCACAGCCAATTTCTACCCCTTTCACTTCC T | CTGATTTGGTCACATTCTCCAACGA |
| 470 | SEQ ID NO: 358 | SEQ ID NO: 335 | SEQ ID NO: 336 |
| | CACCCCTAGTATGATTATAGAAAAGG GACAA | TCCCACAGCCAATTTCTACCCCTTTCACTTCC T | CTGATTTGGTCACATTCTCCAACGA |
| 471 | SEQ ID NO: 359 | SEQ ID NO: 335 | SEQ ID NO: 336 |
| | CCCCTAGTATGATTATAGAAAAGGGA CAAGAC | TCCCACAGCCAATTTCTACCCCTTTCACTTCC T | CTGATTTGGTCACATTCTCCAACGA |
| 472 | SEQ ID NO: 360 | SEQ ID NO: 335 | SEQ ID NO: 336 |
| | GAGCTGCAGCAATCTCCATGAATAAA ATA | TCCCACAGCCAATTTCTACCCCTTTCACTTCC T | CTGATTTGGTCACATTCTCCAACGA |
| 473 | SEQ ID NO: 361 | SEQ ID NO: 335 | SEQ ID NO: 336 |
| | CCCCTAGTATGATTATAGAAAAGGGA CAAGA | TCCCACAGCCAATTTCTACCCCTTTCACTTCC T | CTGATTTGGTCACATTCTCCAACGA |
| 474 | SEQ ID NO: 362 | SEQ ID NO: 335 | SEQ ID NO: 336 |
| | GGCAGGCTCATCAAAAGGAATTAGC | TCCCACAGCCAATTTCTACCCCTTTCACTTCC T | CTGATTTGGTCACATTCTCCAACGA |
| 475 | SEQ ID NO: 363 | SEQ ID NO: 335 | SEQ ID NO: 336 |
| | AAGGAATTAGCAAGGTAGAACAAAAA AGTGATT | TCCCACAGCCAATTTCTACCCCTTTCACTTCC T | CTGATTTGGTCACATTCTCCAACGA |
| 476 | SEQ ID NO: 364 | SEQ ID NO: 335 | SEQ ID NO: 336 |
| | AGCTGCAGCAATCTCCATGAATAAAA TAAT | TCCCACAGCCAATTTCTACCCCTTTCACTTCC T | CTGATTTGGTCACATTCTCCAACGA |
| 477 | SEQ ID NO: 365 | SEQ ID NO: 335 | SEQ ID NO: 336 |
| | CCATCAATTTGCTGAAACAATTGGTGT AAAA | TCCCACAGCCAATTTCTACCCCTTTCACTTCC T | CTGATTTGGTCACATTCTCCAACGA |
| 478 | SEQ ID NO: 366 | SEQ ID NO: 335 | SEQ ID NO: 336 |
| | AGCTGCAGCAATCTCCATGAATAAAA TAA | TCCCACAGCCAATTTCTACCCCTTTCACTTCC T | CTGATTTGGTCACATTCTCCAACGA |
| 479 | SEQ ID NO: 367 | SEQ ID NO: 335 | SEQ ID NO: 336 |
| | CTGCAGCAATCTCCATGAATAAAATA ATGC | TCCCACAGCCAATTTCTACCCCTTTCACTTCC T | CTGATTTGGTCACATTCTCCAACGA |
| 480 | SEQ ID NO: 368 | SEQ ID NO: 335 | SEQ ID NO: 336 |
| | GACGAAGCTTCAAAATATGATAGCCG TAT | TCCCACAGCCAATTTCTACCCCTTTCACTTCC T | CTGATTTGGTCACATTCTCCAACGA |
| 481 | SEQ ID NO: 369 | SEQ ID NO: 335 | SEQ ID NO: 336 |
| | CCATCAATTTGCTGAAACAATTGGTGT AAA | TCCCACAGCCAATTTCTACCCCTTTCACTTCC T | CTGATTTGGTCACATTCTCCAACGA |
| 482 | SEQ ID NO: 370 | SEQ ID NO: 335 | SEQ ID NO: 336 |
| | AGCTGCAGCAATCTCCATGAATAAA | TCCCACAGCCAATTTCTACCCCTTTCACTTCC T | CTGATTTGGTCACATTCTCCAACGA |
| 483 | SEQ ID NO: 371 | SEQ ID NO: 335 | SEQ ID NO: 336 |
| | TTGGTGTAAAAAGCACCCCTAGTATG | TCCCACAGCCAATTTCTACCCCTTTCACTTCC T | CTGATTTGGTCACATTCTCCAACGA |
| 484 | SEQ ID NO: 372 | SEQ ID NO: 335 | SEQ ID NO: 336 |
| | GAAAAGGGACAAGACCTACAAAAAGT CGAT | TCCCACAGCCAATTTCTACCCCTTTCACTTCC T | CTGATTTGGTCACATTCTCCAACGA |
| 485 | SEQ ID NO: 373 | SEQ ID NO: 335 | SEQ ID NO: 336 |
| | AGCTGCAGCAATCTCCATGAATAAAA | TCCCACAGCCAATTTCTACCCCTTTCACTTCC | CTGATTTGGTCACATTCTCCAACGA |
| | TA | T | |
| 486 | SEQ ID NO: 374 | SEQ ID NO: 335 | SEQ ID NO: 336 |
| | GGTGTAAAAAGCACCCCTAGTATGATT | TCCCACAGCCAATTTCTACCCCTTTCACTTCC T | CTGATTTGGTCACATTCTCCAACGA |
| 487 | SEQ ID NO: 375 | SEQ ID NO: 335 | SEQ ID NO: 336 |
| | GCTGCAGCAATCTCCATGAATAAAAT AATGC | TCCCACAGCCAATTTCTACCCCTTTCACTTCC T | CTGATTTGGTCACATTCTCCAACGA |
| 488 | SEQ ID NO: 376 | SEQ ID NO: 335 | SEQ ID NO: 336 |
| | ACCCCTAGTATGATTATAGAAAAGGG ACAA | TCCCACAGCCAATTTCTACCCCTTTCACTTCC T | CTGATTTGGTCACATTCTCCAACGA |
| 489 | SEQ ID NO: 377 | SEQ ID NO: 335 | SEQ ID NO: 336 |
| | AGTATGATTATAGAAAAGGGACAAGA CCTACA | TCCCACAGCCAATTTCTACCCCTTTCACTTCC T | CTGATTTGGTCACATTCTCCAACGA |
| 490 | SEQ ID NO: 378 | SEQ ID NO: 335 | SEQ ID NO: 336 |
| | TGGTGTAAAAAGCACCCCTAGTATG | TCCCACAGCCAATTTCTACCCCTTTCACTTCC T | CTGATTTGGTCACATTCTCCAACGA |
| 491 | SEQ ID NO: 379 | SEQ ID NO: 335 | SEQ ID NO: 336 |
| | AGCTGCAGCAATCTCCATGAATAA | TCCCACAGCCAATTTCTACCCCTTTCACTTCC T | CTGATTTGGTCACATTCTCCAACGA |
| 492 | SEQ ID NO: 380 | SEQ ID NO: 335 | SEQ ID NO: 336 |
| | ATTTGCTGAAACAATTGGTGTAAAAA GCA | TCCCACAGCCAATTTCTACCCCTTTCACTTCC T | CTGATTTGGTCACATTCTCCAACGA |
| 493 | SEQ ID NO: 347 | SEQ ID NO: 335 | SEQ ID NO: 381 |
| | AAAGGGACAAGACCTACAAAAAGTCG AT | TCCCACAGCCAATTTCTACCCCTTTCACTTCC T | CCACAAGACTGATTTGGTCACATTCT |
| 494 | SEQ ID NO: 348 | SEQ ID NO: 335 | SEQ ID NO: 381 |
| | TTGGTGTAAAAAGCACCCCTAGTATGA TT | TCCCACAGCCAATTTCTACCCCTTTCACTTCC T | CCACAAGACTGATTTGGTCACATTCT |
| 495 | SEQ ID NO: 349 | SEQ ID NO: 335 | SEQ ID NO: 381 |
| | TGCAGCAATCTCCATGAATAAAATAAT GCT | TCCCACAGCCAATTTCTACCCCTTTCACTTCC T | CCACAAGACTGATTTGGTCACATTCT |
| 496 | SEQ ID NO: 350 | SEQ ID NO: 335 | SEQ ID NO: 381 |
| | TAGACGAAGCTTCAAAATATGATAGC CGTAT | TCCCACAGCCAATTTCTACCCCTTTCACTTCC T | CCACAAGACTGATTTGGTCACATTCT |
| 497 | SEQ ID NO: 382 | SEQ ID NO: 335 | SEQ ID NO: 381 |
| | TCCATCAATTTGCTGAAACAATTGGTG TAAAA | TCCCACAGCCAATTTCTACCCCTTTCACTTCC T | CCACAAGACTGATTTGGTCACATTCT |
| 498 | SEQ ID NO: 383 | SEQ ID NO: 335 | SEQ ID NO: 381 |
| | GAGCTGCAGCAATCTCCATGA | TCCCACAGCCAATTTCTACCCCTTTCACTTCC T | CCACAAGACTGATTTGGTCACATTCT |
| 499 | SEQ ID NO: 355 | SEQ ID NO: 335 | SEQ ID NO: 381 |
| | GCAATCTCCATGAATAAAATAATGCTC CATCA | TCCCACAGCCAATTTCTACCCCTTTCACTTCC T | CCACAAGACTGATTTGGTCACATTCT |
| 500 | SEQ ID NO: 359 | SEQ ID NO: 335 | SEQ ID NO: 381 |
| | CCCCTAGTATGATTATAGAAAAGGGA CAAGAC | TCCCACAGCCAATTTCTACCCCTTTCACTTCC T | CCACAAGACTGATTTGGTCACATTCT |
| 501 | SEQ ID NO: 360 | SEQ ID NO: 335 | SEQ ID NO: 381 |
| | GAGCTGCAGCAATCTCCATGAATAAA ATA | TCCCACAGCCAATTTCTACCCCTTTCACTTCC T | CCACAAGACTGATTTGGTCACATTCT |
| 502 | SEQ ID NO: 338 | SEQ ID NO: 335 | SEQ ID NO: 381 |
| | AGACGAAGCTTCAAAATATGATAGCC GTAT | TCCCACAGCCAATTTCTACCCCTTTCACTTCC T | CCACAAGACTGATTTGGTCACATTCT |
| 503 | SEQ ID NO: 384 | SEQ ID NO: 335 | SEQ ID NO: 381 |
| | TCAATTTGCTGAAACAATTGGTGTAAA AAGC | TCCCACAGCCAATTTCTACCCCTTTCACTTCC T | CCACAAGACTGATTTGGTCACATTCT |
| 504 | SEQ ID NO: 385 | SEQ ID NO: 335 | SEQ ID NO: 381 |
| | GACAAGACCTACAAAAAGTCGATGAA TTTGC | TCCCACAGCCAATTTCTACCCCTTTCACTTCC T | CCACAAGACTGATTTGGTCACATTCT |
| 505 | SEQ ID NO: 386 | SEQ ID NO: 335 | SEQ ID NO: 381 |
| | GAGCTGCAGCAATCTCCATGAAT | TCCCACAGCCAATTTCTACCCCTTTCACTTCC T | CCACAAGACTGATTTGGTCACATTCT |
| 506 | SEQ ID NO: 341 | SEQ ID NO: 335 | SEQ ID NO: 381 |
| | TTGGTGTAAAAAGCACCCCTAGTATGA | TCCCACAGCCAATTTCTACCCCTTTCACTTCC T | CCACAAGACTGATTTGGTCACATTCT |
| 507 | SEQ ID NO: 342 | SEQ ID NO: 335 | SEQ ID NO: 381 |
| | TTGGTGTAAAAAGCACCCCTAGTATGA T | TCCCACAGCCAATTTCTACCCCTTTCACTTCC T | CCACAAGACTGATTTGGTCACATTCT |
| 508 | SEQ ID NO: 344 | SEQ ID NO: 335 | SEQ ID NO: 381 |
| | TTGGTGTAAAAGCACCCCTAGTATGA TTA | TCCCACAGCCAATTTCTACCCCTTTCACTTCC T | CCACAAGACTGATTTGGTCACATTCT |
| 509 | SEQ ID NO: 387 | SEQ ID NO: 335 | SEQ ID NO: 381 |
| | ATCAATTTGCTGAAACAATTGGTGTAA AAAGC | TCCCACAGCCAATTTCTACCCCTTTCACTTCC T | CCACAAGACTGATTTGGTCACATTCT |

| ***vanD* Sets** | | | |
|---|---|---|---|
| 510 | SEQ ID NO: 388 | SEQ ID NO: 389 | SEQ ID NO: 390 |
| | TGCGCCATACTGGGAAACG | TGATCCACCTCGCCAGCCATGAGATCATTT | AGCCGTGTCTCAGCTCAATC |
| 511 | SEQ ID NO: 391 | SEQ ID NO: 392 | SEQ ID NO: 393 |
| | CTGCGCCATACTGGGAAACG | TCTGATCCACCTCGCCAGCCATGAGA | TTAAAAAAGCCGTGTCTCAGCTCAA |
| 512 | SEQ ID NO: 394 | SEQ ID NO: 392 | SEQ ID NO: 395 |
| | GGTAGGCTGCGCCATACTG | TCTGATCCACCTCGCCAGCCATGAGA | AAAAGCCGTGTCTCAGCTCAA |
| 513 | SEQ ID NO: 396 | SEQ ID NO: 397 | SEQ ID NO: 398 |
| | GCTGCGCCATACTGGGAAAC | TCTGATCCACCTCGCCAGCCATGAGATCATTT | CTTAAAAAAGCCGTGTCTCAGCTCAA |
| 514 | SEQ ID NO: 399 | SEQ ID NO: 400 | SEQ ID NO: 401 |
| | AGATTTTGATTGAAGAGGCCGTTACC | TTCCCAGTATGGCGCAGCCTACCTCAC | CTCGCCAGCCATGAGATCATTT |
| 515 | SEQ ID NO: 399 | SEQ ID NO: 402 | SEQ ID NO: 401 |
| | AGATTTTGATTGAAGAGGCCGTTACC | CCCAGTATGGCGCAGCCTACCTCACT | CTCGCCAGCCATGAGATCATTT |
| 516 | SEQ ID NO: 399 | SEQ ID NO: 403 | SEQ ID NO: 401 |
| | AGATTTTGATTGAAGAGGCCGTTACC | TGGCGCAGCCTACCTCACTCCC | CTCGCCAGCCATGAGATCATTT |
| 517 | SEQ ID NO: 399 | SEQ ID NO: 404 | SEQ ID NO: 401 |
| | AGATTTTGATTGAAGAGGCCGTTACC | TCCCAGTATGGCGCAGCCTACCTCA | CTCGCCAGCCATGAGATCATTT |
| 518 | SEQ ID NO: 399 | SEQ ID NO: 405 | SEQ ID NO: 401 |
| | AGATTTTGATTGAAGAGGCCGTTACC | TCCCAGTATGGCGCAGCCTACCTCAC | CTCGCCAGCCATGAGATCATTT |
| 519 | SEQ ID NO: 399 | SEQ ID NO: 406 | SEQ ID NO: 401 |
| | AGATTTTGATTGAAGAGGCCGTTACC | AGTATGGCGCAGCCTACCTCACTCCC | CTCGCCAGCCATGAGATCATTT |
| 520 | SEQ ID NO: 399 | SEQ ID NO: 407 | SEQ ID NO: 401 |
| | AGATTTTGATTGAAGAGGCCGTTACC | TTTCCCAGTATGGCGCAGCCTACCTCA | CTCGCCAGCCATGAGATCATTT |
| 521 | SEQ ID NO: 399 | SEQ ID NO: 408 | SEQ ID NO: 401 |
| | AGATTTTGATTGAAGAGGCCGTTACC | TTTCCCAGTATGGCGCAGCCTACCTCAC | CTCGCCAGCCATGAGATCATTT |
| 522 | SEQ ID NO: 409 | SEQ ID NO: 410 | SEQ ID NO: 411 |
| | AGGTAGGCTGCGCCATACTG | CTGATCCACCTCGCCAGCCATGAGATCATTT | AAAAAAGCCGTGTCTCAGCTCAAT |
| 523 | SEQ ID NO: 399 | SEQ ID NO: 412 | SEQ ID NO: 401 |
| | AGATTTTGATTGAAGAGGCCGTTACC | CCCAGTATGGCGCAGCCTACCTCAC | CTCGCCAGCCATGAGATCATTT |
| 524 | SEQ ID NO: 399 | SEQ ID NO: 413 | SEQ ID NO: 401 |
| | AGATTTTGATTGAAGAGGCCGTTACC | ATGGCGCAGCCTACCTCACTCCC | CTCGCCAGCCATGAGATCATTT |
| 525 | SEQ ID NO: 399 | SEQ ID NO: 414 | SEQ ID NO: 401 |
| | AGATTTTGATTGAAGAGGCCGTTACC | TTCCCAGTATGGCGCAGCCTACCTCA | CTCGCCAGCCATGAGATCATTT |
| 526 | SEQ ID NO: 415 | SEQ ID NO: 406 | SEQ ID NO: 401 |
| | AAGATTTTGATTGAAGAGGCCGTTACC | AGTATGGCGCAGCCTACCTCACTCCC | CTCGCCAGCCATGAGATCATTT |
| 527 | SEQ ID NO: 415 | SEQ ID NO: 407 | SEQ ID NO: 401 |
| | AAGATTTTGATTGAAGAGGCCGTTACC | TTTCCCAGTATGGCGCAGCCTACCTCA | CTCGCCAGCCATGAGATCATTT |
| 528 | SEQ ID NO: 415 | SEQ ID NO: 408 | SEQ ID NO: 401 |
| | AAGATTTTGATTGAAGAGGCCGTTACC | TTTCCCAGTATGGCGCAGCCTACCTCAC | CTCGCCAGCCATGAGATCATTT |
| 529 | SEQ ID NO: 394 | SEQ ID NO: 410 | SEQ ID NO: 393 |
| | GGTAGGCTGCGCCATACTG | CTGATCCACCTCGCCAGCCATGAGATCATTT | TTAAAAAAGCCGTGTCTCAGCTCAA |
| 530 | SEQ ID NO: 415 | SEQ ID NO: 412 | SEQ ID NO: 401 |
| | AAGATTTTGATTGAAGAGGCCGTTACC | CCCAGTATGGCGCAGCCTACCTCAC | CTCGCCAGCCATGAGATCATTT |
| 531 | SEQ ID NO: 415 | SEQ ID NO: 413 | SEQ ID NO: 401 |
| | AAGATTTTGATTGAAGAGGCCGTTACC | ATGGCGCAGCCTACCTCACTCCC | CTCGCCAGCCATGAGATCATTT |
| 532 | SEQ ID NO: 415 | SEQ ID NO: 414 | SEQ ID NO: 401 |
| | AAGATTTTGATTGAAGAGGCCGTTACC | TTCCCAGTATGGCGCAGCCTACCTCA | CTCGCCAGCCATGAGATCATTT |
| 533 | SEQ ID NO: 415 | SEQ ID NO: 400 | SEQ ID NO: 401 |
| | AAGATTTTGATTGAAGAGGCCGTTACC | TTCCCAGTATGGCGCAGCCTACCTCAC | CTCGCCAGCCATGAGATCATTT |
| 534 | SEQ ID NO: 394 | SEQ ID NO: 389 | SEQ ID NO: 393 |
| | GGTAGGCTGCGCCATACTG | TGATCCACCTCGCCAGCCATGAGATCATTT | TTAAAAAAGCCGTGTCTCAGCTCAA |
| 535 | SEQ ID NO: 415 | SEQ ID NO: 402 | SEQ ID NO: 401 |
| | AAGATTTTGATTGAAGAGGCCGTTACC | CCCAGTATGGCGCAGCCTACCTCACT | CTCGCCAGCCATGAGATCATTT |
| 536 | SEQ ID NO: 415 | SEQ ID NO: 403 | SEQ ID NO: 401 |
| | AAGATTTTGATTGAAGAGGCCGTTACC | TGGCGCAGCCTACCTCACTCCC | CTCGCCAGCCATGAGATCATTT |
| 537 | SEQ ID NO: 415 | SEQ ID NO: 404 | SEQ ID NO: 401 |
| | AAGATTTTGATTGAAGAGGCCGTTACC | TCCCAGTATGGCGCAGCCTACCTCA | CTCGCCAGCCATGAGATCATTT |
| 538 | SEQ ID NO: 394 | SEQ ID NO: 397 | SEQ ID NO: 393 |
| | GGTAGGCTGCGCCATACTG | TCTGATCCACCTCGCCAGCCATGAGATCATTT | TTAAAAAAGCCGTGTCTCAGCTCAA |
| 539 | SEQ ID NO: 415 | SEQ ID NO: 405 | SEQ ID NO: 401 |
| | AAGATTTTGATTGAAGAGGCCGTTACC | TCCCAGTATGGCGCAGCCTACCTCAC | CTCGCCAGCCATGAGATCATTT |
| 540 | SEQ ID NO: 416 | SEQ ID NO: 400 | SEQ ID NO: 417 |
| | CAAGATTTTGATTGAAGAGGCCGTTAC C | TTCCCAGTATGGCGCAGCCTACCTCAC | CCTCGCCAGCCATGAGAT |
| 541 | SEQ ID NO: 396 | SEQ ID NO: 418 | SEQ ID NO: 419 |
| | GCTGCGCCATACTGGGAAAC | CAATCTGATCCACCTCGCCAGCCATGAGA | CCTGATGAATCTTAAAAAAGCCGTGTCT |
| 542 | SEQ ID NO: 396 | SEQ ID NO: 397 | SEQ ID NO: 419 |
| | GCTGCGCCATACTGGGAAAC | TCTGATCCACCTCGCCAGCCATGAGATCATTT | CCTGATGAATCTTAAAAAAGCCGTGTCT |
| 543 | SEQ ID NO: 391 | SEQ ID NO: 420 | SEQ ID NO: 421 |
| | CTGCGCCATACTGGGAAACG | CTCAATCTGATCCACCTCGCCAGCCATGAGA | TCCTGATGAATCTTAAAAAAGCCGTGTCT |
| 544 | SEQ ID NO: 396 | SEQ ID NO: 420 | SEQ ID NO: 419 |
| | GCTGCGCCATACTGGGAAAC | CTCAATCTGATCCACCTCGCCAGCCATGAGA | CCTGATGAATCTTAAAAAAGCCGTGTCT |
| 545 | SEQ ID NO: 396 | SEQ ID NO: 422 | SEQ ID NO: 419 |
| | GCTGCGCCATACTGGGAAAC | CTCAATCTGATCCACCTCGCCAGCCATGAGA T | CCTGATGAATCTTAAAAAAGCCGTGTCT |
| 546 | SEQ ID NO: 396 | SEQ ID NO: 423 | SEQ ID NO: 419 |
| | GCTGCGCCATACTGGGAAAC | CTCAATCTGATCCACCTCGCCAGCCATGAG | CCTGATGAATCTTAAAAAAGCCGTGTCT |
| 547 | SEQ ID NO: 396 | SEQ ID NO: 424 | SEQ ID NO: 419 |
| | GCTGCGCCATACTGGGAAAC | TCAATCTGATCCACCTCGCCAGCCATGAGA | CCTGATGAATCTTAAAAAAGCCGTGTCT |
| 548 | SEQ ID NO: 425 | SEQ ID NO: 406 | SEQ ID NO: 426 |
| | AGCAAGATTTTGATTGAAGAGGCCGTT A | AGTATGGCGCAGCCTACCTCACTCCC | CACCTCGCCAGCCATGA |
| 549 | SEQ ID NO: 396 | SEQ ID NO: 427 | SEQ ID NO: 419 |
| | GCTGCGCCATACTGGGAAAC | TCAATCTGATCCACCTCGCCAGCCATGAGAT | CCTGATGAATCTTAAAAAAGCCGTGTCT |
| 550 | SEQ ID NO: 428 | SEQ ID NO: 429 | SEQ ID NO: 430 |
| | GGCTTGCTTGAATTGTCAGGCATT | CACGGAGCTTTGAATATCGCATCCCACATAC GG | AACGGTATATGCAAGCGCCTTATC |
| 551 | SEQ ID NO: 391 | SEQ ID NO: 431 | SEQ ID NO: 421 |
| | CTGCGCCATACTGGGAAACG | AGCTCAATCTGATCCACCTCGCCAGCC | TCCTGATGAATCTTAAAAAAGCCGTGTCT |
| 552 | SEQ ID NO: 396 | SEQ ID NO: 432 | SEQ ID NO: 419 |
| | GCTGCGCCATACTGGGAAAC | TCAATCTGATCCACCTCGCCAGCCATGAG | CCTGATGAATCTTAAAAAAGCCGTGTCT |
| 553 | SEQ ID NO: 391 | SEQ ID NO: 433 | SEQ ID NO: 434 |
| | CTGCGCCATACTGGGAAACG | TCTGATCCACCTCGCCAGCCATGAGAT | CGGCTGTGCTTCCTGATGA |
| 554 | SEQ ID NO: 435 | SEQ ID NO: 436 | SEQ ID NO: 437 |
| | CCATACAAGGCTTGCTTGAATTGTCA | [?] ACGGAGCTTTGAATATCGCATCCCACATACG GAA | ATACCCGCATTTTTCACAACGGTAT |
| 555 | SEQ ID NO: 438 | SEQ ID NO: 420 | SEQ ID NO: 439 |
| | GGCCGTTACCGGGAGTGA | CTCAATCTGATCCACCTCGCCAGCCATGAGA | TTCCTGATGAATCTTAAAAAAGCCGTGTCT |
| 556 | SEQ ID NO: 440 | SEQ ID NO: 441 | SEQ ID NO: 442 |
| | AGGAAGCACAGCCGGAGAA | CCTCATCCGGTAAGGCGGCTGGAACT | AGCCAAGTATCCGGTAAATCTTCATTG |
| 557 | SEQ ID NO: 443 | SEQ ID NO: 392 | SEQ ID NO: 434 |
| | CCGTTACCGGGAGTGAGGTA | TCTGATCCACCTCGCCAGCCATGAGA | CGGCTGTGCTTCCTGATGA |
| 558 | SEQ ID NO: 399 | SEQ ID NO: 397 | SEQ ID NO: 444 |
| | AGATTTTGATTGAAGAGGCCGTTACC | TCTGATCCACCTCGCCAGCCATGAGATCATTT | TGATGAATCTTAAAAAAGCCGTGTCTCA |
| 559 | SEQ ID NO: 399 | SEQ ID NO: 410 | SEQ ID NO: 444 |
| | AGATTTTGATTGAAGAGGCCGTTACC | CTGATCCACCTCGCCAGCCATGAGATCATTT | TGATGAATCTTAAAAAAGCCGTGTCTCA |
| 560 | SEQ ID NO: 445 | SEQ ID NO: 446 | SEQ ID NO: 447 |
| | CGCTCTCGTATCCGGTCTTTG | CCGTTCCGGCTCCTCTTTTGGCGTGAATAA | TTCCGGCTGTGCTTCCTGAT |
| 561 | SEQ ID NO: 445 | SEQ ID NO: 446 | SEQ ID NO: 448 |
| | CGCTCTCGTATCCGGTCTTTG | CCGTTCCGGCTCCTCTTTTGGCGTGAATAA | CCGGCTGTGCTTCCTGATG |
| 562 | SEQ ID NO: 445 | SEQ ID NO: 446 | SEQ ID NO: 449 |
| | CGCTCTCGTATCCGGTCTTTG | CCGTTCCGGCTCCTCTTTTGGCGTGAATAA | TCCGGCTGTGCTTCCTGAT |
| 563 | SEQ ID NO: 445 | SEQ ID NO: 446 | SEQ ID NO: 450 |
| | CGCTCTCGTATCCGGTCTTTG | CCGTTCCGGCTCCTCTTTTGGCGTGAATAA | TCCTACCTCACTCCCGGAAAC |
| 564 | SEQ ID NO: 445 | SEQ ID NO: 446 | SEQ ID NO: 451 |
| | CGCTCTCGTATCCGGTCTTTG | CCGTTCCGGCTCCTCTTTTGGCGTGAATAA | CCTACCTCACTCCCGGAAAC |
| 565 | SEQ ID NO: 445 | SEQ ID NO: 446 | SEQ ID NO: 452 |
| | CGCTCTCGTATCCGGTCTTTG | CCGTTCCGGCTCCTCTTTTGGCGTGAATAA | GACCGCTGCCTGCAGTTC |
| 566 | SEQ ID NO: 445 | SEQ ID NO: 446 | SEQ ID NO: 453 |
| | CGCTCTCGTATCCGGTCTTTG | CCGTTCCGGCTCCTCTTTTGGCGTGAATAA | ATCCTACCTCACTCCCGGAAAC |
| 567 | SEQ ID NO: 445 | SEQ ID NO: 446 | SEQ ID NO: 454 |
| | CGCTCTCGTATCCGGTCTTTG | CCGTTCCGGCTCCTCTTTTGGCGTGAATAA | CATCCTACCTCACTCCCGGAAA |
| 568 | SEQ ID NO: 445 | SEQ ID NO: 446 | SEQ ID NO: 455 |
| | CGCTCTCGTATCCGGTCTTTG | CCGTTCCGGCTCCTCTTTTGGCGTGAATAA | CCGGCTGTGCTTCCTGAT |
| 569 | SEQ ID NO: 456 | SEQ ID NO: 457 | SEQ ID NO: 458 |
| | GGTTGTGAAAAATGCGGGAATTGAG | CTGCCCGTTCCGGCTCCTCTTTTGG | TCTGCCCGGCATACCTTATTCAC |
| 570 | SEQ ID NO: 445 | SEQ ID NO: 446 | SEQ ID NO: 459 |
| | CGCTCTCGTATCCGGTCTTTG | CCGTTCCGGCTCCTCTTTTGGCGTGAATAA | CAGTATGGCACATCCTACCTCACT |
| 571 | SEQ ID NO: 445 | SEQ ID NO: 446 | SEQ ID NO: 460 |
| | CGCTCTCGTATCCGGTCTTTG | CCGTTCCGGCTCCTCTTTTGGCGTGAATAA | CAGCCAAGTACCCGGTAAATCTTC |
| 572 | SEQ ID NO: 445 | SEQ ID NO: 446 | SEQ ID NO: 461 |
| | CGCTCTCGTATCCGGTCTTTG | CCGTTCCGGCTCCTCTTTTGGCGTGAATAA | GCTGTGCTTCCTGATGGATCTTAAAAA |
| 573 | SEQ ID NO: 462 | SEQ ID NO: 463 | SEQ ID NO: 464 |
| | CGTTCCGGCTCCTCTTTTGG | ACCGCTGCCTGCAGTTCCTCTGC | CGGAAACGGCCTCCTCAAC |
| 574 | SEQ ID NO: 465 | SEQ ID NO: 466 | SEQ ID NO: 467 |
| | GGCTCCTCTTTTGGCGTGAA | TGCAGTTCCTCTGCCCGGCATACCT | CTACCTCACTCCCGGAAACG |
| 575 | SEQ ID NO: 468 | SEQ ID NO: 469 | SEQ ID NO: 470 |
| | CCGTTCCGGCTCCTCTTTT | CTCTGCCCGGCATACCTTATTCACGCC | ACCGCTGCCTGCAGTT |
| 576 | SEQ ID NO: 471 | SEQ ID NO: 472 | SEQ ID NO: 473 |
| | CTGCTTGAGCTGTCCGGCATT | AGAACTCGAAACCCAGGTACCTCAATTCCCG C | TCCAGGCTGTCCCCCTTTT |
| 577 | SEQ ID NO: 474 | SEQ ID NO: 475 | SEQ ID NO: 476 |
| | TAAGGTATGCCGGGCAGAGGAA | TCCCGGAAACGGCCTCCTCAACCAAT | CCCAGTATGGCACATCCTACCT |
| 578 | SEQ ID NO: 477 | SEQ ID NO: 478 | SEQ ID NO: 479 |
| | GTCGCTCGCTTATATGGTTGTGAA | ACTCGAAACCCAGGTACCTCAATTCCCGCAT | AGGCTGTCCCCCTTTTGTAGA |
| 579 | SEQ ID NO: 480 | SEQ ID NO: 481 | SEQ ID NO: 482 |
| | CTCCTCTTTTGGCGTGAATAAGGT | TCTGTGACCGCTGCCTGCAGTTCC | AAACGGCCTCCTCAACCAAT |
| 580 | SEQ ID NO: 483 | SEQ ID NO: 484 | SEQ ID NO: 479 |
| | TGGATAAGTCGCTCGCTTATATGGT | ACTCGAAACCCAGGTACCTCAATTCCCGCA | AGGCTGTCCCCCTTTTGTAGA |
| 581 | SEQ ID NO: 483 | SEQ ID NO: 485 | SEQ ID NO: 486 |
| | TGGATAAGTCGCTCGCTTATATGGT | ACCCAGGTACCTCAATTCCCGCATTTTTCACA | CAGGCTGTCCCCCTTTTGTA |
| 582 | SEQ ID NO: 483 | SEQ ID NO: 487 | SEQ ID NO: 486 |
| | TGGATAAGTCGCTCGCTTATATGGT | AACCCAGGTACCTCAATTCCCGCATTTTTCAC A | CAGGCTGTCCCCCTTTTGTA |
| 583 | SEQ ID NO: 488 | SEQ ID NO: 489 | SEQ ID NO: 490 |
| | CCTCTTTTGGCGTGAATAAGGTATGC | CTCTGTGACCGCTGCCTGCAGTTCC | GAAACGGCCTCCTCAACCA |
| 584 | SEQ ID NO: 491 | SEQ ID NO: 492 | SEQ ID NO: 493 |
| | TCGCTCGCTTATATGGTTGTGAAAA | TCGAAACCCAGGTACCTCAATTCCCGCA | CAGGCTGTCCCCCTTTTGT |
| 585 | SEQ ID NO: 494 | SEQ ID NO: 485 | SEQ ID NO: 486 |
| | ATGGATAAGTCGCTCGCTTATATGGT | ACCCAGGTACCTCAATTCCCGCATTTTTCACA | CAGGCTGTCCCCCTTTTGTA |
| 586 | SEQ ID NO: 494 | SEQ ID NO: 487 | SEQ ID NO: 486 |
| | ATGGATAAGTCGCTCGCTTATATGGT | AACCCAGGTACCTCAATTCCCGCATTTTTCAC A | CAGGCTGTCCCCCTTTTGTA |
| 587 | SEQ ID NO: 495 | SEQ ID NO: 492 | SEQ ID NO: 493 |
| | GATAAGTCGCTCGCTTATATGGTTGT | TCGAAACCCAGGTACCTCAATTCCCGCA | CAGGCTGTCCCCCTTTTGT |
| 588 | SEQ ID NO: 496 | SEQ ID NO: 485 | SEQ ID NO: 486 |
| | TATGGATAAGTCGCTCGCTTATATGGT | ACCCAGGTACCTCAATTCCCGCATTTTTCACA | CAGGCTGTCCCCCTTTTGTA |
| 589 | SEQ ID NO: 496 | SEQ ID NO: 487 | SEQ ID NO: 486 |
| | TATGGATAAGTCGCTCGCTTATATGGT | AACCCAGGTACCTCAATTCCCGCATTTTTCAC A | CAGGCTGTCCCCCTTTTGTA |
| 590 | SEQ ID NO: 497 | SEQ ID NO: 485 | SEQ ID NO: 486 |
| | GTATGGATAAGTCGCTCGCTTATATGG T | ACCCAGGTACCTCAATTCCCGCATTTTTCACA | CAGGCTGTCCCCCTTTTGTA |
| 591 | SEQ ID NO: 497 | SEQ ID NO: 487 | SEQ ID NO: 486 |
| | GTATGGATAAGTCGCTCGCTTATATGG T | AACCCAGGTACCTCAATTCCCGCATTTTTCAC A | CAGGCTGTCCCCCTTTTGTA |
| 592 | SEQ ID NO: 498 | SEQ ID NO: 499 | SEQ ID NO: 486 |
| | TGTATGGATAAGTCGCTCGCTTATATG G | ACCCAGGTACCTCAATTCCCGCATTTTTCACA A | CAGGCTGTCCCCCTTTTGTA |
| 593 | SEQ ID NO: 498 | SEQ ID NO: 485 | SEQ ID NO: 486 |
| | TGTATGGATAAGTCGCTCGCTTATATG G | ACCCAGGTACCTCAATTCCCGCATTTTTCACA | CAGGCTGTCCCCCTTTTGTA |
| 594 | SEQ ID NO: 498 | SEQ ID NO: 487 | SEQ ID NO: 486 |
| | TGTATGGATAAGTCGCTCGCTTATATG G | AACCCAGGTACCTCAATTCCCGCATTTTTCAC A | CAGGCTGTCCCCCTTTTGTA |
| 595 | SEQ ID NO: 500 | SEQ ID NO: 499 | SEQ ID NO: 486 |
| | GTATGGATAAGTCGCTCGCTTATATGG | ACCCAGGTACCTCAATTCCCGCATTTTTCACA A | CAGGCTGTCCCCCTTTTGTA |
| 596 | SEQ ID NO: 500 | SEQ ID NO: 485 | SEQ ID NO: 486 |
| | GTATGGATAAGTCGCTCGCTTATATGG | ACCCAGGTACCTCAATTCCCGCATTTTTCACA | CAGGCTGTCCCCCTTTTGTA |
| 597 | SEQ ID NO: 500 | SEQ ID NO: 487 | SEQ ID NO: 486 |
| | GTATGGATAAGTCGCTCGCTTATATGG | AACCCAGGTACCTCAATTCCCGCATTTTTCAC A | CAGGCTGTCCCCCTTTTGTA |
| 598 | SEQ ID NO: 501 | SEQ ID NO: 499 | SEQ ID NO: 486 |
| | CTGTATGGATAAGTCGCTCGCTTATAT GG | ACCCAGGTACCTCAATTCCCGCATTTTTCACA A | CAGGCTGTCCCCCTTTTGTA |
| 599 | SEQ ID NO: 501 | SEQ ID NO: 485 | SEQ ID NO: 486 |
| | CTGTATGGATAAGTCGCTCGCTTATAT GG | ACCCAGGTACCTCAATTCCCGCATTTTTCACA | CAGGCTGTCCCCCTTTTGTA |
| 600 | SEQ ID NO: 501 | SEQ ID NO: 487 | SEQ ID NO: 486 |
| | CTGTATGGATAAGTCGCTCGCTTATAT GG | AACCCAGGTACCTCAATTCCCGCATTTTTCAC A | CAGGCTGTCCCCCTTTTGTA |
| 601 | SEQ ID NO: 502 | SEQ ID NO: 492 | SEQ ID NO: 493 |
| | TGTATGGATAAGTCGCTCGCTTATATG GTT | TCGAAACCCAGGTACCTCAATTCCCGCA | CAGGCTGTCCCCCTTTTGT |

A PCR primer set for amplifying a *vanC1* gene comprises at least one of the following sets of primer sequences: (1) SEQ ID NOS: 123 and 125; (2) SEQ ID NOS: 127 and 129; (3) SEQ ID NOS: 130 and 132; (4) SEQ ID NOS: 133 and 135; (5) SEQ ID NOS: 133 and 137; (6) SEQ ID NOS: 138 and 140; (7) SEQ ID NOS: 141 and 137; (8) SEQ ID NOS: 141 and 143; (9) SEQ ID NOS: 141 and 147; (10) SEQ ID NOS: 141 and 179; (11) SEQ ID NOS: 144 and 137; (12) SEQ ID NOS: 144 and 146; (13) SEQ ID NOS: 144 and 147; (14) SEQ ID NOS: 144 and 157; (15) SEQ ID NOS: 148 and 137; (16) SEQ ID NOS: 148 and 150; (17) SEQ ID NOS: 151 and 153; (18) SEQ ID NOS: 151 and 155; (19) SEQ ID NOS: 156 and 150; (20) SEQ ID NOS: 158 and 160; (21) SEQ ID NOS: 161 and 137; (22) SEQ ID NOS: 161 and 147; (23) SEQ ID NOS: 161 and 150; (24) SEQ ID NOS: 161 and 153; (25) SEQ ID NOS: 161 and 190; (26) SEQ ID NOS: 161 and 192; (27) SEQ ID NOS: 162 and 164; (28) SEQ ID NOS: 165 and 167; (29) SEQ ID NOS: 168 and 169; (30) SEQ ID NOS: 170 and 169; (31) SEQ ID NOS: 171 and 173; (32) SEQ ID NOS: 171 and 174; (33) SEQ ID NOS: 175 and 177; (34) SEQ ID NOS: 178 and 179; (35) SEQ ID NOS: 180 and 146; (36) SEQ ID NOS: 183 and 150; (37) SEQ ID NOS: 184 and 174; (38) SEQ ID NOS: 185 and 187; (39) SEQ ID NOS: 188 and 137; (40) SEQ ID NOS: 188 and 150; (41) SEQ ID NOS: 188 and 190; (42) SEQ ID NOS: 191 and 137; (43) SEQ ID NOS: 191 and 150; (44) SEQ ID NOS: 191 and 153; (45) SEQ ID NOS: 191 and 190; (46) SEQ ID NOS: 191 and 192; (47) SEQ ID NOS: 193 and 137; (48) SEQ ID NOS: 193 and 150; (49) SEQ ID NOS: 193 and 153; (50) SEQ ID NOS: 193 and 190; (51) SEQ ID NOS: 194 and 147; (52) SEQ ID NOS: 194 and 160; (53) SEQ ID NOS: 196 and 147; (54) SEQ ID NOS: 196 and 160; (55) SEQ ID NOS: 198 and 179; (56) SEQ ID NOS: 200 and 179; (57) SEQ ID NOS: 201 and 179; (58) SEQ ID NOS: 202 and 179; (59) SEQ ID NOS: 203 and 147; (60) SEQ ID NOS: 204 and 179; and (61) SEQ ID NOS: 204 and 187.

A PCR primer set for amplifying a *vanC2*/*3* gene comprises at least one of the following sets of primer sequences: (1) SEQ ID NOS: 206 and 208; (2) SEQ ID NOS: 206 and 209; (3) SEQ ID NOS: 206 and 216; (4) SEQ ID NOS: 206 and 219; (5) SEQ ID NOS: 206 and 227; (6) SEQ ID NOS: 210 and 209; (7) SEQ ID NOS: 210 and 212; (8) SEQ ID NOS: 210 and 215; (9) SEQ ID NOS: 210 and 216; (10) SEQ ID NOS: 210 and 219; (11) SEQ ID NOS: 210 and 223; (12) SEQ ID NOS: 210 and 227; (13) SEQ ID NOS: 213 and 215; (14) SEQ ID NOS: 217 and 209; (15) SEQ ID NOS: 217 and 216; (16) SEQ ID NOS: 217 and 219; (17) SEQ ID NOS: 217 and 223; (18) SEQ ID NOS: 217 and 227; (19) SEQ ID NOS: 220 and 209; (20) SEQ ID NOS: 220 and 219; (21) SEQ ID NOS: 220 and 223; (22) SEQ ID NOS: 220 and 227; (23) SEQ ID NOS: 221 and 209; (24) SEQ ID NOS: 221 and 216; (25) SEQ ID NOS: 221 and 219; (26) SEQ ID NOS: 221 and 227; (27) SEQ ID NOS: 222 and 209; (28) SEQ ID NOS: 222 and 216; (29) SEQ ID NOS: 222 and 219; (30) SEQ ID NOS: 222 and 223; (31) SEQ ID NOS: 222 and 227; (32) SEQ ID NOS: 224 and 212; (33) SEQ ID NOS: 224 and 215; (34) SEQ ID NOS: 224 and 216; (35) SEQ ID NOS: 225 and 209; (36) SEQ ID NOS: 225 and 212; (37) SEQ ID NOS: 225 and 216; (38) SEQ ID NOS: 226 and 209; (39) SEQ ID NOS: 226 and 212; (40) SEQ ID NOS: 226 and 216; (41) SEQ ID NOS: 228 and 215; (42) SEQ ID NOS: 229 and 209; (43) SEQ ID NOS: 229 and 215; (44) SEQ ID NOS: 230 and 219; (45) SEQ ID NOS: 231 and 212; (46) SEQ ID NOS: 231 and 215; (47) SEQ ID NOS: 232 and 216; (48) SEQ ID NOS: 233 and 212; (49) SEQ ID NOS: 234 and 215; (50) SEQ ID NOS: 235 and 215; (51) SEQ ID NOS: 235 and 239; (52) SEQ ID NOS: 235 and 241; (53) SEQ ID NOS: 236 and 216; (54) SEQ ID NOS: 237 and 209; (55) SEQ ID NOS: 237 and 215; (56) SEQ ID NOS: 238 and 215; (57) SEQ ID NOS: 240 and 216; (58) SEQ ID NOS: 242 and 216; and (59) SEQ ID NOS: 243 and 215.

A PCR primer set for amplifying a *vanD* gene comprises at least one of the following sets of primer sequences: (1) SEQ ID NOS: 388 and 390; (2) SEQ ID NOS: 391 and 393; (3) SEQ ID NOS: 391 and 434; (4) SEQ ID NOS: 394 and 393; (5) SEQ ID NOS: 396 and 398; (6) SEQ ID NOS: 396 and 419; (7) SEQ ID NOS: 396 and 419; (8) SEQ ID NOS: 399 and 401; (9) SEQ ID NOS: 399 and 401; (10) SEQ ID NOS: 399 and 401; (11) SEQ ID NOS: 399 and 401; (12) SEQ ID NOS: 399 and 444; (13) SEQ ID NOS: 399 and 444; (14) SEQ ID NOS: 415 and 401; (15) SEQ ID NOS: 416 and 417; (16) SEQ ID NOS: 435 and 437; (17) SEQ ID NOS: 438 and 439; (18) SEQ ID NOS: 440 and 442; (19) SEQ ID NOS: 443 and 434; (20) SEQ ID NOS: 445 and 447; (21) SEQ ID NOS: 445 and 448; (22) SEQ ID NOS: 445 and 449; (23) SEQ ID NOS: 445 and 450; (24) SEQ ID NOS: 445 and 451; (25) SEQ ID NOS: 445 and 452; (26) SEQ ID NOS: 445 and 453; (27) SEQ ID NOS: 445 and 454; (28) SEQ ID NOS: 445 and 455; (29) SEQ ID NOS: 445 and 459; (30) SEQ ID NOS: 445 and 460; (31) SEQ ID NOS: 445 and 461; (32) SEQ ID NOS: 456 and 458; (33) SEQ ID NOS: 462 and 464; (34) SEQ ID NOS: 465 and 467; (35) SEQ ID NOS: 468 and 470; (36) SEQ ID NOS: 471 and 473; (37) SEQ ID NOS: 474 and 476; (38) SEQ ID NOS: 477 and 479; (39) SEQ ID NOS: 480 and 482; (40) SEQ ID NOS: 483 and 479; (41) SEQ ID NOS: 483 and 486; (42) SEQ ID NOS: 488 and 490; (43) SEQ ID NOS: 491 and 493; (44) SEQ ID NOS: 494 and 486; (45) SEQ ID NOS: 495 and 493; (46) SEQ ID NOS: 496 and 486; (47) SEQ ID NOS: 497 and 486; (48) SEQ ID NOS: 498 and 486; (49) SEQ ID NOS: 500 and 486; (50) SEQ ID NOS: 501 and 486; and (51) SEQ ID NOS: 502 and 493.

A PCR primer set for amplifying a *vanE* gene comprises at least one of the following sets of primer sequences: (1) SEQ ID NOS: 334 and 336; (2) SEQ ID NOS: 337 and 336; (3) SEQ ID NOS: 338 and 336; (4) SEQ ID NOS: 338 and 381; (5) SEQ ID NOS: 339 and 336; (6) SEQ ID NOS: 340 and 336; (7) SEQ ID NOS: 341 and 336; (8) SEQ ID NOS: 341 and 381; (9) SEQ ID NOS: 342 and 336; (10) SEQ ID NOS: 342 and 381; (11) SEQ ID NOS: 343 and 336; (12) SEQ ID NOS: 344 and 336; (13) SEQ ID NOS: 344 and 381; (14) SEQ ID NOS: 345 and 336; (15) SEQ ID NOS: 346 and 336; (16) SEQ ID NOS: 347 and 336; (17) SEQ ID NOS: 347 and 381; (18) SEQ ID NOS: 348 and 336; (19) SEQ ID NOS: 348 and 381; (20) SEQ ID NOS: 349 and 336; (21) SEQ ID NOS: 349 and 381; (22) SEQ ID NOS: 350 and 336; (23) SEQ ID NOS: 350 and 381; (24) SEQ ID NOS: 351 and 336; (25) SEQ ID NOS: 352 and 336; (26) SEQ ID NOS: 353 and 336; (27) SEQ ID NOS: 354 and 336; (28) SEQ ID NOS: 355 and 336; (29) SEQ ID NOS: 355 and 381; (30) SEQ ID NOS: 356 and 336; (31) SEQ ID NOS: 357 and 336; (32) SEQ ID NOS: 358 and 336; (33) SEQ ID NOS: 359 and 336; (34) SEQ ID NOS: 359 and 381; (35) SEQ ID NOS: 360 and 336; (36) SEQ ID NOS: 360 and 381; (37) SEQ ID NOS: 361 and 336; (38) SEQ ID NOS: 362 and 336; (39) SEQ ID NOS: 363 and 336; (40) SEQ ID NOS: 364 and 336; (41) SEQ ID NOS: 365 and 336; (42) SEQ ID NOS: 366 and 336; (43) SEQ ID NOS: 367 and 336; (44) SEQ ID NOS: 368 and 336; (45) SEQ ID NOS: 369 and 336; (46) SEQ ID NOS: 370 and 336; (47) SEQ ID NOS: 371 and 336; (48) SEQ ID NOS: 372 and 336; (49) SEQ ID NOS: 373 and 336; (50) SEQ ID NOS: 374 and 336; (51) SEQ ID NOS: 375 and 336; (52) SEQ ID NOS: 376 and 336; (53) SEQ ID NOS: 377 and 336; (54) SEQ ID NOS: 378 and 336; (55) SEQ ID NOS: 379 and 336; (56) SEQ ID NOS: 380 and 336; (57) SEQ ID NOS: 382 and 381; (58) SEQ ID NOS: 383 and 381; (59) SEQ ID NOS: 384 and 381; (60) SEQ ID NOS: 385 and 381; (61) SEQ ID NOS: 386 and 381; and (62) SEQ ID NOS: 387 and 381.

A PCR primer set for amplifying a *vanG* gene comprises at least one of the following sets of primer sequences: (1) SEQ ID NOS: 244 and 246; (2) SEQ ID NOS: 244 and 247; (3) SEQ ID NOS: 244 and 248; (4) SEQ ID NOS: 244 and 250; (5) SEQ ID NOS: 244 and 251; (6) SEQ ID NOS: 244 and 254; (7) SEQ ID NOS: 244 and 258; (8) SEQ ID NOS: 244 and 259; (9) SEQ ID NOS: 244 and 284; (10) SEQ ID NOS: 244 and 286; (11) SEQ ID NOS: 244 and 287; (12) SEQ ID NOS: 249 and 246; (13) SEQ ID NOS: 249 and 248; (14) SEQ ID NOS: 249 and 286; (15) SEQ ID NOS: 249 and 301; (16) SEQ ID NOS: 249 and 306; (17) SEQ ID NOS: 249 and 308; (18) SEQ ID NOS: 249 and 312; (19) SEQ ID NOS: 252 and 246; (20) SEQ ID NOS: 252 and 262; (21) SEQ ID NOS: 253 and 246; (22) SEQ ID NOS: 255 and 246; (23) SEQ ID NOS: 256 and 246; (24) SEQ ID NOS: 260 and 258; (25) SEQ ID NOS: 263 and 258; (26) SEQ ID NOS: 264 and 266; (27) SEQ ID NOS: 267 and 269; (28) SEQ ID NOS: 270 and 266; (29) SEQ ID NOS: 270 and 269; (30) SEQ ID NOS: 271 and 266; (31) SEQ ID NOS: 272 and 274; (32) SEQ ID NOS: 275 and 269; (33) SEQ ID NOS: 276 and 269; (34) SEQ ID NOS: 277 and 269; (35) SEQ ID NOS: 278 and 266; (36) SEQ ID NOS: 279 and 269; (37) SEQ ID NOS: 280 and 266; (38) SEQ ID NOS: 280 and 269; (39) SEQ ID NOS: 281 and 269; (40) SEQ ID NOS: 282 and 266; (41) SEQ ID NOS: 282 and 269; (42) SEQ ID NOS: 283 and 269; (43) SEQ ID NOS: 285 and 259; (44) SEQ ID NOS: 288 and 290; (45) SEQ ID NOS: 288 and 304; (46) SEQ ID NOS: 291 and 290; (47) SEQ ID NOS: 293 and 290; (48) SEQ ID NOS: 293 and 304; (49) SEQ ID NOS: 295 and 258; (50) SEQ ID NOS: 297 and 290; (51) SEQ ID NOS: 298 and 290; (52) SEQ ID NOS: 298 and 304; (53) SEQ ID NOS: 299 and 290; (54) SEQ ID NOS: 300 and 290; (55) SEQ ID NOS: 302 and 274; (56) SEQ ID NOS: 303 and 290; (57) SEQ ID NOS: 303 and 304; (58) SEQ ID NOS: 305 and 274; (59) SEQ ID NOS: 305 and 290; (60) SEQ ID NOS: 307 and 274; (61) SEQ ID NOS: 307 and 290; (62) SEQ ID NOS: 309 and 290; (63) SEQ ID NOS: 310 and 290; (64) SEQ ID NOS: 311 and 258; (65) SEQ ID NOS: 313 and 290; (66) SEQ ID NOS: 314 and 290; (67) SEQ ID NOS: 314 and 320; (68) SEQ ID NOS: 315 and 290; (69) SEQ ID NOS: 316 and 290; (70) SEQ ID NOS: 317 and 290; (71) SEQ ID NOS: 318 and 290; (72) SEQ ID NOS: 321 and 258; (73) SEQ ID NOS: 322 and 324; (74) SEQ ID NOS: 325 and 266; (75) SEQ ID NOS: 325 and 274; (76) SEQ ID NOS: 326 and 274; (77) SEQ ID NOS: 327 and 266; (78) SEQ ID NOS: 328 and 274; (79) SEQ ID NOS: 329 and 274; (80) SEQ ID NOS: 330 and 274; (81) SEQ ID NOS: 331 and 274; (82) SEQ ID NOS: 332 and 266; and (83) SEQ ID NOS: 333 and 266.

The preceding numbering of the sets of primers does not correspond exactly to the "Group" numbering scheme in Table 6 because certain groups use the same primer set, but different internal probes. For example, Groups 213 and 214 of Table 6 each employ the forward primer of SEQ ID NO: 123 and the reverse primer of SEQ ID NO: 125, but different internal probes in each instance, *e.g*., SEQ ID NOS: 124 and 126. Accordingly, primer set "(1)" of the preceding passage relating to the *vanC1* primers implies any one of Groups 213 or 214 of Table 6.

Any set of primers can be used simultaneously in a multiplex reaction with one or more other primer sets, so that multiple amplicons are amplified simultaneously.

A probe for binding to an amplicon(s) of a *vanC, vanD, vanE* and/or *vanG* gene, or to a *vanC, vanD, vanE* and/or *vanG* gene target, comprises at least one of the following probe sequences: SEQ ID NOS: 124, 126, 128, 131, 134, 136, 139, 142, 145, 149, 152, 154, 159, 163, 166, 172, 176, 181, 182, 186, 189, 195, 197, 199, 205, 207, 211 (*vanC* probes); SEQ ID NOS: 389, 392, 397, 400, 402-408, 410, 412-414, 418, 420, 422-424, 427, 429, 431-433, 436, 441, 446, 457, 463, 466, 469, 472, 478, 481, 484, 485, 487, 489, 492, 499 (*vanD* probes); SEQ ID NOS: 335 (*vanE* probe) and SEQ ID NOS: 245, 257, 261, 265, 268, 273, 292, 294, 296, 319, 323 (*vanG* probes).

Any set of primers can be used simultaneously in a multiplex reaction with one or more other primer sets, so that multiple amplicons are amplified simultaneously.

Primer sets for simultaneously amplifying the *vanA* and/or *vanB* and/or *vanC* and/or *vanD* and/or *vanE* and/or *vanG* comprises a nucleotide sequence selected from the primer sets consisting of: Groups 1-609 of Tables 5 and 6. Oligonucleotide probes for binding to the *vanA* and/or *vanB* and/or *vanC* and/or *vanD* and/or *vanE* and/or *vanG* genes comprises a nucleotide sequence selected from the group consisting of: SEQ ID NOS: 2, 4, 7, 9, 11-18, 20, 24, 25, 27, 30, 35, 43, 46, 49, 50, 54, 56, 57, 58 (*vanA* probes); 63, 65, 66, 74, 77, 85-88, 90, 91, 95, 97-102 (*vanB* probes); 124, 126, 128, 131, 134, 136, 139, 142, 145, 149, 152, 154, 159, 163, 166, 172, 176, 181, 182, 186, 189, 195, 197, 199, 205, 207, 211 (*vanC* probes); SEQ ID NOS: 389, 392, 397, 400, 402-408, 410, 412-414, 418, 420, 422-424, 427, 429, 431-433, 436, 441, 446, 457, 463, 466, 469, 472, 478, 481, 484, 485, 487, 489, 492, 499 (*vanD* probes); SEQ ID NOS: 335 (*vanE* probe); and SEQ ID NOS: 245, 257, 261, 265, 268, 273, 292, 294, 296, 319, 323 (*van*G probes).

The internal control is detected by a forward primer (SEQ ID NO: 504), a reverse primer (SEQ ID NO: 506) and a probe (SEQ ID NO: 505). A plasmid vector containing the internal control target sequence (SEQ ID NO: 503):
GCGAAGTGAGAATACGCCGTGTCGCAGTTTCCTTGAGCAGTGTCTCTAAATGCCT CAAACCGTCGCATTTTTGGTTATAGCAGTAACTATATGGAGGTCCGTAGGCGGCG TGCGTGGGGGCACCAAACTCATCCAACGGTCGACTGCGCCTGTAGGGTCTTAAG AAGCGGCACCTCAGACCGATAGCATAGCACTTAAAGAGGAATTGAATAATCAAG ATGGGTATCCGACCGACGCGGAGTGACCGAGGAAGAGGACCCTGCATGTATCCT GAGAGTATAGTTGTCAGAGCAGCAATTGATTCACCACCAAGGGACTTAGTCT is included in the assay. The internal control plasmid is added directly to the reaction mix to monitor the integrity of the PCR reagents and the presence of PCR inhibitors.

**Table 7. Internal Control.**

| **Group No.** | **Forward primer** | **Probe** | **Reverse primer** |
|---|---|---|---|
| 602 | SEQ ID NO: 504 | SEQ ID NO:505 | SEQ ID NO:506 |
| | CAGACCGATAGCATAGCACTTAAA | TGCTGCTCTGACAACTATACTCTCAGGATACA | TCCCTTGGTGGTGAATCAAT |

### Example 5. Enterococcus species-specific markers.

The *vanA* and *vanB* markers, which are carried on a transferrable element, are indicative of the presence of VRE. *vanA* is almost always associated with VRE, while *vanB* is usually associated with VRE. *vanB* can also occur in species other than *Enterococcus* (*e.g. Clostridium*). In either case, a direct link cannot be made between *Enterococcus* and the detection of *vanA* or *vanB* in a mixed flora population. In some cases, detection of *vanA* or *vanB* harboring organisms is followed by an attempt to isolate the vancomycin resistant organism and conclusively identify it as *Enterococcus.*

Thus, in one respect, a species-specific marker is useful for identifying vancomycin-resistant clinical isolates as *Enterococcus faecium* (*E. faecium* or Efm) and/or *Enterococcus faecalis* (*E. faecalis* or Efs), which are the two most common Type A and Type B *Enterococcus* species. These two species are also the most important with regard to VRE.

One embodiment is directed to species-specific markers for the detection of Efm, Efs or both Efm and Efs ("Efm/Efs dual"). Two approaches were utilized within this embodiment. One approach targeted the *sodA* gene, which encodes the enzyme superoxide dismutase A. The *sodA* gene is frequently used as a bacterial species-specific marker. A second approach targeted novel genes from Efm, Efs that were identified through *in silico* analyses. Below are the *sodA* markers for Efm and Efs, the novel marker for Efm and Efs and a dual marker (dual Efm/Efs dual). The dual marker detects both Efm and Efs. Table 8A-12 describe the nucleic acid primers and probes used for detection and screening of Efm and/or Efs based on the target. Below are the sequences of the *sodA* for Efm and Efs, *novel* genes for Efm and Efs, and dual genes for Efm and Efs.
Efm *sodA* (SEQ ID NO: 600) 
TAGAAAGATTATTATCTGATATGGACGCTATTCCAACAGATATCAAGACAGCTGT
ACGTAACAATGGTGGCGGACATGCTAACCATTCATTTTTCTGGGAAATCATGGCA
CCAAATGCTGGTGGCGAACCTACAGGAGAAATAAAAGAAGCGATTAATGAAGCT
TTTGGTGATTTTTCTTCTTTTAAAGAAGAATTCAAAAAAGCAGCCGCTGGACGAT
TTGGTTCTGGATGGGCTTGGCATGTAATGGAAATTGGAAAATTAGCTATTACCTC
TACTGCAAATCAAGATTCTCCATT
Efs *sodA* (SEQ ID NO: 601) 
TCTGTAGAAAACCTAATTTCAGATATGAATGCTATTCCTGAAGATATCCGCACAG
CTGTTCGTAACAATGGTGGCGGTCACGCAAACCATACATTCTTCTGGGAAATTAT
GGCACCAAATGCTGGTGGACAACCAACTGGCGCTATTAAAGAAGCAATCGATGA
AACATTTGGTAGCTTTGATGAAATGAAAGCTGCTTTCAAAACAGCTGCAACTGGC
CGCTTTGGTTCAGGTTGGGCTTGGTTAGTTGTGAATAACGGTAAATTAGAAATCA
CTTCAACACCAA
Efm novel marker (SEQ ID NO: 602) 
ACGCGTTCGTTTCGTTCTCTTCTAGCAAAAATACTCCCCGTATCCCGCTTCAGAG
GTTTCGCCTTCTTATGGATGTCCTTATAGCGATCCATCCGACTCATTGTGGTTCCT
CCTCGTTTCTTACTGATAAATAAAGGATACGATAACCAATCGACAAAAGCCTATC
ATTTTTTGATAATTTAATAAAAAATAACGAAATAAATGCTTCGATACATAAAG
Efs novel marker (SEQ ID NO: 603) 
CTCGTTCCTTTTAGCAAAAGTAATTGGACAAGCGTCGCACATCTTCAGGAGCTA
AATCAATTTTTTAATCGTATGATACAGCGTTTCTAATTGTTTCACTGTCTCTGGA
TTAGGTACTCCGTCAAGTACTCGTACTTGAAAATCATCTAAAATATTTCACCATT
TTCAATATACGCATCTAAAAAAGAAGTATCCAATGACTGTTGCAATAACTGGATT
GCTTGTTCCATTGGCTCGAAAGCCTCTTCAATTTTTTCTGGGAACAAATTAGCACC
TCTATTCTATTCAAAATGTAACAACTTCCATACCTGTTTTATTTTAACGAAAAAAG
AAAGAATAATCAATGCCTTTACTGTCTTTCTTCTAAAATCTAATAATTTGTAGAA
AAACAACGCTATTCCAGTATTGTTTCCGCTTTTTTTTGATGAGAAACTCTCTCTTG
ATATTGGTATTTTCGACCAGATTTGTTTAATAAAACAGTTATTCTTAATTGTTCAT
TTTTGTATGTATAATCGAGGGTCACTTGCGCGTATACGAGCCTTTCTTCTGTTTTT
AACTTTCGTCTATTTTGTTTAACATCTGCAAGAAATAAATGAAAGATACTTTTCCC
TGCATAGTATTCCTGGGTAGCTTGCGTAAATTTTTGTGTTAACTGACGTTCTTCTA
GTAACTGAAGGGCGAGAAAACTCATCAAATAAACAATGGCCATGGCCGTGAACA
ATAAGTTTCCTGAATATTTTTGCCTCATTAAAATTCTCCCTTTATCGGAAAAAAAC
TATCAAATGTTTTTCCAGAAGTAAAAGTAACTTCCAAAGTAAACGATTGTCCTTC
ATTTTTAAACTGTCCATTTTTGATACCAATTAACAGCGGTTGATAGCCTTTACCAT
TGACTCGCTTAATAATTTTATCTTCTTTGATTTGAATTGAAATAGTCTTGTTTGTTT
 
CTGAGTCGAGGAAAGAAATCTCTTGAGCAGAGCCTGTTTGGAAAACGAGCTTTT
GACATTCTTTTCTAATTGAATTAAAAAAATATGCCAGGATTTCGAATCATTGTTT
TTCAAATATTGATTTCCGATAAAACTTTGTTGAATCATCAACTGAAACAATTGAC
ACATACAACTTAAAACGACTAGTGCTACTAAACACTCTAACATCGTAAAACCCGC
ATATTTTTTATTGACCAATCAAAATCGCTCCTCTTGAATCTGTGACTTTTAGTATC
CTTCTTCCATCGTTCTCCATCAAAGAAAATCGGTAAGTTTCTCCTTGATATATCCG
CTCTTTGAAACGTAAATCACCCGTCTGCTCTAAAAATAAAATTGCTTCGTATCCT
AGACGAGTGCGCGTTAATTCTTGCTCCCGTTGATAATTTTGCTGAATTAATTGTGT
AATAGTCAGGGAGAATATCCCTGCTATCACACATACAATACTAAAACTAACGAG
ACTCTCTAATAAAATAAAACCGCTATAAATAGTTAATTTCTTCGTATATTTGCCA
CTCCCCATCT
Efm Efs dual novel marker (from Efm) (SEQ ID NO: 604) 
CGAGCGTTCTCGAGAATACTCGACGCTGAAATTTTTGCACCATTTATTGCTTTATC
GTGACGTGACTTTGCAATAACAAATCCTAAACCAAGACCGACAATCAAACCGAC
GATAGCGAGGAGAATATTTAAAACCATATTTCCACCTCCATACTATCTTTTT
Efm Efs dual novel marker (from Efs) (SEQ ID NO: 605)

GCTGATGATTGTGCACCAGCTATTTCTTTCTCGTGACGTGACTTTGCGACCATAAA
CCCTAAACCAAGACCGACAATTAAACCGATGATAGCGAGGAGAATGTTGAATAC
CATAAAATCCACCTCCATACTATCTTTTT

**Table 8A. Efm sodA gene nucleic acid primers and probes**

| **Primer** |
|---|
| SEQ ID NO: 606 |
| GGACATGCTAACCATTCATTT |
| SEQ ID NO: 607 |
| CGCTGGACGATTTGGTTCTG |
| SEQ ID NO: 608 |
| AGCGATTAATGAAGCTTTTGGTGA |
| SEQ ID NO: 609 |
| GGAAATCATGGCACCGAATG |
| SEQ ID NO: 610 |
| TGGTGGACATGCTAACCATTCA |
| SEQ ID NO: 611 |
| AGCGATTAATGAAGCTTTTGGTGAT |
| SEQ ID NO: 612 |
| CAGTAGAGGTAATAGCTAATTTTCC |
| SEQ ID NO: 613 |
| ATTTCTCCTGTAGGTTCGC |
| SEQ ID NO: 614 |
| GCCAAGCCCATCCAGA |
| SEQ ID NO: 615 |
| CCATTACAAGCCAAGCC |
| SEQ ID NO: 616 |
| AGCTTCATTAATCGCTTCTTTT |
| SEQ ID NO: 617 |
| GCCAAGCCCATCCAGAAC |
| SEQ ID NO: 618 |
| GCCAAGCCCAACCAGA |
| SEQ ID NO: 619 |
| GCTTCATTAATTGCTTCTTTTATTG |
| SEQ ID NO: 620 |
| GGCAGTAGAGGTAATAGCTAA |
| SEQ ID NO: 621 |
| GGCAGTAGAGGTAATAGCTAAT |
| SEQ ID NO: 622 |
| CCAAAAGCTTCATTAATCGCTTCTT |
| SEQ ID NO: 623 |
| CATCCAGAACCAAATCGTCCAG |
| SEQ ID NO: 624 |
| TCACCAAAAGCTTCATTAATCGCT |
| SEQ ID NO: 625 |
| TCTTTTATTTCTCCTGTAGGTTCGC |
| SEQ ID NO: 626 |
| CATTAATCGCTTCTTTTATTTCTCC |
| SEQ ID NO: 627 |
| TAATCGCTTCTTTTATTTCTCCTGT |
| SEQ ID NO: 628 |
| CATTTTCCATTACAAGCCAAGCC |
| SEQ ID NO: 629 |
| CAAGCCCATCCAGAACCAAATC |
| SEQ ID NO: 630 |
| GTCCAGCGGCTGCTTTT |
| SEQ ID NO: 631 |
| CATCCAGAACCAAATCGTCCA |
| SEQ ID NO: 632 |
| TGCCAAGCCCATCCAGAAC |
| SEQ ID NO: 633 |
| CAACCAGAACCAAAACGTCCA |
| SEQ ID NO: 634 |
| TCGCCAAAAGCTTCATTAATTGCTT |
| SEQ ID NO: 635 |
| AAAATCGCCAAAAGCTTCATT |
| SEQ ID NO: 636 |
| GCCAAGCCCAACCAGAAC |
| SEQ ID NO: 637 |
| ATTACAAGCCAAGCCCAACCA |
| SEQ ID NO: 638 |
| ATTCTCCATTACAAGCCAAGCC |
| SEQ ID NO: 639 |
| CGTCCAGCTGCTGCTTTT |
| SEQ ID NO: 640 |
| TAATTGCTTCTTTTATTGCCCCAGT |
| SEQ ID NO: 641 |
| TTTCCCATTCTCCATTACAAGCCA |
| SEQ ID NO: 642 |
| AGCTAATTTCCCATTCTCCATTACA |
| SEQ ID NO: 643 |
| TGGAGAATCTTGATTGGCAGTAGAG |
| SEQ ID NO: 644 |
| TTGATTGGCAGTAGAGGTAATAGC |
| SEQ ID NO: 645 |
| GATTGGCAGTAGAGGTAATAGCTAA |
| SEQ ID NO: 646 |
| TATTACAAGCCAAGCCCATCCAG |
| **Probe** |
| SEQ ID NO: 647 |
| CATGGCACCAAATGCTGGT |
| SEQ ID NO: 648 |
| CGGTGCCATGATTTCCCAGAAAA |
| SEQ ID NO: 649 |
| CCAAAACGTCCAGCTGCTGC |
| SEQ ID NO: 650 |
| ATGGCACCGAATGCGGG |
| SEQ ID NO: 651 |
| AGAAGCAATTAATGAAGCTTTTGGCGA |
| SEQ ID NO: 652 |
| ATTGCTTCTTTTATTGCCCCAGTAGG |
| SEQ ID NO: 653 |
| AGCTAATTTTCCATTTTCCATTACAAGCCAAGCCC |
| SEQ ID NO: 654 |
| TGGGCTTGGCTTGTAATGGAAAATGGAAAATTAGC |
| SEQ ID NO: 655 |
| CCAGCATTTGGTGCCATGATTTCCCAGAAAA |
| SEQ ID NO: 656 |
| TTTTCCATTTTCCATTACAAGCCAAGCCCATCCA |
| SEQ ID NO: 657 |
| TTTTCCATTACAAGCCAAGCCCATCCAGAACC |
| SEQ ID NO: 658 |
| ATTACAAGCCAAGCCCATCCAGAACCAAAT |
| SEQ ID NO: 659 |
| ACCAAATGCTGGTGGCGAACCTACA |
| SEQ ID NO: 660 |
| ATTTGGTTCTGGATGGGCTTGGCTTGTA |
| SEQ ID NO: 661 |
| CAACCAGAACCAAAACGTCCAGCTGC |
| SEQ ID NO: 662 |
| CGCCAAAAGCTTCATTAATTGCTTCTTTTATTGCC |
| SEQ ID NO: 663 |
| CGCATTCGGTGCCATGATTTCCCAGAA |
| SEQ ID NO: 664 |
| TTTTCTGGGAAATCATGGCACCGAATGC |
| SEQ ID NO: 665 |
| TTCTGGGAAATCATGGCACCGAATGCG |
| SEQ ID NO: 666 |
| CAATAAAAGAAGCAATTAATGAAGCTTTTGGCGAT |
| SEQ ID NO: 667 |
| AGCTTCATTAATTGCTTCTTTTATTGCCCCAGTAG |

**Table 8B. Efm sodA gene solutions**

| **Group No.** | **Forward Primer** | **Probe** | **Reverse Primer** |
|---|---|---|---|
| 603 | SEQ ID NO: 610 | SEQ ID NO: 664 | SEQ ID NO: 622 |
| | TGGTGGACATGCTAAC CATTCA | TTTTCTGGGAAATCATGGC ACCAAATGCTGG | CCAAAAGCTTCATTAATC GCTTCTT |
| 604 | SEQ ID NO: 610 | SEQ ID NO: 648 | SEQ ID NO: 622 |
| | TGGTGGACATGCTA ACCATTCA | CGGTGCCATGATTTCCCAG AAAA | CCAAAAGCTTCATTAATC GCTTCTT |
| 605 | SEQ ID NO: 610 | SEQ ID NO: 655 | SEQ ID NO: 622 |
| | TGGTGGACATGCTAAC CATTCA | CCAGCATTTGGTGCCATGA TTTCCCAGAAAA | CCAAAAGCTTCATTAATC GCTTCTT |

**Table 9A. Efs sodA gene nucleic acid primers and probes**

| **Primer** |
|---|
| SEQ ID NO: 668 |
| CTGGCCGCTTTGGTT |
| SEQ ID NO: 669 |
| ACATTCTTCTGGGAAATTATGG |
| SEQ ID NO: 670 |
| TGAATGCTATTCCTGAAGATATCCG |
| SEQ ID NO: 671 |
| AAAGAAGCAATCGATGAAACATTTG |
| SEQ ID NO: 672 |
| GGGAAATTATGGCACCAAAT |
| SEQ ID NO: 673 |
| TTCTGGGAAATTATGGCACCAAATG |
| SEQ ID NO: 674 |
| TGGCGCTATTAAAGAAGCAATCGA |
| SEQ ID NO: 675 |
| TTCTTCTGGGAAATTATGGCACCAA |
| SEQ ID NO: 676 |
| GCAATCGATGAAACATTTGGTAGC |
| SEQ ID NO: 677 |
| CAACTGGCGCTATTAAAGAAGCA |
| SEQ ID NO: 678 |
| ACAGCTGTTCGTAACAATGGTG |
| SEQ ID NO: 679 |
| TCAGATATGAATGCTATTCCTGAAG |
| SEQ ID NO: 680 |
| GGTAGCTTTGATGAAATGAAAGCTG |
| SEQ ID NO: 681 |
| TGCTGGTGGACAACCAACTG |
| SEQ ID NO: 682 |
| ACCAAATGCTGGTGGACAAC |
| SEQ ID NO: 683 |
| GGTCACGCAAACCATACATTCTTC |
| SEQ ID NO: 684 |
| TGAAAGCTGCTTTCAAAACAGCTG |
| SEQ ID NO: 685 |
| TTGATGAAATGAAAGCTGCTTTCAA |
| SEQ ID NO: 686 |
| GAAATGAAAGCTGCTTTCAAAACAG |
| SEQ ID NO: 687 |
| CTATTCCTGAAGATATCCGTACTGC |
| SEQ ID NO: 688 |
| AACATTCTTCTGGGAAATTATGGCA |
| SEQ ID NO: 689 |
| GCAAACCAAACATTCTTCTGGGAAA |
| SEQ ID NO: 690 |
| CAAACATTCTTCTGGGAAATTATGG |
| SEQ ID NO: 691 |
| GGTCACGCAAACCAAACATTCT |
| SEQ ID NO: 692 |
| GATGAAACATTTGGCAGCTTTGATG |
| SEQ ID NO: 693 |
| ACATTTGGCAGCTTTGATGAAATG |
| SEQ ID NO: 694 |
| TGGCGGGCACGCAA |
| SEQ ID NO: 695 |
| CAACCAACTGGCGCTATTAAAGA |
| SEQ ID NO: 696 |
| GGACAACCAACTGGCGCTA |
| SEQ ID NO: 697 |
| TGCGACTGGCCGCTTT |
| SEQ ID NO: 698 |
| TTTCAAAACAGCTGCGACTGG |
| SEQ ID NO: 699 |
| GTTTCATCGATTGCTTCTTTAATAG |
| SEQ ID NO: 700 |
| CCAAAGCGGCCAGT |
| SEQ ID NO: 701 |
| AGAAGAATGTATGGTTTGCG |
| SEQ ID NO: 702 |
| GCCATAATTTCCCAGAAGAATG |
| SEQ ID NO: 703 |
| TTCTAATTTACCGTTATTCACAACT |
| SEQ ID NO: 704 |
| GAAAGCAGCTTTCATTTCATC |
| SEQ ID NO: 705 |
| GGTGATTTCTAATTTACCGTTATTC |
| SEQ ID NO: 706 |
| AGCGCCAGTTGGTTG |
| SEQ ID NO: 707 |
| TTCTTTAATAGCGCCAGTTGGTTG |
| SEQ ID NO: 708 |
| CCAAATGTTTCATCGATTGCTTCTT |
| SEQ ID NO: 709 |
| TTTCATCGATTGCTTCTTTAATAGC |
| SEQ ID NO: 710 |
| TTTGGTGCCATAATTTCCCAGAAGA |
| SEQ ID NO: 711 |
| GATTGCTTCTTTAATAGCGCCAGTT |
| SEQ ID NO: 712 |
| TGAACCAAAGCGGCCAGT |
| SEQ ID NO: 713 |
| CAAAGCGGCCAGTTGCA |
| SEQ ID NO: 714 |
| AGCGGCCAGTTGCAG |
| SEQ ID NO: 715 |
| TACCGTTATTCACAACTAACCAAGC |
| SEQ ID NO: 716 |
| ATTTACCGTTATTCACAACTAACCA |
| SEQ ID NO: 717 |
| AATTTACCGTTATTCACAACTAACC |
| SEQ ID NO: 718 |
| CCGTTATTCACAACTAACCAAGCC |
| SEQ ID NO: 719 |
| AAGCAGCTTTCATTTCATCAAAGC |
| SEQ ID NO: 720 |
| CCAGTTGGTTGTCCACCAG |
| SEQ ID NO: 721 |
| ACAACTAACCAAGCCCAACC |
| SEQ ID NO: 722 |
| CAGTTGCAGCTGTTTTGAAAGCA |
| SEQ ID NO: 723 |
| TAACCAAGCCCAACCTGAACC |
| SEQ ID NO: 724 |
| GCTGTTTTGAAAGCAGCTTTCATT |
| SEQ ID NO: 725 |
| GCCCAACCTGAACCAAAGC |
| SEQ ID NO: 726 |
| TCAAAGCTACCAAATGTTTCATCGA |
| SEQ ID NO: 727 |
| ATTTCCCAGAAGAATGTTTGGTTTG |
| SEQ ID NO: 728 |
| TTTCCCAGAAGAATGTTTGGTTTGC |
| SEQ ID NO: 729 |
| GCTGCCAAATGTTTCATCGATTG |
| SEQ ID NO: 730 |
| TGCCAAATGTTTCATCGATTGCTT |
| SEQ ID NO: 731 |
| TTTCATCAAAGCTGCCAAATGTTTC |
| SEQ ID NO: 732 |
| TTCATCAAAGCTGCCAAATGTTTCA |
| SEQ ID NO: 733 |
| CCAGCATTTGGTGCCATAATTTC |

| **Probe** |
|---|
| SEQ ID NO: 734 |
| CTTCTTTAATAGCGCCAGTTGGTTG |
| SEQ ID NO: 735 |
| CGCTATTAAAGAAGCAATCGATGAAACATTTG |
| SEQ ID NO: 736 |
| CAGGTTGGGCTTGGTTAGTTGT |
| SEQ ID NO: 737 |
| CTGGGAAATTATGGCACCAAATGCT |
| SEQ ID NO: 738 |
| AGAAGCAATCGATGAAACATTTGGTAGCTTTGATG |
| SEQ ID NO: 739 |
| CCAACTGGCGCTATTAAAGAAGCAATCGATGAAAC |
| SEQ ID NO: 740 |
| AATGTTTCATCGATTGCTTCTTTAATAGCGCCAGT |
| SEQ ID NO: 741 |
| ATCGATGAAACATTTGGTAGCTTTGATGAAATGAA |
| SEQ ID NO: 742 |
| ATCGATTGCTTCTTTAATAGCGCCAGTTGGTTG |
| SEQ ID NO: 743 |
| TGGCCGCTTTGGTTCAGGTTGGG |
| SEQ ID NO: 744 |
| TGCCATAATTTCCCAGAAGAATGTATGGTTTGCG |
| SEQ ID NO: 745 |
| CAGCATTTGGTGCCATAATTTCCCAGAAGAATGT |
| SEQ ID NO: 746 |
| CCAACCTGAACCAAAGCGGCCAG |
| SEQ ID NO: 747 |
| CAGCTGTTCGTAACAATGGTGGCGG |
| SEQ ID NO: 748 |
| TTCTTTAATAGCGCCAGTTGGTTGTCCACCAG |
| SEQ ID NO: 749 |
| TAACCAAGCCCAACCTGAACCAAAGCG |
| SEQ ID NO: 750 |
| TGGTGGACAACCAACTGGCGCTATTAAAGAAG |
| SEQ ID NO: 751 |
| AGCAGCTTTCATTTCATCAAAGCTACCAAATGTTT |
| SEQ ID NO: 752 |
| CAAATGCTGGTGGACAACCAACTGGC |
| SEQ ID NO: 753 |
| AAACATTTGGTAGCTTTGATGAAATGAAAGCTGCT |
| SEQ ID NO: 754 |
| CGTGACCGCCACCATTGTTACGAACA |
| SEQ ID NO: 755 |
| CGCCAGTTGGTTGTCCACCAGC |
| SEQ ID NO: 756 |
| CTGCTTTCAAAACAGCTGCAACTGGCC |
| SEQ ID NO: 757 |
| AGAAGAATGTATGGTTTGCGTGACCGCC |
| SEQ ID NO: 758 |
| AAAGCGGCCAGTTGCAGCTGT |
| SEQ ID NO: 759 |
| AACCAAACATTCTTCTGGGAAATTATGGCACCAAA |
| SEQ ID NO: 760 |
| CGCCACCATTGTTACGAACGGCTGT |
| SEQ ID NO: 761 |
| CACGCAAACCATACATTCTTCTGGGAAATTATGGC |
| SEQ ID NO: 762 |
| TTCATTTCATCAAAGCTGCCAAATGTTTCATCGAT |
| SEQ ID NO: 763 |
| CGCTATTAAAGAAGCAATCGATGAAACATTTGGCA |
| SEQ ID NO: 764 |
| AAGCTGCCAAATGTTTCATCGATTGCTTCTTTAAT |
| SEQ ID NO: 765 |
| TCATCAAAGCTGCCAAATGTTTCATCGATTGCTTC |
| SEQ ID NO: 766 |
| CCAGTTGCAGCTGTTTTGAAAGCAGC |
| SEQ ID NO: 767 |
| CATTCTTCTGGGAAATTATGGCACCAAATGCTGG |
| SEQ ID NO: 768 |
| TGGGAAATTATGGCACCAAATGCTGGCG |
| SEQ ID NO: 769 |
| CTGCGACTGGCCGCTTTGGTTCA |
| SEQ ID NO: 770 |
| TGAACCAAAGCGGCCAGTCGCAG |

**Table 9B. Efs sodA gene solutions**

| **Group No**. | **Forward** | **Probe** | **Reverse** |
|---|---|---|---|
| 606 | SEQ ID NO: 670 | SEQ ID NO: 754 | SEQ ID NO: 733 |
| | TGAATGCTATTCCTGAAGATATCCG | CGTGACCGCCACCATTGTTACGAACA | CCAGCATTTGGTGCCATAATTTC |
| 607 | SEQ ID NO: 670 | SEQ ID NO: 747 | SEQ ID NO: 733 |
| | TGAATGCTATTCCTGAAGATATCCG | CAGCTGTTCGTAACAATGGTGGCGG | CCAGCATTTGGTGCCATAATTTC |
| 608 | SEQ ID NO: 693 | SEQ ID NO: 743 | SEQ ID NO: 716 |
| | ACATTTGGCAGCTTTGATGAAATG | TGGCCGCTTTGGTTCAGGTTGGG | ATTTACCGTTATTCACAACTAACCA |
| 609 | SEQ ID NO: 683 | SEQ ID NO: 768 | SEQ ID NO: 730 |
| | GGTCACGCAAACCATACATTCTTC | TGGGAAATTATGGCACCAAATGCTGGCG | TGCCAAATGTTTCATCGATTGCTT |
| 610 | SEQ ID NO: 691 | SEQ ID NO: 737 | SEQ ID NO: 730 |
| | GGTCACGCAAACCAAACATTCT | CTGGGAAATTATGGCACCAAATGCT | TGCCAAATGTTTCATCGATTGCTT |
| 611 | SEQ ID NO: 670 | SEQ ID NO: 757 | SEQ ID NO: 733 |
| | TGAATGCTATTCCTGAAGATATCCG | AGAAGAATGTATGGTTTGCGTGACCGCC | CCAGCATTTGGTGCCATAATTTC |
| 612 | SEQ ID NO: 670 | SEQ ID NO: 760 | SEQ ID NO: 733 |
| | TGAATGCTATTCCTGAAGATATCCG | CGCCACCATTGTTACGAACGGCTGT | CCAGCATTTGGTGCCATAATTTC |
| 613 | SEQ ID NO: 683 | SEQ ID NO: 752 | SEQ ID NO: 730 |
| | GGTCACGCAAACCATACATTCTTC | CAAATGCTGGTGGACAACCAACTGGC | TGCCAAATGTTTCATCGATTGCTT |
| 614 | SEQ ID NO: 683 | SEQ ID NO: 755 | SEQ ID NO: 730 |
| | GGTCACGCAAACCATACATTCTTC | CGCCAGTTGGTTGTCCACCAGC | TGCCAAATGTTTCATCGATTGCTT |
| 615 | SEQ ID NO: 693 | SEQ ID NO: 769 | SEQ ID NO: 717 |
| | ACATTTGGCAGCTTTGATGAAATG | CTGCGACTGGCCGCTTTGGTTCA | AATTTACCGTTATTCACAACTAACC |
| 616 | SEQ ID NO: 692 | SEQ ID NO: 766 | SEQ ID NO: 718 |
| | GATGAAACATTTGGCAGCTTTGATG | CCAGTTGCAGCTGTTTTGAAAGCAGC | CCGTTATTCACAACTAACCAAGCC |
| 617 | SEQ ID NO: 693 | SEQ ID NO: 766 | SEQ ID NO: 717 |
| | ACATTTGGCAGCTTTGATGAAATG | CCAGTTGCAGCTGTTTTGAAAGCAGC | AATTTACCGTTATTCACAACTAACC |
| 618 | SEQ ID NO: 693 | SEQ ID NO: 770 | SEQ ID NO: 717 |
| | ACATTTGGCAGCTTTGATGAAATG | TGAACCAAAGCGGCCAGTCGCAG | AATTTACCGTTATTCACAACTAACC |
| 619 | SEQ ID NO: 692 | SEQ ID NO: 769 | SEQ ID NO: 718 |
| | GATGAAACATTTGGCAGCTTTGATG | CTGCGACTGGCCGCTTTGGTTCA | CCGTTATTCACAACTAACCAAGCC |
| 620 | SEQ ID NO: 692 | SEQ ID NO: 770 | SEQ ID NO: 718 |
| | GATGAAACATTTGGCAGCTTTGATG | TGAACCAAAGCGGCCAGTCGCAG | CCGTTATTCACAACTAACCAAGCC |
| 621 | SEQ ID NO: 679 | SEQ ID NO: 760 | SEQ ID NO: 710 |
| | TCAGATATGAATGCTATTCCTGAAG | CGCCACCATTGTTACGAACGGCTGT | TTTGGTGCCATAATTTCCCAGAAGA |
| 622 | SEQ ID NO: 692 | SEQ ID NO: 758 | SEQ ID NO: 718 |
| | GATGAAACATTTGGCAGCTTTGATG | AAAGCGGCCAGTTGCAGCTGT | CCGTTATTCACAACTAACCAAGCC |
| 623 | SEQ ID NO: 692 | SEQ ID NO: 756 | SEQ ID NO: 718 |
| | GATGAAACATTTGGCAGCTTTGATG | CTGCTTTCAAAACAGCTGCAACTGGCC | CCGTTATTCACAACTAACCAAGCC |
| 624 | SEQ ID NO: 693 | SEQ ID NO: 758 | SEQ ID NO: 717 |
| | ACATTTGGCAGCTTTGATGAAATG | AAAGCGGCCAGTTGCAGCTGT | AATTTACCGTTATTCACAACTAACC |
| 625 | SEQ ID NO: 679 | SEQ ID NO: 754 | SEQ ID NO: 710 |
| | TCAGATATGAATGCTATTCCTGAAG | CGTGACCGCCACCATTGTTACGAACA | TTTGGTGCCATAATTTCCCAGAAGA |
| 626 | SEQ ID NO: 679 | SEQ ID NO: 747 | SEQ ID NO: 710 |
| | TCAGATATGAATGCTATTCCTGAAG | CAGCTGTTCGTAACAATGGTGGCGG | TTTGGTGCCATAATTTCCCAGAAGA |
| 627 | SEQ ID NO: 693 | SEQ ID NO: 756 | SEQ ID NO: 717 |
| | ACATTTGGCAGCTTTGATGAAATG | CTGCTTTCAAAACAGCTGCAACTGGCC | AATTTACCGTTATTCACAACTAACC |

**Table 10A. Efm novel gene nucleic acid primers and probes**

| **PRIMER** |
|---|
| SEQ ID NO: 771 |
| CCTCCTCGTTTCTTACTGAT |
| SEQ ID NO: 772 |
| GGTTCCTCCTCGTTTCTT |
| SEQ ID NO: 773 |
| GTTTTCGTTCTCTTCTAGCAAAA |
| SEQ ID NO: 774 |
| CGATCCATCCGACTCATTG |
| SEQ ID NO: 775 |
| CGATCCATCCGACTCATT |
| SEQ ID NO: 776 |
| TTTCGCCTTCTTATGGATGTCCTTA |
| SEQ ID NO: 777 |
| GATCCATCCGACTCATTGTGGT |
| SEQ ID NO: 778 |
| TTATAGCGATCCATCCGACTCATTG |
| SEQ ID NO: 779 |
| TTATAGCGATCCATCCGACTCATT |
| SEQ ID NO: 780 |
| TCTTATGGATGTCCTTATAGCGATC |
| SEQ ID NO: 781 |
| TGGATGTCCTTATAGCGATCCATC |
| SEQ ID NO: 782 |
| TTGTGGTTCCTCCTCGTTTCTTAC |
| SEQ ID NO: 783 |
| TCATTGTGGTTCCTCCTCGTTTC |
| SEQ ID NO: 784 |
| TCCGACTCATTGTGGTTCCTC |
| SEQ ID NO: 785 |
| TTCAGAGGTTTCGCCTTCTTATGG |
| SEQ ID NO: 786 |
| GCTTCAGAGGTTTCGCCTTCTTA |
| SEQ ID NO: 787 |
| GCTTCAGAGGTTTCGCCTTCTT |
| SEQ ID NO: 788 |
| GTATCCCGCTTCAGAGGTTTC |
| SEQ ID NO: 789 |
| TCCCGCTTCAGAGGTTTCG |
| SEQ ID NO: 790 |
| CGTATCCCGCTTCAGAGG |
| SEQ ID NO: 791 |
| GGTTTCGCCTTCTTATGGATGTC |
| SEQ ID NO: 792 |
| TACTCCCCGTATCCCGCTTCA |
| SEQ ID NO: 793 |
| CCCGTATCCCGCTTCAGA |
| SEQ ID NO: 794 |
| AAGAAACGAGGAGGAACC |
| SEQ ID NO: 795 |
| CGCTATAAGGACATCCATAAGA |
| SEQ ID NO: 796 |
| CGAGGAGGAACCACAATG |
| SEQ ID NO: 797 |
| CTTTTGTCGATTGGTTATCGTA |
| SEQ ID NO: 798 |
| CTTTTGTCGATTGGTTATCGTAT |
| SEQ ID NO: 799 |
| TATCAGTAAGAAACGAGGAGGAACC |
| SEQ ID NO: 800 |
| ATGATAGGCTTTTGTCGATTGGTTA |
| SEQ ID NO: 801 |
| CACAATGAGTCGGATGGATCG |
| SEQ ID NO: 802 |
| ACCACAATGAGTCGGATGGA |
| SEQ ID NO: 803 |
| CAATGAGTCGGATGGATCGCTAT |
| SEQ ID NO: 804 |
| CAATGAGTCGGATGGATCGCTA |
| SEQ ID NO: 805 |
| GTCGGATGGATCGCTATAAGG |
| |
| SEQ ID NO: 806 |
| ATTTATCAGTAAGAAACGAGGAGGA |
| |
| SEQ ID NO: 807 |
| GGATGGATCGCTATAAGGACATCC |
| SEQ ID NO: 808 |
| TAAGAAACGAGGAGGAACCACAAT |
| SEQ ID NO: 809 |
| GAGGAGGAACCACAATGAGTCG |
| SEQ ID NO: 810 |
| ATAAGGACATCCATAAGAAGGCGAA |
| SEQ ID NO: 811 |
| ATCGCTATAAGGACATCCATAAGAA |
| SEQ ID NO: 812 |
| TGTATCGAAGCATTTATTTCGTTAT |
| SEQ ID NO: 813 |
| GGCTTTTGTCGATTGGTTATCGTAT |
| SEQ ID NO: 814 |
| GGCTTTTGTCGATTGGTTATCGTA |
| SEQ ID NO: 815 |
| TTTGTCGATTGGTTATCGTATCCTT |
| SEQ ID NO: 816 |
| TGTCGATTGGTTATCGTATCCTTTA |
| SEQ ID NO: 817 |
| CGAGGAGGAACCACAATGAG |
| SEQ ID NO: 818 |
| AAATGATAAGCTTTTGTCGATTGGT |

| **PROBE** |
|---|
| SEQ ID NO: 819 |
| CGGATGGATCGCTATAAGGACATCCAT |
| SEQ ID NO: 820 |
| AGGATACGATAACCAATCGACAAAAGC |
| SEQ ID NO: 821 |
| CGATCCATCCGACTCATTGTGGT |
| SEQ ID NO: 822 |
| ACTGATAAATAAAGGATACGATAACCAATCGACAA |
| SEQ ID NO: 823 |
| CCCGTATCCCGCTTCAGAGG |
| SEQ ID NO: 824 |
| AGGCGAAACCTCTGAAGCG |
| SEQ ID NO: 825 |
| TTCTTATGGATGTCCTTATAGCGATCCATCCGACT |
| SEQ ID NO: 826 |
| TGGATGTCCTTATAGCGATCCATCCGACTCATTG |
| SEQ ID NO: 827 |
| TGGTTCCTCCTCGTTTCTTACTGATAAATAAAGGA |
| SEQ ID NO: 828 |
| TTCGCCTTCTTATGGATGTCCTTATAGCGATCCA |
| SEQ ID NO: 829 |
| AATGATAGGCTTTTGTCGATTGGTTATCGTATCCT |
| SEQ ID NO: 830 |
| CAATGAGTCGGATGGATCGCTATAAGGACATCCAT |
| SEQ ID NO: 831 |
| CCTTTATTTATCAGTAAGAAACGAGGAGGAACCAC |
| SEQ ID NO: 832 |
| TTATAGCGATCCATCCGACTCATTGTGGTTCCTC |
| SEQ ID NO: 833 |
| TCAGTAAGAAACGAGGAGGAACCACAATGAGTCG |
| SEQ ID NO: 834 |
| TCGCTATAAGGACATCCATAAGAAGGCGAAACCTC |
| SEQ ID NO: 835 |
| CTCCTCGTTTCTTACTGATAAATAAAGGATACGAT |
| SEQ ID NO: 836 |
| TCGTATCCTTTATTTATCAGTAAGAAACGAGGAGG |
| SEQ ID NO: 837 |
| AAATAAAGGATACGATAACCAATCGACAAAAGCCT |
| SEQ ID NO: 838 |
| ATAAATAAAGGATACGATAACCAATCGACAAAAGC |
| SEQ ID NO: 839 |
| CTTCAGAGGTTTCGCCTTCTTATGGATGTCCTTAT |
| SEQ ID NO: 840 |
| TAAGGACATCCATAAGAAGGCGAAACCTCTGAAGC |
| SEQ ID NO: 841 |
| TTGTCGATTGGTTATCGTATCCTTTATTTATCAGT |
| |
| SEQ ID NO: 842 |
| CATCCATAAGAAGGCGAAACCTCTGAAGCG |
| |
| SEQ ID NO: 843 |
| CGAGGAGGAACCACAATGAGTCGGATG |
| SEQ ID NO: 844 |
| CGACTCATTGTGGTTCCTCCTCGTTTCTTACTG |
| SEQ ID NO: 845 |
| AGGATACGATAACCAATCGACAAAAGCTTATCATT |
| SEQ ID NO: 846 |
| AAGCTTTTGTCGATTGGTTATCGTATCCTTTATTT |

**Table 10B. Efm novel gene solutions**

| **Group No.** | **Forward** | **Probe** | **Reverse** |
|---|---|---|---|
| 628 | SEQ ID NO: 780 | SEQ ID NO: 843 | SEQ ID NO: 816 |
| | TCTTATGGATGTCCTTATAGCGATC | CGAGGAGGAACCACAATGAGTCGGATG | TGTCGATTGGTTATCGTATCCTTTA |
| | | | |
| 629 | SEQ ID NO: 780 | SEQ ID NO: 843 | SEQ ID NO: 813 |
| | TCTTATGGATGTCCTTATAGCGATC | CGAGGAGGAACCACAATGAGTCGGATG | GGCTTTTGTCGATTGGTTATCGTAT |
| | | | |
| 630 | SEQ ID NO: 776 | SEQ ID NO: 821 | SEQ ID NO: 816 |
| | TTTCGCCTTCTTATGGATGTCCTTA | CGATCCATCCGACTCATTGTGGT | TGTCGATTGGTTATCGTATCCTTTA |
| | | | |
| 631 | SEQ ID NO: 776 | SEQ ID NO: 843 | SEQ ID NO: 816 |
| | TTTCGCCTTCTTATGGATGTCCTTA | CGAGGAGGAACCACAATGAGTCGGATG | TGTCGATTGGTTATCGTATCCTTTA |
| | | | |
| 632 | SEQ ID NO: 780 | SEQ ID NO: 843 | SEQ ID NO: 800 |
| | TCTTATGGATGTCCTTATAGCGATC | CGAGGAGGAACCACAATGAGTCGGATG | ATGATAGGCTTTTGTCGATTGGTTA |
| | | | |
| 633 | SEQ ID NO: 776 | SEQ ID NO: 821 | SEQ ID NO: 813 |
| | TTTCGCCTTCTTATGGATGTCCTTA | CGATCCATCCGACTCATTGTGGT | GGCTTTTGTCGATTGGTTATCGTAT |
| | | | |
| 634 | SEQ ID NO: 776 | SEQ ID NO: 843 | SEQ ID NO: 813 |
| | TTTCGCCTTCTTATGGATGTCCTTA | CGAGGAGGAACCACAATGAGTCGGATG | GGCTTTTGTCGATTGGTTATCGTAT |
| | | | |
| 635 | SEQ ID NO: 785 | SEQ ID NO: 821 | SEQ ID NO: 816 |
| | TTCAGAGGTTTCGCCTTCTTATGG | CGATCCATCCGACTCATTGTGGT | TGTCGATTGGTTATCGTATCCTTTA |
| | | | |
| 636 | SEQ ID NO: 785 | SEQ ID NO: 843 | SEQ ID NO: 816 |
| | TTCAGAGGTTTCGCCTTCTTATGG | CGAGGAGGAACCACAATGAGTCGGATG | TGTCGATTGGTTATCGTATCCTTTA |

**Table 11A. Efs novel gene nucleic acid primers and probes**

| **PRIMER** |
|---|
| SEQ ID NO: 847 |
| CATCTTCAGGAGCTAAATCAAT |
| SEQ ID NO: 848 |
| AAGTACTCGTACTTGAAAATCATCT |
| SEQ ID NO: 849 |
| GTCGCACATCTTCAGGAGCTAAA |
| SEQ ID NO: 850 |
| CTAATTGTTTCACTGTCTCTGGATT |
| |
| SEQ ID NO: 851 |
| AAGAAGTATCCAATGACTGTTGCAA |
| |
| SEQ ID NO: 852 |
| TCCAATGACTGTTGCAATAACTGGA |
| SEQ ID NO: 853 |
| TTCACTGTCTCTGGATTAGGTACTC |
| SEQ ID NO: 854 |
| AATTGTTTCACTGTCTCTGGATTAG |
| SEQ ID NO: 855 |
| TCACCATTTTCAATATACGCATCTA |
| SEQ ID NO: 856 |
| TTCACCATTTTCAATATACGCATCT |
| SEQ ID NO: 857 |
| CGTATGATACAGCGTTTCTAATTGT |
| SEQ ID NO: 858 |
| AGCGTTTCTAATTGTTTCACTGTCT |
| SEQ ID NO: 859 |
| CAGCGTTTCTAATTGTTTCACTGTC |
| SEQ ID NO: 860 |
| TTAGGTACTCCGTCAAGTACTCGT |
| SEQ ID NO: 861 |
| GACAAGCGTCGCACATCTTC |
| SEQ ID NO: 862 |
| AAAAGAAGTATCCAATGACTGTTGC |
| SEQ ID NO: 863 |
| TTTTAATCGTATGATACAGCGTTTC |
| SEQ ID NO: 864 |
| GGAACAAATTAGCACCTCTATTCTA |
| SEQ ID NO: 865 |
| GATTGCTTGTTCCATTGGCT |
| SEQ ID NO: 866 |
| TGTTGCAATAACTGGATTGCTTGTT |
| SEQ ID NO: 867 |
| TCTGGATTAGGTACTCCGTCAAGT |
| SEQ ID NO: 868 |
| TAACTGGATTGCTTGTTCCATTGG |
| SEQ ID NO: 869 |
| TAATTGGGACAAGCGTCGCA |
| SEQ ID NO: 870 |
| TTTCTGGGAACAAATTAGCACCTCT |
| SEQ ID NO: 871 |
| TCCGTCAAGTACTCGTACTTGAAAA |
| SEQ ID NO: 872 |
| ATTGTTTCACTGTCTCTGGATTAGG |
| SEQ ID NO: 873 |
| GCTTTCGAGCCAATGGAACA |
| SEQ ID NO: 874 |
| CGGAGTACCTAATCCAGAG |
| SEQ ID NO: 875 |
| TTCGTTAAAATAAAACAGGTATGGA |
| SEQ ID NO: 876 |
| AGTAAAGGCATTGATTATTCTTTCT |
| SEQ ID NO: 877 |
| ACAGTAAAGGCATTGATTATTCTTT |
| SEQ ID NO: 878 |
| ATGGAAGTTGTTACATTTTGAATAG |
| SEQ ID NO: 879 |
| TTGCAACAGTCATTGGATACTTCTT |
| SEQ ID NO: 880 |
| TTAGATGATTTTCAAGTACGAGTAC |
| SEQ ID NO: 881 |
| GAGCCAATGGAACAAGCAATCCA |
| SEQ ID NO: 882 |
| GAGGTGCTAATTTGTTCCCAGAAAA |
| SEQ ID NO: 883 |
| GTACGAGTACTTGACGGAGTACCTA |
| SEQ ID NO: 884 |
| AAAATAAAACAGGTATGGAAGTTG |
| SEQ ID NO: 885 |
| TCCAGTTATTGCAACAGTCATTGGA |
| SEQ ID NO: 886 |
| AGAAGAAAGACAGTAAAGGCATTGA |
| SEQ ID NO: 887 |
| TAAAACAGGTATGGAAGTTGTTACA |
| |
| SEQ ID NO: 888 |
| TTTCGAGCCAATGGAACAAGCA |
| |
| SEQ ID NO: 889 |
| TGGAACAAGCAATCCAGTTATTGCA |
| SEQ ID NO: 890 |
| TGAATAGAATAGAGGTGCTAATTTG |
| SEQ ID NO: 891 |
| GAGCCAATGGAACAAGCAAT |
| SEQ ID NO: 892 |
| AACAGGTATGGAAGTTGTTACATTT |
| SEQ ID NO: 893 |
| CAAGCAATCCAGTTATTGCAACAG |
| SEQ ID NO: 894 |
| AGACAGTAAAGGCATTGATTATTCT |
| SEQ ID NO: 895 |
| GCAACAGTCATTGGATACTTCTTTT |
| SEQ ID NO: 896 |
| TAGAGGTGCTAATTTGTTCCCAGAA |
| SEQ ID NO: 897 |
| AAAGGCATTGATTATTCTTTCTTTT |
| SEQ ID NO: 898 |
| GAATAGAATAGAGGTGCTAATTTGT |
| SEQ ID NO: 899 |
| ATAGAATAGAGGTGCTAATTTGTTC |
| SEQ ID NO: 900 |
| GTACTTGACGGAGTACCTAATCCAG |
| SEQ ID NO: 901 |
| TTTTCAAGTACGAGTACTTGACGGA |
| SEQ ID NO: 902 |
| AGATGCGTATATTGAAAATGGTGAA |

| **PROBE** |
|---|
| SEQ ID NO: 903 |
| CCAGTTATTGCAACAGTCATTGGATACTTC |
| SEQ ID NO: 904 |
| CGTTAAAATAAAACAGGTATGGAAGTTGTTACATT |
| SEQ ID NO: 905 |
| CCGTCAAGTACTCGTACTTGAAAATCATCTAAAAT |
| SEQ ID NO: 906 |
| ATGTAACAACTTCCATACCTGTTTTATTTTAACGA |
| SEQ ID NO: 907 |
| ACATTTTGAATAGAATAGAGGTGCTAATTTGTTCC |
| SEQ ID NO: 908 |
| AATAGAGGTGCTAATTTGTTCCCAGAAAA |
| SEQ ID NO: 909 |
| CCAGAGACAGTGAAACAATTAGAAACGCTGTATCA |
| SEQ ID NO: 910 |
| CGTATGATACAGCGTTTCTAATTGTTTCACTGTCT |
| SEQ ID NO: 911 |
| AGAAGTATCCAATGACTGTTGCAATAACTGGATTG |
| SEQ ID NO: 912 |
| ACAGTGAAACAATTAGAAACGCTGTATCATACGAT |
| SEQ ID NO: 913 |
| CTGTCTCTGGATTAGGTACTCCGTCAAGTACTCGT |
| SEQ ID NO: 914 |
| CTGGATTAGGTACTCCGTCAAGTACTCGTACTTGA |
| SEQ ID NO: 915 |
| AGGTACTCCGTCAAGTACTCGTACTTGAAAATCAT |
| SEQ ID NO: 916 |
| AGCGTTTCTAATTGTTTCACTGTCTCTGGATTAGG |
| SEQ ID NO: 917 |
| ATCCAATGACTGTTGCAATAACTGGATTGCTTGTT |
| SEQ ID NO: 918 |
| AAACAGGTATGGAAGTTGTTACATTTTGAATAGAA |
| SEQ ID NO: 919 |
| AATGGAACAAGCAATCCAGTTATTGCAACAGTCAT |
| SEQ ID NO: 920 |
| CTTTCGAGCCAATGGAACAAGCAATCCAGTTATT |
| SEQ ID NO: 921 |
| ACCTAATCCAGAGACAGTGAAACAATTAGAAACGC |
| SEQ ID NO: 922 |
| AGCACCTCTATTCTATTCAAAATGTAACAACTTCC |
| SEQ ID NO: 923 |
| ATTGTTTCACTGTCTCTGGATTAGGTACTCCGTCA |
| |
| SEQ ID NO: 924 |
| TGTTGCAATAACTGGATTGCTTGTTCCATTGGCTC |
| |
| SEQ ID NO: 925 |
| TAACTGGATTGCTTGTTCCATTGGCTCGAAAGC |
| SEQ ID NO: 926 |
| ATTCTATTCAAAATGTAACAACTTCCATACCTGTT |
| SEQ ID NO: 927 |
| ATGATTTTCAAGTACGAGTACTTGACGGAGTACCT |
| SEQ ID NO: 928 |
| CTGGGAACAAATTAGCACCTCTATTCTATTCAAAA |
| SEQ ID NO: 929 |
| CAAGTACGAGTACTTGACGGAGTACCTAATCCAGA |
| SEQ ID NO: 930 |
| TGACGGAGTACCTAATCCAGAGACAGTGAAACAAT |
| SEQ ID NO: 931 |
| TGGAAGTTGTTACATTTTGAATAGAATAGAGGTGC |
| SEQ ID NO: 932 |
| TGAATAGAATAGAGGTGCTAATTTGTTCCCAGAAA |
| SEQ ID NO: 933 |
| CGAGTACTTGACGGAGTACCTAATCCAGAGACAG |
| SEQ ID NO: 934 |
| ATTTTAGATGATTTTCAAGTACGAGTACTTGACGG |
| SEQ ID NO: 935 |
| AGCAATCCAGTTATTGCAACAGTCATTGGATACTT |

**Table 11B. Efs novel gene solutions**

| **Group No.** | **Forward** | **Probe** | **Reverse** |
|---|---|---|---|
| 637 | SEQ ID NO: 851 | SEQ ID NO: 925 | SEQ ID NO: 896 |
| | AAGAAGTATCCAATGACTGTTGCAA | TAACTGGATTGCTTGTTCCATTGGCTCGAAAGC | TAGAGGTGCTAATTTGTTCCCAGAA |
| | | | |
| 638 | SEQ ID NO: 868 | SEQ ID NO: 908 | SEQ ID NO: 878 |
| | TAACTGGATTGCTTGTTCCATTGG | AATAGAGGTGCTAATTTGTTCCCAGAAAA | ATGGAAGTTGTTACATTTTGAATAG |
| | | | |
| 639 | SEQ ID NO: 865 | SEQ ID NO: 908 | SEQ ID NO: 892 |
| | GATTGCTTGTTCCATTGGCT | AATAGAGGTGCTAATTTGTTCCCAGAAAA | AACAGGTATGGAAGTTGTTACATTT |
| | | | |
| 640 | SEQ ID NO: 851 | SEQ ID NO: 925 | SEQ ID NO: 890 |
| | AAGAAGTATCCAATGACTGTTGCAA | TAACTGGATTGCTTGTTCCATTGGCTCGAAAGC | TGAATAGAATAGAGGTGCTAATTTG |
| | | | |
| 641 | SEQ ID NO: 868 | SEQ ID NO: 908 | SEQ ID NO: 892 |
| | TAACTGGATTGCTTGTTCCATTGG | AATAGAGGTGCTAATTTGTTCCCAGAAAA | AACAGGTATGGAAGTTGTTACATTT |
| | | | |
| 642 | SEQ ID NO: 865 | SEQ ID NO: 908 | SEQ ID NO: 884 |
| | GATTGCTTGTTCCATTGGCT | AATAGAGGTGCTAATTTGTTCCCAGAAAA | AAAATAAAACAGGTATGGAAGTTG |
| | | | |
| | SEQ ID NO: 866 | SEQ ID NO: 908 | SEQ ID NO: 878 |
| 643 | TGTTGCAATAACTGGATTGCTTGTT | AATAGAGGTGCTAATTTGTTCCCAGAAAA | ATGGAAGTTGTTACATTTTGAATAG |

**Table 12. Efm/Efs dual nucleic acid primers and probes**

| **Group No.** | **Forward** | **Probe** | **Reverse** |
|---|---|---|---|
| 644 | SEQ ID NO: 936 | SEQ ID NO: 937 | SEQ ID NO: 938 |
| | TTGCTTTATCGTGACGTGACTTTG | CATTCTCCTCGCTATCATCGGTTTAATTGTCGG | GATAGTATGGAGGTGGAAATATGGT |
| | | | SEQ ID NO: 939 |
| | | | TATGGAGGTGGATTTTATGGTATTC |

### Example 6. Testing vanA and vanB primer and probe sequences for their ability to amplify their intended target sequences.

The oligonucleotide sequences listed in Table 5 were tested for their ability to amplify their intended target sequences. About 25 µL PCRs were formulated using iQTM Supermix for qPCR (BioRad) and oligos at a final concentration of 400 nM each.

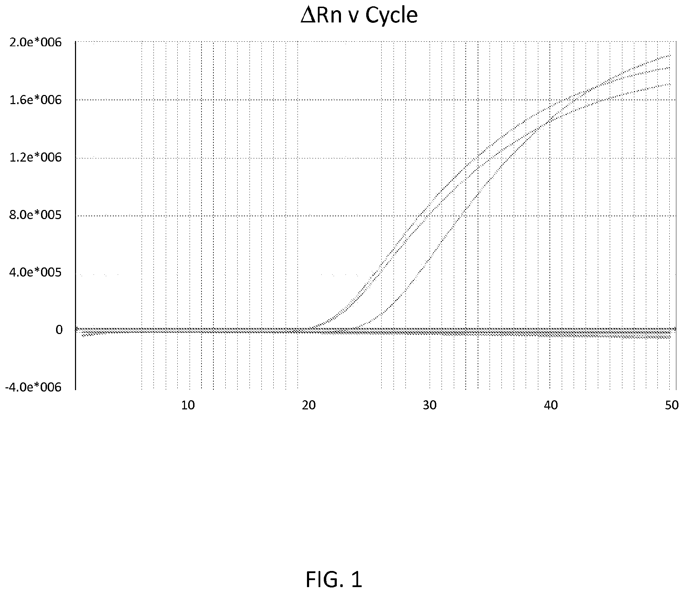

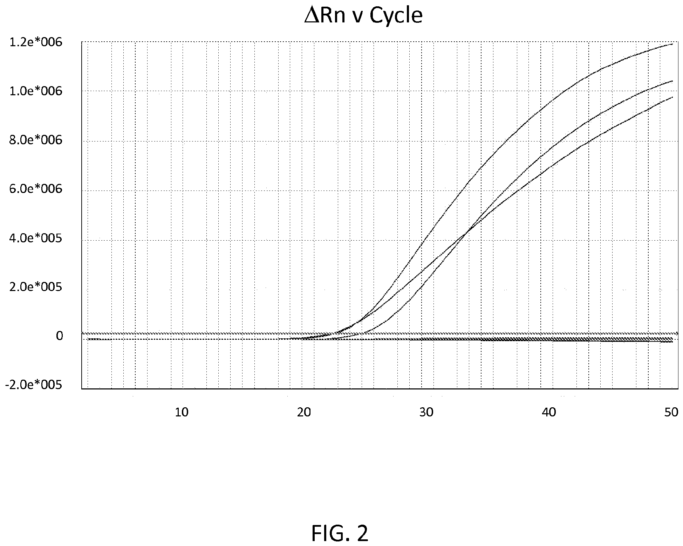

Genomic DNA isolated from *E. faecium* (ATCC No. 5159, strain MMC4, *vanA*) and *E. faecalis* (ATCC No. 700802, strain V583, *vanB*) were loaded into real-time PCRs using oligonucleotide solutions specific for *vanA* and *vanB.* The specificity of the oligonucleotide solutions was assessed by attempting to amplify *E. faecalis* (*vanB*) gDNA with *vanA* oligos and *E. faecium* (*vanA*) gDNA with *vanB* oligos. Amplification plots are illustrated in FIGS. 1 and 2, which show detection of *vanA and vanB.* Synthetic constructs encoding the *vanA* and *vanB* targets were amplified and compared to duplicate reactions where *E. faecium* (ATCC No. 5159, strain MMC4, vanA) and *E. faecalis* (ATCC No. 700802, strain V583, vanB) gDNA was amplified.

PCRs were run on the ABI 7500 with the following Thermal protocol:
- 95 °C 5 minute, initial denaturation
- 50 cycles of:
   ∘ 95 °C 15 sec, denaturation, and
   ∘ 60 °C 1 min, annealing/extension

In FIG. 1, the *vanA* solution (SEQ ID NO: 1, 2, and 5) included amplification of *E*. *faecium* gDNA (*) and the *E. faecium vanA* synthetic construct (§), while the *vanB* solution (SEQ ID NOS: 103, 108, and 66) in FIG. 2 included amplification of the *E. faecalis vanB* synthetic construct (¥) and *E. faecalis* gDNA (+). PCR products were not evident in any of the No template controls (NTC). Ct values corresponding to the presence of PCR product are shown in Table 13 below.

**Table 13. Ct values showing presence of vanA and vanB.**

| | **Oligo solution** | |
|---|---|---|
| **Input** | ***Van*A Ct** | ***vanB* Ct** |
| Synthetic construct | 23.36 | 24.04 |
| NTC | UND | UND |
| E. faecalis (ATCC No. 700802, strain V583, *van*B) | UND | 21.85 |
| | UND | 21.53 |
| E. faecium (ATCC No. 5159, strain MMC4; *vanA*) | 19.67 | UND |
| | 19.80 | UND |

### Example 7. Gel electrophoresis analysis for vanA and vanB.

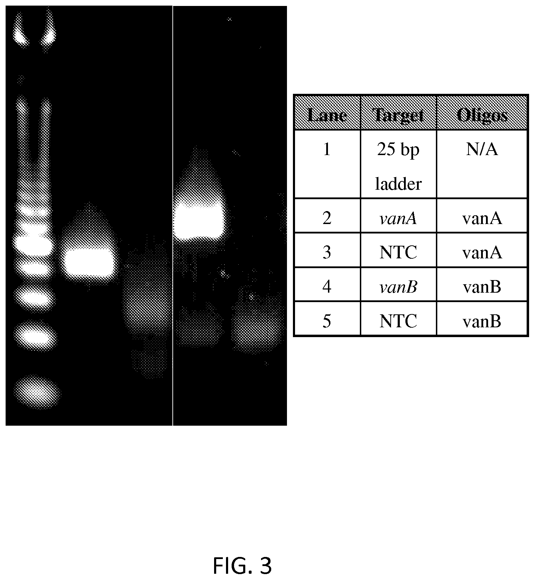

A gel electrophoresis analysis was performed using 20 µL aliquots of post-amplification *vanA* and *vanB* PCR products on an agarose gel plate. The gel electrophoresis of FIG. 3 illustrates that the molecular weight marker was a 25 bp ladder (Invitrogen); gel, 4% agarose e-gel (Invitrogen), EtBr stained; inputs are shown in the table of FIG. 3. The *arrows* on the gel point to the 100 bp and 150 bp markers. The gel illustrates that the *vanA* and *vanB* PCR products migrate according to their predicted sizes.

### Example 8. Testing sodA and Efm/EFs dual sequences for ability to amplify their intended target sequences.

The nucleic acid primers and probes listed in Tables 8B, 9B and 12 were tested for their ability to amplify their intended target sequences. About 25 µL PCRs were formulated using iQTM Supermix for qPCR (BioRad) and oligonucleotides at a final concentration of 400 nM each.

In this example, 1×10⁴ copies of genomic DNA from *E. faecium* (ATCC No. 51559), *E. faecalis* (ATCC. No 700802) and C. *difficile* (ATCC No. 43598) were loaded into real-time PCRs using oligonucleotide solutions directed against *E. faecium sodA, E. faecalis sodA* novel markers for both *E. faecalis* and *E. faecium* (Efm/Efs dual). PCRs were run on the ABI 7500 with the following Thermal protocol:
- 95 °C 5 minute, initial denaturation
- 50 cycles of:
   ∘ 95 °C 15 sec, denaturation
   ∘ 60 °C 1 min, annealing/extension

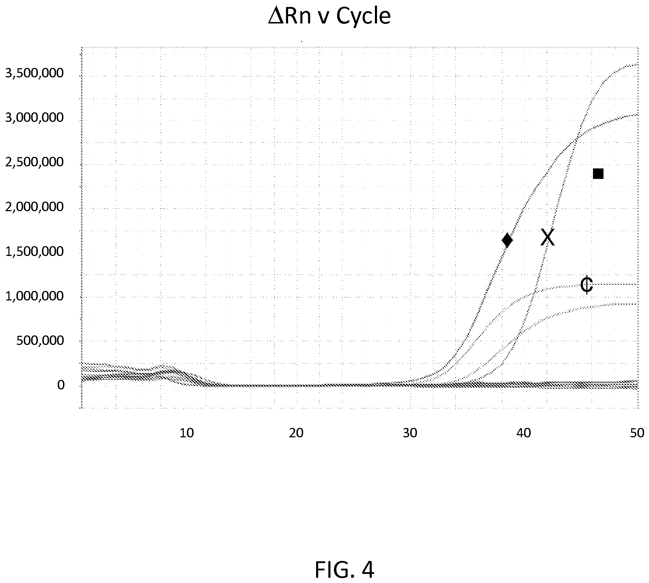

In FIG. 4, amplification plots show detection of *E. faecium* and not *E. faecalis* or C. *difficile* using the *E. faecium sodA* oligonucleotide solution (◆ - SEQ ID NO: 610, 664, and 622); detection of *E. faecalis* and not *E. faecium* or C. *difficile* using the *E. faecalis sodA* oligonulceotide solution (X - SEQ ID NO: 692, 756, and 718); and detection of both *E. faecium* and *E. faecalis* but not C. *difficile* using the Efm/Efs dual oligonucleotide solution (· - SEQ ID NO: 936, 937, 938 and 939). Ct values corresponding to the presence of PCR product are shown in Table 14 below. PCR products were not evident in any of the No template controls (NTC).

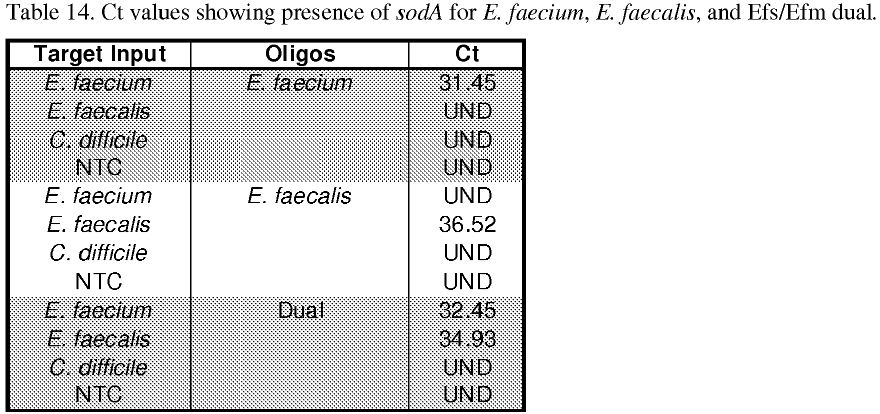

### Example 9. Gel electrophoresis analysis for soda of E. faecium, E. faecalis, and Efm/Efs dual.

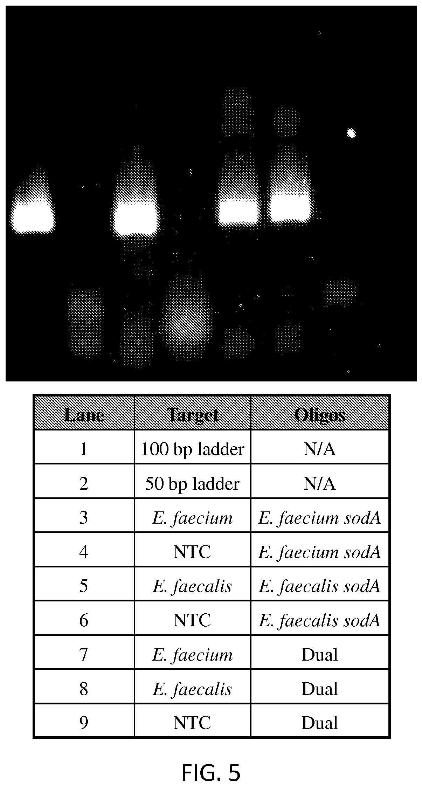

A gel electrophoresis analysis was performed using 20 µL aliquots of post-amplification PCR *sodA* and dual Efs/Efm products on an agarose gel plate. The gel electrophoresis was shown in FIG. 5 where molecular weight markers were the 100 bp ladder and 50 bp ladder (Invitrogen); gel, 4% agarose e-gel (Invitrogen), EtBr stained; inputs as shown in the table of FIG 5. The *arrow* on the gel of FIG. 5 points to the 100 bp marker. The gel illustrates that the PCR products migrate according to their predicted sizes.

## Claims

1. A primer set comprising
(a) at least one forward primer comprising the sequence of SEQ ID NO:1;
(b) at least one reverse primer comprising a sequence selected from the group consisting of SEQ ID NOs: 3, 5, and 32.

2. A method of producing a nucleic acid product, comprising contacting:
a) at least one forward primer comprising the sequence of SEQ ID NO:1; and
b) at least one reverse primer comprising a sequence selected from the group consisting of SEQ ID NOs:3, 5, and 32;
with a sample comprising a *vanA* gene under conditions suitable for nucleic acid polymerization.

3. A method for detecting the vancomycin-resistance gene *vanA* in a sample, comprising:
a) contacting the sample with at least one forward primer comprising the sequence of SEQ ID NO:1 and at least one reverse primer comprising a sequence selected from the group consisting of SEQ ID NOs:3, 5, and 32 under conditions such that nucleic acid amplification occurs to yield an amplicon; and
b) contacting the amplicon with one or more probes comprising one or more sequences selected from the group consisting of SEQ ID NOs:2, 4, and 25 under conditions such that hybridization of the probe to the amplicon occurs, wherein hybridization of the probe is indicative of a *vanA* gene in the sample.

4. The method of Claim 3, wherein each of the one or more probes is labeled with a different detectable label.

5. The method of Claim 3, wherein the one or more probes are labeled with the same detectable label.

6. A kit for detecting vancomycin-resistance gene *vanA* in a sample, comprising
a) one or more probes comprising a sequence selected from the group consisting of SEQ ID NOs:2, 4, and 25;
b) at least one forward primer comprising the sequence of SEQ ID NO:1; and
c) at least one reverse primer comprising a sequence selected from the group consisting of SEQ ID NOs:3, 5, and 32.

7. The kit of Claim 6, further comprising reagents for sequencing a vancomycin-resistance gene in the sample.

8. The kit of Claim 6, wherein the one or more probes are labeled with different detectable labels.

9. The kit of Claim 6, wherein the one or more probes are labeled with the same detectable label.

10. An *in vitro* method of diagnosing a condition, syndrome or disease in a human associated with a vancomycin-resistant organism, comprising:
a) contacting a sample with a forward primer comprising the sequence of SEQ ID NOs:1 and at least one reverse primer comprising a sequence selected from the group consisting of SEQ ID NO:3, 5, and 32;
b) conducting an amplification reaction, thereby producing an amplicon; and
c) detecting the amplicon using one or more probes comprising a sequence selected from the group consisting of SEQ ID NOs:2, 4, and 25;
wherein the detection of an amplicon is indicative of the presence of a vancomycin-resistant organism in the sample.

11. The method of Claim 10, wherein the probe is labeled with a detectable label selected from the group consisting of: a fluorescent label, a chemiluminescent label, a quencher, a radioactive label, biotin and gold.

## Patentansprüche

1. Primer-Set, das Folgendes umfasst:
(a) mindestens einen Vorwärts-Primer, welcher die Sequenz von SEQ ID NO:1 umfasst;
(b) mindestens einen Rückwärts-Primer, welcher eine Sequenz umfasst, die ausgewählt ist aus der Gruppe bestehend aus SEQ ID NOs:3, 5 und 32.

2. Verfahren zur Herstellung eines Nukleinsäure-Produkts, bei dem man
a) mindestens einen Vorwärts-Primer, welcher die Sequenz von SEQ ID NO:1 umfasst; und
b) mindestens einen Rückwärts-Primer, welcher eine Sequenz umfasst, die ausgewählt ist aus der Gruppe bestehend aus SEQ ID NOs:3, 5 und 32,
mit einer Probe, die ein *vanA*-Gen umfasst, unter Bedingungen in Kontakt bringt, die für eine Nukleinsäure-Polymerisation geeignet sind.

3. Verfahren zur Detektion des Vancomycin-Resistenz-Gens *vanA* in einer Probe, bei dem man
a) die Probe mit mindestens einem Vorwärts-Primer, welcher die Sequenz von SEQ ID NO:1 umfasst, und mindestens einem Rückwärts-Primer, der eine Sequenz umfasst, die ausgewählt ist aus der Gruppe bestehend aus SEQ ID NOs: 3, 5 und 32, unter Bedingungen in Kontakt bringt, die eine Nukleinsäure-Amplifikation erlauben, sodass ein Amplikon erhalten wird; und
b) das Amplikon mit einer oder mehreren Sonde(n), die eine oder mehrere Sequenzen umfasst/umfassen, welche ausgewählt ist/sind aus der Gruppe bestehend aus SEQ ID NOs: 2, 4 und 25, unter Bedingungen in Kontakt bringt, bei denen eine Hybridisierung der Sonde an das Amplikon erfolgt, wobei die Hybridisierung der Sonde darauf verweist, dass ein *vanA*-Gen in der Probe ist.

4. Verfahren gemäß Anspruch 3, bei dem jede der einen oder mehreren Sonde(n) mit einer unterschiedlichen nachweisbaren Markierung versehen ist/sind.

5. Verfahren gemäß Anspruch 3, bei dem die eine oder mehreren Sonde(n) mit derselben nachweisbaren Markierung versehen ist/sind.

6. Kit zur Detektion des Vancomycin-Resistenz-Gens *vanA* in einer Probe, das Folgendes umfasst:
a) eine oder mehrere Sonde(n), die eine Sequenz umfasst/umfassen, welche ausgewählt ist aus der Gruppe bestehend aus SEQ ID NOs:2, 4 und 25;
b) mindestens einen Vorwärts-Primer, welcher die Sequenz von SEQ ID NO:1 umfasst; und
c) mindestens einen Rückwärts-Primer, welcher eine Sequenz ausgewählt aus der Gruppe bestehend aus SEQ ID NOs:3, 5 und 32 umfasst.

7. Kit gemäß Anspruch 6, das ferner ein Reagenz für die Sequenzierung eines in der Probe enthaltenen Vancomycin-Resistenz-Gens umfasst.

8. Kit gemäß Anspruch 6, wobei die eine oder mehreren Sonde(n) mit unterschiedlichen nachweisbaren Markierungen versehen ist/sind.

9. Kit gemäß Anspruch 6, wobei die eine oder mehreren Sonde(n) mit derselben nachweisbaren Markierung versehen ist/sind.

10. *In* vitro-Verfahren zur Diagnose eines Zustands, eines Syndroms oder einer Krankheit der/das/die mit einem Vancomycin-resistenten Organismus assoziiert ist, in einem Menschen, bei dem man
a) eine Probe mit einem Vorwärts-Primer, welcher die Sequenz von SEQ ID NO:1 umfasst, und mit mindestens einem Rückwärts-Primer, der eine Sequenz ausgewählt aus der Gruppe bestehend aus SEQ ID NOs:3, 5 und 32 umfasst, in Kontakt bringt;
b) eine Amplifikationsreaktion durchführt, wodurch ein Amplikon hergestellt wird; und
c) das Amplikon unter Verwendung von einer oder mehreren Sonde(n) nachweist, die eine Sequenz ausgewählt aus der Gruppe bestehend aus SEQ ID NOs:2, 4 und 25 umfasst/umfassen;
wobei die Detektion des Amplikons auf das Vorhandensein eines Vancomycin-resistenten Organismus in der Probe verweist.

11. Verfahren gemäß Anspruch 10, wobei die Sonde mit einer nachweisbaren Markierung versehen ist, die ausgewählt ist aus der Gruppe bestehend aus einer fluoreszenten Markierung, einer chemilumineszenten Markierung, einem Quencher, einer radioaktiven Markierung, Biotin und Gold.

## Revendications

1. Jeu d'amorces comprenant
a) au moins une amorce sens comprenant la Séquence N° 1,
b) et au moins une amorce anti-sens comprenant une séquence choisie dans l'ensemble formé par les Séquences N° 3, N° 5 et N° 32.

2. Procédé de production d'un produit acide nucléique, comportant le fait de mettre
a) au moins une amorce sens comprenant la Séquence N° 1,
b) et au moins une amorce anti-sens comprenant une séquence choisie dans l'ensemble formé par les Séquences N° 3, N° 5 et N° 32,
en contact avec un échantillon comprenant un gène *vanA,* dans des conditions appropriées pour une polymérisation d'acide nucléique.

3. Procédé de détection du gène *vanA* de résistance à la vancomycine dans un échantillon, comprenant les étapes suivantes :
a) mettre l'échantillon en contact avec au moins une amorce sens comprenant la Séquence N° 1 et au moins une amorce anti-sens comprenant une séquence choisie dans l'ensemble formé par les Séquences N° 3, N° 5 et N° 32, dans des conditions telles qu'il y ait amplification d'acide nucléique donnant un amplicon ;
b) et mettre cet amplicon en contact avec une ou plusieurs sonde(s) comprenant une ou plusieurs séquence(s) choisie(s) dans l'ensemble formé par les Séquences N° 2, N° 4 et N° 25, dans des conditions telles qu'il y ait hybridation de la sonde avec l'amplicon, cette hybridation de la sonde étant l'indice de la présence d'un gène *vanA* dans l'échantillon.

4. Procédé conforme à la revendication 3, dans lequel chacune de la ou des sonde(s) est marquée avec un marqueur détectable différent.

5. Procédé conforme à la revendication 3, dans lequel la ou les sonde(s) est ou sont marquée(s) avec le même marqueur détectable.

6. Trousse servant à détecter un gène *van*A de résistance à la vancomycine dans un échantillon, comprenant
a) une ou plusieurs sonde(s) comprenant une séquence choisie dans l'ensemble formé par les Séquences N° 2, N° 4 et N° 25,
b) au moins une amorce sens comprenant la Séquence N° 1,
c) et au moins une amorce anti-sens comprenant une séquence choisie dans l'ensemble formé par les Séquences N° 3, N° 5 et N° 32.

7. Trousse conforme à la revendication 6, comprenant en outre des réactifs servant à séquencer un gène de résistance à la vancomycine présent dans l'échantillon.

8. Trousse conforme à la revendication 6, dans laquelle la ou les sonde(s) est ou sont marquée(s) avec des marqueurs détectables différents.

9. Trousse conforme à la revendication 6, dans laquelle la ou les sonde(s) est ou sont marquée(s) avec le même marqueur détectable.

10. Procédé *in vitro* permettant le diagnostic, chez un humain, d'un état, d'un syndrome ou d'une maladie associé(e) à un organisme résistant à la vancomycine, comportant les étapes suivantes :
a) mettre un échantillon en contact avec une amorce sens comprenant la Séquence N° 1 et au moins une amorce anti-sens comprenant une séquence choisie dans l'ensemble formé par les Séquences N° 3, N° 5 et N° 32,
b) réaliser une réaction d'amplification, produisant ainsi un amplicon ;
c) et détecter cet amplicon à l'aide d'une ou de plusieurs sonde(s) comprenant une séquence choisie dans l'ensemble formé par les Séquences N° 2, N° 4 et N° 25,
étant entendu que la détection d'un amplicon est l'indice de la présence, dans l'échantillon, d'un organisme résistant à la vancomycine.

11. Procédé conforme à la revendication 10, dans lequel la sonde est marquée avec un marqueur détectable choisi dans l'ensemble formé par un marqueur fluorescent, un marqueur chimioluminescent, un extincteur, un marqueur radioactif, la biotine et l'or.
